# EUROPEAN PATENT APPLICATION

(11) **EP 4 613 748 A1**
(43) Date of publication of application: **10.09.2025**
(21) Application number: 23885114.1
(22) Date of filing: 03.11.2023
(51) Int. Cl.: C07D 471/10, C07D 403/12, C07D 401/14, C07D 239/22, A61P 37/02, A61P 37/06, A61K 31/395, A61K 31/513

(54) **IRAK4 DEGRADATION AGENT AND USE THEREOF**

(30) Priority: 04.11.2022 CN 202211374883
(71) Applicant: Leadingtac Pharmaceutical (Shaoxing) Co., Ltd., Shaoxing, Zhejiang 312030 (CN); Shanghai Leadingtac Qifan Pharmaceutical Co., Ltd., Shanghai 201203 (CN)
(72) Inventor: FENG, Yan, Shanghai 201203 (CN); YE, Zhengqing, Shanghai 201203 (CN); LI, Shiqiang, Shanghai 201203 (CN); DING, Chenli, Shanghai 201203 (CN); CHEN, Haiji, Shanghai 201203 (CN); XU, Juan, Shanghai 201203 (CN); WANG, Qiao, Shanghai 201203 (CN); WEI, Hong, Shanghai 201203 (CN); XIE, Shuchen, Shanghai 201203 (CN); YAN, Shuang, Shanghai 201203 (CN)
(74) Representative: dompatent von Kreisler Selting Werner - Partnerschaft von Patent- und Rechtsanwälten mbB
(86) International application number: PCT/CN2023/129712
(87) International publication number: WO 2024/094190

(57) **Abstract**

The present invention relates to use of compounds of formula I and formula II, and specifically relates to use of compound A1-A52, B1-B5, or C1-C36 in the preparation of a drug for treating and/or preventing an inflammatory or immune disease. The present invention relates to use of compound A1-A52, B1-B5, or C1-C36 in the preparation of a drug for treating and/or preventing diseases such as rheumatoid arthritis, atopic dermatitis, hidradenitis suppurativa, vitiligo, dermatomyositis, alopecia areata, urticaria, multiple myositis, an interstitial lung disease, systemic lupus erythematosus, systemic sclerosis, and psoriasis.

## Description

TECHNICAL FIELD: The present disclosure relates to a compound that regulates one or more interleukin-1 receptor associated kinase 4 (IRAK4) by ubiquitination and/or degradation and use in a medicament for immunological and/or inflammatory diseases thereof.

BACKGROUND: Interleukin-1 receptor kinase 4 (IRAK4) is a serine/threonine-specific protein kinase with biologically significant kinase activity, playing an important role in activating the immune system. Studies have shown that IRAK4 is a key factor downstream in the signaling pathways of the receptors of the interleukin (IL)-1β family (including IL-1R, IL-18R, IL-33R, and IL-36R) and Toll-like receptors (TLRs), and that both IRAK4-deficient mice and IRAK4-deficient patients are unresponsive to stimulation by TLRs (except TLR3) and the IL-1β family (Suzuki, Suzuki et al., Nature, 2002; Davidson, Currie et al., The Journal of Immunology, 2006; Kuvon Bemtuth et al., JEM, 2007; Kim, Staschke et al., JEM, 2007).

According to whether MyD88 is involved or not, the TLR/IL-1β-mediated signaling pathways can be classified into MyD88-dependent signaling pathways and MyD88-independent signaling pathways. The signaling transduction pathways mediated by IL-1R and TLR2, TLR4, TLR7/8, and TLR9 all rely on MyD88 as a regulatory factor to activate downstream inflammatory signaling pathways. Upon binding to a ligand, TLR/IL-1β recruits MyD88, which, through its N-terminal death domain, further recruits IRAK4 to the TLR/IL-1B complex, and interacts with IRAK1 or IRAK2 and activates it (Kollewe, Mackensen et al., Journal of Biological Chemistry, 2004; Precious et al., J. Biol. Chem., 2009). This initiates downstream signaling to the E3 ubiquitin ligase TNF receptor-associated factor (TRAF6), activates the serine/threonine kinase TAK1, and thus triggers the NF-κB and MAPK signaling pathways (Wang, Deng et al., Nature, 2001), resulting in the release of various inflammatory cytokines and anti-apoptotic molecules. The IRAK4-dependent TLR/IL-1β signaling pathway has been shown to be associated with a variety of diseases, for example, multiple sclerosis, atherosclerosis, myocardial infarction, myocarditis (Valaperti, Nishii et al., Circulation, 2013), Vogt-Koyanagi-Harada syndrome, systemic lupus erythematosus (SLE), obesity (Ahmad, R., P. Shihab et al., Diabetology & Metabolic Syndrome, 2015), type 1 diabetes, rheumatoid arthritis, spondyloarthritis (particularly psoriatic spondyloarthritis and Bekhterev's disease), lupus erythematosus, psoriasis, vitiligo, giant cell arteritis, chronic inflammatory bowel diseases, and viral diseases such as human immunodeficiency virus (HIV) and hepatitis virus (Staschke et al., The Journal of Immunology, 2009; Marquez et al., Ann Rheum Dis, 2014; Zambrano-Zaragoza et al., International Journal of Inflammation, 2014; Wang et al., Experimental and Therapeutic Medicine, 2015; Ciccia et al., Rheumatology, 2015); skin diseases such as psoriasis, atopic dermatitis, Kindler's syndrome, bullous pemphigoid, allergic contact dermatitis, alopecia areata, acne inversa, and acne vulgaris; other inflammatory diseases such as allergy, Behcet's disease, gout, adult-onset Still's disease, pericarditis, chronic inflammatory bowel diseases such as ulcerative colitis and Crohn's disease, and transplant rejection and graft-versus-host reactions; gynecological diseases such as adenomyosis, dysmenorrhea, dyspareunia, and endometriosis, particularly pain associated with adenomyosis and other symptoms associated with adenomyosis such as dysmenorrhea, dyspareunia, dysuria, and dyschezia (Akoum, Lawson et al., Human Reproduction, 2007; Allhorn, Boing et al., Reproductive Biology and Endocrinology, 2008; Lawson, Bourcier et al., Journal of Reproductive Immunology, 2008; Sikora, Mielczarek-Palacz et al., American Journal of Reproductive Immunology, 2012; Khan, Kitajima et al., Journal of Obstetrics and Gynaecology Research, 2013; Santulli, Borghese et al., Human Reproduction, 2013); eye diseases such as retinal ischemia, keratitis, allergic conjunctivitis, keratoconjunctivitis sicca, macular degeneration, and uveitis (Kaarniranta and Salminen, J Mol Med (Berl), 2009; Sun and Pearlman, Investigative Ophthalmology & Visual Science, 2009; Redfern and McDermott, Experimental Eye Research, 2010; Kezic, Taylor et al., J Leukoc Biol, 2011; Chang, McCluskey et al., Clinical & Experimental Ophthalmology, 2012; Guo, Gao et al., Immunol Cell Biol, 2012; Lee, Hattori et al., Investigative Ophthalmology & Visual Science, 2012; Qi, Zhao et al., Investigative Ophthalmology & Visual Science, 2014); fibrotic diseases such as liver fibrosis, myocarditis, primary biliary cirrhosis, and cystic fibrosis (Zhao, Zhao et al., Scand J Gastroenterol, 2011; Benias, Gopal et al., Clin Res Hepatol Gastroenterol, 2012; Yang, L. and E. Seki, Front Physiol, 2012; Liu, Hu et al., Biochim Biophys Acta., 2015); chronic liver diseases such as fatty liver hepatitis, particularly nonalcoholic fatty liver disease (NAFLD) and/or nonalcoholic steatohepatitis (NASH), and alcoholic steatohepatitis (ASH) (Nozaki, Saibara et al., Alcohol Clin Exp Res, 2004; Csak, T., A. Velayudham et al., Am J Physiol Gastrointest Liver Physiol, 2011; Miura, Kodama et al., Gastroenterology, 2010; Kamari, Shaish et al., J Hepatol, 2011; Ye, Li et al., Gut, 2012; Roh, Seki, J Gastroenterol Hepatol, 2013; Ceccarelli, S., V. Nobili et al., World J Gastroenterol, 2014; Miura, Ohnishi, World J Gastroenterol, 2014; Stojsavljevic, Palcic et al., World J Gastroenterol, 2014); cardiovascular diseases and neurological disorders such as myocardial reperfusion injury, myocardial infarction, and hypertension (Oyama, Blais et al., Circulation, 2004; Timmers, Sluijter et al., Circulation Research, 2008; Fang and Hu, MedSci Monit, 2011; Bijani, International Reviews of Immunology, 2012; Bomfim, DosSantos et al., Clin Sci (Lond), 2012; Christia and Frangogiannis, European Journal of Clinical Investigation, 2013; Thompson and Webb, Clin Sci (Lond), 2013; Hernanz, Martinez-Revelles et al., British Journal of Pharmacology, 2015; Frangogiannis, Curr Opin Cardiol, 2015; Bomfim, Echem et al., Life Sciences, 2015), as well as Alzheimer's disease, stroke, craniocerebral trauma, amyotrophic lateral sclerosis (ALS), and Parkinson's disease (Brough, Tyrrell et al., Trends in Pharmacological Sciences, 2011; Carty and Bowie, Biochemical Pharmacology, 2011; Denes, Kitazawa, Cheng et al., The Journal of Immunology, 2011; Lim, Kou et al., The American Journal of Pathology, 2011; Béraud and Maguire-Zeiss, Parkinsonism & Related Disorders, 2012; Denes, Wilkinson et al., Disease Models & Mechanisms, 2013; Noelker, Morel et al., Sci. Rep., 2013; Wang, Wang et al., Stroke, 2013; Xiang, Chao et al., Rev Neurosci, 2015; Lee, Lee et al., J Neuroinflammation, 2015); itching and pain (including acute, chronic, inflammatory, and neuropathic pain) such as hyperalgesia, allodynia, premenstrual pain, pain associated with adenomyosis, postoperative pain, interstitial cystitis, complex regional pain syndrome (CRPS), trigeminal neuralgia, prostatitis, pain caused by spinal cord injury, pain induced by inflammation, lower back pain, cancer pain, chemotherapy-associated pain, HIV treatment-induced neuropathy, pain caused by burns, and chronic pain (Wolf, Livshits et al., Brain, Behavior, and Immunity, 2008; Kim, Lee et al., Toll-like Receptors: Roles in Infection and Neuropathology, 2009; del Rey, Apkarian et al., Annals of the New York Academy of Sciences, 2012; Guerrero, Cunha et al., European Journal of Pharmacology, 2012; Kwok, Hutchinson et al., PLoS ONE, 2012; Nicotra, Loram et al., Experimental Neurology, 2012; Chopra and Cooper, J Neuroimmune Pharmacol, 2013; David, Ratnayake et al., Neurobiology of Disease, 2013; Han, Zhao et al., Neuroscience, 2013; Liu and Ji, Pflugers Arch., 2013; Stokes, Cheung et al., Journal of Neuroinflammation, 2013; Zhao, Zhang et al., Neuroscience, 2013; Liu, Zhang et al., Cell Research, 2014; Park, Stokes et al., Cancer Chemother Pharmacol, 2014; Van der Watt, Wilkinson et al., BMC Infect Dis, 2014; Won, K. A., M. J. Kim et al., J Pain, 2014; Min, Ahmad et al., Photochem Photobiol., 2015; Schrepf, Bradley et al., Brain Behav Immun, 2015; Wong, L., J. D. Done et al., Prostate, 2015); tumor diseases such as certain lymphomas: activated B-cell diffuse large cell B-cell lymphoma (ABC-DLBCL), mantle cell lymphoma, and Waldenstrom's macroglobulinemia, as well as chronic lymphocytic leukemia, melanoma, pancreatic tumors and hepatocellular carcinoma (Ngo, Young et al., Nature, 2011; Puente, Pinyol et al., Nature, 2011; Ochi, Nguyen et al., J Exp Med, 2012; Srivastava, Geng et al., Cancer Research, 2012; Treon, Xu et al., New England Journal of Medicine, 2012; Choi, Kim et al., Human Pathology, 2013; Liang, Chen et al., Clinical Cancer Research, 2013), ras-dependent tumors, breast cancer, ovarian cancer, colorectal cancer, head and neck cancer, lung cancer, and prostate cancer.

Regulation of IRAK4-mediated signaling pathways is primarily associated with its kinase function. However, there are also some reports indicating that in certain cell types, signaling regulation by IRAK4 of downstream processes is associated with the non-kinase function of IRAK4. Cushing et al. have indicated that despite reduced levels of IRAK4 phosphorylation in IL-1β-stimulated human skin fibroblasts, pharmacological inhibition of IRAK4 does not result in inhibition of IL-6 and TNF-α. The results are supported by the observation that IRAK4-deficient fibroblasts, compared with wild-type cells, demonstrate that the scaffolding function of IRAK4 is important for IL1 signalling. Chiang et al. have also indicated that the kinase activity of IRAK4 is not essential in human B cells and T cells, dendritic cells, and monocytes, and siRNA gene excision also shows that IRAK4 can function as a scaffold in these cells. A variety of potent selective inhibitors against IRAK4 have been reported, such as CA-4948, BAY-1834845, BMS-986126, and PF-06650833. These inhibitors can selectively inhibit the kinase activity of IRAK4 and are mainly used for the prevention and treatment of autoimmune diseases, inflammatory diseases, and tumor diseases. However, since IRAK4 can function as a scaffold protein and an active kinase, and traditional small molecule inhibitors easily lead to drug resistance, inhibiting only the kinase activity of IRAK4 may not be sufficient to produce a therapeutic effect. Proteolysis targeting chimera (PROTAC) is a technology different from the traditional small molecule inhibitors, which typically act on the active site of a target protein to inhibit its activity. A PROTAC is a heterogeneous bifunctional molecule, one end of which is a small molecule inhibitor capable of recognizing the target protein, and the other end of which is an E3 ubiquitin ligase ligand that recognizes E3 ubiquitin ligase. These two ends are connected by a linking chain. Such a bifunctional molecule recognizes the target protein and the E3 ubiquitin ligase *in vivo,* bringing the target protein and the E3 ubiquitin ligase closer to form a ternary complex. The target protein is then ubiquitinated and degraded via the ubiquitin-proteasome pathway *in vivo.* Compared with the traditional small molecule inhibitors, in one aspect, the PROTAC only needs to draw the target protein and the E3 ubiquitin ligase closer to degrade the substrate, and the action mode enables the technology to be applied to some non-druggable targets; in another aspect, the PROTAC molecules can be released to continue to participate in the degradation process of the next protein after the target protein is degraded, so that the degradation effect with a catalytic effect can realize efficient degradation with less dose of the PROTAC medicament; in yet another aspect, the traditional small molecule inhibitors easily generate drug resistance often because a point mutation occurs, so that the small molecule inhibitors lose the inhibitory effect on the target, while the PROTAC can directly degrade the target protein, so that the drug resistance generated by the point mutation can be avoided to a certain extent. Therefore, compared with the traditional small molecule inhibitors, the PROTAC technology has high advantages and feasibility in the research and development of small molecule new drugs, and these drugs are expected to become next-generation promising new drugs. The PROTAC technology has also been applied to the modification of various target drugs, such as androgen receptors, estrogen protein receptors, and BTK. Several types of IRAK4-targeting degraders are disclosed in US2019/0151295, US2019/0192688, WO2019/160915, and WO2020/113233. There is an urgent need to develop more IRAK4-targeting degraders.

### SUMMARY OF THE INVENTION

The prior art discloses a number of IRAK4 inhibitors (see: Annual Reports in Medical Chemistry (2014), 49, 117-133).

The following compound (the reference compound (I) in the present disclosure) is disclosed in the example of WO2016/083433A1:

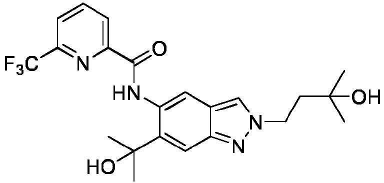

The following compound (the reference compound (II) in the present disclosure) is disclosed in the example of WO2015/150995A1:

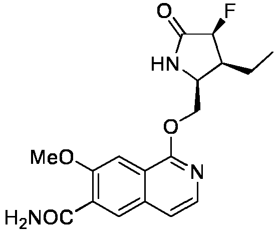

As described herein, the inventors have found that IRAK4 degraders are suitable for the treatment and prevention of immunological diseases in animals characterized by an overreacting immune system, particularly showing very good therapeutic or preventive effects on the following diseases: rheumatoid arthritis, atopic dermatitis, hidradenitis suppurativa (HS), vitiligo, dermatomyositis, alopecia areata, urticaria, multiple myositis, interstitial lung disease, systemic lupus erythematosus, systemic sclerosis, and/or psoriasis.

### SUMMARY

Compounds A1-A52 correspond to the following formulas, respectively:

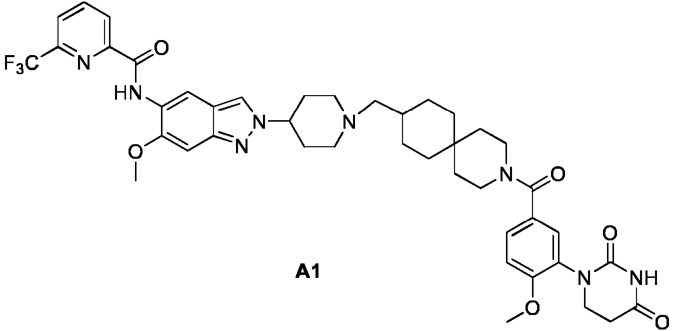

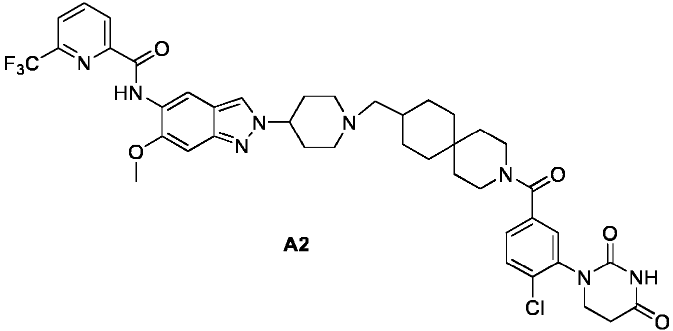

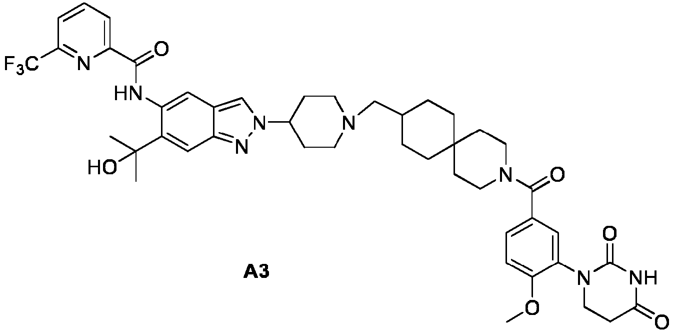

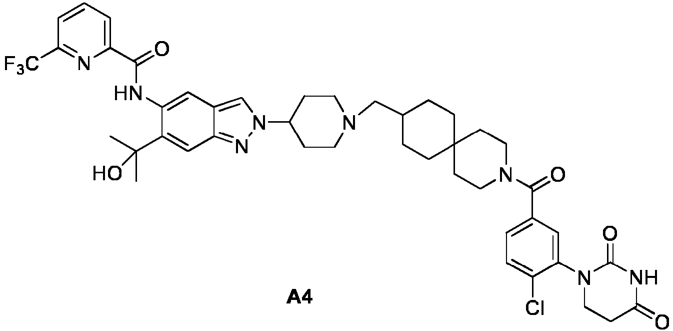

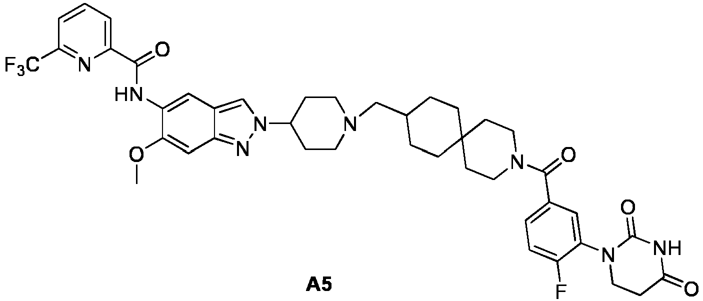

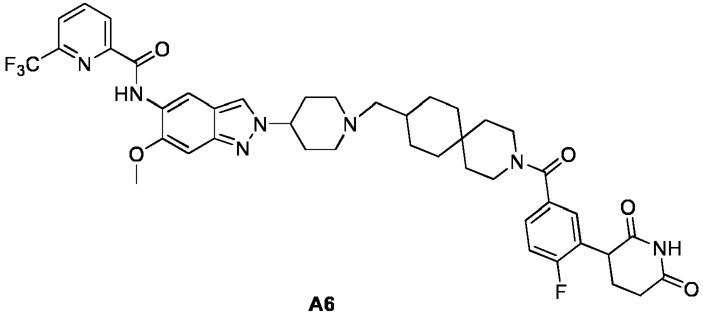

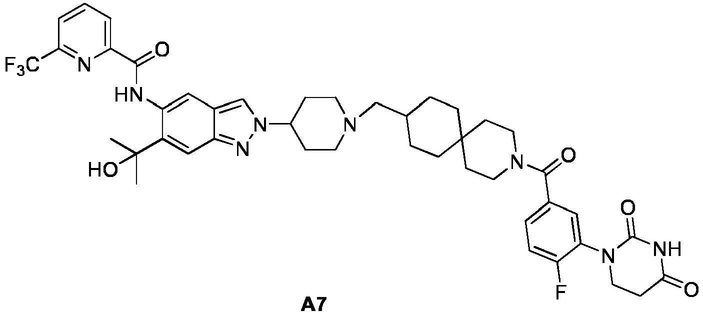

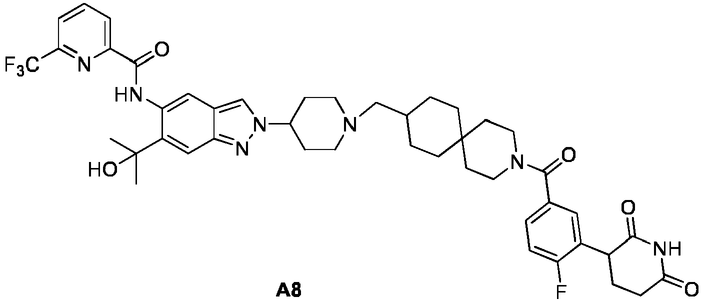

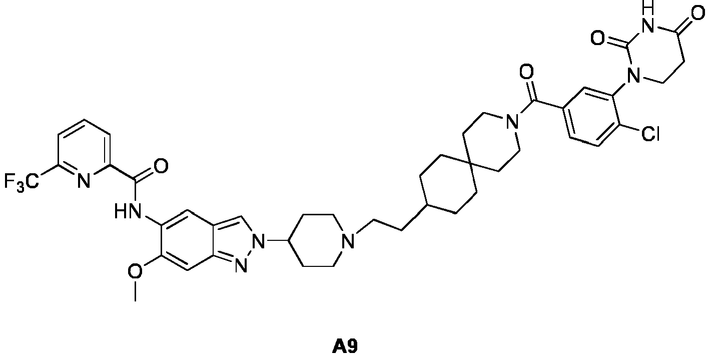

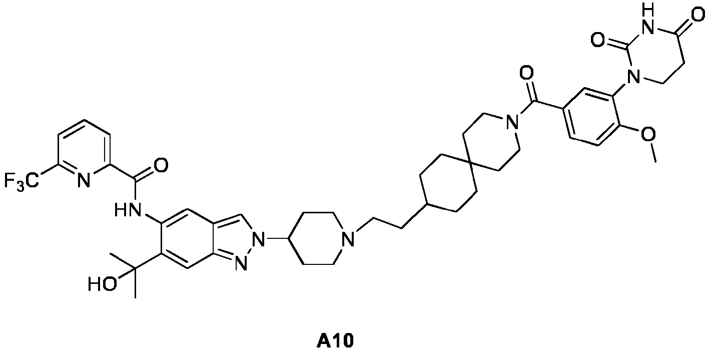

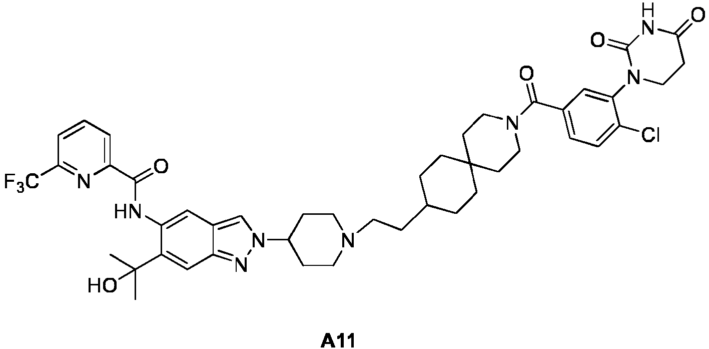

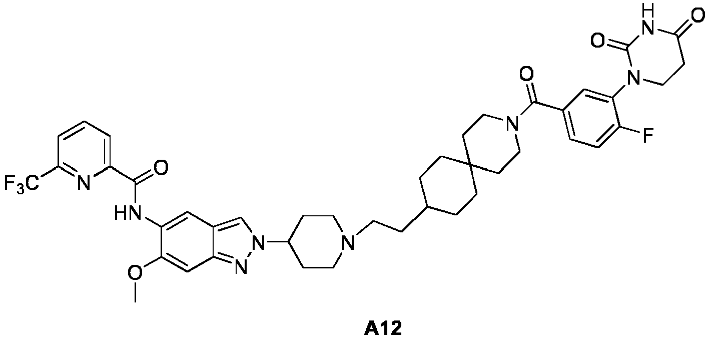

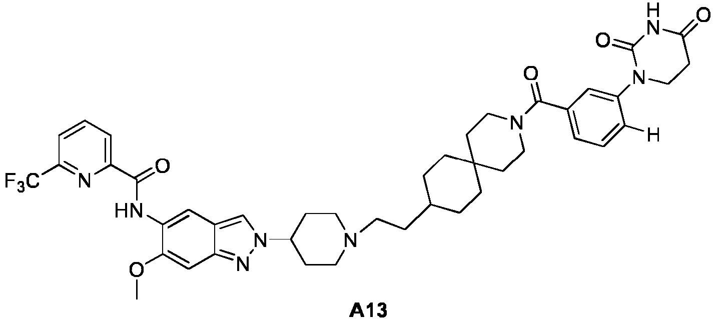

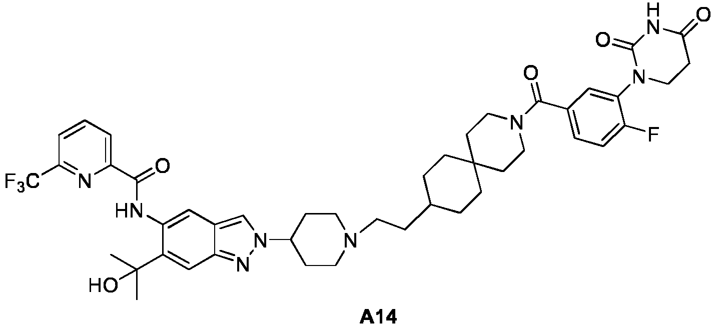

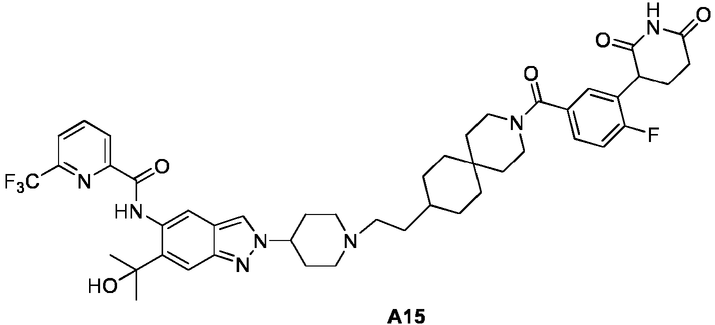

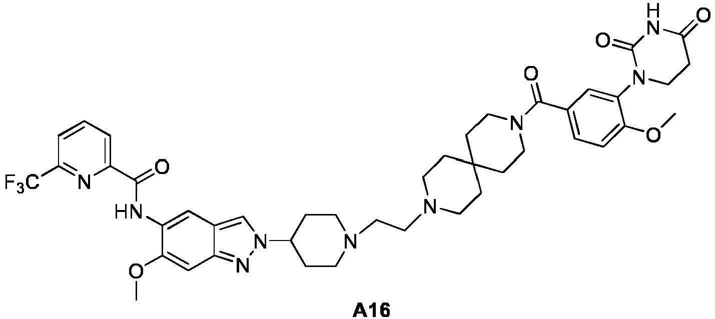

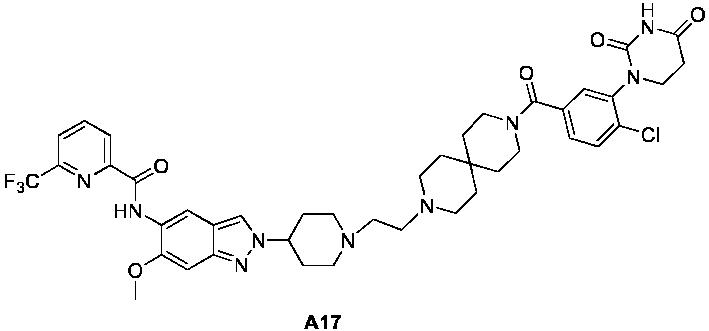

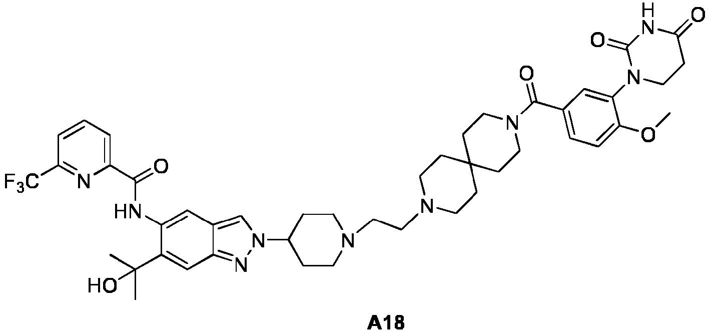

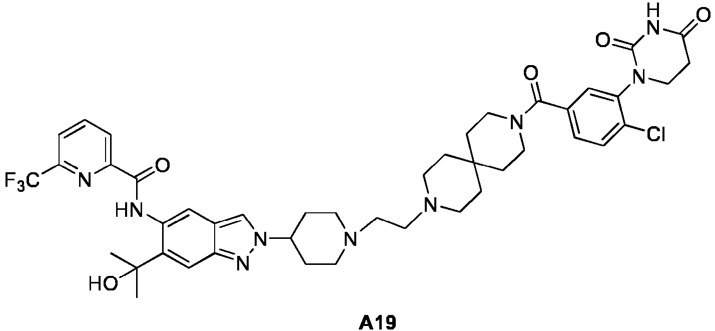

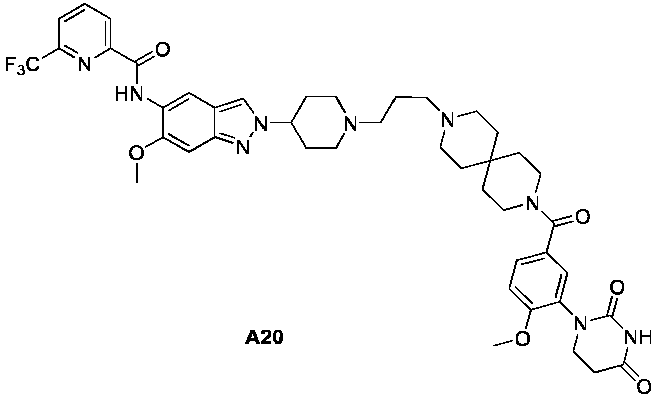

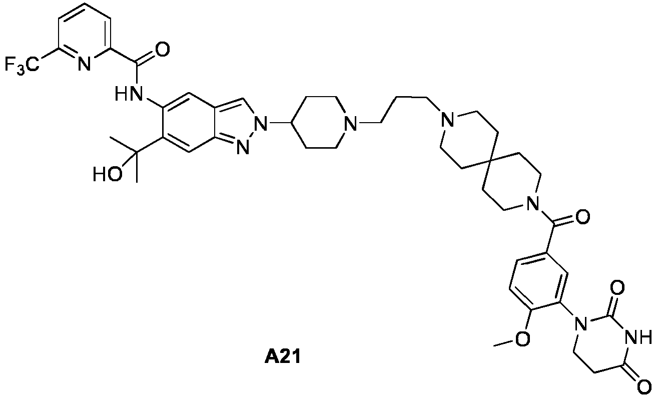

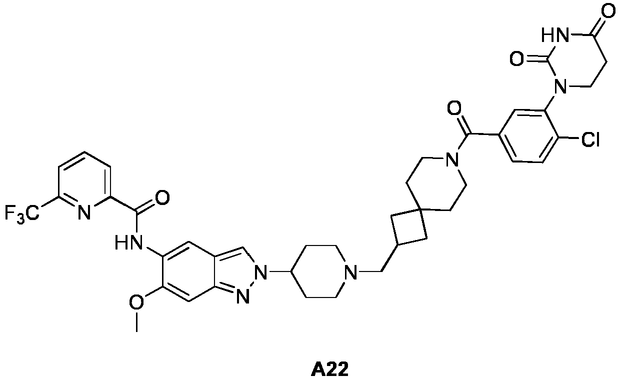

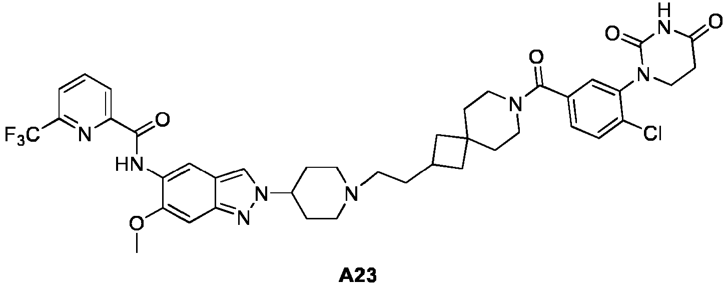

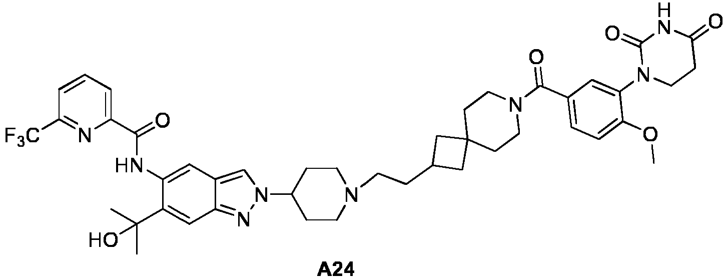

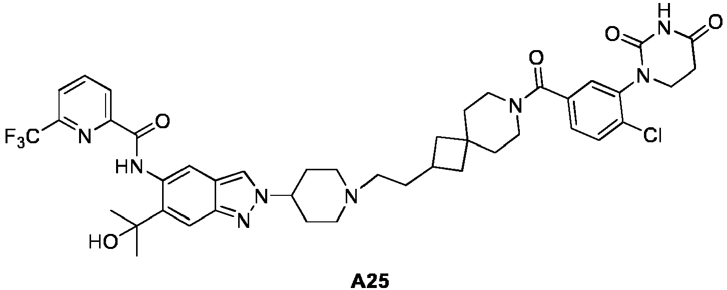

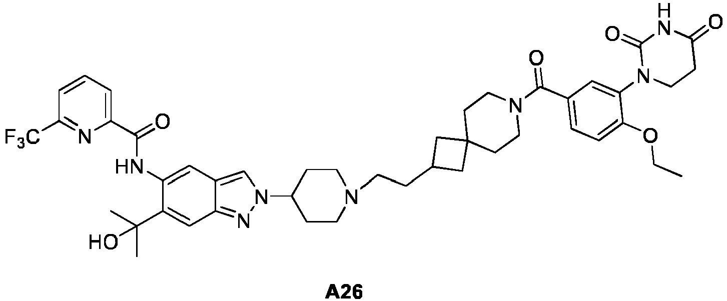

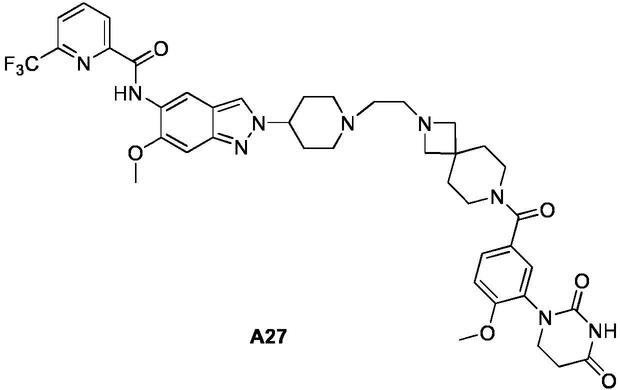

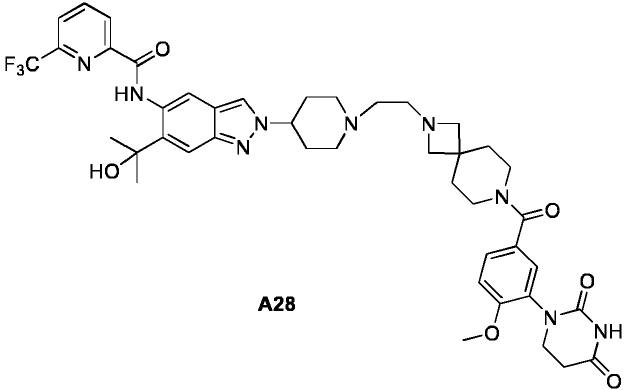

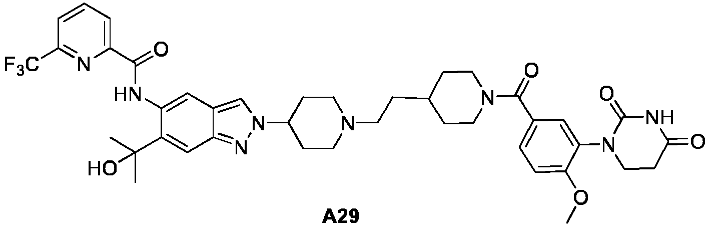

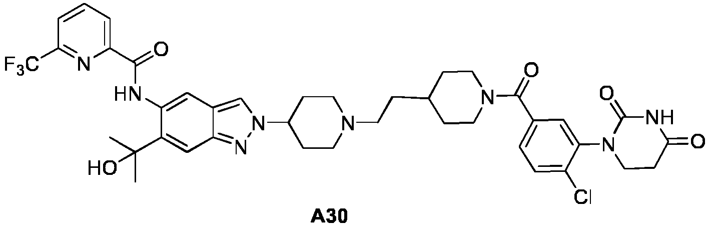

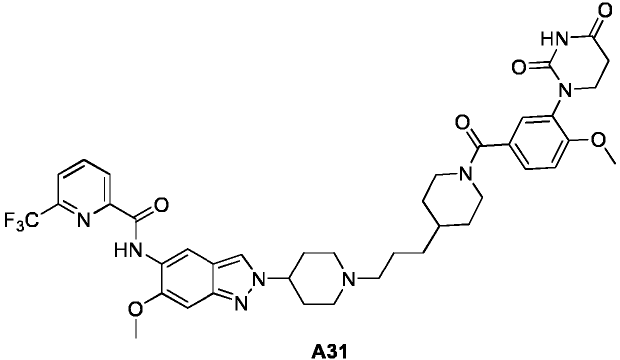

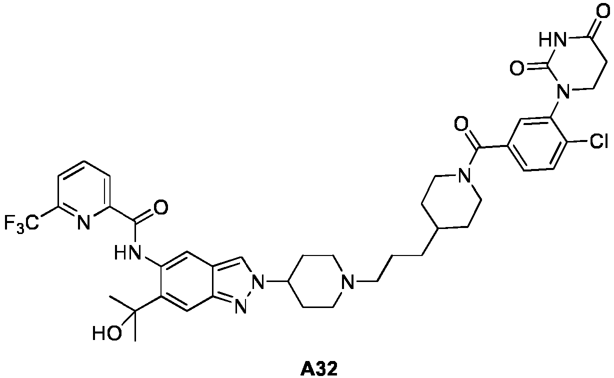

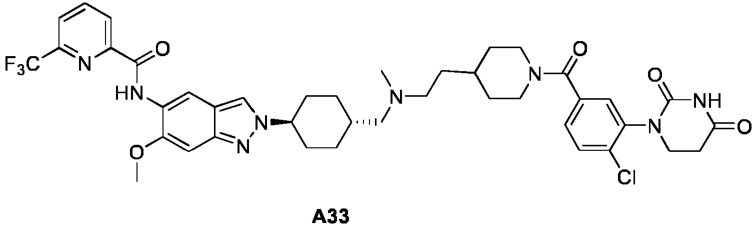

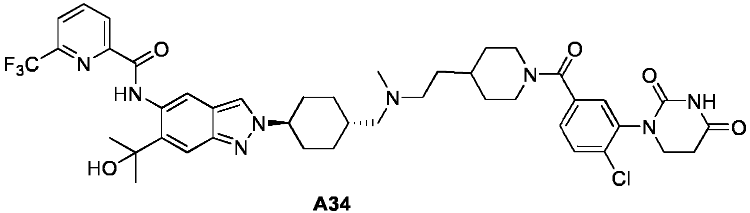

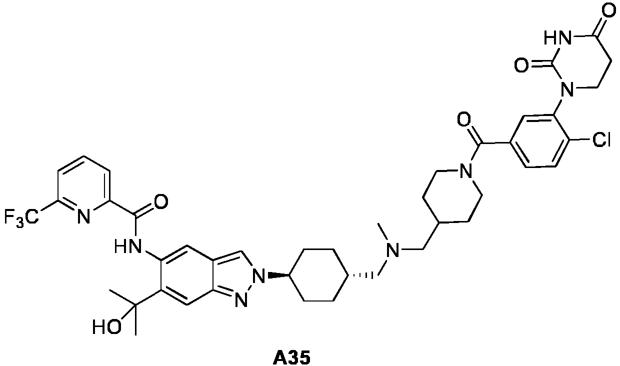

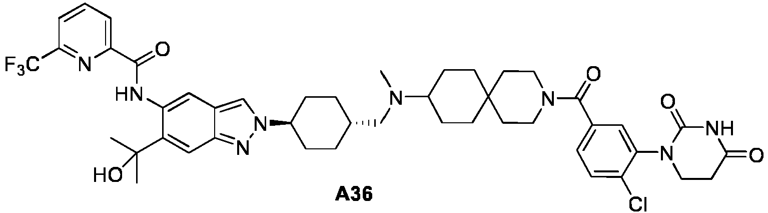

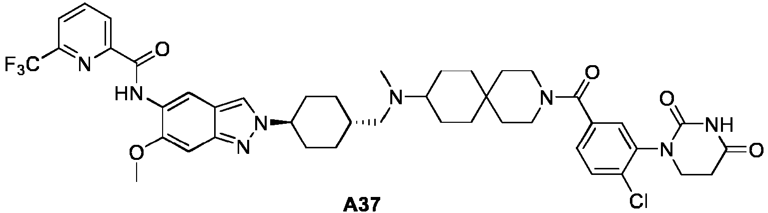

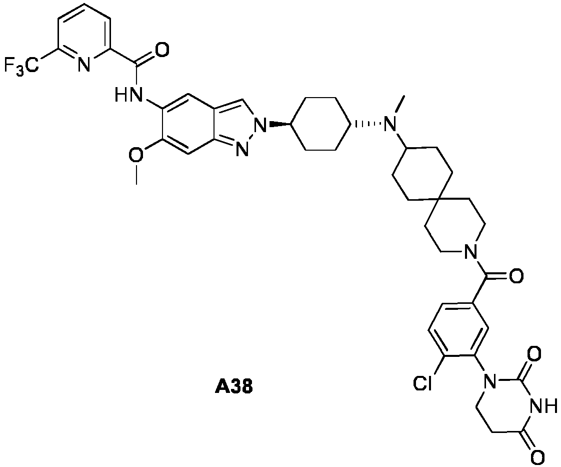

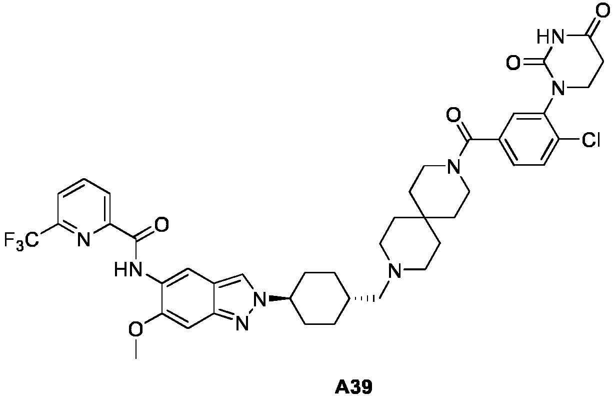

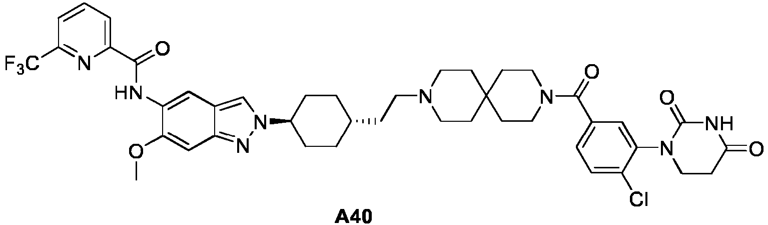

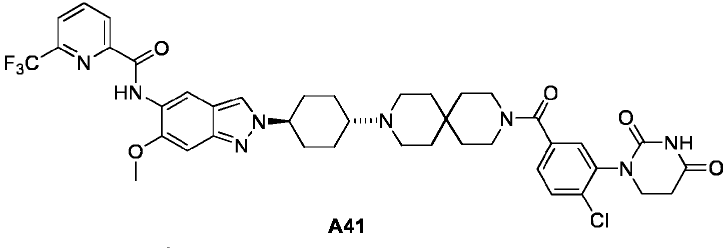

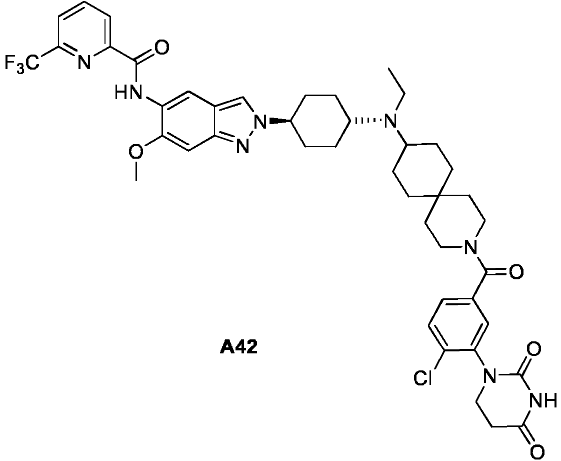

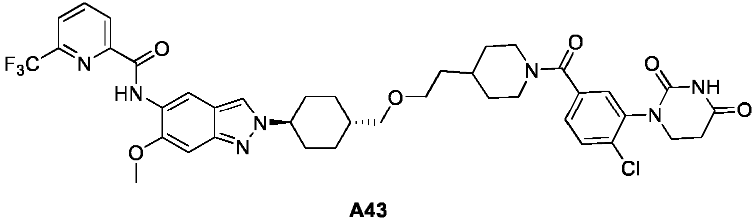

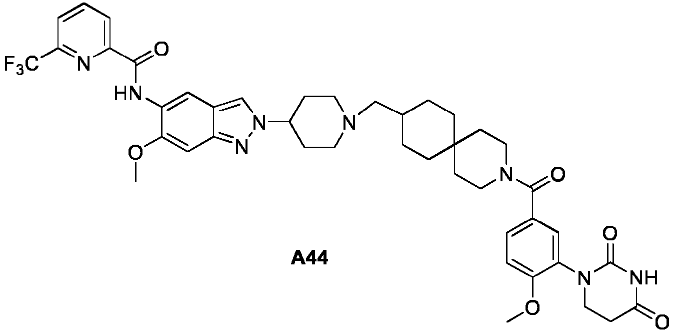

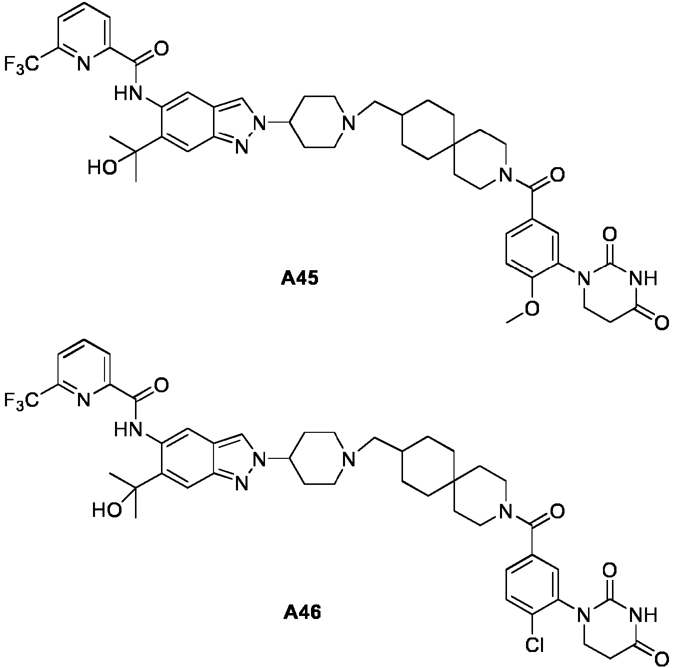

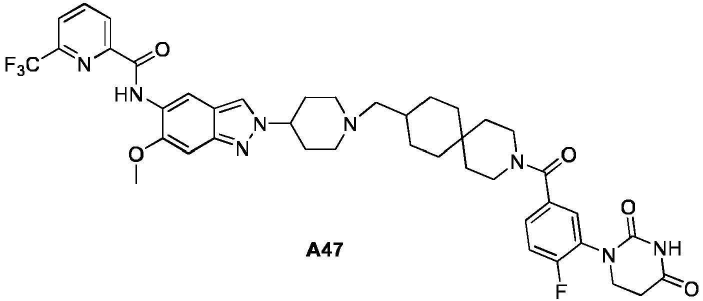

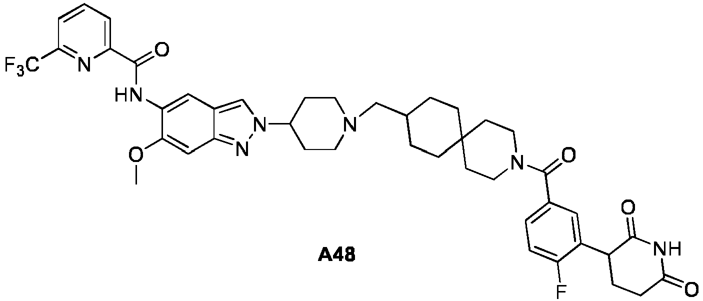

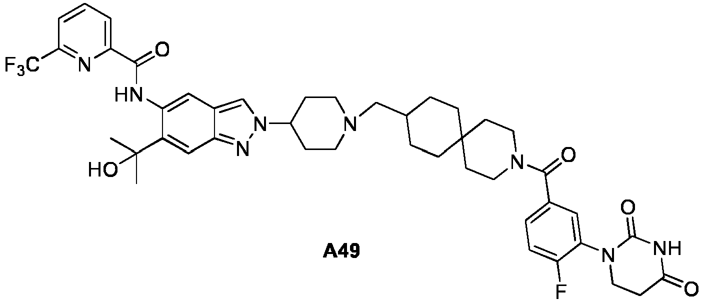

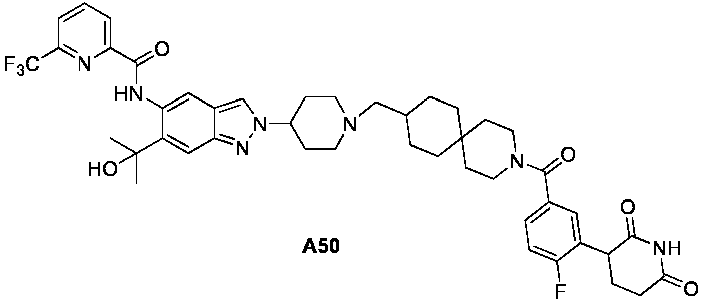

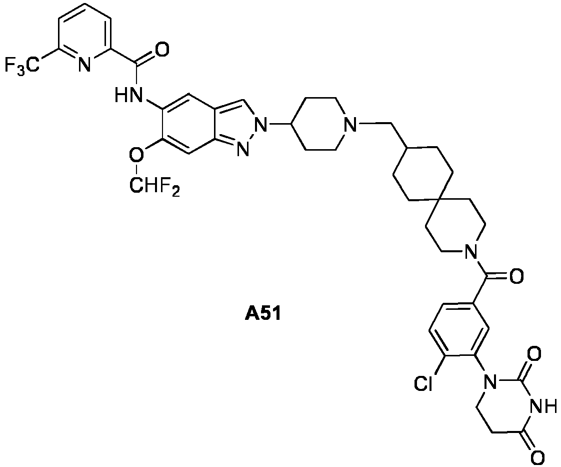

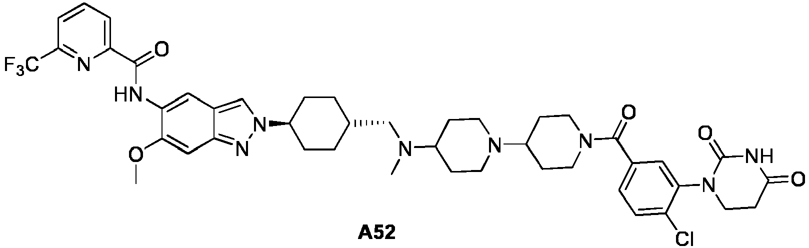

The preparation and use as a medicament of compounds A1-A52 are disclosed in WO2022/088551A1. Compounds B1-B5 correspond to the following formulas, respectively:

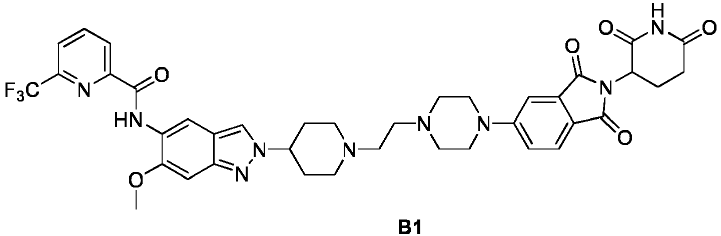

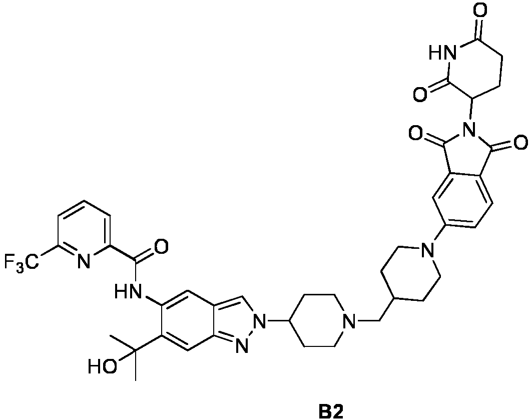

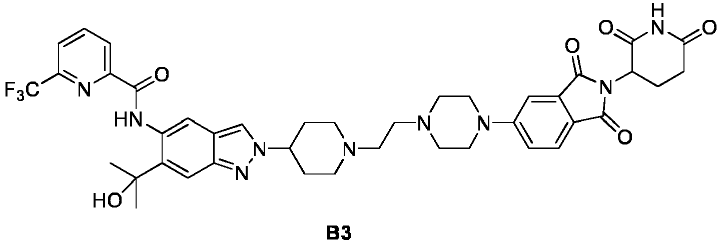

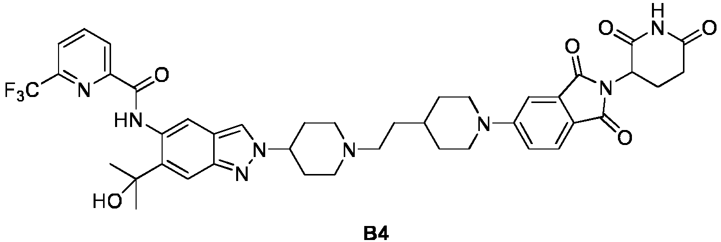

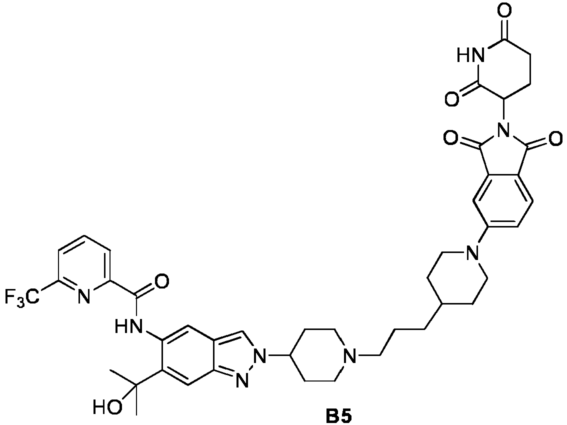

The preparation and use as a medicament of compounds B1-B5 are disclosed in WO2022/028547A1. Compounds C1-C36 correspond to the following formulas, respectively:

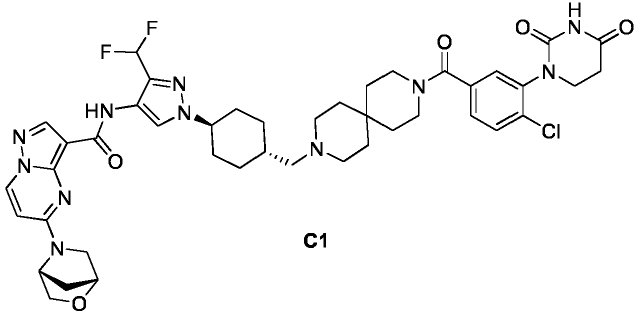

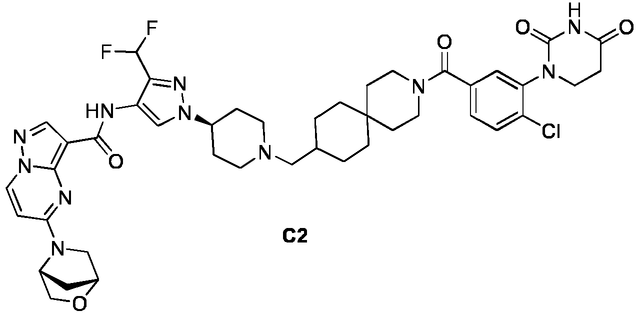

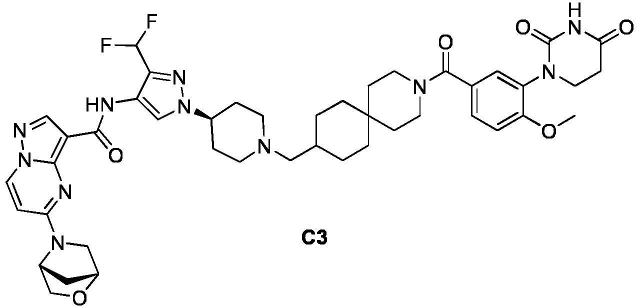

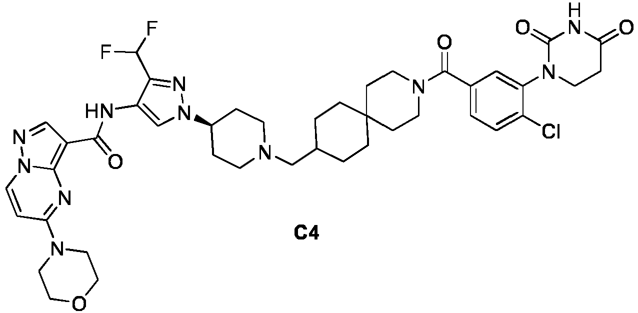

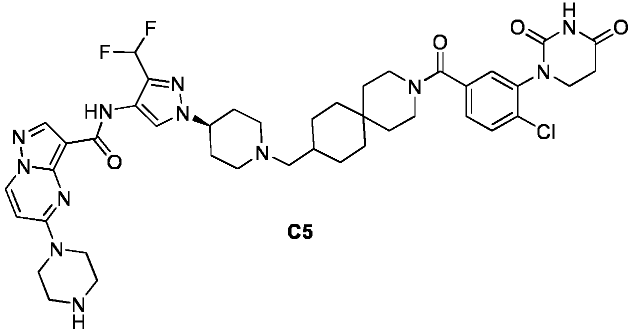

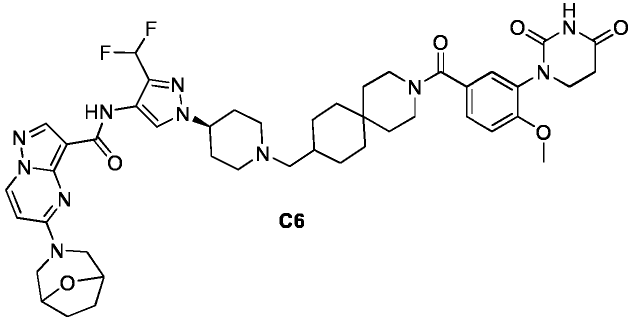

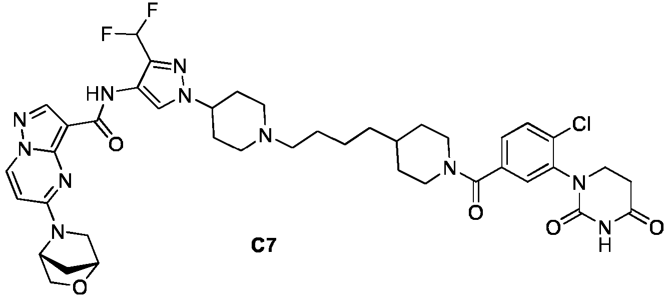

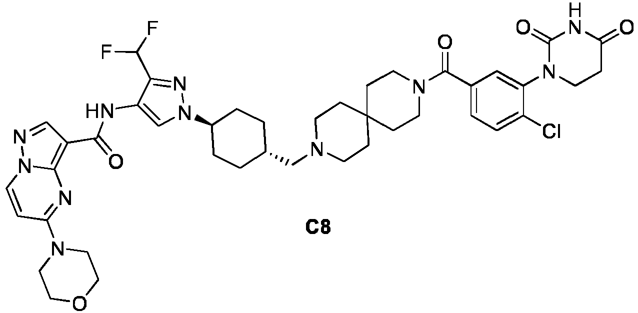

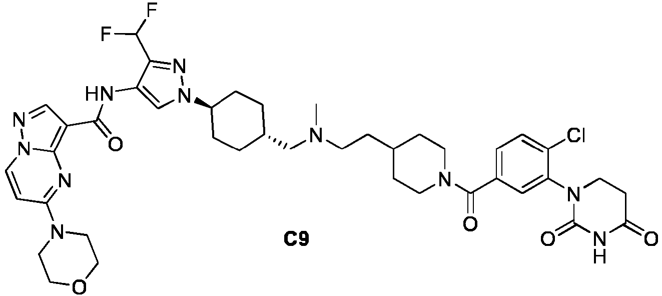

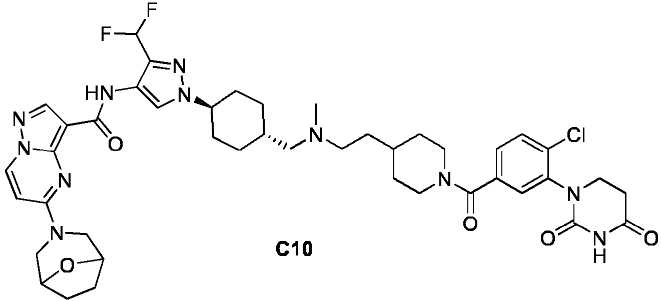

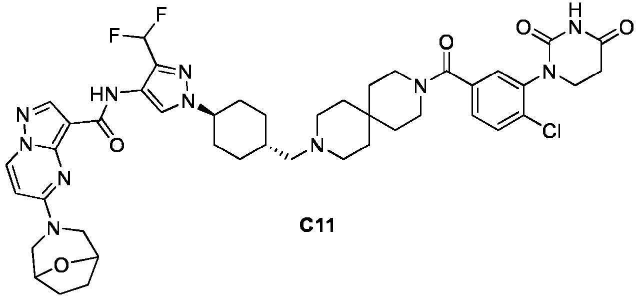

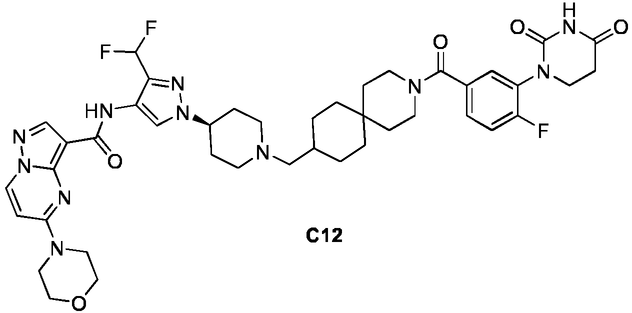

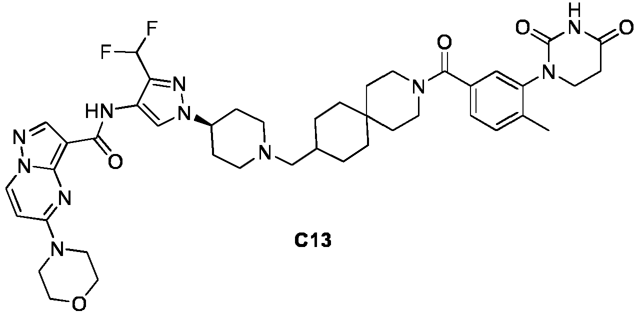

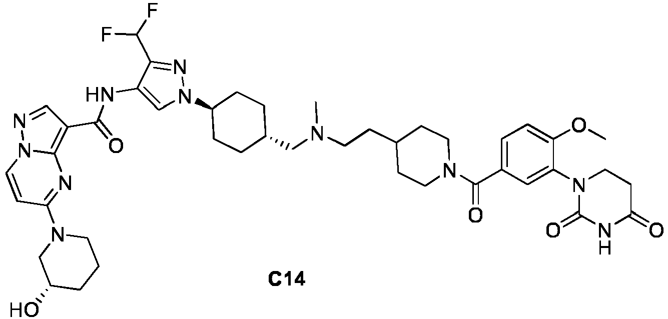

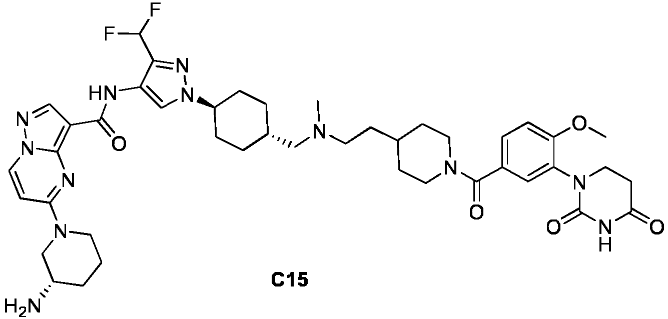

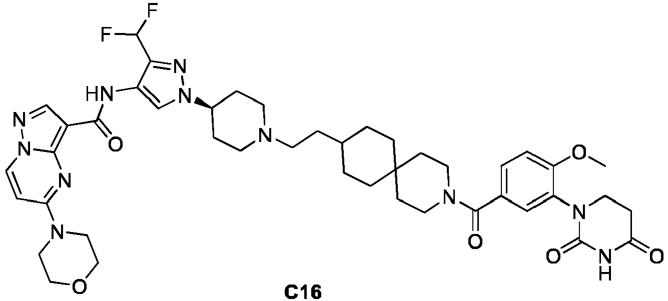

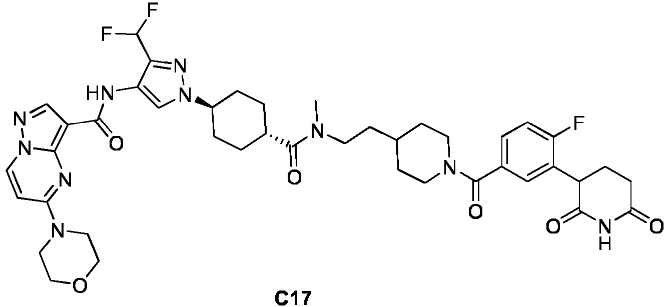

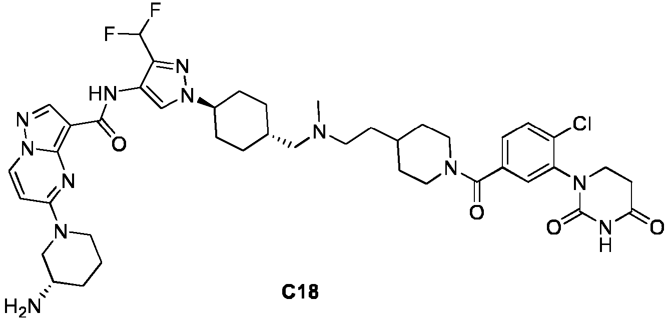

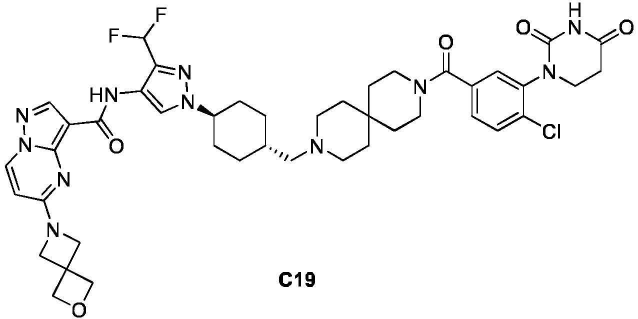

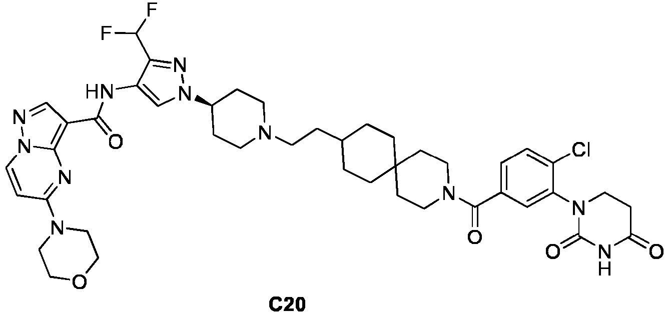

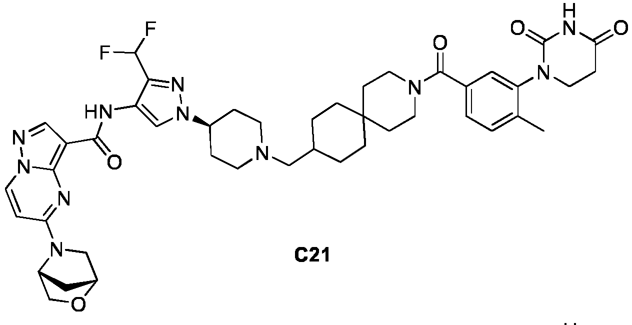

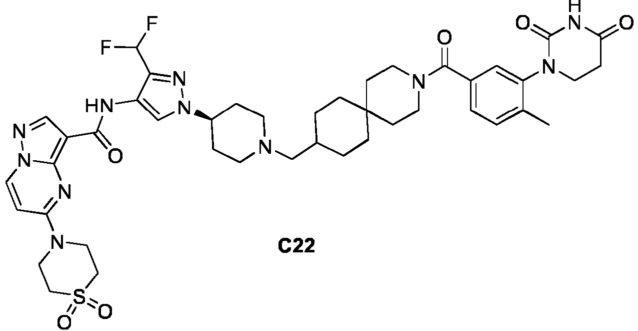

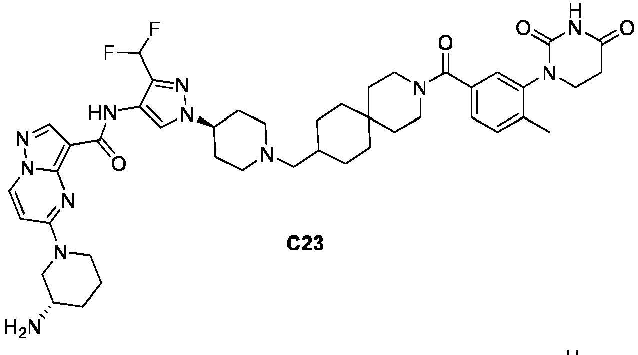

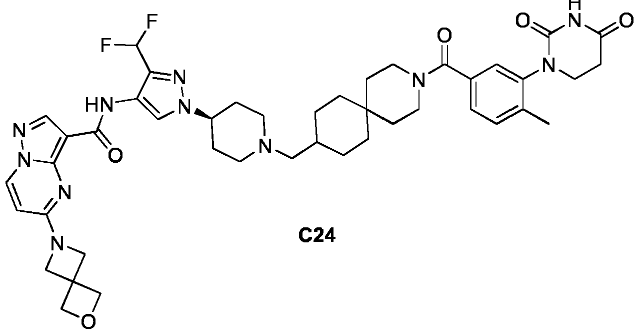

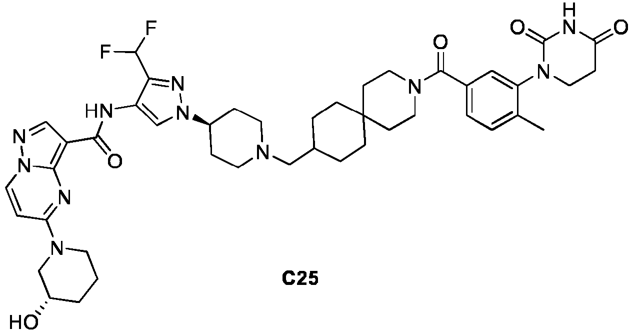

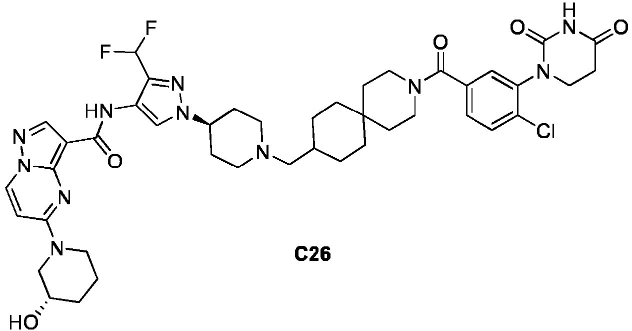

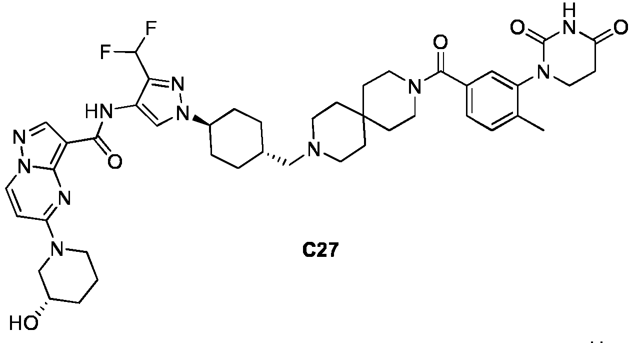

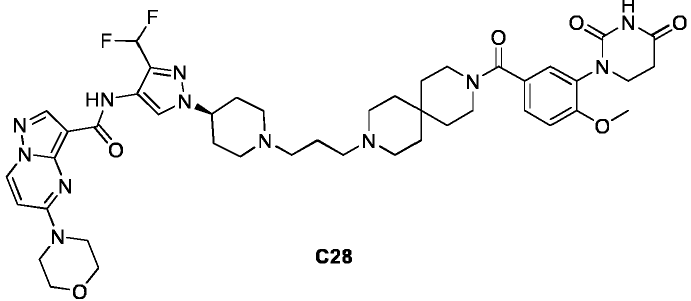

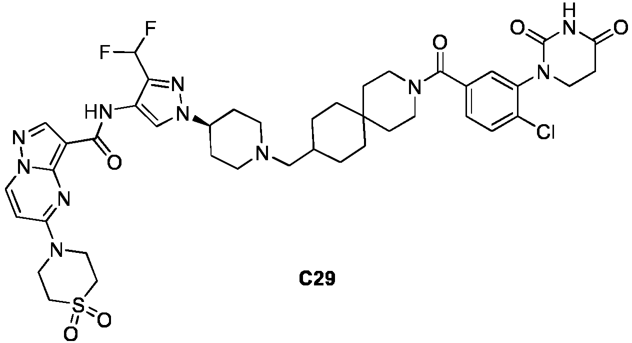

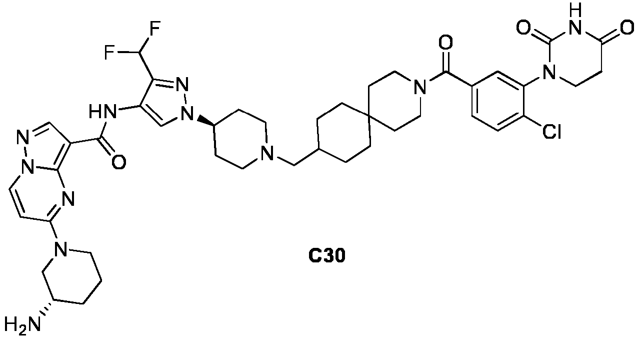

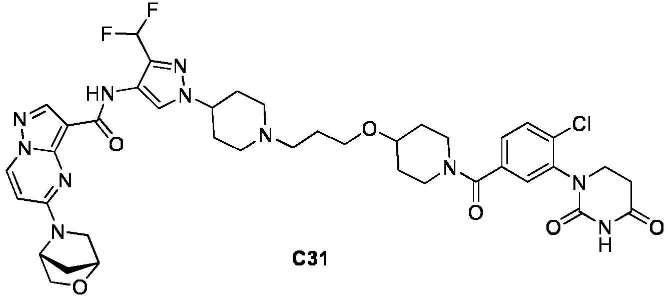

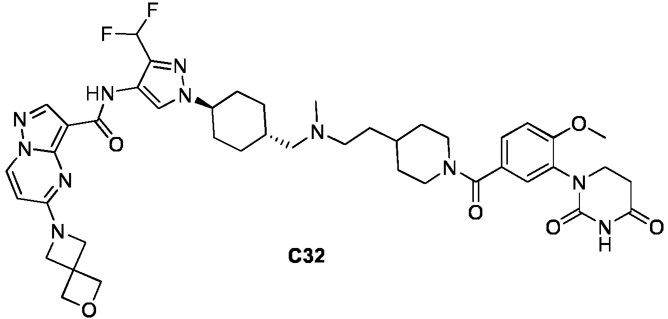

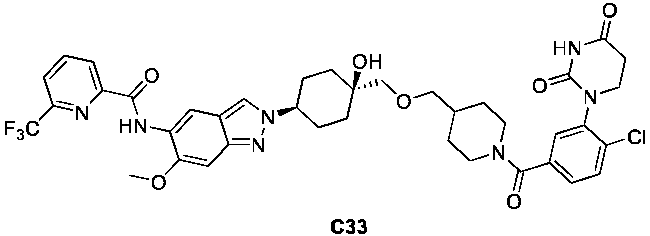

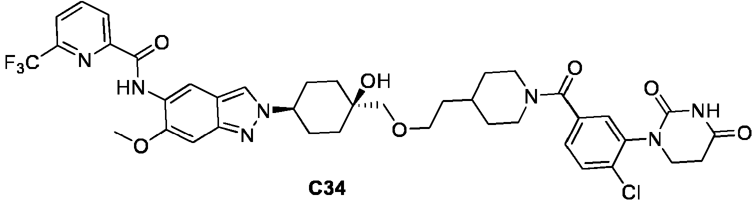

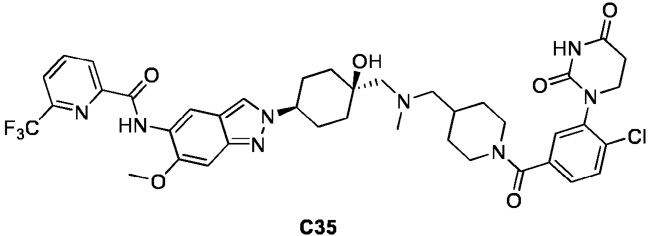

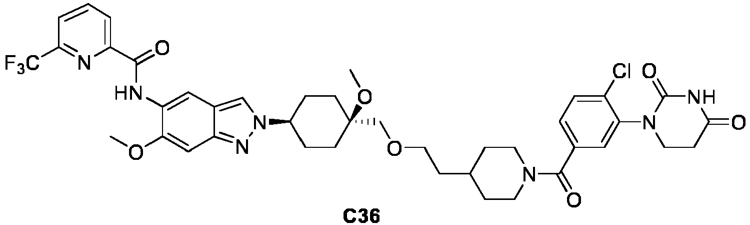

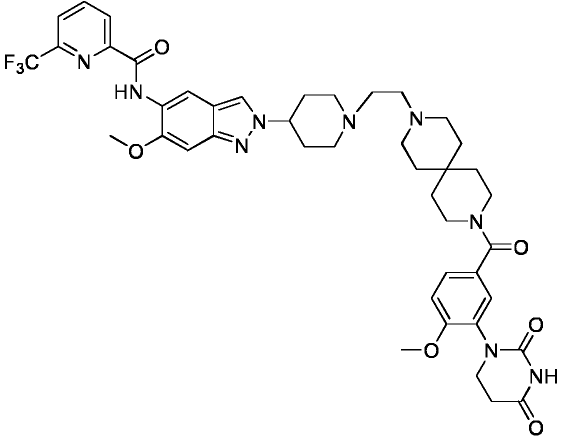

The preparation and use as a medicament of compounds C1-C36 are described in the specification part of the present disclosure.

The present disclosure provides IRAK4, and/or a stereoisomer, an enantiomer, a diastereomer, a deuteride, a hydrate, a solvate, or a prodrug thereof and/or a pharmaceutically acceptable salt thereof, and use thereof for the treatment and/or prevention of an immunological and/or inflammatory disease.

The present disclosure provides IRAK4, and/or a stereoisomer, an enantiomer, a diastereomer, a deuteride, a hydrate, a solvate, or a prodrug thereof and/or a pharmaceutically acceptable salt thereof, and use thereof for the treatment and/or prevention of a disease caused by abnormal expression of IRAK4 and IRAK4-associated proteins in an IL-1R/TLR pathway and/or abnormal secretion or proliferation of chemical factors, cytokines, or immune cells mediated by IRAK4.

In some embodiments of the present disclosure, the IRAK4 degrader is a compound of formula I, formula II, or formula III or a pharmaceutically acceptable salt thereof: 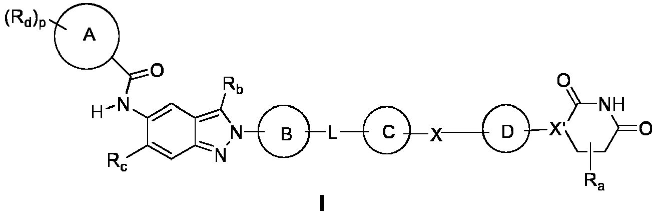 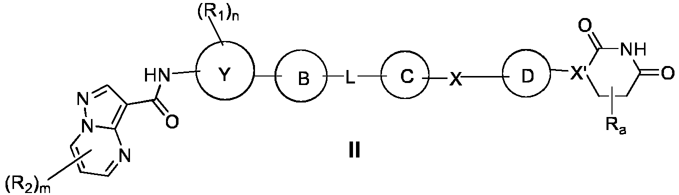 wherein:
ring A is phenyl or pyridyl;
ring B is C₆-C₁₀ cycloalkyl or 6- to 10-membered heterocycloalkyl containing 1-2 heteroatoms selected from N, O, and S, wherein the C₆-C₁₀ cycloalkyl and 6- to 10-membered heterocycloalkyl are optionally substituted with substituents selected from halogen, oxo, cyano, amino, hydroxy, C₁-C₆ alkyl, C₁-C₆ haloalkyl, C₁-C₆ hydroxyalkyl, and -O-(C₁-C₆ alkyl);
ring C is C₆-C₁₂ cycloalkyl or 6- to 12-membered heterocycloalkyl containing 1-2 heteroatoms selected from N, O, and S, wherein the C₆-C₁₂ cycloalkyl and 6- to 12-membered heterocycloalkyl are optionally substituted with substituents selected from halogen, cyano, amino, hydroxy, C₁-C₆ alkyl, C₁-C₆ haloalkyl, C₁-C₆ hydroxyalkyl, and -O-(C₁-C₆ alkyl);
ring D is C₆-C₁₀ aryl, wherein the C₆-C₁₀ aryl is optionally substituted with substituents of halogen, cyano, amino, hydroxy, C₁-C₆ alkyl, C₁-C₆ haloalkyl, C₁-C₆ hydroxyalkyl, or -O-(C₁-C₆ alkyl);
ring Y is 5- to 6-membered heteroaryl;
X is a bond, -O-, -NH-, -C(O)-, -OC(O)-, -C(O)O-, -NHC(O)-, or -C(O)NH-;
L is -(CH₂)ⱼ-, wherein 1 or more methylene groups in the -(CH₂)ⱼ- are optionally replaced with a group selected from -NR^{3'}-, -O-, -S-, -S(O)-, -S(O)₂-, -S(O)₂NR3^{'}-, -CR^{1'}R^{2'}-, -C(O)-, -C(O)O-, -OC(O)-, -NR^{3'}C(O)O-,-OC(O)NR^{3'}-, -C(O)NR^{3'}-, -NR^{3'}C(O)-, -NR^{4'}C(O)NR^{3'}-, ethenylene, and ethynylene;
R^{1'} and R^{2'} are each independently halogen, -OH, -NH₂, C₁-C₄ alkyl, C₁-C₄ haloalkyl, C₁-C₄ hydroxyalkyl,-O(C₁-C₄ alkyl), or -NH(C₁-C₄ alkyl);
R^{3'} and R^{4'} are each independently hydrogen or C₁-C₆ alkyl;
each R_{d} is independently hydrogen, deuterium, halogen, cyano, or C₁-C₆ alkyl, wherein the alkyl is optionally substituted with 1 or more groups selected from halogen, hydroxy, and amino;
R_{c} is -O(C₁-C₃ alkyl), -N(C₁-C₃ alkyl)₁₋₂, or C₁-C₆ alkyl, wherein the alkyl is optionally substituted with 1 or more groups independently selected from hydroxy, amino, halogen, cyano, and -O-(C₁-C₃ alkyl);
R_{b} is hydrogen or C₁-C₆ alkyl, wherein the alkyl is optionally substituted with 1 or more groups independently selected from hydroxy, amino, halogen, and cyano;
Rₐ is hydrogen, halogen, C₁-C₆ alkyl, or -O-(C₁-C₆ alkyl), wherein the alkyl is optionally substituted with halogen or hydroxy;
each R₁ is independently selected from C₁-C₄ alkyl, -O(C₁-C₄ alkyl), -N(C₁-C₄ alkyl)₁₋₂, CN, halogen, -OH, and -NH₂, wherein the alkyl is optionally substituted with a group selected from halogen, cyano, -OH, C₁-C₄ alkyl, and -O(C₁-C₄ alkyl);
each R₂ is independently hydrogen, C₁-C₄ alkyl, -O(C₁-C₄ alkyl), C₃-C₈ cycloalkyl, 3- to 8-membered heterocycloalkyl, 6- to 10-membered aryl, 5- to 6-membered heteroaryl, CN, halogen, or -OH, wherein the alkyl, cycloalkyl, heterocycloalkyl, aryl, and heteroaryl are optionally substituted with a group selected from halogen, cyano, -OH, -NH₂, C₁-C₄ alkyl, and -O(C₁-C₄ alkyl);
X' is CH or N;
m is 0, 1, 2, 3, or 4;
n is 0, 1, 2, 3, or 4;
p is 1 or 2;
j is 0, 1, 2, 3, 4, or 5.

In some preferred embodiments of the present disclosure, the IRAK4 degrader is a compound of formula I-1, formula I-2, or formula II-1 or a pharmaceutically acceptable salt thereof: 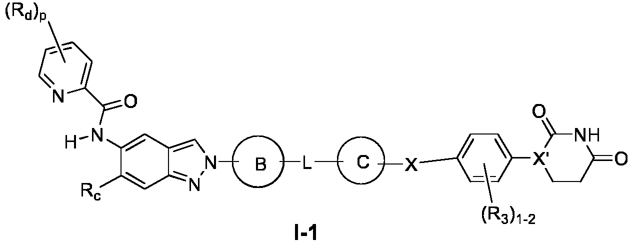 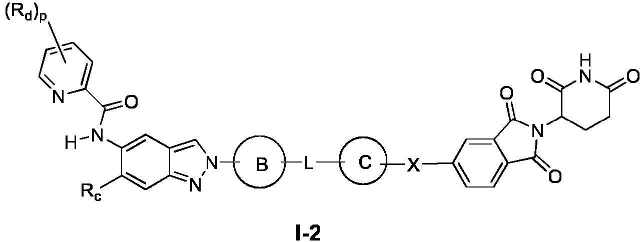 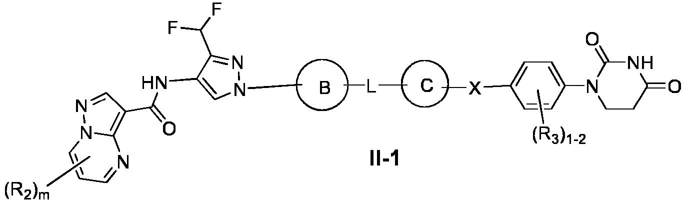 wherein:
R₃ is H, halogen, C₁-C₆ alkyl, or -O-(C₁-C₆ alkyl);
R_{c}, R_{d}, ring B, L, ring C, X, X', p, R₂, and m are as defined in the present disclosure.

In some preferred embodiments of the present disclosure, the disease is characterized by the use of an IRAK4 degrader for the treatment and/or prevention of an immunological disease.

In some preferred embodiments of the present disclosure, the IRAK4 degrader is used for an immunological disease selected from: adult-onset Still's disease, alopecia areata, ankylosing spondylitis, autoimmune hepatitis, autoimmune myocarditis, autoimmune pancreatitis, autoimmune retinopathy, autoimmune urticaria, behcet's disease, benign mucosal pemphigoid (mucosal pemphigoid), bullous pemphigoid, Castleman's disease (CD), celiac disease, coxsackie myocarditis, Crohn's disease, dermatitis herpetiformis, atopic dermatitis, dermatomyositis, discoid lupus, adenomyosis, eosinophilic fasciitis, erythema nodosum, fibrosing alveolitis, primary glomerulonephritis, Goodpasture's syndrome, autoimmune hemolytic anemia, Henoch-Schonlein purpura (HSP), hidradenitis suppurativa (HS), IgG4-associated sclerosing diseases, inclusion body myositis (IBM), interstitial cystitis (IC), Lambert-Eaton syndrome, linear IgA disease (LAD), systemic lupus erythematosus, chronic lyme disease, multiple sclerosis, systemic sclerosis, idiopathic inflammatory myopathy (IIM), ocular cicatricial pemphigoid optic neuritis, palindromic rheumatism (PR), pemphigus, autoimmune encephalomyelitis, POEMS syndrome, polyarteritis nodosa, primary biliary cholangitis, primary sclerosing cholangitis, psoriasis, psoriatic arthritis, reactive arthritis, relapsing polychondritis, retroperitoneal fibrosis, rheumatic fever, rheumatoid arthritis, sarcoidosis, autoimmune scleritis, morphea, Sjogren's syndrome, Takayasu arteritis, temporal arteritis, idiopathic thrombocytopenic purpura (ITP), thrombotic thrombocytopenic purpura (TTP), thyroid eye disease (Ted), Tolosa-Hunt syndrome (THS), transverse myelitis, ulcerative colitis (UC), undifferentiated connective tissue disease (UCTD), uveitis, systemic vasculitis, vitiligo, Vogt-Koyanagi-Harada disease, myasthenia gravis, gout, spondyloarthropathy, crystal arthropathy, osteoarthritis, rheumatoid arthritis, multiple myositis, interstitial lung disease, giant cell arteritis, polymyalgia rheumatica, granulomatous vasculitis, eosinophilic granulomatous vasculitis, eosinophilic vasculitis, microscopic polyangiitis, cryoglobulinemia, cutaneous leukocytoclastic vasculitis, mixed connective tissue disease, Steven-Johnson syndrome, pulmonary hypertension, endocarditis, atherosclerosis, erythema multiforme, acute coronary syndrome, idiopathic pulmonary fibrosis, non-alcoholic fatty liver, renal fibrosis, type 1 diabetes, primary dryness, Kawasaki disease, pustular psoriasis, chronic granulomatous disease, neuromyelitis optica urticaria, palmoplantar pustulosis, septicemia, bullous skin disease, Alzheimer's disease, chronic spontaneous urticaria, chronic psoriasis, juvenile idiopathic arthritis, axial spondyloarthritis, and Graves' disease.

In some preferred embodiments of the present disclosure, the IRAK4 degrader is used for an immunological disease selected from: rheumatoid arthritis, atopic dermatitis, hidradenitis suppurativa (HS), vitiligo, dermatomyositis, alopecia areata, urticaria, multiple myositis, interstitial lung disease, systemic lupus erythematosus, systemic sclerosis, and/or psoriasis.

In some preferred embodiments of the present disclosure, the disease is characterized by the use of an IRAK4 degrader for the treatment and/or prevention of an inflammatory disease.

In some preferred embodiments of the present disclosure, the IRAK4 degrader is used for an immunological disease, and the inflammatory disease is selected from: familial Mediterranean fever, tumor necrosis factor-associated periodic syndrome, mevalonate kinase deficiency, pyrin-associated autoinflammation with neutrophilic dermatosis (PAAND), pyogenic aseptic arthritis, pyoderma gangrenosum, and acne (PAPA), familial cold autoinflammatory syndrome (FCAS), familial keratosis lichenoides chronica (FKLC), NLRP1-associated autoinflammation with arthritis and dyskeratosis (NAIAD), deficiency of the IL-1 receptor antagonist (DIRA), deficiency of the IL-36 receptor antagonist (DITRA), allergic contact dermatitis, CAR-T cell-induced cytokine release syndrome, Crohn's disease, chronic bronchitis, COPD, active ankylosing spondylitis, axial spondyloarthritis, pityriasis rubra pilaris, inflammatory bowel disease, spinal arthritis, acute lung injury, generalized pustular psoriasis, acne vulgaris, and colitis.

In some preferred embodiments of the present disclosure, the IRAK4 degrader is for use for the treatment and/or prevention of a disease mediated by IL-2R alpha, IL-6, IFA-alpha2, IFN-gamma, IL-1ra, MCP-3, IL-16, IL-12 (p40), LIF, IL-5, GM-CSF, TNF-alpha, IL-2, IL-1alpha, IL-1beta, IL-18, Eotaxin, Basic FGF, beta-NGF, PDGF-BB, IL-4, MCP-1, IL-8, IL-10, GRO-alpha, HGF, IL-1alpha, IL-1beta, IL-3, SCF, TRAIL, M-CSF, CTACK, IL-15, IL-12 (P70), IL-17, IL-23, IL-33, and/or IL-36 cytokines.

In some preferred embodiments of the present disclosure, the IRAK4 degrader is for use for the treatment and/or prevention of a disease mediated by IL-4, IL-6, IL-12(p40), GM-CSF, TNF-alpha, IL-2, IL-1alpha, IL-1beta, IL-18, IL-8, IL-10, IL-17, IL-23, IL-33, and/or IL-36 cytokines.

In some preferred embodiments of the present disclosure, the sample is a spleen, skin, and/or blood sample. In some preferred embodiments of the present disclosure, the blood sample is normal human whole blood and/or patient whole blood.

In some preferred embodiments of the present disclosure, the method for the disease comprises administering to a subject an effective amount of an IRAK4 degrader.

In some preferred embodiments of the present disclosure, provided is the method for the treatment of the disease, wherein the IRAK4 degrader is the single compound or in combination with an additional drug.

According to the present disclosure, IRAK4 kinase activity tests prove that the compounds A1-A52, B1-B5, or C1-C36 described herein are able to effectively bind to the IRAK4 target protein and have degradation and/or inhibitory effects, and it is proved that the compounds A1-A52, B1-B5, or C1-C36 described herein is able to effectively and specifically degrade the IRAK4 protein in THP-1 cells by means of Western-Blot. The compounds A1-A52, B1-B5, or C1-C36, and/or the stereoisomers, the enantiomers, the diastereomers, the deuterides, the hydrates, the solvates, the metabolites, or the prodrugs thereof and/or the pharmaceutically acceptable salts thereof described herein can effectively degrade the IRAK4 protein, thereby achieving the effect of preventing or treating a disease or disorder associated with IRAK4.

The present disclosure proves experimentally that the compound described herein can significantly reduce the expression of IRAK4 in whole blood, skin, or liver, and that the IRAK4 degrader can be used for treating immunological diseases.

In the present disclosure, the medicament comprises an IRAK4 degrader with it as an active ingredient; the medicament described herein also optionally comprises a pharmaceutically acceptable carrier, diluent, or excipient.

In the present disclosure, the medicament IRAK4 degrader can be used alone or combined with additional drugs to realize a better therapeutic effect on inflammatory or immunological diseases.

In the present disclosure, the dosage form of the medicament may be tablets, pills, capsules, pulvis, granules, emulsions, suspensions, dispersions, solutions, syrups, elixirs, ointments, drops, suppositories, inhalants, or sprays. The medicament taking the IRAK4 degrader as the active ingredient can be prepared into any one of the medicament dosage forms described above according to actual needs, and the medicaments of each dosage form can be prepared according to the conventional method in the pharmaceutical field.

In the present disclosure, the administration route of the medicament may be selected from any one of oral administration, sublingual administration, intravenous injection, intraperitoneal injection, intramuscular injection, subcutaneous injection, nasal administration, transdermal administration, parenteral administration, inhalation administration, intratracheal administration, intrapulmonary administration, and bronchial administration, according to actual needs.

The present disclosure also provides pharmaceutically acceptable salts of the compounds A1-A52, B1-B5, or C1-C36. The term "pharmaceutically acceptable salt" refers to a relatively non-toxic acid addition salt or base addition salt of the compound of the present disclosure. The acid addition salts are salts formed from the compounds A1-A52, B1-B5, or C1-C36 of the present disclosure and a suitable inorganic acid or organic acid. These salts may be prepared during the final isolation and purification of the compounds or by reacting the purified compounds A1-A52, B1-B5, or C1-C36 in their free base form with a suitable organic acid or inorganic acid. Representative acid addition salts include hydrobromide, hydrochloride, sulfate, bisulfate, sulfite, acetate, oxalate, valerate, oleate, palmitate, stearate, laurate, borate, benzoate, lactate, phosphate, biphosphate, carbonate, bicarbonate, toluate, citrate, maleate, fumarate, succinate, tartrate, benzoate, methanesulfonate, p-toluenesulfonate, gluconate, lactobionate, lauryl sulfonate, and the like. The base addition salts are salts formed from the compounds A1-A52, B1-B5, or C1-C36 and a suitable inorganic base or organic base, including, for example, salts formed with alkali metal, alkaline earth metal, and quaternary ammonium cations, such as sodium salts, lithium salts, potassium salts, calcium salts, magnesium salts, tetramethyl quaternary ammonium salts, tetraethyl quaternary ammonium salts, and the like; amine salts include salts formed with ammonia (NH3) and primary, secondary, or tertiary amines, such as methylamine salts, dimethylamine salts, trimethylamine salts, triethylamine salts, ethylamine salts, and the like.

The compound or the pharmaceutically acceptable salt thereof of the present disclosure may be administered to mammals including humans, and may be administered orally, rectally, parenterally (intravenously, intramuscularly, or subcutaneously), topically (powders, ointments, or drops), or intratumorally.

The compound of the present disclosure may be administered at a dose of about 0.05-300 mg/kg body weight/day, preferably 10-300 mg/kg body weight/day, and more preferably 10-150 mg/kg body weight/day. The compound or the pharmaceutically acceptable salt thereof of the present disclosure may be formulated into a solid dosage form for oral administration, including but not limited to, capsules, tablets, pills, pulvis, granules, and the like. In these solid dosage forms, the compounds A1-A52, B1-B5, or C1-C36 of the present disclosure, as an active ingredient, are mixed with at least one conventional inert excipient (or carrier), such as sodium citrate or dicalcium phosphate, or with the following ingredients: (1) fillers or extenders, such as starch, lactose, sucrose, glucose, mannitol, and silicic acid; (2) binders, such as hydroxymethyl cellulose, alginate, gelatin, polyvinylpyrrolidone, sucrose, and acacia; (3) humectants, such as glycerol; (4) disintegrants, such as agar, calcium carbonate, potato or tapioca starch, alginic acid, certain complex silicates, and sodium carbonate; (5) solution retarders, such as paraffin; (6) absorption accelerators, such as quaternary ammonium compounds; (7) wetting agents, such as cetyl alcohol and glycerol monostearate; (8) adsorbents, such as kaolin; and (9) lubricants, such as talc, calcium stearate, magnesium stearate, solid polyethylene glycol, sodium lauryl sulfate, or mixtures thereof. Capsules, tablets, and pills may further comprise buffers. The solid dosage forms such as tablets, dragees, capsules, pills, and granules may be coated or microencapsulated using coatings and shells such as enteric coatings and other materials well known in the art. They may comprise opacifying agents, and the active ingredient in such a composition may be released in a certain part of the digestive tract in a delayed manner. Examples of embedding components that can be used are polymeric substances and wax-based substances. If necessary, the active ingredient can also be in microcapsule form with one or more of the above-mentioned excipients.

The compound or the pharmaceutically acceptable salt thereof of the present disclosure may be formulated into a liquid dosage form for oral administration, including but not limited to, pharmaceutically acceptable emulsions, solutions, suspensions, syrups, tinctures, and the like. In addition to the compounds A1-A52, B1-B5, or C1-C36 or the pharmaceutically acceptable salts thereof as an active ingredient, the liquid dosage form may comprise inert diluents commonly used in the art, such as water and other solvents, solubilizers, and emulsifiers, for example, ethanol, isopropanol, ethyl carbonate, ethyl acetate, propylene glycol, 1,3-butanediol, dimethylformamide, and oils, especially cottonseed oil, peanut oil, corn germ oil, olive oil, castor oil, sesame oil, and the like, or mixtures of these substances. In addition to these inert diluents, the liquid dosage form of the present disclosure may further comprise conventional adjuvants, such as wetting agents, emulsifiers and suspending agents, sweeteners, flavoring agents, perfuming agents, and the like. The suspending agents include, for example, ethoxylated isostearyl alcohols, polyoxyethylene sorbitol and sorbitan esters, microcrystalline cellulose, aluminum methylate, agar, and the like, or mixtures of these substances.

The compound or the pharmaceutically acceptable salt thereof of the present disclosure may be formulated into a dosage form for parenteral injection, including but not limited to, physiologically acceptable sterile aqueous or water-free solutions, dispersions, suspensions, or emulsions, and sterile powders for re-dissolution to form sterile injectable solutions or dispersions. Suitable carriers, diluents, solvents, or excipients include water, ethanol, polyols, and suitable mixtures thereof.

The compound or the pharmaceutically acceptable salt thereof of the present disclosure may also be formulated into a dosage form for topical administration, including, for example, ointments, pulvis, suppositories, drops, sprays, inhalants, and the like. The compounds A1-A52, B1-B5, or C1-C36 or the pharmaceutically acceptable salts thereof of the present disclosure as an active ingredient are mixed under sterile conditions with a physiologically acceptable carrier and optional preservatives, buffers, or propellants that may be required if necessary.

The present disclosure also provides a pharmaceutical composition comprising the compounds A1-A52, B1-B5, or C1-C36 or the pharmaceutically acceptable salts thereof of the present disclosure as an active ingredient, and a pharmaceutically acceptable carrier, excipient, or diluent. In the preparation of the pharmaceutical composition, the compounds A1-A52, B1-B5, or C1-C36 or the pharmaceutically acceptable salts thereof of the present disclosure are typically mixed with the pharmaceutically acceptable carrier, excipient, or diluent. The composition of the present disclosure may be formulated into a conventional pharmaceutical formulation according to a conventional preparation method. Examples include tablets, pills, capsules, pulvis, granules, emulsions, suspensions, dispersions, solutions, syrups, elixirs, ointments, drops, suppositories, inhalants, sprays, and the like.

The compound or the pharmaceutically acceptable salt thereof described herein may be administered alone or, if desired, in combination with additional pharmaceutically acceptable therapeutic agents, such as additional anti-tumor drugs, anti-inflammatory drugs, or autoimmune drugs. The ingredients to be combined may be administered simultaneously or sequentially, and administered in a single formulation form or in different formulation forms. The combination may include not only a combination of the compound of the present disclosure and one additional active agent but also a combination of the compound of the present disclosure and two or more additional active agents.

The pharmaceutically acceptable described herein means that the substance or composition must be compatible with the additional ingredients of the formulation and not deleterious to the patient.

The treatment described herein comprises both prophylactic and palliative treatment.

In some embodiments of the present disclosure, the sample is a patient or normal human blood sample; in some embodiments, the sample is a patient or normal human plasma sample; in some embodiments, the sample is a patient or normal human peripheral blood mononuclear cell sample.

In some embodiments of the present disclosure, measuring the level of an immune biomarker in a sample comprises using the AlphaLISA method. In some embodiments, measuring the level of an immune biomarker in a sample comprises using a method selected from those described in the examples.

Detailed description: Unless otherwise stated, the following terms used in the specification and claims have the following meanings.

"Alkyl" refers to a saturated aliphatic hydrocarbon group, including linear or branched alkyl; C₁-C₈ alkyl refers to an alkyl group containing 1-8 carbon atoms, e.g., methyl, ethyl, *n*-propyl, isopropyl, n-butyl, isobutyl, *tert-*butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n-*octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, or various branched isomers thereof, preferably C₁-C₆ alkyl, and more preferably, C₁-C₄ alkyl. The alkyl may be substituted or unsubstituted. In some embodiments, the alkyl is C₁, C₂, C₃, C₄, C₅, C₆, C₇, or C₈ alkyl.

"Cycloalkyl" refers to a saturated or partially unsaturated monocyclic or polycyclic hydrocarbon substituent; "C₃-C₁₁ cycloalkyl" refers to a cycloalkyl group containing 3 to 11 carbon atoms; "C₃-C₈ cycloalkyl" refers to a cycloalkyl group containing 3 to 8 carbon atoms; "C₅-C₁₀ cycloalkyl" refers to a cycloalkyl group containing 5 to 10 carbon atoms.

Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like, preferably cyclopropyl, cyclobutyl, cyclopentyl, and cyclohexyl, preferably C₃-C₈ cycloalkyl, and more preferably C₃-C₆ cycloalkyl.

Polycyclic cycloalkyl includes spiro, fused, and bridged cycloalkyl. "Spirocycloalkyl" refers to a polycyclic group in which a carbon atom (referred to as a spiro atom) is shared between monocyclic rings; they may contain one or more double bonds, but none of the rings has a fully conjugated π-electron system. According to the number of spiro atoms shared between rings, the spirocycloalkyl may include monospirocycloalkyl, bispirocycloalkyl, or polyspirocycloalkyl, and is preferably 7- to 12-membered bispirocycloalkyl. Non-limiting examples of the spirocycloalkyl include: 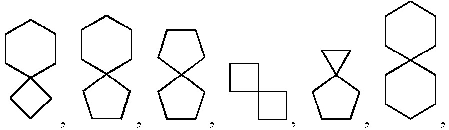 etc.

"Fused cycloalkyl" refers to an all-carbon polycyclic group in which each ring shares a pair of adjacent carbon atoms with the other rings in the system, wherein one or more of the rings may contain one or more double bonds, but none of the rings has a fully conjugated π-electron system. According to the number of constituent rings, the fused cycloalkyl may include bicyclic, tricyclic, tetracyclic, or polycyclic fused cycloalkyl, and is preferably bicyclic fused cycloalkyl. Non-limiting examples of the fused cycloalkyl include: 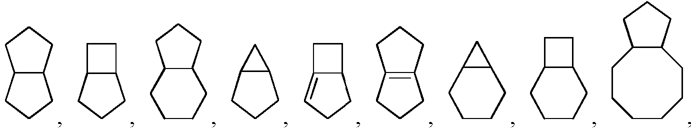 etc.

"Bridged cycloalkyl" refers to an all-carbon polycyclic group in which any two rings share two carbon atoms not directly connected; they may contain one or more double bonds, but none of the rings has a fully conjugated π-electron system. According to the number of constituent rings, the bridged cycloalkyl may include bicyclic, tricyclic, tetracyclic, or polycyclic bridged cycloalkyl. Non-limiting examples of the bridged cycloalkyl include: 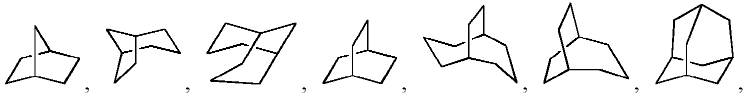 etc.

The cycloalkyl ring may be fused to an aryl, heteroaryl, or heterocycloalkyl ring, wherein the ring attached to the parent structure is cycloalkyl; non-limiting examples include indanyl, tetrahydronaphthyl, benzocycloheptyl, etc. The cycloalkyl may be optionally substituted or unsubstituted.

In some embodiments, the cycloalkyl is C₃, C₄, C₅, C₆, C₇, C₈, C₉, C₁₀, C₁₁ or C₁₂ monocyclic or polycyclic (e.g., spiro, fused or bridged) cycloalkyl.

"Heterocycloalkyl" refers to a saturated or partially unsaturated monocyclic or polycyclic hydrocarbon substituent, wherein one or more (e.g., 2, 3, 4, or 5) of the ring atoms are selected from nitrogen, oxygen, and S(O)ᵣ (wherein r is an integer of 0, 1, or 2), excluding ring portions of -O-O-, -O-S-, or -S-S-, and the remaining ring atoms are carbon atoms. "3- to 11-membered heterocycloalkyl" refers to a cyclic group containing 3 to 11 ring atoms, "5- to 10-membered heterocycloalkyl" refers to a cyclic group containing 5 to 10 ring atoms, and "3- to 8-membered heterocycloalkyl" refers to a cyclic group containing 3 to 8 ring atoms, preferably "3- to 11-membered heterocycloalkyl" containing 1-2 heteroatoms selected from N, O, and S, and more preferably "3- to 11-membered heterocycloalkyl" containing 1 or 2 N atoms.

Monocyclic heterocycloalkyl is preferably 3- to 8-membered monocyclic heterocycloalkyl containing 1-2 N heteroatoms; non-limiting examples of the monocyclic heterocycloalkyl include pyrrolidinyl, piperidinyl, piperazinyl, morpholinyl, thiomorpholinyl, homopiperazinyl, etc., preferably piperidinyl and piperazinyl. The polycyclic heterocycloalkyl includes spiro, fused, and bridged heterocycloalkyl. "Spiroheterocycloalkyl" refers to a polycyclic heterocycloalkyl group in which an atom (called spiro atom) is shared among monocyclic rings, wherein one or more of the ring atoms are selected from nitrogen, oxygen, and S(O)ᵣ (wherein r is an integer of 0, 1, or 2), and the remaining ring atoms are carbon atoms. They may contain one or more double bonds, but none of the rings has a fully conjugated π-electron system. According to the number of spiro atoms shared between rings, the spirocycloalkyl may include monospiroheterocycloalkyl, bispiroheterocycloalkyl, or polyspiroheterocycloalkyl, and is preferably saturated "3- to 11-membered bispiroheterocycloalkyl" containing 1-2 heteroatoms selected from N, O, and S, and more preferably saturated "7- to 11-membered bispiroheterocycloalkyl" containing 1 or 2 N atoms. Non-limiting examples of the spiroheterocycloalkyl include:

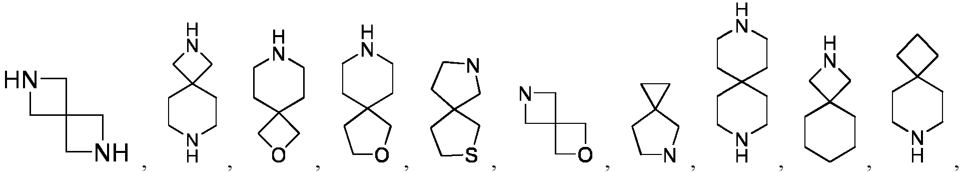

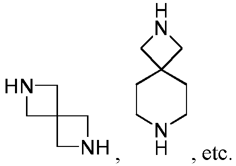

"Fused heterocycloalkyl" refers to a polycyclic heterocycloalkyl group in which each ring shares a pair of adjacent atoms with the other rings in the system, wherein one or more of the rings may contain one or more double bonds, but none of the rings has a fully conjugated π-electron system, wherein one or more of the ring atoms are selected from nitrogen, oxygen, and S(O)ᵣ (wherein r is an integer of 0, 1, or 2), and the remaining ring atoms are carbon atoms. According to the number of constituent rings, the fused heterocycloalkyl may include bicyclic, tricyclic, tetracyclic, or polycyclic fused heterocycloalkyl, and is preferably "3- to 11-membered bicyclic fused heterocycloalkyl" containing 1-3 heteroatoms selected from N, O, and S, and more preferably saturated "3- to 11-membered bicyclic fused heterocycloalkyl" containing 1 or 2 N atoms. Non-limiting examples of the fused heterocycloalkyl include: 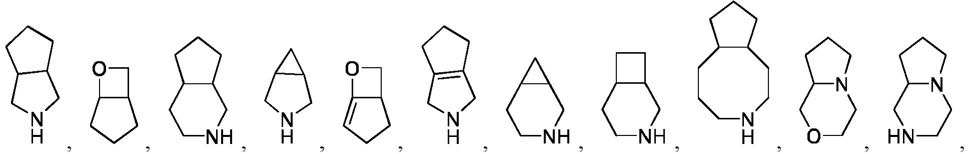 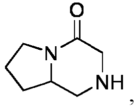 etc.

"Bridged heterocycloalkyl" refers to a polycyclic heterocycloalkyl group in which any two rings share two atoms not directly attached, and they may contain one or more double bonds, but none of the rings has a fully conjugated π-electron system, wherein one or more of the ring atoms are selected from nitrogen, oxygen, and S(O)ᵣ (wherein r is an integer of 0, 1, or 2), and the remaining ring atoms are carbon atoms. According to the number of constituent rings, the bridged heterocycloalkyl may include bicyclic, tricyclic, tetracyclic, or polycyclic bridged cycloalkyl; non-limiting examples of the bridged heterocycloalkyl include: 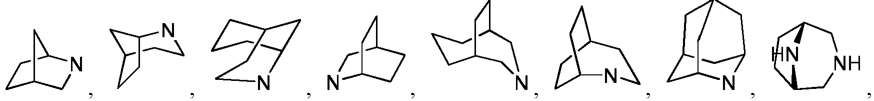 etc.

The heterocycloalkyl ring may be fused to an aryl, heteroaryl, or cycloalkyl ring, wherein the ring attached to the parent structure is heterocycloalkyl; non-limiting examples include: 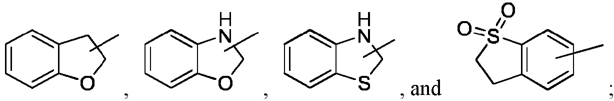 the heterocycloalkyl may be optionally substituted or unsubstituted.

In some embodiments, the heterocycloalkyl is 3-, 4-, 5-, 6-, 7-, 8-, 9-, 10-, 11-, or 12-membered monocyclic or polycyclic (e.g., spiro, fused, or bridged) heterocycloalkyl, wherein the number of the heteroatoms may be 1, 2, 3, 4, or 5, each heteroatom being independently nitrogen, oxygen, or S(O)ᵣ (wherein r is an integer of 0, 1, or 2).

"Aryl" refers to an all-carbon monocyclic or fused polycyclic group (i.e., rings that share pairs of adjacent carbon atoms) and a polycyclic group having a conjugated π-electron system; "C₆-C₁₀ aryl" refers to an all-carbon aryl group containing 6-10 carbon atoms, such as phenyl and naphthyl, preferably phenyl. The aryl ring may be fused to a heteroaryl, heterocycloalkyl, or cycloalkyl ring, wherein the ring attached to the parent structure is an aryl ring; non-limiting examples include: 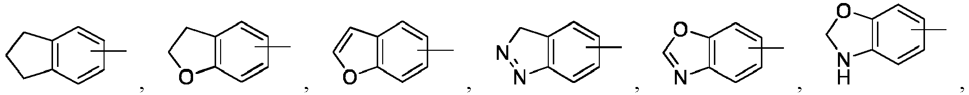 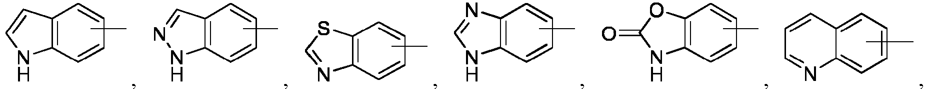 and 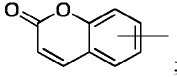 the aryl may be optionally substituted or unsubstituted. In some embodiments, the aryl is 6- to 10-membered aryl.

"Heteroaryl" refers to a heteroaromatic system containing 1 to 4 heteroatoms, and the heteroatoms include nitrogen, oxygen, or S(O)ᵣ (wherein r is an integer of 0, 1, or 2); 5- to 6-membered heteroaryl refers to a heteroaromatic system containing 5-6 ring atoms; 5- to 10-membered heteroaryl refers to a heteroaromatic system containing 5-10 ring atoms; preferably 5- to 6-membered heteroaryl; more preferably 5- to 6-membered heteroaryl containing 1 or 2 N atoms; non-limiting examples include furanyl, thienyl, pyridyl, pyrrolyl, N-alkylpyrrolyl, pyrimidinyl, pyrazinyl, pyrazole, imidazolyl, triazolyl, tetrazolyl, and the like, preferably pyridyl. The heteroaryl ring may be fused to an aryl, heterocycloalkyl, or cycloalkyl ring, wherein the ring attached to the parent structure is a heteroaryl ring; non-limiting examples include: 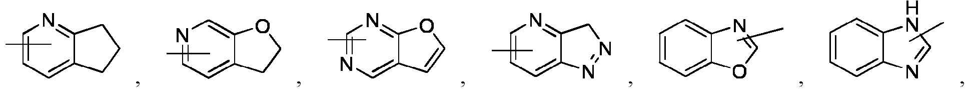 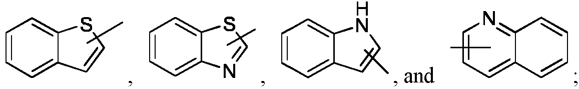 the heteroaryl may be optionally substituted or unsubstituted. In some embodiments, the heteroaryl is 5-, 6-, 7-, 8-, 9-, or 10-membered heteroaryl, wherein the number of the heteroatoms may be 1, 2, 3, 4, or 5, each heteroatom being independently nitrogen, oxygen, or S.

Unless otherwise indicated, the structure described herein also means all isomeric (e.g., enantiomeric, diastereomeric, and geometric (or conformational) forms of the structure; the R and S configurations of each asymmetric center; the Z and E double bond isomers; the Z and E conformational isomers. Thus, monolayer chemical isomers as well as enantiomeric, diastereomeric, and geometric (or conformational) mixtures of the compound of the present disclosure are within the scope of the present disclosure. Unless otherwise indicated, all tautomeric forms of the compound of the present disclosure are within the scope of the present disclosure. In addition, unless otherwise indicated, the structure described herein also means to include compounds that differ only in the presence of one or more isotopically enriched atoms. For example, it is within the scope of the present disclosure for compounds having the structure to include replacement of hydrogen with deuterium or tritium, or replacement of carbon with ¹³C or ¹⁴C. These compounds are useful as analytical tools, as probes in bioassays, or as therapeutic agents according to the present disclosure. "Stereoisomer" includes all isomers of a single compound which differ only in the orientation of their atoms in space. The term stereoisomer includes the enantiomers of a compound (including enantiomers of the compound in the (R-) or (S-) configuration), mixtures of enantiomers (physical mixtures of enantiomers, as well as racemates or racemic mixtures), isomers of geometric (cis/trans or E/Z, R/S) compounds, and isomers of compounds having multiple chiral centers that are not mirror images of each other (diastereomers). The chiral centers of the compounds can epimerize *in vivo,* and thus, for these compounds, administration of the compounds in the (R-) form is considered equivalent to administration of the compounds in the (S-) form. Thus, the compound of the present disclosure may be prepared and used as a single isomer and substantially free of other isomers, or as a mixture of various isomers, e.g., prepared and used as a racemic mixture of stereoisomers. In some embodiments, the bifunctional compound of the present disclosure is an isotopic derivative because it has at least one desired isotopic substitution of atoms in an amount above the natural abundance, i.e., enrichment, of the isotope. In one embodiment, the compound comprises deuterium or a plurality of deuterium atoms. Substitution with heavier isotopes such as deuterium, i.e., 2H, may afford certain therapeutic advantages resulting from greater metabolic stability, e.g., increased *in vivo* half-life or reduced dose requirements, and hence may be advantageous in some circumstances. Furthermore, the bifunctional compound of the present disclosure comprises N-oxides, crystalline forms (also referred to as polymorphs), active metabolites of compounds having the same type of activity, tautomers, as well as unsolvated, solvated, and hydrated forms of pharmaceutically acceptable solvents such as water, ethanol, and the like in compounds. The solvated form of a conjugate provided herein is also considered disclosed herein.

"Pharmaceutical composition" refers to a mixture containing one or more of the compounds or the physiologically/pharmaceutically acceptable salts or prodrugs thereof described herein and other chemical components, as well as other components such as physiologically/pharmaceutically acceptable carriers and excipients. The pharmaceutical composition is intended to promote the administration to an organism, so as to facilitate the absorption of the active ingredient, thereby exerting biological activity.

"Control sample" or "reference sample" refers to an individual or sample of a group of individuals not suffering from a disease or disorder (e.g., arthritis, rheumatoid arthritis, myositis, lupus erythematosus, and/or systemic sclerosis) or internal control, as determined by techniques known in the art. In some embodiments, the control or baseline level is determined first, or measured prior to measurement in a sample, or obtained from a database of such a control sample.

"Reference compound (I)" and "reference (I)" are used interchangeably herein.

"Reference compound (II)" and "reference (II)" are used interchangeably herein.

"Induction" and "stimulation" are used interchangeably herein.

The "effective amount" refers to an amount that, when administered to a subject, produces a beneficial or desired result, including a clinical result, e.g., degrades, inhibits, or reduces symptoms of a condition being treated in the subject as compared to a control.

"Pharmaceutically acceptable carrier, adjuvant, or carrier" refers to a non-toxic carrier, adjuvant, or carrier that does not destroy the pharmacological activity of the compound with which it is formulated. Pharmaceutically acceptable carriers, adjuvants, or carriers which may be used in the composition of the present disclosure include, but are not limited to, ion exchangers, aluminium oxide, stearates, lecithin, serum proteins such as human serum albumin, phosphates, and other buffer substances, glycine, sorbic acid, potassium sorbate, saturated vegetable fatty acids, water, partial glycerol mixtures of salts or electrolytes, such as guanidine sulfate, disodium phosphate, disodium phosphate, sodium chloride, zinc salts, colloidal silica, magnesium glucaluminate, polyvinylpyrrolidone, cellulose-based substances, polyethylene glycol, carboxymethyl cellulose, polyacrylates, waxes, polyethylene-polyoxypropylene-block polymers, polyethylene glycol, and wool fat.

"Patient" refers to an animal, preferably a mammal, and most preferably a human.

"Cytokine" refers to a cytokine selected from IL-2Ralpha, MIG, MIP-1beta, IL-6, IFN-alpha2, IFN-gamma, SDF-1alpha, IL-1ra, MCP-3, IL-16, IL-12(p40), LIF, TNF-beta, IL-5, GM-CSF, MIF, TNF-alpha, RANTES, IL-2, IL-1beta, IL-18, Eotaxin, BasicFGF, VEGF, beta-NGF, PDGF-BB, IP-10, IL-13, IL-4, MCP-1, IL-8, MIP-1alpha, IL-10, G-CSF, GRO-alpha, HGF, IL-1alpha, IL-3, SCF, TRAIL, M-CSF, CTACK, IL-15, IL-7, IL-12(p70), IL-17, IL-9, SCGF-beta, KC, IL-17A, and MCP-17A. In some embodiments, measuring the cytokine level in a normal human or mouse sample comprises determining by using cultured peripheral blood mononuclear cells (PBMCs). In some embodiments, measuring the cytokine level in a normal human or mouse sample comprises determining by using the Luminex method.

The following illustrative embodiments are provided to illustrate the present disclosure. The present disclosure is not limited to these examples.

The present disclosure is further explained in detail below with reference to examples; however, the examples are not intended to limit the present disclosure, and the present disclosure is not limited to the contents of the examples. The starting materials in the examples of the present disclosure are known and commercially available, or may be synthesized by using or following methods known in the art. Unless otherwise stated, experimental methods without specific conditions indicated in the examples of the present disclosure were generally conducted under conventional conditions or conditions recommended by the manufacturers of the starting materials or commercial products.

Definition of abbreviations

"DMSO" refers to *N,N*-dimethylformamide.

"Glucose" refers to glucose.

"Solutol" refers to polyethylene glycol-15 hydroxystearate.

"PO" refers to oral administration.

"LPS" refers to lipopolysaccharide.

"R848" refers to resiquimod.

"PBMC" refers to a human peripheral blood mononuclear cell.

"IRAK4" refers to interleukin 1 receptor associated kinase 4.

"IL" refers to interleukin.

"IMQ" refers to imiquimod.

"Topical" refers to topical administration.

"BID" refers to 2 times daily.

"QD" refers to once daily.

### BRIEF DESCRIPTION OF THE DRAWINGS

FIG. 1 IRAK4 degradation by compound A as shown in FIGs. 1A and 1B, respectively.
FIG. 2 A graph showing the intracellular IRAK4 protein expression level in PBMCs of various patients.
FIG. 3 IL-6 inhibition in LPS-induced normal human PMBCs by compound A.
FIG. 4 Inhibition of LPS-stimulated cytokine secretion from human peripheral blood mononuclear cells by compound A.
FIG. 5 Inhibition of R848-stimulated cytokine secretion from human peripheral blood mononuclear cells by compound A.
FIG. 6 IL-6 inhibition in normal whole blood and patient whole blood by compound A as shown in FIGs. 6A, 6B, 6C, and 6D, respectively.
FIG. 7 IRAK4 degradation in PBMCs, spleen, and skin of C57 mice by compound A as shown in FIGs. 7A, 7B, and 7C, respectively.
FIG. 8 Efficacy of compound A on imiquimod-induced C57 mouse psoriasis model as shown in FIGs. 8A, 8B, and 8C, respectively.

### DETAILED DESCRIPTION:

### I. Compound Preparation Examples:

### Example 1: 5-((1R,4R)-2-oxa-5-azabicyclo[2.2.1]heptan-5-yl)-N-(1-(1-((3-(4-chloro-3-(2,4-dioxotetrahydropyrimidin-1(2H)-yl)benzoyl)-3-azaspiro[5.5]undecan-9-yl)methyl)piperidin-4-yl)-3-(difluoromethyl)-1H-pyrazol-4-yl)pyrazolo[1,5-a]pyrimidine-3-carboxamide

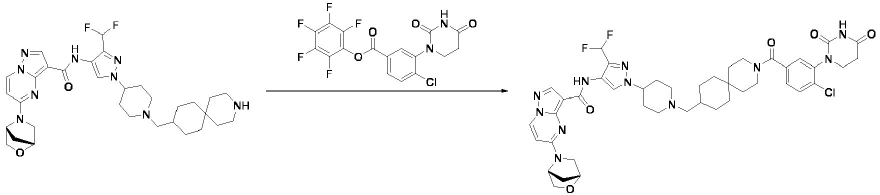

A solution of*N*-(1-(1-((3-azaspiro[5.5]undecan-9-yl)methyl)piperidin-4-yl)-3-(difluoromethyl)-1*H*-pyrazol-4-yl)-5-((1*R*,4*R*)-2-oxa-5-azabicyclo[2.2.1]heptan-5-yl)pyrazolo[1,5-*a*]pyrimidine-3-carboxamide (150 mg, crude, 0.18 mmol), pentafluorophenyl 4-chloro-3-(2,4-dioxotetrahydropyrimidin-1(2*H*)-yl)benzoate (78 mg, 0.18 mmol), and *N,N*-diisopropylethylamine (116 mg, 0.9 mmol) in dimethyl sulfoxide (5 mL) was stirred at room temperature overnight. The resulting mixture was poured into water (50 mL), and the mixture was stirred for 10 min. The mixture was filtered, and the filter cake was purified by preparative HPLC to give the target product, 5-((1*R*,4*R*)-2-oxa-5-azabicyclo[2.2.1]heptan-5-yl)-*N*-(1-(1-((3-(4-chloro-3-(2,4-dioxotetrahydropyrimidin-1(2*H*)-yl)benzoyl)-3-azaspiro[5.5]undecan-9-yl)methyl)piperidin-4-yl)-3-(difluoromethyl)-1*H*-pyrazol-4-yl)pyrazolo[1,5-*a*]pyrimidine-3-carboxamide.

LC-MS: (ESI, *m*/*z):* [M+H] ⁺ = 874.5.

¹H NMR (400 MHz, DMSO-*d*₆) δ 10.51 (s, 1H), 9.50 (d, *J =* 5.9 Hz, 1H), 8.78 (d, *J =* 7.7 Hz, 1H), 8.39 (d, *J* = 4.0 Hz, 1H), 8.26 (d, *J =* 5.7 Hz, 1H), 7.63 (d, *J =* 8.2 Hz, 1H), 7.55 (d, *J* = 1.9 Hz, 1H), 7.39 (dd, *J* = 8.3, 2.0 Hz, 1H), 7.27-6.96 (m, 1H), 6.90 - 6.41 (m, 1H), 5.30-5.05 (m, 1H), 4.77 (d, *J =* 17.4 Hz, 1H), 4.26-4.14 (m, 1H), 3.86 - 3.70 (m, 3H), 3.66 - 3.41 (m, 5H), 3.30-3.25 (m, 2H), 2.96-2.84 (m, 2H), 2.79 - 2.70 (m, 2H), 2.22-2.08 (m, 2H), 2.06 - 1.83 (m, 8H), 1.75-1.65 (m, 2H), 1.63 - 1.24 (m, 7H), 1.19 - 0.91 (m, 4H).

### Example 2: 5-((1R,4R)-2-oxa-5-azabicyclo[2.2.1]heptan-5-yl)-N-(1-((1r,4R)-4-((9-(4-chloro-3-(2,4-dioxotetrahydropyrimidin-1(2H)-yl)benzoyl)-3,9-diazaspiro[5.5]undecan-3-yl)methyl)cyclohexyl)-3-(difluoromethyl)-1H-pyrazol-4-yl)pyrazolo[1,5-a]pyrimidine-3-carboxamide

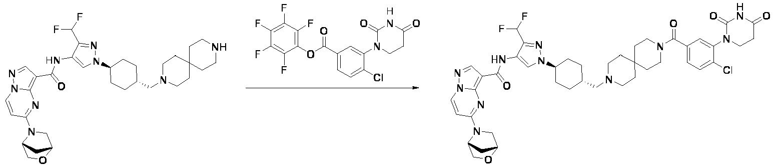

The preparation method refers to Example 1.
LC-MS: (ESI, *m*/*z):* [M+H] ⁺ = 874.4
¹H NMR (400 MHz, DMSO-*d*₆) δ 10.51 (s, 1H), 9.50 (d, *J =* 6.3 Hz, 1H), 8.78 (d, *J =* 7.7 Hz, 1H), 8.38 (d, *J* = 4.4 Hz, 1H), 8.25 (d, *J =* 5.7 Hz, 1H), 7.64 (d, *J =* 8.2 Hz, 1H), 7.55 (d, *J =* 1.9 Hz, 1H), 7.39 (dd, *J =* 8.2, 1.9 Hz, 1H), 7.26-6.95 (m, 1H), 6.91-6.41 (m, 1H), 5.32-5.03 (m, 1H), 4.77 (d, *J =* 17.4 Hz, 1H), 4.17 (t, *J =* 12.9 Hz, 1H), 3.84 - 3.71 (m, 3H), 3.67-3.57 (m, 4H), 3.45 (d, *J* = 9.8 Hz, 1H), 3.30-3.25 (m, 2H), 2.79 - 2.71 (m, 2H), 2.38-2.24 (m, 4H), 2.13 - 1.77 (m, 8H), 1.79-1.66 (m, 2H), 1.60-1.35 (m, 9H), 1.12-0.92 (m, 2H).

### Example 3: 5-((1R,4R)-2-oxa-5-azabicyclo[2.2.1]heptan-5-yl)-N-(3-(difluoromethyl)-1-(1-((3-(3-(2,4-dioxotetrahydropyrimidin-1(2H)-yl)-4-methoxybenzoyl)-3-azaspiro[5.5]undecan-9-yl)methyl)piperidin-4-yl)-1H-pyrazol-4-yl)pyrazolo[1,5-a]pyrimidine-3-carboxamide

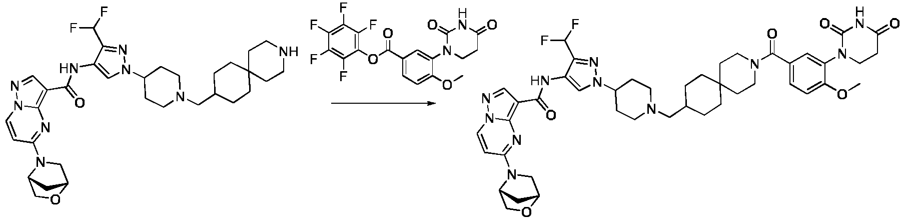

A mixture of *N*-(1-(1-((3-azaspiro[5.5]undecan-9-yl)methyl)piperidin-4-yl)-3-(difluoromethyl)-1*H*-pyrazol-4-yl)-5-((1*R*,4*R*)-2-oxa-5-azabicyclo[2.2.1]heptan-5-yl)pyrazolo[1,5-*a*]pyrimidine-3-carboxamide (160 mg, 0.26 mmol), pentafluorophenyl 3-(2,4-dioxotetrahydropyrimidin-1(2*H*)-yl)-4-methoxybenzoate (110 mg, 0.26 mmol), and DIEA (168 mg, 1.3 mmol) in DMSO (5 mL) was stirred at room temperature overnight. The reaction liquid was poured into water (50 mL), and the mixture was stirred for 10 min. The mixture was filtered, and the filter cake was purified by Prep-HPLC to give 5-((1*R*,4*R*)-2-oxa-5-azabicyclo[2.2.1]heptan-5-yl)-*N*-(3-(difluoromethyl)-1-(1-((3-(3-(2,4-dioxotetrahydropyrimidin-1(2*H*)-yl)-4-methoxybenzoyl)-3-azaspiro[5.5]undecan-9-yl)methyl)piperidin-4-yl)-1*H*-pyrazol-4-yl)pyrazolo[1,5-*a*]pyrimidine-3-carboxamide.

LC-MS: (ESI, *m*/*z*): [M+H] ⁺ = 870.3.

¹H NMR (400 MHz, DMSO-*d*₆) δ 10.33 (s, 1H), 9.50 (d, *J =* 5.7 Hz, 1H), 8.79 (d, *J =* 7.7 Hz, 1H), 8.39 (d, *J* = 4.0 Hz, 1H), 8.26 (d, *J =* 5.6 Hz, 1H), 7.41 - 7.29 (m, 2H), 7.27 - 6.95 (m, 2H), 6.91-6.40 (m, 1H), 5.31-5.00 (m, 1H), 4.77 (d, *J =* 17.5 Hz, 1H), 4.27-4.15 (m, 1H), 3.86 - 3.71 (m, 5H), 3.65 - 3.39 (m, 8H), 2.89 (d, *J =* 10.4 Hz, 2H), 2.68 (t, *J* = 6.5 Hz, 2H), 2.15 (d, *J* = 6.1 Hz, 2H), 2.08 - 1.87 (m, 8H), 1.76-1.65 (m, 2H), 1.63-1.38 (m, 5H), 1.36-1.21 (m, 2H), 1.19-0.95 (m, 4H).

### Example 4: N-(1-(1-((3-(4-chloro-3-(2,4-dioxotetrahydropyrimidin-1(2H)-yl)benzoyl)-3-azaspiro[5.5]undecan-9-yl)methyl)piperidin-4-y1)-3-(difluoromethyl)-1H-pyrazol-4-yl)-5-morpholinopyrazolo[1,5-a]pyrimidine-3-carboxamide

### Step 1: preparation of tert-butyl 4-(3-(difluoromethyl)-4-(5-morpholinopyrazolo[1,5-a]pyrimidine-3-carboxamido)-1H-pyrazol-1-yl)piperidine-1-carboxylate

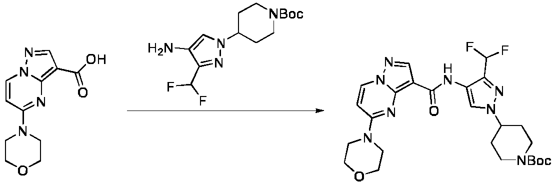

HATU (900 mg, 2.4 mmol) was added to a solution of 5-morpholinopyrazolo[1,5-*a*]pyrimidine-3-carboxylic acid (470 mg, 1.9 mmol) and DIEA (1 mL, 4.7 mmol) in DMF (20 mL) at 25 °C. After the mixture was stirred at 25 °C for 2 h, *tert*-butyl 4-(4-amino-3-(difluoromethyl)-1*H*-pyrazol-1-yl)piperidine-1-carboxylate (0.5 g, 1.5 mmol) was added, and the reaction liquid was then stirred at 25 °C overnight. Water (30 mL) was added to the reaction liquid, and then the mixture was extracted with EA (50 mL × 3). The organic phases were combined, washed with saturated brine, dried over anhydrous Na₂SO₄, filtered, and concentrated under reduced pressure. The resulting crude product was purified by flash column chromatography (EA) to give *tert*-butyl 4-(3-(difluoromethyl)-4-(5-morpholinopyrazolo[1,5-*a*]pyrimidine-3-carboxamido)-1*H*-pyrazol-1-yl)piperidine-1-carboxylate.

LC-MS: (ESI, *m*/*z):* [M+H] ⁺ = 547.4.

### Step 2: preparation of N-(3-(difluoromethyl)-1-(piperidin-4-yl)-1Hpyrazol-4-yl)-5-morpholinopyrazolo[1,5-a]pyrimidine-3-carboxamide

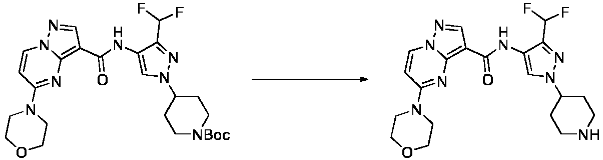

TFA (1 mL) was added to a solution of *tert-butyl* 4-(3-(difluoromethyl)-4-(5-morpholinopyrazolo[1,5-*a*]pyrimidine-3-carboxamido)-1*H*-pyrazol-1-yl)piperidine-1-carboxylate (0.2 g, 0.366 mmol) in DCM (3 mL) at 25 °C. The reaction liquid was stirred at 25 °C for 1 h. The reaction liquid was directly concentrated under reduced pressure to give *N*-(3-(difluoromethyl)-1-(piperidin-4-yl)-1*H*-pyrazol-4-yl)-5-morpholinopyrazolo[1,5-*a*]pyrimidine-3-carboxamide.

LC-MS: (ESI, *m*/*z):* [M+H] ⁺ = 447.2.

### Step 3: preparation of tert-butyl 9-((4-(3-(difluoromethyl)-4-(5-morpholinopyrazolo[1,5-a]pyrimidine-3-carboxamido)-1H-pyrazol-1-yl)piperidin-1-yl)methyl)-3-azaspiro[5.5]undecane-3-carboxylate

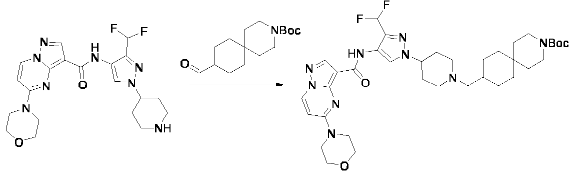

*tert*-Butyl 9-formyl-3-azaspiro[5.5]undecane-3-carboxylate (150 mg, 0.45 mmol) and NaBH₃CN (100 mg, 2.25 mmol) were added to a solution of N-(3-(difluoromethyl)-1-(piperidin-4-yl)-1*H*-pyrazol-4-yl)-5-morpholinopyrazolo[1,5-*a*]pyrimidine-3-carboxamide (200 mg, 0.45 mmol) and DIEA (0.5 mL, 1.35 mmol) in MeOH (5 mL) at 25 °C. The reaction liquid was stirred at 25 °C for 16 h. After filtration and concentration under reduced pressure, the resulting crude product was purified by flash column chromatography (MeOH:DCM = 3:97) to give *tert*-butyl 9-((4-(3-(difluoromethyl)-4-(5-morpholinopyrazolo[1,5-*a*]pyrimidine-3-carboxamido)-1*H*-pyrazol-1-yl)piperidin-1-yl)methyl)-3-azaspiro[5.5]undecane-3-carboxylate.

LC-MS: (ESI, *m*/*z):* [M+H] ⁺ = 712.4.

### Step 4: preparation of N-(1-(1-((3-azaspiro[5.5]undecan-9-yl)methyl)piperidin-4-yl)-3-(difluoromethyl)-1H-pyrazol-4-yl)-5-morpholinopyrazolo[1,5-a]pyrimidine-3-carboxamide

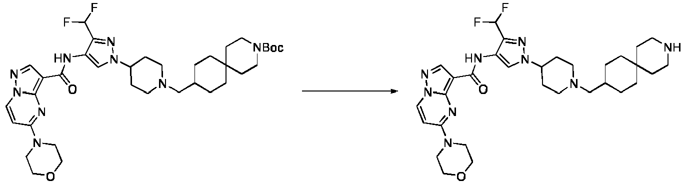

TFA (1 mL) was added to a solution of *tert*-butyl 9-((4-(3-(difluoromethyl)-4-(5-morpholinopyrazolo[1,5-*a*]pyrimidine-3-carboxamido)-1*H*-pyrazol-1-yl)piperidin-1-yl)methyl)-3-azaspiro[5.5]undecane-3-carboxylate (0.14 g, 0.2 mmol) in DCM (3 mL) at 25 °C. The reaction liquid was stirred at 25 °C for 1 h. The reaction liquid was directly concentrated under reduced pressure to give *N*-(1-(1-((3-azaspiro[5.5]undecan-9-yl)methyl)piperidin-4-yl)-3-(difluoromethyl)-1*H-*pyrazol-4-yl)-5-morpholinopyrazolo[1,5-*a*]pyrimidine-3-carboxamide. The resulting product was directly used in the next step.

LC-MS: (ESI, *m*/*z):* [M+H] ⁺ = 612.5.

### Step 5: preparation of N-(1-(1-((3-(4-chloro-3-(24-dioxotetrahydropyrimidin-1(2H)-yl)benzoyl)-3-azaspiro[5.5]undecan-9-yl)methyl)piperidin-4-yl)-3-(difluoromethyl)-1H-pyrazol-4-yl)-5-morpholinopyrazolo[1,5-a]pyrimidine-3-carboxamide

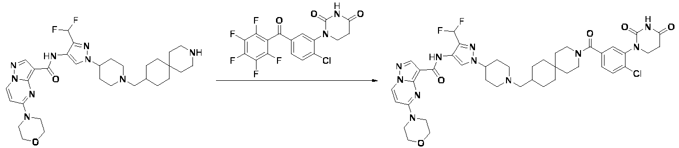

DIEA (0.1 mL, 0.54 mmol) was added to a solution of *N*-(1-(1-((3-azaspiro[5.5]undecan-9-yl)methyl)piperidin-4-yl)-3-(difluoromethyl)-1*H*-pyrazol-4-yl)-5-morpholinopyrazolo[1,5-*a*]pyrimidine-3-carboxamide (110 mg, 0.18 mmol) and 1-(2-chloro-5-pentafluorobenzoyl)phenyl)dihydropyrimidine-2,4(1*H*,3*H*)-dione (90 mg, 0.19 mmol) in DMSO (2 mL) at 25 °C. The reaction liquid was stirred at 25 °C for 16 h. The reaction liquid was poured into water (10 mL), and the mixture was filtered. The filter cake was purified by Prep-HPLC to give.

LC-MS: (ESI, *m*/*z):* [M+H] ⁺ = 862.4.

¹H NMR (400 MHz, DMSO-*d*₆) δ 10.51 (s, 1H), 9.40 (s, 1H), 8.83 (d, *J =* 7.9 Hz, 1H), 8.39 (s, 1H), 8.29 (s, 1H), 7.63 (d, *J =* 8.2 Hz, 1H), 7.55 (d, *J* = 1.9 Hz, 1H), 7.39 (dd, *J =* 8.2, 1.8 Hz, 1H), 7.25-6.95 (m, 1H), 6.91 (d, *J =* 8.0 Hz, 1H), 4.26-4.14 (m, 1H), 3.87 - 3.68 (m, 9H), 3.66 - 3.50 (m, 3H), 3.31 - 3.20 (m, 2H), 2.95-2.83 (m, 2H), 2.80 - 2.68 (m, 2H), 2.21-2.08 (m, 2H), 2.07 - 1.87 (m, 6H), 1.70 (d, *J =* 8.7 Hz, 2H), 1.63 - 1.41 (m, 5H), 1.38 - 1.25 (m, 2H), 1.16 - 1.02 (m, 4H).

### Example 5: N-(1-(1-((3-(4-chloro-3-(2,4-dioxotetrahydropyrimidin-1(2H)-yl)benzoyl)-3-azaspiro[5.5]undecan-9-yl)methyl)piperidin-4-yl)-3-(difluoromethyl)-1H-pyrazol-4-yl)-5-(piperazin-1-yl)pyrazolo[1,5-a]pyrimidine-3-carboxamide

### Step 1: preparation of ethyl 5-(4-((benzyloxy)carbonyl)piperazin-1-yl)pyrazolo[1,5-a]pyrimidine-3-carboxylate

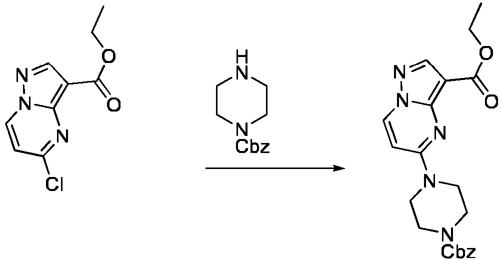

Ethyl 5-chloropyrazolo[1,5-*a*]pyrimidine-3-carboxylate (3.0 g, 13.3 mmol) and benzyl piperazine-1-carboxylate (2.9 g, 13.3 mmol) were dissolved in a solution of ACN (30 mL). DIEA (5.2 g, 40 mmol) was added, and the reaction liquid was heated to 60 °C, stirred for 2 h, and concentrated under reduced pressure. The crude product was dissolved in ethyl acetate (100 mL), washed with saturated brine, and concentrated under reduced pressure. The crude product was purified by column chromatography (PE:EA = 2:1) to give ethyl 5-(4-((benzyloxy)carbonyl)piperazin-1-yl)pyrazolo[1,5-*a*]pyrimidine-3-carboxylate.

LC-MS: (ESI, *m*/*z):* [M+H] ⁺ = 410.2.

¹H NMR (400 MHz, DMSO-*d*₆) δ 8.76 (d, J = 7.9 Hz, 1H), 8.22 (s, 1H), 7.41 - 7.33 (m, 5H), 6.86 (d, J = 7.9 Hz, 1H), 5.13 (s, 2H), 4.19 (q, J = 7.1 Hz, 2H), 3.88-3.74 (m, 4H), 3.62-3.49 (m, 4H), 1.28 (t, J = 7.1 Hz, 3H).

### Step 2: preparation of 5-(4-((benzyloxy)carbonyl)piperazin-1-yl)pyrazolo[1,5-a]pyrimidine-3-carboxylic acid

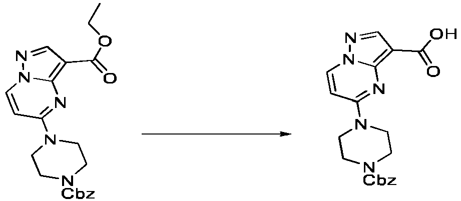

Ethyl 5-(4-((benzyloxy)carbonyl)piperazin-1-yl)pyrazolo[1,5-*a*]pyrimidine-3-carboxylate (2.6 g, 6.3 mmol) was dissolved in methanol (26 mL), lithium hydroxide (1.0 g, 25.2 mmol) was added, and the reaction liquid was stirred at 60 °C for 2 h. After the reaction was completed, dilute hydrochloric acid (2 mol/L) was added to the reaction liquid to adjust the pH of the reaction liquid to 2. Then the mixture was filtered, and the resulting filter cake was washed with water and dried under vacuum to give 5-(4-((benzyloxy)carbonyl)piperazin-1-yl)pyrazolo[1,5-*a*]pyrimidine-3-carboxylic acid.

LC-MS: (ESI, *m*/*z):* [M+H] ⁺ = 382.2.

### Step 3: preparation of benzyl 4-(3-((1-(1-(tert-butoxycarbonyl)piperidin-4-yl)-3-(difluoromethyl)-1H-pyrazol-4-yl)carbamoyl)pyrazolo[1,5-a]pyrimidin-5-yl)piperazine-1-carboxylate

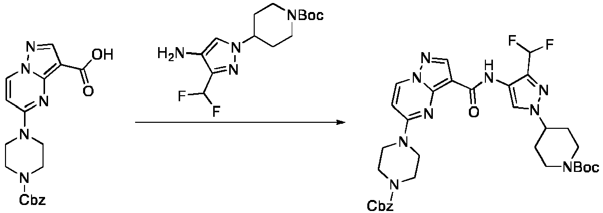

HATU (380 mg, 0.50 mmol) was added to a solution of 5-(4-((benzyloxy)carbonyl)piperazin-1-yl)pyrazolo[1,5-*a*]pyrimidine-3-carboxylic acid (270 mg, 0.4 mmol) and DIEA (0.5 mL, 1.0 mmol) in DMF (5 mL) at 25 °C. After the mixture was stirred at room temperature for 2 h, *tert*-butyl 4-(4-amino-3-(difluoromethyl)-1*H*-pyrazol-1-yl)piperidine-1-carboxylate (0.21 g, 0.3 mmol) was added, and the reaction liquid was stirred at 25 °C overnight. Water (30 mL) was added to quench the reaction, and the mixture was extracted with EA (50 mL × 3). The organic phase was washed with saturated brine, dried over anhydrous Na₂SO₄, filtered, and concentrated under reduced pressure. The resulting crude product was purified by column chromatography (MeOH:DCM = 0-3:97) to give benzyl 4-(3-((1-(1-(*tert*-butoxycarbonyl)piperidin-4-yl)-3-(difluoromethyl)-1*H*-pyrazol-4-yl)carbamoyl)pyrazolo[1,5-*a*]pyrimidin-5-yl)piperazine-1-carboxylate.

LC-MS: (ESI, *m*/*z):* [M+H] ⁺ = 680.4.

### Step 4: preparation of benzyl 4-(3-((3-(difluoromethyl)-1-(piperidin-4-yl)-1H-pyrazol-4-yl)carbonyl)pyrazolo[1,5-a]pyrimidin-5-yl)piperazine-1-carboxylate

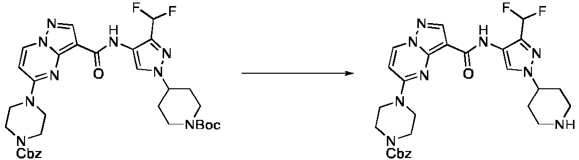

TFA (1 mL) was added to a solution of benzyl 4-(3-((1-(1-(tert-butoxycarbonyl)piperidin-4-yl)-3-(difluoromethyl)-1*H-*pyrazol-4-yl)carbamoyl)pyrazolo[1,5-*a*]pyrimidin-5-yl)piperazine-1-carboxylate (0.29 g, 0.42 mmol) in DCM (5 mL), and then the reaction liquid was stirred at room temperature for 2 h. The reaction liquid was directly concentrated under reduced pressure to give benzyl 4-(3-((3-(difluoromethyl)-1-(piperidin-4-yl)-1*H*-pyrazol-4-yl)carbonyl)pyrazolo[1,5-*a*]pyrimidin-5-yl)piperazine-1-carboxylate.

LC-MS: (ESI, *m*/*z):* [M+H] ⁺ = 580.5.

### Step 5: preparation of tert-butyl 9-((4-(4-(5-(4-((benzyloxy)carbonyl)piperazin-1-yl)pyrazolo[1,5-a]pyrimidine-3-carboxamido)-3-(difluoromethyl)-1H-pyrazol-1-yl)piperidin-1-yl)methyl)-3-azaspiro[5.5] undecane-3-carboxylate

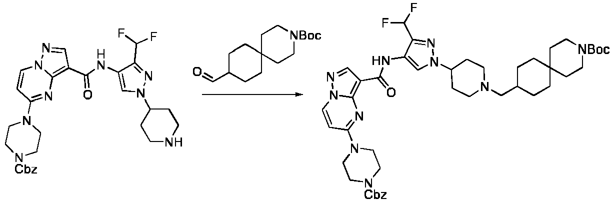

*tert*-Butyl 9-formyl-3-azaspiro[5.5]undecane-3-carboxylate (144 mg, 0.45 mmol) and NaBH₃CN (138 mg, 1.2 mmol) were added to a solution of benzyl 4-(3-((3-(difluoromethyl)-1-(piperidin-4-yl)-1*H*-pyrazol-4-yl)carbonyl)pyrazolo[1,5-*a*]pyrimidin-5-yl)piperazine-1-carboxylate (230 mg, 0.4 mmol) and DIEA (0.35 mL, 2.0 mmol) in MeOH (10 mL). The reaction liquid was stirred at 25 °C overnight. Water (30 mL) was added to quench the reaction, and then the mixture was extracted with EA (50 mL × 3). The organic phase was washed with saturated brine, dried over anhydrous Na₂SO₄, filtered, and concentrated under reduced pressure. The resulting crude product was purified by flash column chromatography (MeOH:DCM = 0-3:97) to give *tert-*butyl 9-((4-(4-(5-(4-((benzyloxy)carbonyl)piperazin-1-yl)pyrazolo[1,5-*a*]pyrimidine-3-carboxamido)-3-(difluoromethyl)-1*H*-pyrazol-1-yl)piperidin-1-yl)methyl)-3-azaspiro[5.5]undecane-3-carboxylate.

LC-MS: (ESI, *m*/*z):* [M+H] ⁺ = 845.5.

### Step 6: preparation of benzyl 4-(3-((1-(1-((3-azaspiro[5.5]undecan-9-yl)methyl)piperidin-4-yl)-3-(difluoromethyl)-1H-pyrazol-4-yl)carbonyl)pyrazolo[1,5-a]pyrimidin-5-yl)piperazine-1-carboxylate

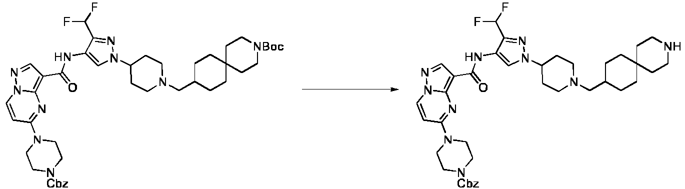

TFA (1 mL) was added to a solution of *tert-butyl* 9-((4-(4-(5-(4-((benzyloxy)carbonyl)piperazin-1-yl)pyrazolo [1,5-α]pyrimidine-3-carboxamido)-3-(difluoromethyl)-1*H*-pyrazol-1-yl)piperidin-1-yl)methyl)-3-azaspiro[5.5]undecane-3-carboxylate (0.29 g, 0.34 mmol) in DCM (3 mL), and the mixture was stirred at 25 °C for 1 h. The reaction liquid was directly concentrated under reduced pressure to give benzyl 4-(3-((1-(1-((3-azaspiro[5.5]undecan-9-yl)methyl)piperidin-4-yl)-3-(difluoromethyl)-1*H*-pyrazol-4-yl)carbonyl)pyrazolo[1,5-*a*] pyrimidin-5-yl)piperazine-1-carboxylate.

LC-MS: (ESI, *m*/*z):* [M+H] ⁺ = 745.4.

### Step 7: preparation of benzyl 4-(3-((1-(1-((3-(4-chloro-3-(2,4-dioxotetrahydropyrimidin-1(2H)-yl)benzoyl)-3-azaspiro[5.5]undecan-9-yl)methyl)piperidin-4-yl)-3-(difluoromethyl)-1H-pyrazol-4-yl)carbonyl)pyrazolo[1,5-α]pyrimidin-5-yl)piperazine-1-carboxylate

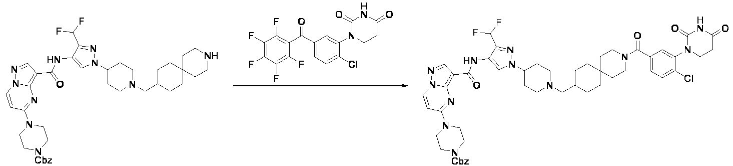

DIEA (0.1 mL, 0.6 mmol) was added to a solution of benzyl 4-(3-((1-(1-((3-azaspiro[5.5]undecan-9-yl)methyl)piperidin-4-yl)-3-(difluoromethyl)-1*H-*pyrazol-4-yl)carbonyl)pyrazolo[1,5-*a*]pyrimidin-5-yl)piperazine-1-carboxylate (240 mg, 0.32 mmol) and pentafluorophenyl 4-chloro-3-(2,4-dioxotetrahydropyrimidin-1(2*H*)-yl)benzoate (144 mg, 0.33 mmol) in DMSO (5 mL). The reaction liquid was then stirred at 25 °C for 16 h. Water (30 mL) was added to the reaction liquid, and the mixture was extracted with EA (50 mL × 3). The organic phase was washed with saturated brine, dried over anhydrous sodium sulfate, filtered, and concentrated under reduced pressure. The resulting crude product was purified by flash column chromatography (MeOH:DCM = 0-3:97) to give benzyl 4-(3-((1-(1-((3-(4-chloro-3-(2,4-dioxotetrahydropyrimidin-1(2*H*)-yl)benzoyl)-3-azaspiro [5.5]undecan-9-yl)methyl)piperidin-4-yl)-3-(difluoromethyl)-1*H*-pyrazol-4-yl)carbonyl) pyrazolo[1,5-*a*]pyrimidin-5-yl)piperazine-1-carboxylate.

LC-MS: (ESI, *m*/*z):* [M+H] ⁺ = 995.0.

### Step 8: preparation of N-(1-(1-((3-(4-chloro-3-(2,4-dioxotetrahydropyrimidin-1(2H)-yl)benzoyl)-3-azaspiro[5.5]undecan-9-yl)methyl)piperidin-4-yl)-3-(difluoromethyl)-1H-pyrazol-4-yl)-5-(piperazin-1-yl)pyrazolo[1,5-a]pyrimidine-3-carboxamide

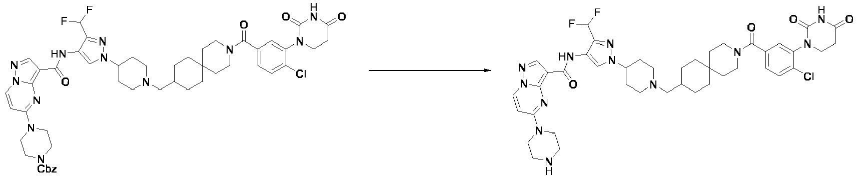

Iodotrimethylsilane (0.85 mL, 0.6 mmol) was added to a solution of benzyl 4-(3-((1-(1-((3-(4-chloro-3-(2,4-dioxotetrahydropyrimidin-1(2*H*)-yl)benzoyl)-3-azaspiro[5.5]undecan-9-yl)methyl)piperidin-4-yl)-3-(difluoromethyl)-1*H*-pyrazol-4-yl)carbonyl)pyrazolo[1,5-*a*]pyrimidin-5-yl)piperazine-1-carboxylate (290 mg, 0.29 mmol) in DCM (15 mL) at 0 °C, and then the reaction liquid was stirred at 25 °C for 16 h. The reaction liquid was poured into water, and the mixture was extracted with DCM (20 mL × 3), dried over anhydrous Na₂SO₄, filtered, and concentrated under reduced pressure. The resulting crude product was purified by Prep-HPLC to give *N*-(1-(1-((3-(4-chloro-3-(2,4-dioxotetrahydropyrimidin-1(2*H*)-yl)benzoyl)-3-azaspiro[5.5]undecan-9-yl)methyl)piperidin-4-yl)-3-(difluoromethyl)-1*H*-pyrazol-4-yl)-5-(piperazin-1-yl)pyrazolo [1,5-*a*] pyrimidine-3-carboxamide.

LC-MS: (ESI, *m*/*z):* [M+H] ⁺ = 861.1.

¹H NMR (400 MHz, MeOD) δ 8.62 (d, J = 7.9 Hz, 1H), 8.43 (brs, 2.67H, FA), 8.35 (s, 1H), 7.64 (d, J = 8.3 Hz, 1H), 7.54 (d, J = 1.6 Hz, 1H), 7.43 (d, J = 8.2 Hz, 1H), 7.08 - 6.77 (m, 2H), 4.57-4.43 (m, 1H), 4.15-4.03 (m, 4H), 3.79 (t, J = 6.4 Hz, 2H), 3.75-3.65 (m, 2H), 3.63-3.48 (m, 2H), 3.47-3.37 (m, 2H), 3.37-3.30 (m, 2H), 3.05-2.95 (m, 2H), 2.95-2.77 (m, 4H), 2.43-2.27 (m, 4H), 1.92-1.75 (m, 3H), 1.74-1.65 (m, 3H), 1.65-1.45 (m, 2H), 1.39-1.12 (m, 5H).

Example 6: 5-(8-oxa-3-azabicyclo[3.2.1]octan-3-yl)-N-(3-(difluoromethyl)-1-(1-((3-(3-(2,4-dioxotetrahydropyrimidin-1(2*H*)-yl)-4-methoxybenzoyl)-3-azaspiro[5.5]undecan-9-yl)methyl)piperidin-4-yl)-1*H-*pyrazol-4-yl)pyrazolo[1,5-*a*]pyrimidine-3-carboxamide

### Step 1: preparation of ethyl 5-(8-oxa-3-azabicyclo[3.2.1]octan-3-yl)pyrazolo[1,5-a]pyrimidine-3-carboxylate

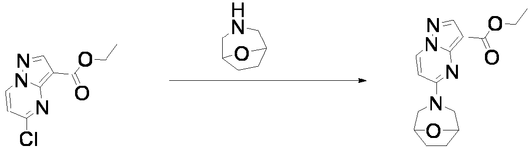

8-Oxa-3-azabicyclo[3.2.1]octane (1.3 g, 8.9 mmol) and DIEA (3.4 g, 26 mmol) were added to a solution of ethyl 5-chloropyrazolo[1,5-*a*]pyrimidine-3-carboxylate (2.0 g, 8.9 mmol) in ACN (20 mL), and the reaction liquid was heated to 60 °C and reacted for 2 h. Water (200 mL) was added to quench the reaction, and the mixture was extracted with EA (200 mL × 2). The organic phase was dried over anhydrous Na₂SO₄, filtered, and concentrated under reduced pressure. The resulting crude product was purified by column chromatography (PE:EA = 1-5:1) to give ethyl 5-(8-oxa-3-azabicyclo[3.2.1]octan-3-yl)pyrazolo[1,5-*a*]pyrimidine-3-carboxylate.

LC-MS: (ESI, *m*/*z):* [M+H] ⁺ = 303.2.

### Step 2: preparation of 5-(8-oxa-3-azabicyclo[3.2.1]octan-3-yl)pyrazolo[1,5-a]pyrimidine-3-carboxylic acid

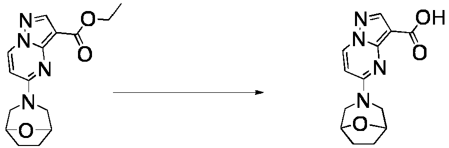

LiOH (1.4 g, 34 mmol) was added to a mixed solution of ethyl 5-(8-oxa-3-azabicyclo[3.2.1]octan-3-yl)pyrazolo[1,5-*a*]pyrimidine-3-carboxylate (2.6 g, 8.6 mmol) in H₂O/MeOH (3 mL/26 mL), and then the reaction liquid was heated to 60 °C and reacted for 12 h. The reaction liquid was filtered through celite, and the filtrate was concentrated under reduced pressure. The resulting crude product was purified by Prep-HPLC (acetonitrile/0.05% aqueous NH₄OH solution, 5%-95%) to give 5-(8-oxa-3-azabicyclo[3.2.1]octan-3-yl)pyrazolo[1,5-*a*]pyrimidine-3-carboxylic acid.

LC-MS: (ESI, *m*/*z*): [M+H] ⁺ = 275.3.

### Step 3: preparation of tert-butyl 4-(4-(5-(8-oxa-3-azabicyclo[3.2.1]octan-3-yl)pyrazolo[1,5-a]pyrimidine-3-carboxamido)-3-(difluoromethyl)-1H-pyrazol-1-yl)piperidine-1-carboxylate

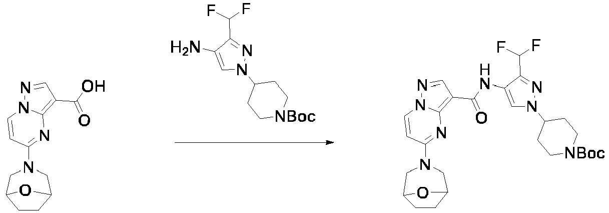

*tert*-Butyl 4-(4-amino-3-(difluoromethyl)-1*H*-pyrazol-1-yl)piperidine-1-carboxylate (316 mg, 1 mmol), HATU (456 mg, 1.2 mmol), and DIEA (387 mg, 3 mmol) were added to a solution of 5-(8-oxa-3-azabicyclo[3.2.1]octan-3-yl)pyrazolo[1,5-*a*]pyrimidine-3-carboxylic acid (274 mg, 1 mmol) in DMF (5 mL), and then the reaction liquid was reacted at 25 °C for 12 h. The reaction liquid was poured into water and then the mixture was extracted with EA (50 mL × 2), and the organic phase was dried over anhydrous Na₂SO₄, filtered, and concentrated under reduced pressure. The resulting crude product was purified by column chromatography (MeOH:DCM = 0-1:9) to give *tert*-butyl 4-(4-(5-(8-oxa-3-azabicyclo[3.2.1]octan-3-yl)pyrazolo[1,5-*a*]pyrimidine-3-carboxamido)-3-(difluoromethyl)-1*H*-pyrazol-1-yl)piperidine-1-carboxylate. LC-MS: (ESI, *m*/*z*): [M+H] ⁺ = 573.4.

### Step 4: preparation of 5-(8-oxa-3-azabicyclo[3.2.1]octan-3-yl)-N-(3-(difluoromethyl)-1-(piperidin-4-yl)-1H-pyrazol-4-yl)pyrazolo[1,5-a]pyrimidine-3-carboxamide

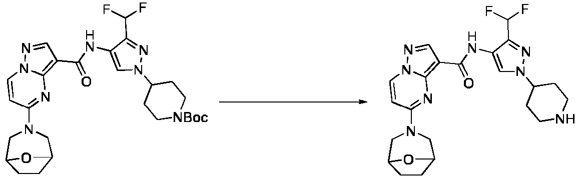

A solution of *tert*-butyl 4-(4-(5-(8-oxa-3-azabicyclo[3.2.1]octan-3-yl)pyrazolo[1,5-*a*]pyrimidine-3-carboxamido)-3-(difluoromethyl)-1*H*-pyrazol-1-yl)piperidine-1-carboxylate (240 mg, 0.42 mmol) in HCl/1,4-dioxane (5 mL) was stirred at room temperature for 1 h. The reaction liquid was directly concentrated under reduced pressure to give 5-(8-oxa-3-azabicyclo[3.2.1]octan-3-yl)-N (3-(difluoromethyl)-1-(piperidin-4-yl)-1*H*-pyrazol-4-yl)pyrazolo[1,5-*a*]pyrimidine-3-carboxamide.

LC-MS: (ESI, *m*/*z):* [M+H] ⁺ = 473.4.

### Step 5: preparation of tert-butyl 9-((4-(4-(5-(8-oxa-3-azabicyclo[3.2.1]octan-3-yl)pyrazolo[1,5-a]pyrimidine-3-carboxatnido)-3-(difluoromethyl)-1H-pyrazol-1-yl)piperidin-1-yl)methyl)-3-azaspiro[5.5]undecane-3-carboxylate

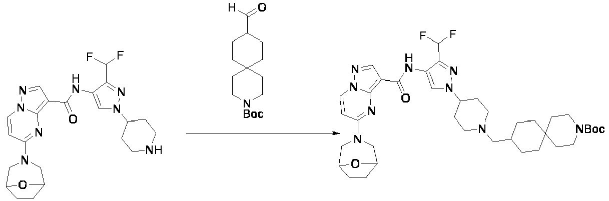

*tert-Butyl* 9-formyl-3-azaspiro[5.5]undecane-3-carboxylate (77 mg, 0.27 mmol) and STAB (175 mg, 0.8 mmol) were added to a solution of 5-(8-oxa-3-azabicyclo[3.2.1]octan-3-yl)-*N*-(3-(difluoromethyl)-1-(piperidin-4-yl)-1*H*-pyrazol-4-yl)pyrazolo[1,5-*a*]pyrimidine-3-carboxamide (130 mg, 0.27 mmol) in THF (10 mL). The reaction liquid was then stirred at 25 °C for 12 h. The reaction liquid was filtered through celite, and the filtrate was concentrated under reduced pressure. The resulting crude product was purified by column chromatography (MeOH:DCM = 0-1:9) to give *tert*-butyl 9-((4-(4-(5-(8-oxa-3-azabicyclo[3.2.1]octan-3-yl)pyrazolo [1,5-*a*]pyrimidine-3-carboxamido)-3-(difluoromethyl)-1*H*-pyrazol-1-yl)piperidin-1-yl)methyl)-3-azaspiro[5.5]undecane-3-carboxylate.

LC-MS: (ESI, *m*/*z):* [M+H] ⁺ = 738.3.

### Step 6: preparation of N (1-(1-((3-azaspiro[5.5]undecan-9-yl)methyl)piperidin-4-yl)-3-(difluoromethyl)-1H-pyrazol-4-yl)-5-(8-oxa-3-azabicyclo[3.2.1]octan-3-yl)pyrazolo[1,5-a]pyrimidine-3-carboxamide

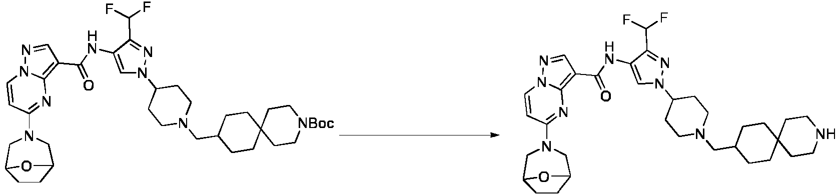

A solution of *tert*-butyl 9-((4-(4-(5-(8-oxa-3-azabicyclo[3.2.1]octan-3-yl)pyrazolo[1,5-*a*]pyrimidine-3-carboxamido)-3-(difluoromethyl)-1*H*-pyrazol-1-yl)piperidin-1-yl)methyl)-3-azaspiro[5.5]undecane-3-carboxylate (100 mg, 0.14 mmol) in HCl/1,4-dioxane (4 mL) was stirred at 25 °C for 1 h. Then the reaction liquid was directly concentrated under reduced pressure to give *N*-(1-(1-((3-azaspiro[5.5]undecan-9-yl)methyl)piperidin-4-yl)-3-(difluoromethyl)-1*H-*pyrazol-4-yl)-5-(8-oxa-3-azabicyclo[3.2.1]octan-3-yl)pyrazolo[1,5-*a*]pyrimidine-3-carboxamide.

LC-MS: (ESI, *m*/*z):* [M+H] ⁺ = 638.3.

### Step 7: preparation of 5-(8-oxa-3-azabicyclo[3.2.1]octan-3-yl)-N-(3-(difluoromethyl)-1-(1-((3-(3-(2,4-dioxotetrahydropyrimidin-1(2H)-yl)-4-methoxybenzoyl)-3-azaspiro[5.5]undecan-9-yl)methyl)piperidin-4-yl)-1H-pyrazol-4-yl)pyrazolo[1,5-a]pyrimidine-3-carboxamide

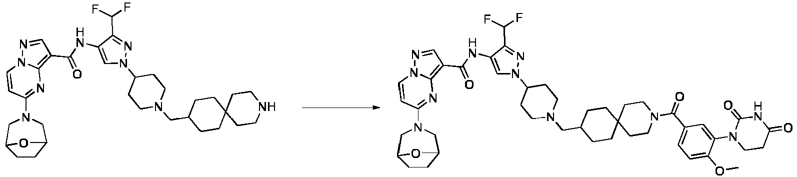

Pentafluorophenyl 3-(2,4-dioxotetrahydropyrimidin-1(2*H*)-yl)-4-methoxybenzoate (123 mg, 0.28 mmol) and DIEA (93 mg, 0.72 mmol) were added to a solution of *N*-(1-(1-((3-azaspiro[5.5]undecan-9-yl)methyl)piperidin-4-yl)-3-(difluoromethyl)-1*H*-pyrazol-4-yl)-5-(8-oxa-3-azabicyclo[3.2.1]octan-3-yl)pyrazolo[1,5-*a*]pyrimidine-3-carboxamide (150.0 mg, 0.24 mmol) in DMSO (2 mL), and then the reaction liquid was stirred at room temperature for 12 h. The reaction liquid was poured into water (20 mL), and the mixture was extracted with EA (30 mL × 2). The organic phase was dried over anhydrous Na₂SO₄ and concentrated under reduced pressure. The resulting crude product was purified by Prep-HPLC (acetonitrile/0.05% aqueous NH₄HCO₃ solution, 5%-95%) to give 5-(8-oxa-3-azabicyclo[3.2.1]octan-3-yl)-*N-*(3-(difluoromethyl)-1-(1-((3-(3-(2,4-dioxotetrahydropyrimidin-1(2*H*)-yl)-4-methoxybenzoyl)-3-azaspiro[5.5] undecan-9-yl)methyl)piperidin-4-yl)-1*H*-pyrazol-4-yl)pyrazolo[1,5-*a*]pyrimidine-3-carboxatnide.

LC-MS: (ESI, *m*/*z):* [M+H] ⁺ = 884.5.

¹H NMR (400 MHz, DMSO-*d*₆) δ 10.33 (s, 1H), 9.41 (s, 1H), 8.81 (d, *J =* 7.8 Hz, 1H), 8.39 (s, 1H), 8.28 (d, *J* = 1.3 Hz, 1H), 7.37 (d, *J* = 8.4 Hz, 1H), 7.32 (s, 1H), 7.27 - 6.95 (m, 2H), 6.82 (d, *J =* 7.9 Hz, 1H), 4.50-4.37 (m, 2H), 4.30-4.00 (m, 2H), 3.84 (s, 3H), 3.68-3.34 (m, 6H), 3.28-3.11 (m, 2H), 2.98-2.78 (m, 2H), 2.73-2.60 (m, 2H), 2.22 - 1.63 (m, 14H), 1.61 - 1.17 (m, 8H), 1.18-0.93 (m, 4H).

### Example 7: 5-((1R,4R)-2-oxa-5-azabicyclo[2.2.1]heptan-5-yl)-N-(1-(1-(4-(1-(4-chloro-3-(2,4-dioxotetrahydropyrimidin-1(2H)-yl)benzoyl)piperidin-4-yl)butyl)piperidin-4-yl)-3-(difluoromethyl)-1H-pyrazol-4-yl)pyrazolo[1,5-a]pyrimidine-3-carboxamide

### Step 1: preparation of tert-butyl 4-(4-(4-(4-(5-((1R,4R)-2-oxa-5-azabicyclo[2.2.1]heptan-5-yl)pyrazolo[1,5-a]pyrimidine-3-carboxamido)-3-(difluoromethyl)-1H-pyrazol-1-yl)piperidin-1-yl)butyl)piperidine-1-carboxylate

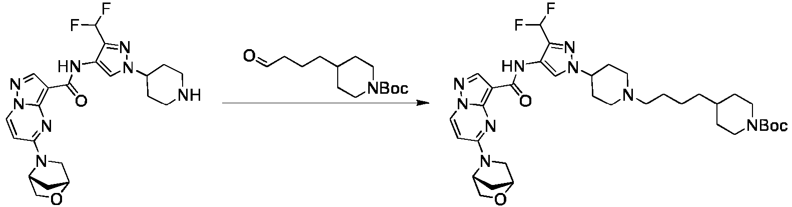

*tert*-Butyl 4-(4-oxobutyl)piperidine-1-carboxylate (100 mg, 0.36 mmol) and STAB (170 mg, 0.71 mmol) were added to a solution of 5-((1*R*,4*R*)-2-oxa-5-azabicyclo[2.2.1]heptan-5-yl)-*N*-(3-(difluoromethyl)-1-(piperidin-4-yl)-1*H*-pyrazol-4-yl)pyrazolo[1,5-*a*]pyrimidine-3-carboxamide (110 mg, 0.24 mmol) in THF (5 mL). The reaction liquid was stirred at 25 °C for 2 h. Water (30 mL) was added to quench the reaction, and the mixture was extracted with DCM (30 mL × 3). The organic phase was dried over anhydrous Na₂SO₄, filtered, and concentrated under reduced pressure. The resulting crude product was purified by flash column chromatography (MeOH:DCM = 0-3:97) to give *tert*-butyl 4-(4-(4-(4-(5-((1*R*,4*R*)-2-oxa-5-azabicyclo[2.2.1]heptan-5-yl)pyrazolo[1,5-*a*]pyrimidine-3-carboxamido)-3-(difluoromethyl)-1*H*-pyrazol-1-yl)piperidin-1-yl)butyl)piperidine-1-carboxylate.

LC-MS: (ESI, *m*/*z*): [M+H] ⁺ = 698.0.

### Step 2: preparation of 5-((1R,4R)-2-oxa-5-azabicyclo[2.2.1]heptan-5-yl)-N-(3-(difluoromethyl)-1-(1-(4-(piperidin-4-yl)butyl)piperidin-4-yl)-1H-pyrazol-4-yl)pyrazolo[1,5-a]pyrimidine-3-carboxamide

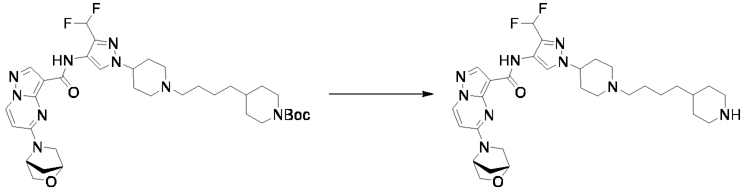

TFA (1 mL) was added to a solution of *tert*-butyl 4-(4-(4-(4-(5-((1*R*,4*R*)-2-oxa-5-azabicyclo[2.2.1]heptan-5-yl)pyrazolo [1,5-*a*]pyrimidine-3-carboxamido)-3-(difluoromethyl)-1*H*-pyrazol-1-yl)piperidin-1-yl)butyl) piperidine-1-carboxylate (0.2 g, 0.26 mmol) in DCM (5 mL). The reaction liquid was stirred at 25 °C for 2 h. The reaction liquid was directly concentrated under reduced pressure to give 5-((1*R*,4*R*)-2-oxa-5-azabicyclo[2.2.1]heptan-5-yl)-*N-*(3-(difluoromethyl)-1-(1-(4-(piperidin-4-yl)butyl)piperidin-4-yl)-1*H-*pyrazol-4-yl)pyrazolo[1,5-*a*]pyrimidine-3-carboxamide.

LC-MS: (ESI, *m*/*z):* [M+H] ⁺ = 598.1.

### Step 3: preparation of 5-((1R,4R)-2-oxa-5-azabicyclo[2.2.1]heptan-5-yl)-N-(1-(1-(4-(1-(4-chloro-3-(2,4-dioxotetrahydropyrimidin-1(2H)-yl)benzoyl)piperidin-4-yl)butyl)piperidin-4-yl)-3-(difluoromethyl)-1H-pyrazol-4-yl)pyrazolo[1,5-a]pyrimidine-3-carboxamide

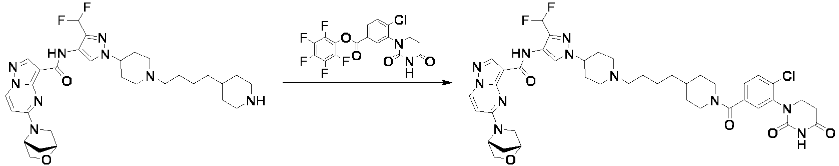

DIEA (0.14 mL, 0.78 mmol) was added to a solution of 5-((1*R*,4*R*)-2-oxa-5-azabicyclo[2.2.1]heptan-5-yl)-*N-*(3-(difluoromethyl)-1-(1-(4-(piperidin-4-yl)butyl)piperidin-4-yl)-1*H*-pyrazol-4-yl)pyrazolo [1,5-*a*]pyrimidine-3-carboxamide (160 mg, 0.26 mmol) and pentafluorophenyl 4-chloro-3-(2,4-dioxotetrahydropyrimidin-1(2*H*)-yl)benzoate (130 mg, 0.26 mmol) in DMSO (4 mL). The reaction liquid was stirred at 25 °C for 2 h. Water (10 mL) was added to quench the reaction, and the mixture was extracted with DCM (20 mL × 3), dried over anhydrous Na₂SO₄, filtered, and concentrated under reduced pressure. The resulting crude product was purified by Prep-HPLC to give 5-((1*R*,4*R*)-2-oxa-5-azabicyclo[2.2.1]heptan-5-yl)-*N-*(1-(1-(4-(1-(4-chloro-3-(2,4-dioxotetrahydropyrimidin-1(2*H*)-yl)benzoyl)piperidin-4-yl)butyl)piperidin-4-yl)-3-(difluoromethyl)-1*H-*pyrazol-4-yl)pyrazolo[1,5-*a*]pyrimidine-3-carboxamide.

LC-MS: (ESI, *m*/*z):* [M+H] ⁺ = 848.3.

¹H NMR (400 MHz, DMSO-*d*₆) δ 10.49 (s, 1H), 9.50 (d, J = 5.3 Hz, 1H), 8.78 (d, J = 7.7 Hz, 1H), 8.40 (s, 1H), 8.25 (d, J = 5.0 Hz, 1H), 7.63 (d, J = 8.3 Hz, 1H), 7.54 (s, 1H), 7.38 (d, J = 8.1 Hz, 1H), 7.20-7.00 (m, 1H), 6.98-6.45 (m, 1H), 5.40-5.05 (m, 1H), 4.77 (d, J = 17.3 Hz, 1H), 4.54-4.34 (m, 1H), 4.28-4.12 (m, 1H), 3.83 - 3.72 (m, 3H), 3.67 - 3.57 (m, 3H), 3.45 (d, J = 9.6 Hz, 1H), 3.02-2.85 (m, 3H), 2.80-2.70 (m, 2H), 2.36-2.26 (m, 2H), 2.08 - 1.88 (m, 8H), 1.76 - 1.47 (m, 4H), 1.47-1.22 (m, 6H), 1.18-1.02 (m, 2H).

### Example 8: N-(1-((1R,4R)-4-((9-(4-chloro-3-(2,4-dioxotetrahydropyrimidin-1(2H)-yl)benzoyl)-3,9-diazaspiro [5.5]undecan-3-yl)methyl)cyclohexyl)-3-(difluoromethyl)-1H-pyrazol-4-yl)-5-morpholinopyrazolo[1,5-a]pyrimidine-3-carboxamide

### Step 1: preparation of tert-butyl 9-(((1R,4R)-4-(3-(difluoromethyl)-4-(5-morpholinopyrazolo[1,5-a]pyrimidine-3-carboxamido)-1H-pyrazol-1-yl)cyclohexyl)methyl)-3,9-diazaspiro[5.5]undecane-3-carboxylate

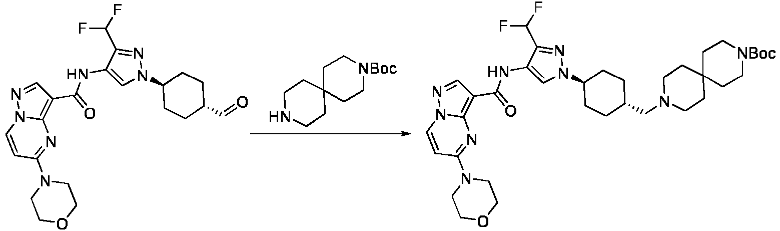

*N*-(3-(Difluoromethyl)-1-((1*R*,4*R*)-4-(formyl)cyclohexyl)-1*H*-pyrazol-4-yl)-5-morpholinopyrazolo[1,5-*a*]pyrimidine-3-carboxamide (50 mg, 0.10 mmol) and *tert*-butyl 3,9-diazaspiro[5.5]undecane-3-carboxylate (24 mg, 0.10 mmol) were dissolved in THF (3 mL). Sodium triacetoxyborohydride (67 mg, 0.32 mmol) was added to the reaction liquid with stirring, and the reaction liquid was stirred at room temperature for 2 h. Water (50 mL) was added to the reaction liquid, and the mixture was extracted with ethyl acetate (30 mL × 3). The organic phases were combined, dried over anhydrous sodium sulfate, and concentrated under reduced pressure. The crude product was purified by normal phase column chromatography to give *tert*-butyl 9-(((1R,4R)-4-(3-(difluoromethyl)-4-(5-morpholinopyrazolo[1,5-*a*]pyrimidine-3-carboxamido)-1*H*-pyrazol-1-yl)cyclohexyl)methyl)-3,9-diazaspiro[5.5]undecane-3-carboxylate.

LC-MS: (ESI, m/z): [M+H] ⁺ = 712.3.

### Step 2: preparation of N-(1-((1R,4R)-4-((3,9-diazaspiro[5.5]undecan-3-yl)methyl)cyclohexyl)-3-(difluoromethyl)-1H-pyrazol-4-yl)-5-morpholinopyrazolo[1,5-a]pyrimidine-3-carboxamide

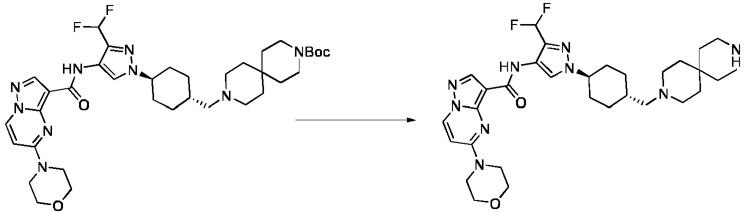

*tert*-Butyl 9-(((1*R*,4*R*)-4-(3-(difluoromethyl)-4-(5-morpholinopyrazolo[1,5-*a*]pyrimidine-3-carboxamido)-1*H-*pyrazol-1-yl)cyclohexyl)methyl)-3,9-diazaspiro[5.5]undecane-3-carboxylate (95 mg, 0.13 mmol) was dissolved in DCM (3 mL). TFA (1 mL) was added, and the reaction liquid was stirred at room temperature for 1 h. The reaction liquid was directly concentrated under reduced pressure to give *N*-(1-((1*R*,4*R*)-4-((3,9-diazaspiro[5.5]undecan-3-yl)methyl)cyclohexyl)-3-(difluoromethyl)-1*H*-pyrazol-4-yl)-5-morpholinopyrazolo[1,5-*a*]pyrimidine-3-carboxamide (crude), which was directly used in the next step.

LC-MS: (ESI, m/z): [M+H] ⁺ = 612.3.

### Step 3: preparation of N-(1-((1R,4R)-4-((9-(4-chloro-3-(2,4-dioxotetrahydropyrimidin-1(2H)-yl)benzoyl)-3,9-diazaspiro[5.5]undecan-3-yl)methyl)cyclohexyl)-3-(difluoromethyl)-1H-pyrazol-4-yl)-5-morpholinopyrazolo[1,5-a]pyrimidine-3-carboxamide

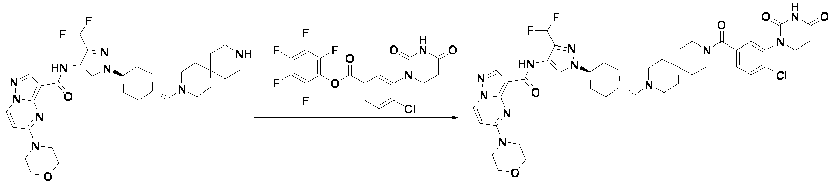

*N*-(1-((1*R*,4*R*)-4-((3,9-Diazaspiro[5.5]undecan-3-yl)methyl)cyclohexyl)-3-(difluoromethyl)-1*H*-pyrazol-4-yl)-5-morpholinopyrazolo[1,5-*a*]pyrimidine-3-carboxamide (130 mg, 0.13 mmol) and pentafluorophenyl 4-chloro-3-(2,4-dioxotetrahydropyrimidin-1(2*H*)-yl)benzoate (57 mg, 0.13 mmol) were dissolved in DMSO (5 mL). DIEA (84 mg, 0.65 mmol) was added to the reaction liquid with stirring, and the reaction liquid was stirred at room temperature for 1 h. After the reaction was completed, the reaction liquid was poured into water (50 mL), and the mixture was extracted with EA (30 mL × 3). The organic phases were combined, washed with saturated brine (80 mL), dried over anhydrous sodium sulfate, and filtered. The filtrate was concentrated under reduced pressure and separated and purified by prep-HPLC to give *N-*(1-((1*R*,4*R*)-4-((9-(4-chloro-3-(2,4-dioxotetrahydropyrimidin-1(2*H*)-yl)benzoyl)-3,9-diazaspiro[5.5]undecan-3-yl)methyl)cyclohexyl)-3-(difluoromethyl)-1*H-*pyrazol-4-yl)-5-morpholinopyrazolo[1,5-*a*]pyrimidine-3-carboxamide.

LC-MS: (ESI, m/z): [M+H] ⁺ = 862.3.

¹H NMR (400 MHz, DMSO-*d*₆) δ 10.50 (s, 1H), 9.39 (s, 1H), 8.82 (d, *J =* 6.3 Hz, 1H), 8.37 (s, 1H), 8.28 (s, 1H), 7.63 *(d, J* = 7.9 Hz, 1H), 7.55 (s, 1H), 7.39 (d, *J =* 7.9 Hz, 1H), 7.25-6.95 (m, 1H), 6.90 (d, *J* = 7.6 Hz, 1H), 4.27-4.07 (m, 1H), 3.90-3.50 (m, 12H), 3.40-3.30 (m, 2H), 2.79-2.70 (m, 2H), 2.43-2.23 (m, 4H), 2.20-1.95 (m, 4H), 1.93-1.83 (m, 2H), 1.82-1.30 (m, 11H), 1.09-0.94 (m, 2H).

### Example 9: N-(1-((1R,4R)-4-(((2-(1-(4-chloro-3-(2,4-dioxotetrahydropyrimidin-1(2H)-yl)benzoyl)piperidin-4-yl)ethyl)(methyl)amino)methyl)cyclohexyl)-3-(difluoromethyl)-1H-pyrazol-4-yl)-5-morpholinopyrazolo [1,5-a]pyrimidine-3-carboxamide

### Step 1: preparation of tert-butyl 4-(2-((((1R,4R)-4-(3-(difluoromethyl)-4-(5-morpholinopyrazolo[1,5-a]pyrimidine-3-carboxamido)-1H-pyrazol-1-yl)cyclohexyl)methyl)(methyl)amino)ethyl)piperidine-1-carboxylate

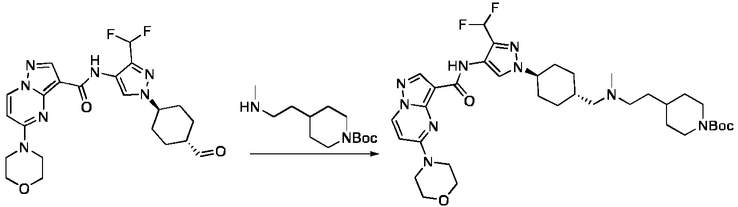

*N*-(3-(Difluoromethyl)-1-((1*R*,4*R*)-4-(formyl)cyclohexyl)-1*H*-pyrazol-4-yl)-5-morpholinopyrazolo[1,5-*a*]pyrimidine-3-carboxamide (110 mg, 0.23 mmol) was dissolved in THF (10 mL). *tert*-Butyl 4-(2-(methylamino)ethyl)piperidine-1-carboxylate (56 mg, 0.23 mmol) and STAB (146 mg, 0.69 mmol) were added, and the mixture was reacted at room temperature for 2 h. The mixture was diluted with water (50 mL), and the resulting mixture was extracted with ethyl acetate (50 mL × 3). The organic phase was washed with saturated brine (100 mL), dried over anhydrous sodium sulfate, filtered, and concentrated under reduced pressure. The crude product was purified by column chromatography (MeOH:DCM = 1:11) to give *tert*-butyl 4-(2-((((1*R*,4*R*)-4-(3-(difluoromethyl)-4-(5-morpholinopyrazolo[1,5-*a*]pyrimidine-3-carboxamido)-1*H-*pyrazol-1-yl)cyclohexyl)methyl)(methyl)amino)ethyl)piperidine-1-carboxylate.

LC-MS: (ESI, m/z): [M+H] ⁺ = 700.4.

### Step 2: preparation of N-(3-(difluoromethyl)-1-((1R,4R)-4-((methyl(2-(piperidin-4-yl)ethyl)atnino)methyl) cyclohexyl)-1H-pyrazol-4-yl)-5-morpholinopyrazolo[1,5-a]pyrimidine-3-carboxamide

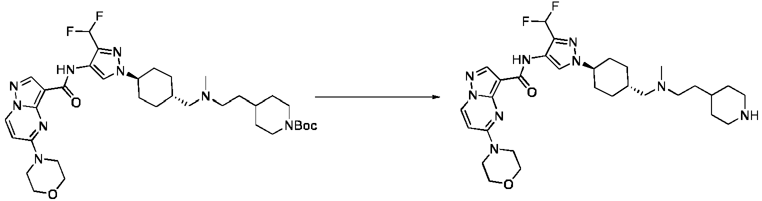

*tert*-Butyl 4-(2-((((1*R*,4*R*)-4-(3-(difluoromethyl)-4-(5-morpholinopyrazolo[1,5-*a*]pyrimidine-3-carboxamido)-1H-pyrazol-1-yl)cyclohexyl)methyl)(methyl)amino)ethyl)piperidine-1-carboxylate (95 mg, 0.14 mmol) was dissolved in DCM (3 mL). TFA (1 mL) was added, and the mixture was reacted at room temperature for 1 h. The reaction liquid was directly concentrated under reduced pressure to give *N*-(3-(difluoromethyl)-1-((1*R*,4*R*)-4-((methyl(2-(piperidin-4-yl)ethyl)amino)methyl)cyclohexyl)-1*H*-pyrazol-4-yl)-5-morpholinopyrazolo[1,5-*a*]pyrimidine-3-carboxamide, which was directly used in the next step.

LC-MS: (ESI, m/z): [M+H] ⁺ = 600.3.

### Step 3: preparation of N-(1-((1R,4R)-4-(((2-(1-(4-chloro-3-(2,4-dioxotetrahydropyrimidin-1(2H)-yl)benzoyl) piperidin-4-yl)ethyl)(methyl)amino)methyl)cyclohexyl)-3-(difluoromethyl)-1H-pyrazol-4-yl)-5-morpholinopyrazolo[1,5-a]pyrimidine-3-carboxamide

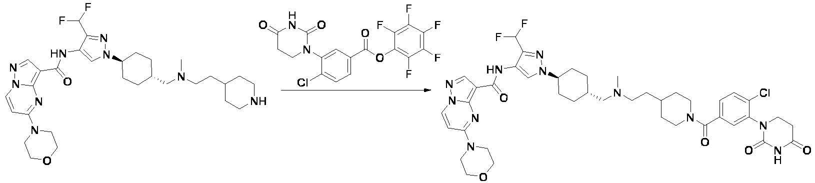

*N-*(3-(Difluoromethyl)-1-((1*R*,4*R*)-4-((methyl(2-(piperidin-4-yl)ethyl)amino)methyl)cyclohexyl)-1*H*-pyrazol-4-yl)-5-morpholinopyrazolo[1,5-*a*]pyrimidine-3-carboxamide (95 mg, 0.14 mmol) was dissolved in DMSO (5 mL). DIEA (77 mg, 0.60 mmol) and pentafluorophenyl 4-chloro-3-(2,4-dioxotetrahydropyrimidin-1(2*H*)-yl)benzoate (61 mg, 0.14 mmol) were added, and the mixture was reacted at room temperature for 2 h. Water (50 mL) was added, and the mixture was stirred for 5 min and extracted with ethyl acetate (50 mL × 3). The organic phase was washed with saturated brine (100 mL), dried over anhydrous sodium sulfate, filtered, and concentrated under reduced pressure. The crude product was purified by preparative reversed-phase chromatography to give *N-(1-((1R,4R)-4-(((2-(1-(4-chloro-3-(2,4-dioxotetrahydropyrimidin-1(2H)-*yl)benzoyl)piperidin-4-yl)ethyl)(methyl)amino)methyl)cyclohexyl)-3-(difluoromethyl)-1*H*-pyrazol-4-yl)-5-morpholinopyrazolo[1,5-*a*]pyrimidine-3-carboxamide.

LC-MS: (ESI, m/z): [M+H] ⁺ = 850.4.

¹H NMR (400 MHz, DMSO-*d*₆) δ 10.50 (s, 1H), 9.39 (s, 1H), 8.83 (d, *J =* 7.9 Hz, 1H), 8.38 (s, 1H), 8.29 (s, 1H), 7.63 (d, *J=* 8.2 Hz, 1H), 7.54 (d, *J=* 1.8 Hz, 1H), 7.38 (dd, *J =* 8.2, 1.8 Hz, 1H), 7.24-6.97 (m, 1H), 6.91 (d, *J=* 8.0 Hz, 1H), 4.49 - 4.38 (m, 1H), 4.23-4.13 (m, 1H), 3.83 - 3.76 (m, 4H), 3.75 - 3.70 (m, 4H), 3.66 - 3.52 (m, 2H), 3.10 - 2.95 (m, 1H), 2.84-2.67 (m, 3H), 2.29 (t, *J=* 7.0 Hz, 2H), 2.14 - 2.01 (m, 7H), 1.93-1.84 (m, 2H), 1.82 - 1.46 (m, 7H), 1.41 - 1.32 (m, 2H), 1.17 - 0.95 (m, 4H).

### Example 10: 5-(8-oxa-3-azabicyclo[3.2.1]octan-3-yl)-N-(1-((1R,4R)-4-(((2-(1-(4-chloro-3-(2,4-dioxotetrahydropyrimidin-1(2H)-yl)benzoyl)piperidin-4-yl)ethyl)(methyl)amino)methyl)cyclohexyl)-3-(difluoromethyl)-1H-pyrazol-4-yl)pyrazolo[1,5-a]pyrimidine-3-carboxamide

### Step 1: preparation of 5-(8-oxa-3-azabicyclo[3.2.1]octan-3-y1)-N-(3-(difluoromethyl)-1-((1R,4R)-4-(hydroxymethyl)cyclohexyl)-1H-pyrazol-4-yl)pyrazolo[1,5-a]pyrimidine-3-carboxamide

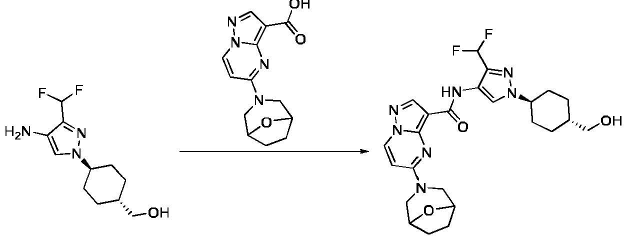

((1*R*,4*R*)-4-(4-Amino-3-(difluoromethyl)-1*H*-pyrazol-1-yl)cyclohexyl)methanol (410 mg, 1.67 mmol) was dissolved in DMF (10 mL). 5-(8-Oxa-3-azaspirobicyclo[3.2.1]octan-3-yl)pyrazolo[1,5-*a*]pyrimidine-3-carboxylic acid (459 mg, 1.67 mmol), HATU (825 mg, 2.17 mmol), and DIEA (646 mg, 5.01 mmol) were added, and the mixture was reacted at room temperature for 2 h. Water (50 mL) was added, and the mixture was stirred for 5 min and extracted with ethyl acetate (50 mL × 3). The organic phase was washed with saturated brine (100 mL), dried over anhydrous sodium sulfate, filtered, and concentrated under reduced pressure. The crude product was purified by column chromatography to give 5-(8-oxa-3-azabicyclo[3.2.1]octan-3-yl)-*N-*(3-(difluoromethyl)-1-((1*R*,4*R*)-4-(hydroxymethyl)cyclohexyl)-1*H*-pyrazol-4-yl)pyrazolo[1,5-*a*]pyrimidine-3-carboxamide.

LC-MS: (ESI, m/z): [M+H] ⁺ = 502.3.

### Step 2: preparation of 5-(8-oxa-3-azabicyclo[3.2.1]octan-3-yl)-N-(3-(difluoromethyl)-1-((1R,4R)-4-formylcyclohexyl)-1H-pyrazol-4-yl)pyrazolo[1,5-a]pyrimidine-3-carboxamide

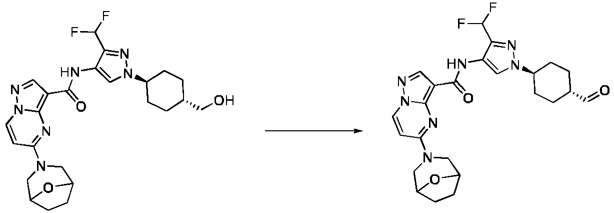

5-(8-Oxa-3-azabicyclo[3.2.1]octan-3-yl)-*N*-(3-(difluoromethyl)-1-((1*R*,4*R*)-4-(hydroxymethyl)cyclohexyl)-1*H*-pyrazol-4-yl)pyrazolo[1,5-*a*]pyrimidine-3-carboxamide (290 mg, 0.58 mmol) was dissolved in ACN (10 mL). IBX (324 mg, 1.16 mmol) was added, and the mixture was reacted at 85 °C for 1.5 h and concentrated under reduced pressure to remove ACN. Water (30 mL) was added, and the mixture was extracted with ethyl acetate (30 mL × 3). The organic phase was washed with saturated brine (50 mL), dried over anhydrous sodium sulfate, filtered, and concentrated under reduced pressure. The crude product was purified by column chromatography to give 5-(8-oxa-3-azabicyclo[3.2.1]octan-3-yl)-*N*-(3-(difluoromethyl)-1-((1*R*,4*R*)-4-formylcyclohexyl)-1*H*-pyrazol-4-yl)pyrazolo[1,5-*a*]pyrimidine-3-carboxamide.

LC-MS: (ESI, m/z): [M+H] ⁺ = 500.3.

### Step 3: preparation of tert-butyl 4-(2-((((1R,4R)-4-(4-(5-(8-oxa-3-azabicyclo[3.2.1]octan-3-yl)pyrazolo[1,5-a]pyrimidine-3-carboxamido)-3-(difluoromethyl)-1H-pyrazol-1-yl)cyclohexyl)methyl)(methyl)amino) ethyl)piperidine-1-carboxylate

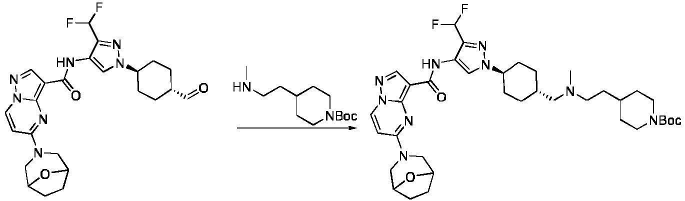

5-(8-Oxa-3-azabicyclo[3.2.1]octan-3-yl)-*N*-(3-(difluoromethyl)-1-((1*R*,4*R*)-4-formylcyclohexyl)-1*H*-pyrazol-4-yl)pyrazolo[1,5-*a*]pyrimidine-3-carboxamide (100 mg, 0.20 mmol) was dissolved in THF (10 mL). *tert*-Butyl 4-(2-(methylamino)ethyl)piperidine-1-carboxylate (48 mg, 0.20 mmol) and STAB (127 mg, 0.6 mmol) were added, and the mixture was reacted at room temperature for 2 h. The mixture was diluted with water (50 mL), and the resulting mixture was extracted with ethyl acetate (50 mL × 3). The organic phase was washed with saturated brine (100 mL), dried over anhydrous sodium sulfate, filtered, and concentrated under reduced pressure. The crude product was purified by column chromatography (MeOH:DCM = 1:11) to give *tert*-butyl 4-(2-((((1*R*,4*R*)-4-(4-(5-(8-oxa-3-azabicyclo[3.2.1]octan-3-yl)pyrazolo[1,5-*a*]pyrimidine-3-carboxamido)-3-(difluoromethyl)-1*H*-pyrazol-1-yl)cyclohexyl)methyl)(methyl)amino)ethyl)piperidine-1-carboxylate. LC-MS: (ESI, m/z): [M+H] ⁺ = 726.4.

### Step 4: preparation of 5-(8-oxa-3-azabicyclo[3.2.1]octan-3-yl)-N-(3-(difluoromethyl)-1-((1R,4R)-4-((methyl(2-(piperidin-4-yl)ethyl)amino)methyl)cyclohexyl)-1H-pyrazol-4-yl)pyrazolo[1,5-a]pyrimidine-3-carboxamide

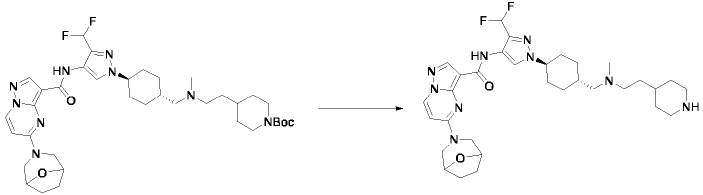

*tert-Butyl* 4-(2-((((1*R*,4*R*)-4-(4-(5-(8-oxa-3-azabicyclo[3.2.1]octan-3-yl)pyrazolo[1,5-*a*]pyrimidine-3-carboxamido)-3-(difluoromethyl)-1*H*-pyrazol-1-yl)cyclohexyl)methyl)(methyl)amino)ethyl)piperidine-1-carboxylate (110 mg, 0.15 mmol) was dissolved in DCM (3 mL). TFA (1 mL) was added, and the mixture was reacted at room temperature for 1 h and concentrated under reduced pressure to give 5-(8-oxa-3-azabicyclo[3.2.1]octan-3-yl)-*N*-(3-(difluoromethyl)-1-((1*R*,4*R*)-4-((methyl(2-(piperidin-4-yl)ethyl)amino) methyl)cyclohexyl)-1*H*-pyrazol-4-yl)pyrazolo[1,5-*a*]pyrimidine-3-carboxamide, which was directly used in the next step.

LC-MS: (ESI, m/z): [M+H] ⁺ = 626.3.

### Step 5: preparation of 5-(8-oxa-3-azabicyclo[3.2.1]octan-3-yl)-N-(1-((1R,4R)-4-(((2-(1-(4-chloro-3-(2,4-dioxotetrahydropyrimidin-1(2H)-yl)benzoyl)piperidin-4-yl)ethyl)(methyl)amino)methyl)cyclohexyl)-3-(difluoromethyl)-1H-pyrazol-4-yl)pyrazolo[1,5-a]pyrimidine-3-carboxamide

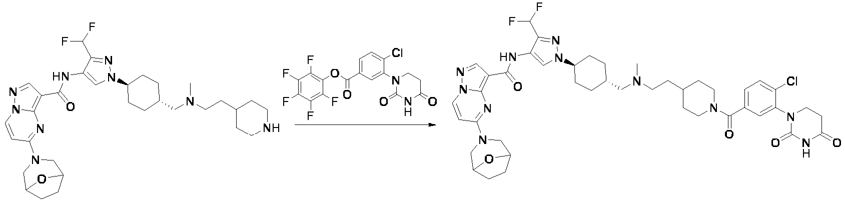

5-(8-Oxa-3-azabicyclo[3.2.1]octan-3-yl)-*N-*(3-(difluoromethyl)-1-((1*R*,4*R*)-4-((methyl(2-(piperidin-4-yl)ethyl)amino)methyl)cyclohexyl)-1*H*-pyrazol-4-yl)pyrazolo[1,5-*a*]pyrimidine-3-carboxamide (110 mg, 0.15 mmol) was dissolved in DMSO (5 mL). DIEA (97 mg, 0.75 mmol) and pentafluorophenyl 4-chloro-3-(2,4-dioxotetrahydropyrimidin-1(2*H*)-yl)benzoate (65 mg, 0.15 mmol) were added, and the mixture was reacted at room temperature for 2 h. Water (50 mL) was added, and the mixture was stirred for 5 min and extracted with ethyl acetate (50 mL × 3). The organic phase was washed with saturated brine (100 mL), dried over anhydrous sodium sulfate, filtered, and concentrated under reduced pressure. The crude product was purified by preparative reversed-phase chromatography to give 5-(8-oxa-3-azabicyclo[3.2.1]octan-3-yl)-*N*-(1-((1*R*,4*R*)-4-(((2-(1-(4-chloro-3-(2,4-dioxotetrahydropyrimidin-1(2*H*)-yl)benzoyl)piperidin-4-yl)ethyl)(methyl)amino) methyl)cyclohexyl)-3-(difluoromethyl)-1*H*-pyrazol-4-yl)pyrazolo[1,5-*a*]pyrimidine-3-carboxamide. LC-MS: (ESI, m/z): [M+H] ⁺ = 876.2.

¹H NMR (400 MHz, DMSO-*d*₆) δ 10.50 (s, 1H), 9.41 (s, 1H), 8.82 (d, *J =* 7.9 Hz, 1H), 8.38 (s, 1H), 8.28 (s, 1H), 7.63 (d, *J =* 8.2 Hz, 1H), 7.54 (d, *J =* 1.9 Hz, 1H), 7.38 (dd, *J =* 8.2, 2.0 Hz, 1H), 7.30-6.95 (m, 1H), 6.82 (d, *J =* 8.0 Hz, 1H), 4.48 - 4.42 (m, 2H), 4.26 - 4.09 (m, 2H), 3.79 - 3.50 (m, 4H), 3.27 - 3.17 (m, 2H), 3.10 - 2.90 (m, 1H), 2.85-2.70 (m, 3H), 2.29 (t, *J =* 6.8 Hz, 2H), 2.15 - 2.07 (m, 5H), 2.07-2.01 (m, 2H), 1.95 -1.83 (m, 4H), 1.82 - 1.47 (m, 9H), 1.41 - 1.31 (m, 2H), 1.17 - 0.96 (m, 4H).

### Example 11: 5-(8-oxa-3-azabicyclo[3.2.1]octan-3-yl)-N-(1-((1R,4R)-4-((9-(4-chloro-3-(2,4-dioxotetrahydropyrimidin-1(2H)-yl)benzoyl)-3,9-diazaspiro[5.5]undecan-3-yl)methyl)cyclohexyl)-3-(difluoromethyl)-1H-pyrazol-4-yl)pyrazolo[1,5-a]pyrimidine-3-carboxamide

### Step 1: preparation of tert-butyl 9-(((1R,4R)-4-(4-(5-(8-oxa-3-azabicyclo[3.2.1]octan-3-yl)pyrazolo[1,5-a]pyrimidine-3-carboxamido)-3-(difluoromethyl)-1H-pyrazol-1-yl)cyclohexyl)methyl)-3,9-diazaspiro[5.5] undecane-3-carboxylate

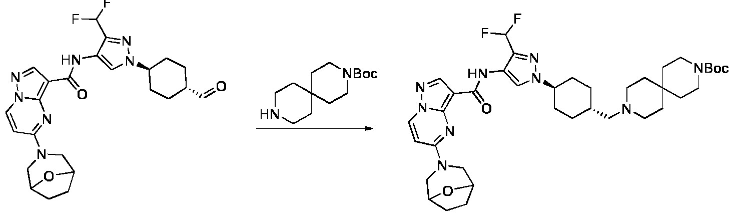

5-(8-Oxa-3-azabicyclo[3.2.1]octan-3-yl)-*N*-(3-(difluoromethyl)-1-((1*R,*4*R*)-4-formylcyclohexyl)-1*H*-pyrazol-4-yl)pyrazolo[1,5-*a*]pyrimidine-3-carboxamide (100 mg, 0.20 mmol) was dissolved in THF (10 mL). *tert*-Butyl 3,9-diazaspiro[5.5]undecane-3-carboxylate (48 mg, 0.20 mmol) and STAB (127 mg, 0.6 mmol) were added, and the mixture was reacted at room temperature for 2 h. The mixture was diluted with water (50 mL), and the resulting mixture was extracted with ethyl acetate (50 mL × 3). The organic phase was washed with saturated brine (100 mL), dried over anhydrous sodium sulfate, filtered, and concentrated under reduced pressure. The crude product was purified by column chromatography (MeOH:DCM = 1:11) to give *tert*-butyl 9-(((1*R*,4*R*)-4-(4-(5-(8-oxa-3-azabicyclo[3.2.1]octan-3-yl)pyrazolo[1,5-*a*]pyrimidine-3-carboxamido)-3-(difluoromethyl)-1*H*-pyrazol-1-yl)cyclohexyl)methyl)-3,9-diazaspiro[5.5]undecane-3-carboxylate.

LC-MS: (ESI, m/z): [M+H] ⁺ = 738.5.

### Step 2: preparation of N-(1-((1R,4R)-4-((3,9-diazaspiro[5.5]undecan-3-yl)methyl)cyclohexyl)-3-(difluoromethyl)-1H-pyrazol-4-yl)-5-(8-oxa-3-azabicyclo[3.2.1]octan-3-yl)pyrazolo[1,5-a]pyrimidine-3-carboxamide

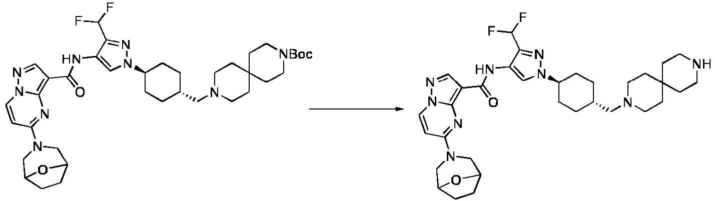

*tert*-Butyl 9-(((1*R*,4*R*)-4-(4-(5-(8-oxa-3-azabicyclo[3.2.1]octan-3-yl)pyrazolo[1,5-*a*]pyrimidine-3-carboxamido)-3-(difluoromethyl)-1*H*-pyrazol-1-yl)cyclohexyl)methyl)-3,9-diazaspiro[5.5]undecane-3-carboxylate (105 mg, 0.14 mmol) was dissolved in DCM (3 mL). TFA (1 mL) was added, and the mixture was reacted at room temperature for 1 h. The mixture was concentrated under reduced pressure to give *N*-(1-((1*R*,4*R*)-4-((3,9-diazaspiro[5.5]undecan-3-yl)methyl)cyclohexyl)-3-(difluoromethyl)-1*H*-pyrazol-4-yl)-5-(8-oxa-3-azabicyclo [3.2.1]octan-3-yl)pyrazolo[1,5-*a*]pyrimidine-3-carboxamide, which was directly used in the next step.

### Step 3: preparation of 5-(8-oxa-3-azabicyclo[3.2.1]octan-3-yl)-N-(1-((1R,4R)-4-((9-(4-chloro-3-(2,4-dioxotetrahydropyrimidin-1(2H)-yl)benzoyl)-3,9-diazaspiro[5.5]undecan-3-yl)methyl)cyclohexyl)-3-(difluoromethyl)-1H-pyrazol-4-yl)pyrazolo[1,5-a]pyrimidine-3-carboxamide

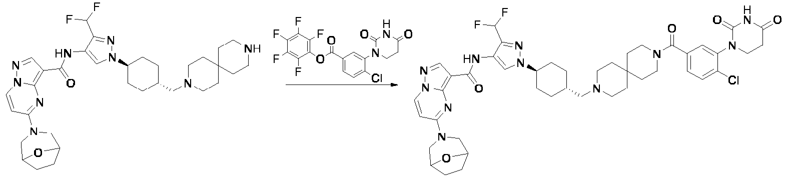

*N*-(1-((1*R*,4*R*)-4-((3,9-Diazaspiro[5.5]undecan-3-yl)methyl)cyclohexyl)-3-(difluoromethyl)-1*H*-pyrazol-4-yl)-5-(8-oxa-3-azabicyclo[3.2.1]octan-3-yl)pyrazolo[1,5-*a*]pyrimidine-3-carboxamide (110 mg, 0.14 mmol) was dissolved in DMSO (5 mL). DIEA (90 mg, 0.70 mmol) and pentafluorophenyl 4-chloro-3-(2,4-dioxotetrahydropyrimidin-1(2*H*)-yl)benzoate (61 mg, 0.14 mmol) were added, and the mixture was reacted at room temperature for 2 h. Water (50 mL) was added, and the mixture was stirred for 5 min and extracted with ethyl acetate (50 mL × 3). The organic phase was washed with saturated brine (100 mL), dried over anhydrous sodium sulfate, filtered, and concentrated under reduced pressure. The crude product was purified by preparative reversed-phase chromatography to give 5-(8-oxa-3-azabicyclo[3.2.1]octan-3-yl)-*N*-(1-((1*R*,4*R*)-4-((9-(4-chloro-3-(2,4-dioxotetrahydropyrimidin-1(2*H*)-yl)benzoyl)-3,9-diazaspiro[5.5]undecan-3-yl)methyl) cyclohexyl)-3-(difluoromethyl)-1*H*-pyrazol-4-yl)pyrazolo[1,5-*a*]pyrimidine-3-carboxamide.

LC-MS: (ESI, m/z): [M+H] ⁺ = 888.2.

¹H NMR (400 MHz, DMSO-*d*₆) δ 10.51 (s, 1H), 9.41 (s, 1H), 8.81 (d, *J =* 7.9 Hz, 1H), 8.37 (s, 1H), 8.28 (s, 1H), 7.64 (d, *J =* 8.2 Hz, 1H), 7.55 (d, *J =* 1.8 Hz, 1H), 7.39 (dd, *J =* 8.2, 1.9 Hz, 1H), 7.24-6.97 (m, 1H), 6.82 (d, *J* = 8.0 Hz, 1H), 4.47-4.41 (m, 2H), 4.31-3.93 (m, 3H), 3.81 - 3.72 (m, 1H), 3.67 - 3.51 (m, 3H), 3.25-3.15 (m, 2H), 2.77-2.72 (m, 2H), 2.35-2.25 (m, 4H), 2.15-2.07 (m, 2H), 2.16-2.98 (m, 2H), 1.96-1.29 (m, 19H), 1.10-0.92 (m, 2H).

### Example 12: N-(3-(difluoromethyl)-1-(1-((3-(3-(2,4-dioxotetrahydropyrimidin-1(2H)-yl)-4-fluorobenzoyl)-3-azaspiro[5.5]undecan-9-yl)methyl)piperidin-4-yl)-1H-pyrazol-4-yl)-5-morpholinopyrazolo[1,5-a]pyrimidine-3-carboxamide

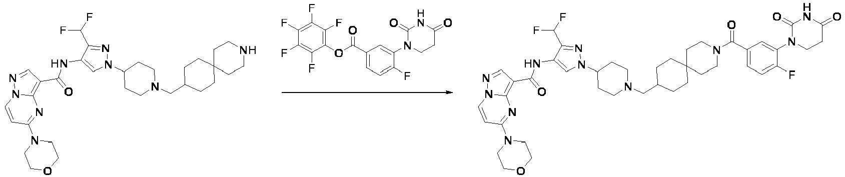

*N*-(1-(1-((3-Azaspiro[5.5]undecan-9-yl)methyl)piperidin-4-yl)-3-(difluoromethyl)-1*H*-pyrazol-4-yl)-5-morpholinopyrazolo[1,5-*a*]pyrimidine-3-carboxamide (100 mg, 0.14 mmol) was dissolved in dimethyl sulfoxide (3 mL). Pentafluorophenyl 3-(2,4-dioxotetrahydropyrimidin-1(2*H*)-yl)-4-fluorobenzoate (58 mg, 0.14 mmol) and *N*,*N*-diisopropylethylamine (54 mg, 0.42 mmol) were added with stirring. After the mixture was stirred at room temperature for 2 h, water (50 mL) was added. The mixture was filtered, and the filter cake was purified by pre-HPLC (CH₃CN/0.08% aqueous NH₄HCO₃ solution, 5%-95%) to give *N*-(3-(difluoromethyl)-1-(1-((3-(3-(2,4-dioxotetrahydropyrimidin-1(2*H*)-yl)-4-fluorobenzoyl)-3-azaspiro[5.5] undecan-9-yl)methyl)piperidin-4-yl)-1*H*-pyrazol-4-yl)-5-morphofinopyrazolo[1,5-*a*]pyrimidine-3-carboxamide.

LC-MS: (ESI, m/z): [M+H] ⁺ = 846.4.

¹H NMR (400 MHz, DMSO-*d*₆) δ 10.53 (s, 1H), 9.40 (s, 1H), 8.83 (d, *J =* 7.9 Hz, 1H), 8.39 (s, 1H), 8.29 (s, 1H), 7.50 (d, *J =* 7.7 Hz, 1H), 7.36 (d, *J* = 4.0 Hz, 2H), 7.23-6.97 (m, 1H), 6.91 (d, *J =* 8.0 Hz, 1H), 4.25 - 4.15 (m, 1H), 3.84 - 3.69 (m, 10H), 3.65-3.49 (m, 2H), 3.31 - 3.24 (m, 2H), 2.89 (d, *J =* 10.3 Hz, 2H), 2.73 (t, *J =* 6.6 Hz, 2H), 2.20-2.04 (m, 2H), 2.04-1.80 (m, 6H), 1.70-1.43 (m, 7H), 1.43-1.20 (m, 2H), 1.20-0.96 (m, 4H).

### Example 13: N-(3-(difluoromethyl)-1-(1-((3-(3-(2,4-dioxotetrahydropyrimidin-1(2H)-yl)-4-methylbenzoyl)-3-azaspiro[5.5]undecan-9-yl)methyl)piperidin-4-yl)-1H-pyrazol-4-yl)-5-morpholinopyrazolo[1,5-a]pyrimidine-3-carboxamide

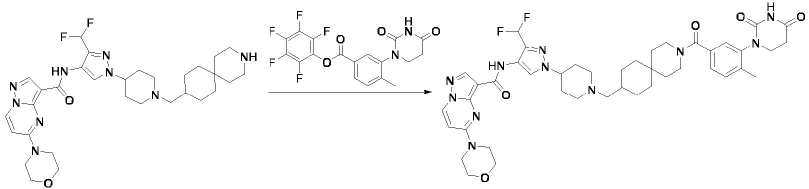

*N*-(1-(1-((3-Azaspiro[5.5]undecan-9-yl)methyl)piperidin-4-yl)-3-(difluoromethyl)-1*H*-pyrazol-4-yl)-5-morpholinopyrazolo[1,5-*a*]pyrimidine-3-carboxamide (100 mg, 0.12 mmol) was dissolved in dimethyl sulfoxide (3 mL). Pentafluorophenyl 3-(2,4-dioxotetrahydropyrimidin-1(2*H*)-yl)-4-methylbenzoate (58 mg, 0.14 mmol) and *N*,*N*-diisopropylethylamine (46 mg, 0.36 mmol) were added with stirring. After the mixture was stirred at room temperature for 2 h, water (50 mL) was added. The mixture was filtered, and the filter cake was purified by pre-HPLC (CH₃CN/0.08% aqueous NH₄HCO₃ solution, 5%-95%) to give *N*-(3-(difluoromethyl)-1-(1-((3-(3-(2,4-dioxotetrahydropyrimidin-1(2*H*)-yl)-4-methylbenzoyl)-3-azaspiro[5.5] undecan-9-yl)methyl)piperidin-4-yl)-1*H*-pyrazol-4-yl)-5-morpholinopyrazolo[1,5-*a*]pyrimidine-3-carboxamide. LC-MS: (ESI, m/z): [M+H] ⁺ = 842.4.

¹H NMR (400 MHz, DMSO-*d*₆) δ 10.37 (s, 1H), 9.40 (s, 1H), 8.83 (d, *J =* 7.9 Hz, 1H), 8.39 (s, 1H), 8.29 (s, 1H), 7.37 - 7.29 (m, 2H), 7.28-6.96 (m, 2H), 6.91 (d, *J =* 7.9 Hz, 1H), 4.25-4.15 (m, 1H), 3.83-3.63 (m, 9H), 3.61-3.47 (m, 3H), 3.32 - 3.24 (m, 2H), 2.95-2.65 (m, 4H), 2.25-1.85 (m, 11H), 1.70 (d, *J* = 9.3 Hz, 2H), 1.58-1.15 (m, 7H), 1.12-0.91 (m, 4H).

### Example 14: N-(3-(difluoromethyl)-1-((1R,4S)-4-(((2-(1-(3-(2,4-dioxotetrahydropyrimidin-1(2H)-yl)-4-methoxybenzoyl)piperidin-4-yl)ethyl)(methyl)amino)methyl)cyclohexyl)-1H-pyrazol-4-yl)-5-((S)-3-hydroxypiperidin-1-yl)pyrazolo[1,5-a]pyrimidine-3-carboxamide

### Step 1: preparation of tert-butyl 4-(2-((((1S,4R)-4-(3-(difluoromethyl)-4-(5-((3S)-3-((tetrahydro-2H-pyran-2-yl)oxy)piperidin-1-yl)pyrazolo[1,5-a]pyrimidine-3-carboxamido)-1H-pyrazol-1-yl)cyclohexyl)methyl) (methyl)amino)ethyl)piperidine-1-carboxylate

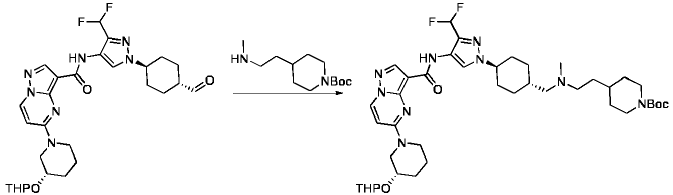

*N*-(3-(Difluoromethyl)-1-((1*R*,4*S*)-4-formylcyclohexyl)-1*H*-pyrazol-4-yl)-5-((3*S*)-3-((tetrahydro-2*H*-pyran-2-yl)oxy)piperidin-1-yl)pyrazolo[1,5-*a*]pyrimidine-3-carboxamide (120 mg, 0.21 mmol) and *tert*-butyl 4-(2-(methylamino)ethyl)piperidine-1-carboxylate (51 mg, 0.21 mmol) were dissolved in tetrahydrofuran (10 mL). Sodium triacetoxyborohydride (222 mg, 1.05 mmol) was added with stirring. The mixture was stirred at room temperature overnight. Water (50 mL) was added, and the mixture was extracted with dichloromethane (20 mL × 3). The organic phase was washed with saturated brine (60 mL), dried over anhydrous sodium sulfate, filtered, and concentrated under reduced pressure. The crude product was purified by column chromatography (methanol:dichloromethane = 1:9) to give *tert*-butyl 4-(2-((((1*S*,4*R*)-4-(3-(difluoromethyl)-4-(5-((3*S*)-3-((tetrahydro-2*H*-pyran-2-yl)oxy)piperidin-1-yl)pyrazolo[1,5-*a*]pyrimidine-3-carboxamido)-1*H*-pyrazol-1-yl)cyclohexyl)methyl)(methyl)amino)ethyl)piperidine-1-carboxylate.

LC-MS: (ESI, m/z): [M+H] ⁺ = 798.5.

### Step 2: preparation of N-(3-(difluoromethyl)-1-((1R,4S)-4-((methyl(2-(piperidin-4-yl)ethyl)amino)methyl) cyclohexyl)-1H-pyrazol-4-yl)-5-((S)-3-hydroxypiperidin-1-yl)pyrazolo[1,5-a]pyrimidine-3-carboxamide

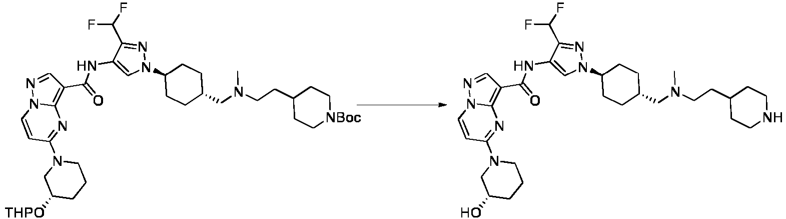

*tert*-Butyl 4-(2-((((1*S*,4*R*)-4-(3-(difluoromethyl)-4-(5-((3*S*)-3-((tetrahydro-2*H*-pyran-2-yl)oxy)piperidin-1-yl)pyrazolo[1,5-*a*]pyrimidine-3-carboxamido)-1*H*-pyrazol-1-yl)cyclohexyl)methyl)(methyl)amino)ethyl) piperidine-1-carboxylate (80 mg, 0.10 mmol) was dissolved in dichloromethane (3 mL). Trifluoroacetic acid (1 mL) was added, and the mixture was stirred at room temperature for 2 h. The reaction liquid was directly concentrated under reduced pressure to give *N*-(3-(difluoromethyl)-1-((1*R*,4*S*)-4-((methyl(2-(piperidin-4-yl)ethyl)amino)methyl)cyclohexyl)-1*H*-pyrazol-4-yl)-5-((*S*)-3-hydroxypiperidin-1-yl)pyrazolo[1,5-*a*]pyrimidine-3-carboxamide, which was directly used in the next step without purification.

LC-MS: (ESI, m/z): [M+H] ⁺ = 614.3.

### Step 3: preparation of N-(3-(difluoromethyl)-1-((1R,4S)-4-(((2-(1-(3-(2,4-dioxotetrahydropyrimidin-1(2H)-yl)-4-methoxybenzoyl)piperidin-4-yl)ethyl)(methyl)amino)methyl)cyclohexyl)-1H-pyrazol-4-yl)-5-((S)-3-hydroxypiperidin-1-yl)pyrazolo[1,5-a]pyrimidine-3-carboxamide

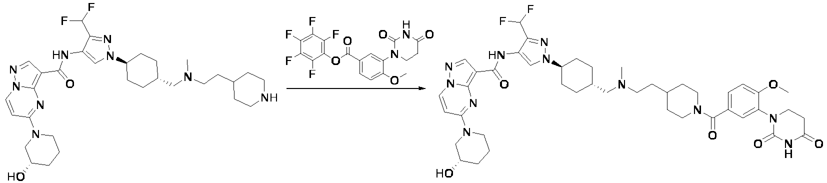

*N*-(3-(Difluoromethyl)-1-((1*R*,4*S*)-4-((methyl(2-(piperidin-4-yl)ethyl)amino)methyl)cyclohexyl)-1*H*-pyrazol-4-yl)-5-((*S*)-3-hydroxypiperidin-1-yl)pyrazolo[1,5-*a*]pyrimidine-3-carboxamide (80 mg, 0.10 mmol) was dissolved in dimethyl sulfoxide (3 mL). Pentafluorophenyl 3-(2,4-dioxotetrahydropyrimidin-1(2*H*)-yl)-4-methoxybenzoate (47 mg, 0.11 mmol) and *N*,*N*-diisopropylethylamine (38 mg, 0.30 mmol) were added with stirring. The mixture was stirred at room temperature overnight. Water (50 mL) was added. The mixture was filtered, and the filter cake was purified by pre-HPLC (CH₃CN/0.08% aqueous NH₄HCO₃ solution, 5%-95%) to give *N*-(3-(difluoromethyl)-1-((1*R*,4*S*)-4-(((2-(1-(3-(2,4-dioxotetrahydropyrimidin-1(2*H*)-yl)-4-methoxybenzoyl) piperidin-4-yl)ethyl)(methyl)amino)methyl)cyclohexyl)-1*H*-pyrazol-4-yl)-5-((*S*)-3-hydroxypiperidin-1-yl)pyrazolo[1,5-*a*]pyrimidine-3-carboxamide.

LC-MS: (ESI, m/z): [M+H] ⁺ = 860.4.

¹H NMR (400 MHz, DMSO-*d*₆) δ 10.34 (s, 1H), 9.37 (s, 1H), 8.72 (d, *J =* 8.0 Hz, 1H), 8.37 (s, 1H), 8.24 (d, *J* = 5.1 Hz, 1H), 7.36 (dd, *J =* 8.5, 2.1 Hz, 1H), 7.32 (d, *J =* 2.1 Hz, 1H), 7.24 - 6.94 (m, 2H), 6.86 (d, *J =* 8.1 Hz, 1H), 4.89 (d, *J* = 4.1 Hz, 1H), 4.50-4.15 (m, 2H), 4.03-3.80 (m, 5H), 3.72-3.53 (m, 4H), 3.51-3.40 (m, 1H), 3.10-2.65 (m, 4H), 2.30 (t, *J* = 7.0 Hz, 2H), 2.15 -1.95 (m, 7H), 1.92-1.25 (m, 15H), 1.17 - 0.95 (m, 4H).

### Example 15: 5-((S)-3-aminopiperidin-1-yl)-N-(3-(difluoromethyl)-1-((1R,4S)-4-(((2-(1-(3-(2,4-dioxotetrahydropyrimidin-1(2H)-yl)-4-methoxybenzoyl)piperidin-4-yl)ethyl)(methyl)amino)methyl) cyclohexyl)-1H-pyrazol-4-yl)pyrazolo[1,5-a]pyrimidine-3-carboxamide

### Step 1: preparation of tert-butyl ((S)-1-(3-((3-(difluoromethyl)-1-((1R,4S)-4-(hydroxymethyl)cyclohexyl)-1H-pyrazol-4-yl)carbamoyl)pyrazolo[1,5-a]pyrimidin-5-yl)piperidin-3-yl)carboxylate

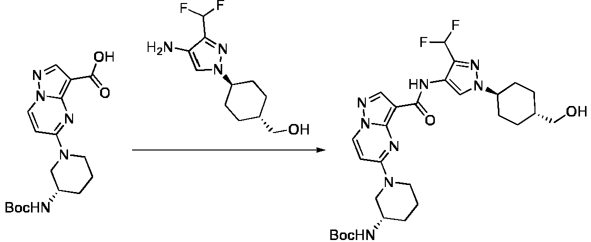

(*S*)-5-(3-((*tert*-Butoxycarbonyl)amino)piperidin-1-yl)pyrazolo[1,5-*a*]pyrimidine-3-carboxylic acid (150 mg, 0.42 mmol) was dissolved in DMF (10 mL). ((1*R*,4*R*)-4-(4-Amino-3-(difluoromethyl)-1*H*-pyrazol-1-yl)cyclohexyl)methanol (112 mg, 0.46 mmol), DIEA (163 mg, 1.26 mmol), and py-BOP (284 mg, 0.55 mmol) were added, and the mixture was reacted at room temperature for 2 h. Water (50 mL) was added, and the mixture was stirred for 5 min and extracted with ethyl acetate (50 mL × 3). The organic phase was washed with saturated brine (100 mL), dried over anhydrous sodium sulfate, filtered, and concentrated under reduced pressure. The crude product was purified by column chromatography (EA:PE = 0-1) to give *tert*-butyl ((*S*)-1-(3-((3-(difluoromethyl)-1-((1*R*,4*S*)-4-(hydroxymethyl)cyclohexyl)-1*H*-pyrazol-4-yl)carbamoyl)pyrazolo[1,5-a]pyrimidin-5-yl)piperidin-3-yl)carboxylate.

LC-MS: (ESI, m/z): [M+H] ⁺ = 589.3.

### Step 2: preparation of tert-butyl ((S)-1-(3-((3-(difluoromethyl)-1-((1R,4S)-4-formylcyclohexyl)-1H-pyrazol-4-yl)carbamoyl)pyrazolo[1,5-a]pyrimidin-5-yl)piperidin-3-yl)carboxylate

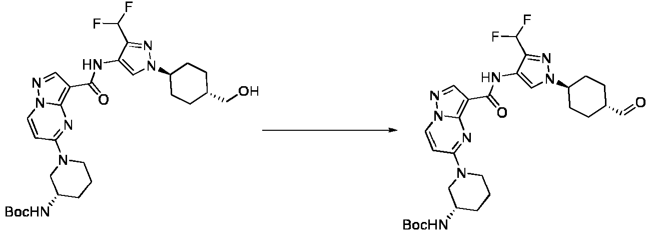

*tert-Butyl* ((*S*)-1-(3-((3-(difluoromethyl)-1-((1*R*,4*S*)-4-(hydroxymethyl)cyclohexyl)-1*H*-pyrazol-4-yl)carbamoyl) pyrazolo[1,5-a]pyrimidin-5-yl)piperidin-3-yl)carboxylate (190 mg, 0.32 mmol) was dissolved in ACN (10 mL). IBX (181 mg, 0.65 mmol) was added, and the mixture was reacted at 85 °C for 1.5 h. Water (30 mL) was added to the reaction liquid, and the mixture was extracted with ethyl acetate (30 mL × 3). The organic phase was washed with saturated brine (50 mL), dried over anhydrous sodium sulfate, filtered, and concentrated under reduced pressure. The crude product was purified by column chromatography (EA:PE = 0-1) to give *tert-butyl* ((*S*)-1-(3-((3-(difluoromethyl)-1-((1*R*,4*S*)-4-formylcyclohexyl)-1*H*-pyrazol-4-yl)carbamoyl)pyrazolo[1,5-*a*]pyrimidin-5-yl)piperidin-3-yl)carboxylate.

LC-MS: (ESI, m/z): [M+H] ⁺ = 586.6.

### Step 3: preparation of N-benzyl-N-methyl-2-(piperidin-4-yl)ethan-1-amine

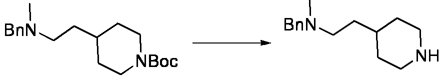

*tert*-Butyl 4-(2-(benzyl(methyl)amino)ethyl)piperidine-1-carboxylate (345 mg, 1.04 mmol) was dissolved in DCM (5 mL). TFA (1 mL) was added, and the mixture was reacted at room temperature for 1 h. The reaction liquid was directly concentrated under reduced pressure to give *N*-benzyl-*N*-methyl-2-(piperidin-4-yl)ethan-1-amine, which was directly used in the next step.

LC-MS: (ESI, m/z): [M+H] ⁺ = 233.2.

### Step 5: preparation of 1-(5-(4-(2-(benzyl(methyl)amino)ethyl)piperidine-1-carbonyl)-2-methoxyphenyl) dihydropyrimidine-2,4(1H,3H)-dione

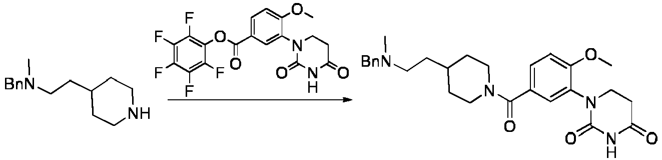

*N*-Benzyl-*N*-methyl-2-(piperidin-4-yl)ethan-1-amine (250 mg, 1.04 mmol) was dissolved in DMSO (10 mL). DIEA (671 mg, 5.2 mmol) and pentafluorophenyl 3-(2,4-dioxotetrahydropyrimidin-1(2*H*)-yl)-4-methoxybenzoate (451 mg, 1.04 mmol) were added, and the mixture was reacted at room temperature for 2 h. Water (100 mL) was added to the reaction liquid, and the mixture was stirred for 5 min and extracted with ethyl acetate (100 mL × 3). The organic phase was washed with saturated brine (150 mL), dried over anhydrous sodium sulfate, filtered, and concentrated under reduced pressure. The crude product was purified by column chromatography (MeOH:DCM = 0-1:9) to give 1-(5-(4-(2-(benzyl(methyl)amino)ethyl)piperidine-1-carbonyl)-2-methoxyphenyl)dihydropyrimidine-2,4(1*H*,3*H*)-dione.

LC-MS: (ESI, m/z): [M+H] ⁺ = 479.2.

### Step 6: preparation of 1-(2-methoxy-5-(4-(2-(methylamino)ethyl)piperidine-1-carbonyl)phenyl) dihydropyrimidine-2,4(1H,3H)-dione

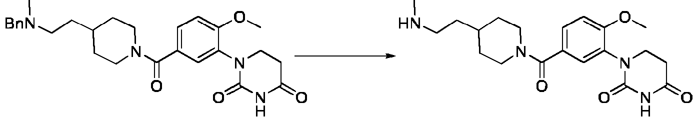

1-(5-(4-(2-(Benzyl(methyl)amino)ethyl)piperidine-1-carbonyl)-2-methoxyphenyl)dihydropyrimidine-2,4(1*H*,3*H*)-dione (240 mg, 0.5 mmol) was dissolved in MeOH (10 mL). Pd/C (72 mg, 30%) was added, and the mixture was reacted at room temperature for 2 h. The mixture was filtered and concentrated under reduced pressure to give 1-(2-methoxy-5-(4-(2-(methylamino)ethyl)piperidine-1-carbonyl)phenyl)dihydropyrimidine-2,4(1H,3H)-dione, which was directly used in the next step.

LC-MS: (ESI, m/z): [M+Na] ⁺ = 411.1.

### Step 7: preparation of tert-butyl ((S)-1-(3-((3-(difluoromethyl)-1-((1R,4S)-4-(((2-(1-(3-(2,4-dioxotetrahydropyrimidin-1(2H)-yl)-4-methoxybenzoyl)piperidin-4-yl)ethyl)(methyl)amino)methyl) cyclohexyl)-1H-pyrazol-4-yl)carbonyl)pyrazolo[1.5-a]pyrimidin-5-yl)piperidin-3-yl)carboxylate

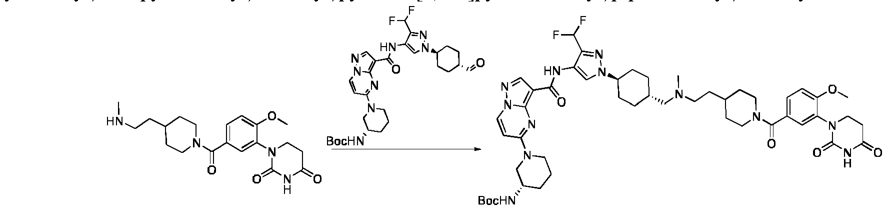

1-(2-Methoxy-5-(4-(2-(methylamino)ethyl)piperidine-1-carbonyl)phenyl)dihydropyrimidine-2,4(1H,3H)-dione (95 mg, 0.24 mmol) was dissolved in THF (10 mL). *tert*-Butyl ((*S*)-1-(3-((3-(difluoromethyl)-1-((1*R*,4*S*)-4-formylcyclohexyl)-1*H*-pyrazol-4-yl)carbamoyl)pyrazolo[1,5-*a*]pyrimidin-5-yl)piperidin-3-yl)carboxylate

(120 mg, 0.20 mmol) and STAB (127 mg, 0.60 mmol) were added, and the mixture was reacted at room temperature for 2 h. The reaction liquid was diluted with water (50 mL), and the mixture was extracted with ethyl acetate (50 mL × 3). The organic phase was washed with saturated brine (100 mL), dried over anhydrous sodium sulfate, filtered, and concentrated under reduced pressure. The crude product was purified by column chromatography (MeOH:DCM = 0-1:9) to give *tert*-butyl ((S)-1-(3-((3-(difluoromethyl)-1-((1*R*,4*S*)-4-(((2-(1-(3-(2,4-dioxotetrahydropyrimidin-1(2*H*)-yl)-4-methoxybenzoyl)piperidin-4-yl)ethyl)(methyl)amino)methyl) cyclohexyl)-1*H*-pyrazol-4-yl)carbonyl)pyrazolo[1,5-*a*]pyrimidin-5-yl)piperidin-3-yl)carboxylate.

LC-MS: (ESI, m/z): [M+H] ⁺ = 959.4.

### Step 8: preparation of 5-((S)-3-aminopiperidin-1-yl)-N-(3-(difluoromethyl)-1-((1R,4S)-4-(((2-(1-(3-(2,4-dioxotetrahydropyrimidin-1(2H)-yl)-4-methoxybenzoyl)piperidin-4-yl)ethyl)(methyl)amino)methyl) cyclohexyl)-1H-pyrazol-4-yl)pyrazolo[1,5-a]pyrimidine-3-carboxamide

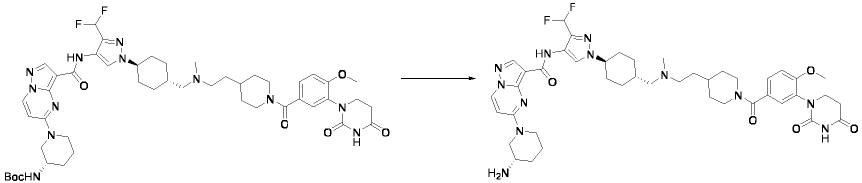

*tert*-Butyl ((*S*)-1-(3-((3-(difluoromethyl)-1-((1*R*,4*S*)-4-(((2-(1-(3-(2,4-dioxotetrahydropyrimidin-1(2*H*)-yl)-4-methoxybenzoyl)piperidin-4-yl)ethyl)(methyl)amino)methyl)cyclohexyl)-1*H*-pyrazol-4-yl)carbonyl)pyrazolo [1,5-*a*]pyrimidin-5-yl)piperidin-3-yl)carboxylate (100 mg, 0.10 mmol) was dissolved in DCM (5 mL). TFA (1 mL) was added, and the mixture was reacted at room temperature for 1 h. The reaction liquid was directly concentrated under reduced pressure, and the crude product was purified by preparative reversed-phase chromatography to give 5-((*S*)-3-aminopiperidin-1-yl)-*N*-(3-(difluoromethyl)-1-((1*R*,4*S*)-4-(((2-(1-(3-(2,4-dioxotetrahydropyrimidin-1(2*H*)-yl)-4-methoxybenzoyl)piperidin-4-yl)ethyl)(methyl)amino)methyl) cyclohexyl)-1*H*-pyrazol-4-yl)pyrazolo[1,5-*a*]pyrimidine-3-carboxamide.

LC-MS: (ESI, m/z): [M+H] ⁺= 859.4.

¹H NMR (400 MHz, DMSO-*d*₆) δ 10.33 (s, 1H), 9.36 (s, 1H), 8.74 (d, *J* = 8.0 Hz, 1H), 8.38 (s, 1H), 8.24 (s, 1H), 7.36 (dd, *J =* 8.4, 2.1 Hz, 1H), 7.32 (d, *J* = 2.1 Hz, 1H), 7.27 - 6.97 (m, 2H), 6.88 (d, *J =* 8.1 Hz, 1H), 4.52-4.15 (m, 4H), 3.84 (s, 3H), 3.59 (t, *J =* 6.6 Hz, 2H), 3.24 - 2.93 (m, 3H), 2.80-2.60 (m, 4H), 2.40-2.25 (m, 2H), 2.16 - 1.99 (m, 8H), 1.95-1.80 (m, 3H), 1.80 - 1.29 (m, 13H), 1.17 - 0.96 (m, 4H).

### Example 16: N-(3-(difluoromethyl)-1-(1-(2-(3-(3-(2,4-dioxotetrahydropyrimidin-1(2H)-yl)-4-methoxybenzoyl)-3-azaspiro[5.5]undecan-9-yl)ethyl)piperidin-4-yl)-1H-pyrazol-4-yl)-5-morpholinopyrazolo [1,5-a]pyrimidine-3-carboxamide

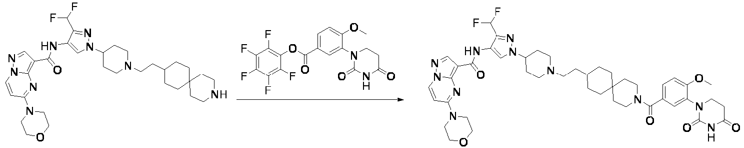

*N*-(1-(1-(2-(3-Azaspiro[5.5]undecan-9-yl)ethyl)piperidin-4-yl)-3-(difluoromethyl)-1*H*-pyrazol-4-yl)-5-morpholinopyrazolo[1,5-*a*]pyrimidine-3-carboxamide (103 mg, 0.165 mmol) and pentafluorophenyl 3-(2,4-dioxotetrahydropyrimidin-1(2H)-yl)-4-methoxybenzoate (85 mg, 0.198 mmol) were added to DMSO (3 mL). Then DIEA (63.8 mg, 0.495 mmol) was added. The mixture was stirred at room temperature for 16 h, and then water (20 mL) was added. The mixture was extracted with ethyl acetate (25 mL × 4), washed with saturated brine, dried over anhydrous sodium sulfate, and concentrated under reduced pressure. The crude product was purified by Prep-HPLC (acetonitrile/0.5% aqueous FA solution, 5%-95%) to give the product of *N*-(3-(difluoromethyl)-l-(1-(2-(3-(3-(2,4-dioxotetrahydropyrimidin-1(2*H*)-yl)-4-methoxybenzoyl)-3-azaspiro[5.5] undecan-9-yl)ethyl)piperidin-4-yl)-1*H*-pyrazol-4-yl)-5-morpholinopyrazolo[1,5-*a*]pyrimidine-3-carboxamide. LC-MS: (ESI, m/z): [M+H] ⁺= 872.5.

¹H NMR (400 MHz, DMSO-*d*₆) δ 10.34 (s, 1H), 9.40 (s, 1H), 8.83 (d, *J* = 7.9 Hz, 1H), 8.39 (s, 1H), 8.29 (s, 1H), 8.27 (s, 0.62H, HCOOH), 7.42 - 7.28 (m, 2H), 7.26 - 6.94 (m, 2H), 6.91 (d, *J* = 7.9 Hz, 1H), 4.30-4.10 (m, 1H), 3.84 (s, 3H), 3.79-3.61(m, 8H), 3.62 - 3.54 (m, 6H), 2.97-2.91 (m, 2H), 2.71-2.65 (m, 2H), 2.37-2.17 (m, 2H), 2.05 - 1.85 (m, 6H), 1.75-1.62 (m, 2H), 1.55 - 1.17 (m, 9H), 1.17-0.93 (m, 4H).

### Example 17: N-(3-(difluoromethyl)-1-((1R,4R)-4-((2-(1-(3-(2,6-dioxopyridin-3-yl)-4-fluorobenzoyl) piperidin-4-yl)ethyl)(methyl)carbamoyl)cyclohexyl)-1H-pyrazol-4-yl)-5-morpholinopyrazolo[1,5-a]pyrimidine-3-carboxamide

### Step 1: preparation of methyl (1R,4R)-4-(3-(difluoromethyl)-4-(5-morpholinopyrazolo[1,5-a]pyrimidine-3-carboxamido)-1H-pyrazol-1-yl)cyclohexane-1-carboxylate

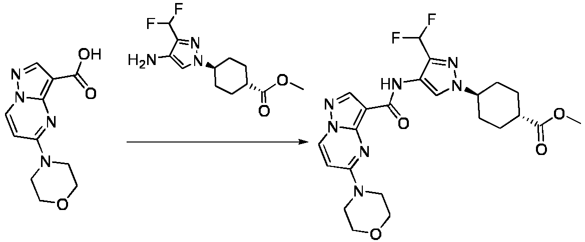

5-Morpholinopyrazolo[1,5-*a*]pyrimidine-3-carboxylic acid (200 mg, 0.73 mmol) was dissolved in DMF (4 mL). Methyl (1*R*,4*R*)-4-(4-amino-3-(difluoromethyl)-1*H*-pyrazol-1-yl)cyclohexane-1-carboxylate (182 mg, 0.73 mmol), HATU (139 mg, 0.95 mmol), and DIEA (377 mg, 2.92 mmol) were added, and the reaction liquid was stirred at room temperature overnight. The reaction liquid was diluted with water (50 mL), and the mixture was extracted with ethyl acetate (40 mL × 3). The organic phase was washed with saturated brine, dried over anhydrous sodium sulfate, filtered, and concentrated under reduced pressure. The crude product was purified by column chromatography (EA:PE = 9:1) to give methyl (1*R*,4*R*)-4-(3-(difluoromethyl)-4-(5-morpholinopyrazolo[1,5-*a*]pyrimidine-3-carboxamido)-1*H*-pyrazol-1-yl)cyclohexane-1-carboxylate.

LC-MS: (ESI, m/z): [M+H] ⁺= 504.1.

### Step 2: preparation of (1R,4R)-4-(3-(difluoromethyl)-4-(5-morpholinopyrazolo[1,5-a]pyrimidine-3-carboxamido)-1H-pyrazol-1-yl)cyclohexane-1-carboxylic acid

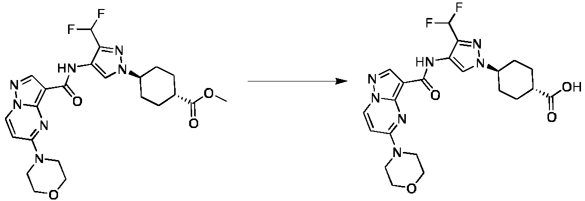

Methyl (1*R*,4*R*)-4-(3-(difluoromethyl)-4-(5-morpholinopyrazolo[1,5-*a*]pyrimidine-3-carboxatnido)-1*H*-pyrazol-1-yl)cyclohexane-1-carboxylate (230 mg, 0.46 mmol) was dissolved in a mixed solution of methanol/water (5 mL, V/V = 5/1). Lithium hydroxide (44 mg, 1.84 mmol) was added, and the reaction liquid was heated to 60 °C and stirred for 2 h. The reaction liquid was adjusted to pH = 4 with dilute hydrochloric acid (2 mol/L), and the mixture was extracted with ethyl acetate (40 mL × 3). The organic phase was washed with saturated brine, dried over anhydrous sodium sulfate, filtered, and concentrated under reduced pressure to give (1*R*,4*R*)-4-(3-(difluoromethyl)-4-(5-morpholinopyrazolo[1,5-*a*]pyrimidine-3-carboxamido)-1*H*-pyrazol-1-yl)cyclohexane-1-carboxylic acid, which was directly used in the next step.

LC-MS: (ESI, m/z): [M+H] ⁺= 490.6.

### Step 3: preparation of tert-butyl 4-(2-((1R,4R)-4-(3-(difluoromethyl)-4-(5-morpholinopyrazolo[1,5-a]pyrimidine-3-carboxamido)-1H-pyrazol-1-yl)-N-methylcyclohexane-1-carboxamido)ethyl)piperidine-1-carboxylate

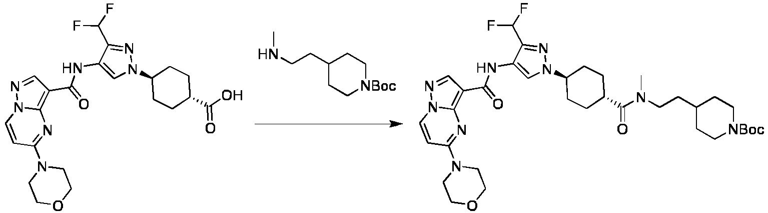

(1*R*,4*R*)-4-(3-(Difluoromethyl)-4-(5-morpholinopyrazolo[1,5-*a*]pyrimidine-3-carboxamido)-1*H*-pyrazol-1-yl)cyclohexane-1-carboxylic acid (140 mg, 0.29 mmol) and *tert-*butyl 4-(2-(methylamino)ethyl)piperidine-1-carboxylate (70 mg, 0.29 mmol) were dissolved in DMF (4 mL). HATU (144 mg, 0.38 mmol) and DIEA (155 mg, 1.2 mmol) were added, and the reaction liquid was stirred at room temperature overnight. The reaction liquid was diluted with water (40 mL), and the mixture was extracted with ethyl acetate (40 mL × 4). The organic phase was washed with saturated brine, dried over anhydrous sodium sulfate, filtered, and concentrated under reduced pressure. The crude product was purified by column chromatography (EA) to give *tert*-butyl 4-(2-((1*R*,4*R*)-4-(3-(difluoromethyl)-4-(5-morpholinopyrazolo[1,5-*a*]pyrimidine-3-carboxamido)-1*H*-pyrazol-1-yl)-*N*-methylcyclohexane-1-carboxamido)ethyl)piperidine-1-carboxylate.

LC-MS: (ESI, m/z): [M+H] ⁺= 714.8.

### Step 4: preparation of N-(3-(difluoromethyl)-1-((1R,4R)-4-(methyl(2-(piperidin-4-yl)ethyl)carbamoyl) cyclohexyl)-1H-pyrazol-4-yl)-5-morpholinopyrazolo[1,5-a]pyrimidine-3-carboxamide

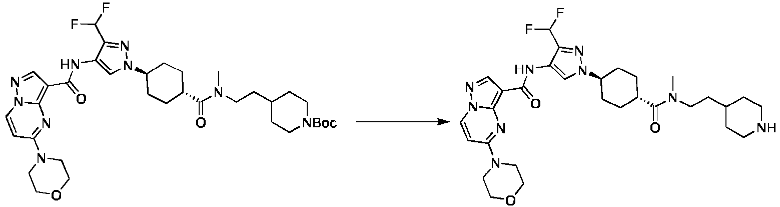

*tert-*Butyl 4-(2-((1*R*,4*R*)-4-(3-(difluoromethyl)-4-(5-morpholinopyrazolo[1,5-*a*]pyrimidine-3-carboxamido)-1*H*-pyrazol-1-yl)-*N*-methylcyclohexane-1-carboxamido)ethyl)piperidine-1-carboxylate (130 mg, 0.18 mmol) was dissolved in dichloromethane (3 mL). Trifluoroacetic acid (0.3 mL) was added, and the mixture was stirred at room temperature for 1 h. The reaction liquid was directly concentrated under reduced pressure to give *N-*(3-(difluoromethyl)-1-((1*R*,4*R*)-4-(methyl(2-(piperidin-4-yl)ethyl)carbamoyl)cyclohexyl)-1*H*-pyrazol-4-yl)-5-morpholinopyrazolo[1,5-*a*]pyrimidine-3-carboxamide, which was directly used in the next step without purification.

LC-MS: (ESI, m/z): [M+H] ⁺= 614.2.

### Step 5: preparation of N-(3-(difluoromethyl)-1-((1R,4R)-4-((2-(1-(3-(2,6-dioxopyridin-3-yl)-4-fluorobenzoyl) piperidin-4-yl)ethyl)(methyl)carbamoyl)cyclohexyl)-1H-pyrazol-4-yl)-5-morpholinopyrazolo[1,5-a]pyrimidine-3-carboxamide

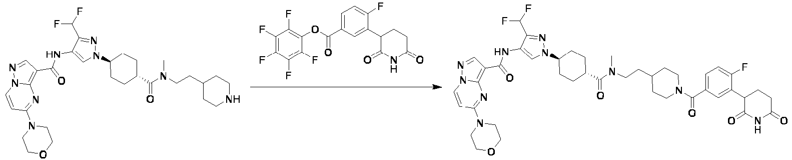

*N*-(3-(Difluoromethyl)-1-((1*R*,4*R*)-4-(methyl(2-(piperidin-4-yl)ethyl)carbamoyl)cyclohexyl)-1*H*-pyrazol-4-yl)-5-morpholinopyrazolo[1,5-*a*]pyrimidine-3-carboxamide (130 mg, 0.18 mmol) and pentafluorophenyl 3-(2,6-dioxopiperidin-3-yl)-4-fluorobenzoate (75.3 mg, 0.18 mmol) were dissolved in DMSO (2 mL). DIEA (93 mg, 0.72 mmol) was added, and the reaction liquid was stirred at room temperature for 3 h. The reaction liquid was diluted with water (20 mL), and the mixture was extracted with ethyl acetate (20 mL × 3). The organic phase was washed with saturated brine (30 mL), dried over anhydrous sodium sulfate, filtered, and concentrated under reduced pressure. The crude product was purified by pre-HPLC (CH₃CN/O.08% aqueous NH₄HCO₃ solution, 5%-95%) to give *N*-(3-(difluoromethyl)-1-((1*R*,4*R*)-4-((2-(1-(3-(2,6-dioxopyridin-3-yl)-4-fluorobenzoyl)piperidin-4-yl)ethyl)(methyl)carbatnoyl)cyclohexyl)-1*H*-pyrazol-4-yl)-5-morpholinopyrazolo [1,5-*a*]pyrimidine-3-carboxamide.

LC-MS: (ESI, m/z): [M+H] ⁺= 847.4.

¹H NMR (400 MHz, DMSO-*d*₆) δ 10.90 (s, 1H), 9.40 (s, 1H), 8.83 (d, *J =* 7.9 Hz, 1H), 8.40 (d, *J =* 5.5 Hz, 1H), 8.29 (s, 1H), 7.40 - 7.33 (m, 2H), 7.31 - 7.23 (m, 1H), 7.23-6.96 (m, 1H), 6.91 (d, *J =* 8.0 Hz, 1H), 4.52-4.20 (m, 2H), 4.12 (dd, *J* = 12.7, 4.9 Hz, 1H), 3.88-3.66 (m, 8H), 3.62-3.48 (m, 1H), 3.433.34 (m, 1H), 3.01 (s, 3H), 2.82 - 2.62 (m, 4H), 2.60-2.54 (m, 1H), 2.29-2.17 (m, 1H), 2.08-1.98 (m, 3H), 1.95 - 1.67 (m, 6H), 1.66 - 1.35 (m, 6H), 1.21 - 1.02 (m, 2H).

### Example 18: 5-((S)-3-aminopiperidin-1-yl)-N-(1-((1R,4S)-4-(((2-(1-(4-chloro-3-(2,4-dioxotetrahydropyrimidin-1(2H)-yl)benzoyl)piperidin-4-yl)ethyl)(methyl)amino)methyl)cyclohexyl)-3-(difluoromethyl)-1H-pyrazol-4-yl)pyrazolo[1,5-a]pyrimidine-3-carboxamide

### Step 1: preparation of tert-butyl 4-(2-((((9H-fluoren-9-yl)methoxy)carbonyl)(methyl)amino)ethyl)piperidine-1-carboxylate

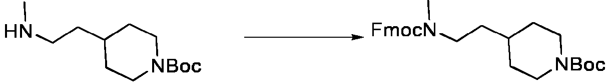

*tert-*Butyl 4-(2-(methylamino)ethyl)piperidine-1-carboxylate (240 mg, 1.0 mmol) was dissolved in DCM (20 mL). FmocCl (311 mg, 1.2 mmol) and pyridine (237 mg, 3.0 mmol) were added, and the mixture was reacted at room temperature overnight. The reaction liquid was diluted with water (50 mL), and the mixture was extracted with DCM (30 mL × 3). The organic phase was dried over anhydrous sodium sulfate, filtered, and concentrated under reduced pressure. The crude product was purified by column chromatography (EA:PE = 0-4:1) to give *tert-*butyl 4-(2-((((9*H*-fluoren-9-yl)methoxy)carbonyl)(methyl)amino)ethyl)piperidine-1-carboxylate.

LC-MS: (ESI, m/z): [M+Na] ⁺= 487.3.

### Step 2: preparation of (9H-fluoren-9-yl)methyl methyl(2-(piperidin-4-yl)ethyl)carbamate

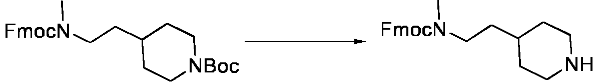

*tert-*Butyl 4-(2-((((9*H*-fluoren-9-yl)methoxy)carbonyl)(methyl)amino)ethyl)piperidine-1-carboxylate (210 mg, 0.45 mmol) was dissolved in DCM (4 mL). TFA (1 mL) was added, and the mixture was reacted at room temperature for 1 h. The reaction liquid was directly concentrated under reduced pressure to give (9*H*-fluoren-9-yl)methyl methyl(2-(piperidin-4-yl)ethyl)carbamate, which was directly used in the next step.

LC-MS: (ESI, m/z): [M+H] ⁺= 365.2.

### Step 3: preparation of 1-(2-chloro-5-(4-(2-(methylamino)ethyl)piperidine-1-carbonyl)phenyl) dihydropyrimidine-2,4(1H,3H)-dione

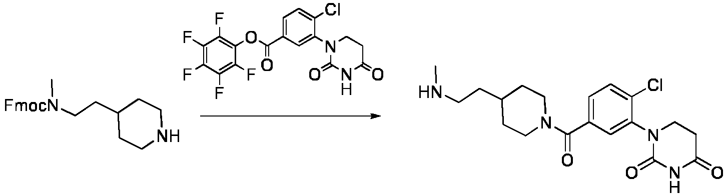

(9*H*-Fluoren-9-yl)methyl methyl(2-(piperidin-4-yl)ethyl)carbamate (210 mg, 0.45 mmol) was dissolved in DMSO (5 mL). DIEA (264 mg, 2.05 mmol) and pentafluorophenyl 4-chloro-3-(2,4-dioxotetrahydropyrimidin-1(*2H*)-yl)benzoate (178 mg, 0.41 mmol) were added, and the mixture was reacted at room temperature overnight. The reaction solution was purified by reversed-phase chromatography (45% ACN/0.1% aqueous NH₄HCO₃ solution) to give 1-(2-chloro-5-(4-(2-(methylamino)ethyl)piperidine-1-carbonyl)phenyl) dihydropyrimidine-2,4(1*H*,3*H*)-dione.

LC-MS: (ESI, m/z): [M+H] ⁺= 393.1.

### Step 4: preparation of tert-butyl ((S)-1-(3-((1-((1R,4S)-4-(((2-(1-(4-chloro-3-(2,4-dioxotetrahydropyrimidin-1(2H)-y)benzoyl)piperidin-4-yl)ethyl)(methyl)amino)methyl)cyclohexyl)-3-(difluoromethyl)-1H-pyrazol-4-yl)carbamoyl)pyrazolo[1,5-a]pyrimidin-5-yl)piperidin-3-yl)carbamate

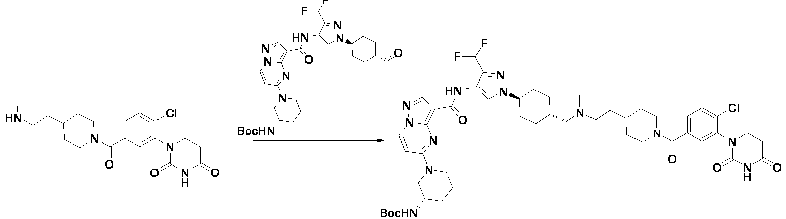

1-(2-Chloro-5-(4-(2-(methylamino)ethyl)piperidine-1-carbonyl)phenyl)dihydropyrimidine-2,4(1*H*,3*H*)-dione (50 mg, 0.13 mmol) was dissolved in THF (10 mL). *tert-*Butyl ((*S*)-1-(3-((3-(difluoromethyl)-1-((1*R*,4*S*)-4-formyleyclohexyl)-1*H*-pyrazol-4-yl)carbamoyl)pyrazolo[1,5-*a*]pyrimidin-5-yl)piperidin-3-yl)carbamate (30 mg, 0.05 mmol) and STAB (83 mg, 0.39 mmol) were added. After the mixture was reacted for 0.5 h, *tert*-butyl ((*S*)-1-(3-((3-(difluoromethyl)-1-((1*R*,4*S*)-4-formylcyclohexyl)-1*H*-pyrazol-4-yl)carbamoyl)pyrazolo[1,5-*a*]pyrimidin-5-yl)piperidin-3-yl)carbamate (60 mg, 0.10 mmol) was added in two portions within 1 h, and the mixture was reacted at room temperature for 2 h. The reaction liquid was diluted with water (50 mL), and the mixture was extracted with ethyl acetate (50 mL × 3). The organic phase was washed with saturated brine (100 mL), dried over anhydrous sodium sulfate, filtered, and concentrated under reduced pressure. The crude product was purified by column chromatography (MeOH:DCM = 0-1:9) to give *tert-*butyl ((*S*)-1-(3-((1-((1*R*,4*S*)-4-(((2-(1-(4-chloro-3-(2,4-dioxotetrahydropyrimidin-1(2*H*)-yl)benzoyl)piperidin-4-yl)ethyl)(methyl)amino) methyl)cyclohexyl)-3-(difluoromethyl)-1*H*-pyrazol-4-yl)carbamoyl)pyrazolo[1,5-*a*]pyrimidin-5-yl)piperidin-3-yl)carbamate.

LC-MS: (ESI, m/z): [M+H] ⁺= 963.3.

### Step 5: preparation of 5-((S)-3-aminopiperidin-1-yl)-N-(1-((1R,4S)-4-(((2-(1-(4-chloro-3-(2,4-dioxotetrahydropyrimidin-1(2H)-yl)benzoyl)piperidin-4-yl)ethyl)(methyl)amino)methyl)cyclohexyl)-3-(difluoromethyl)-1H-pyrazol-4-yl)pyrazolo[1,5-a]pyrimidine-3-carboxamide

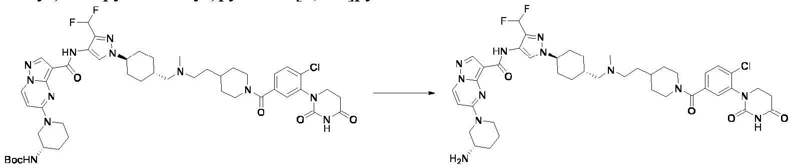

*tert-*Butyl ((*S*)-1-(3-((1-((1*R*,4*S*)-4-(((2-(1-(4-chloro-3-(2,4-dioxotetrahydropyrimidin-1(2*H*)-yl)benzoyl) pipendin-4-yl)ethyl)(methyl)amino)methyl)cyclohexyl)-3-(difluoromethyl)-1*H*-pyrazol-4-yl)carbamoyl) pyrazolo[1,5-*a*]pyrimidin-5-yl)piperidin-3-yl)carbamate (100 mg, 0.10 mmol) was dissolved in DCM (5 mL). TFA (1 mL) was added, and the mixture was reacted at room temperature for 1 h. The reaction liquid was directly concentrated under reduced pressure. Then a saturated NaHCO₃ solution was added to adjust the pH to 8-9, and the mixture was extracted with MeOH/DCM (10%, 20 mL × 3). The organic phase was dried over anhydrous sodium sulfate, filtered, and concentrated. The crude product was purified by preparative reversed-phase chromatography to give 5-((*S*)-3-aminopiperidin-1-yl)-*N*-(1-((1*R*,4*S*)-4-(((2-(1-(4-chloro-3-(2,4-dioxotetrahydropyrimidin-1(2*H*)-yl)benzoyl)piperidin-4-yl)ethyl)(methyl)amino)methyl)cyclohexyl)-3-(difluoromethyl)-1*H*-pyrazol-4-yl)pyrazolo[1,5-*a*]pyrimidine-3-carboxamide.

LC-MS: (ESI, m/z): [M+H] ⁺= 863.1.

¹H NMR (400 MHz, DMSO-*d*₆) δ 11.18 - 9.87 (s, 1H), 9.36 (s, 1H), 8.74 (d, *J* = 8.0 Hz, 1H), 8.37 (s, 1H), 8.24 (s, 1H), 7.63 (d, *J =* 8.2 Hz, 1H), 7.54 (d, *J =* 2.0 Hz, 1H), 7.38 (dd, *J* = 8.2, 2.0 Hz, 1H), 7.26-6.97 (m, 1H), 6.87 (d, *J* = 8.0 Hz, 1H), 4.50-4.25 (m, 2H), 4.18 (t, *J =* 11.9 Hz, 2H), 3.80-3.70 (m, 1H), 3.70-3.51 (m, 2H), 3.26 - 3.18 (m, 1H), 3.08-2.97 (m, 2H), 2.85 - 2.64 (m, 5H), 2.30 (t, *J =* 7.2 Hz, 2H), 2.14-2.07 (m, 5H), 2.07-1.97 (m, 2H), 1.95-1.83 (m, 3H), 1.80-1.30 (m, 12H), 1.18 - 0.95 (m, 4H).

### Example 19: N-(1-((1R,4R)-4-((9-(4-chloro-3-(2,4-dioxotetrahydropyrimidin-1(2H)-yl)benzoyl)-3,9-diazaspiro[5.5]undecan-3-yl)methyl)cyclohexyl)-3-(difluoromethyl)-1H-pyrazol-4-yl)-5-(2-oxa-6-azaspiro[3.3]heptan-6-yl)pyrazolo[1,5-a]pyrimidine-3-carboxamide

### Step 1: preparation of tert-butyl 9-(4-chloro-3-(2,4-dioxotetrahydropyrimidin-1(2H)-yl)benzoyl)-3,9-diazaspiro [5.5]undecane-3-carboxylate

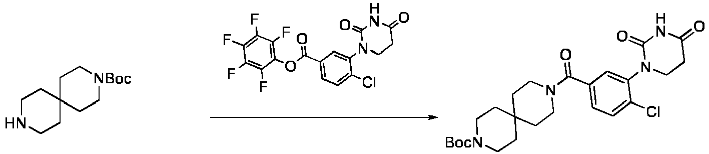

*tert-*Butyl 3,9-diazaspiro[5.5]undecane-3-carboxylate (100 mg, 0.39 mmol) was dissolved in dimethyl sulfoxide (3 mL). Pentafluorophenyl 4-chloro-3-(2,4-dioxotetrahydropyrimidin-1(2*H*)-yl)benzoate (205 mg, 0.47 mmol) and *N,N*-diisopropylethylamine (153 mg, 1.18 mmol) were added with stirring, and the mixture was stirred at room temperature overnight. Water (50 mL) was added to the reaction liquid, and the mixture was extracted with ethyl acetate (30 mL × 3). The organic phase was washed with saturated brine (100 mL), dried over anhydrous sodium sulfate, filtered, and concentrated under reduced pressure. The crude product was purified by column chromatography (ethyl acetate) to give *tert-*butyl 9-(4-chloro-3-(2,4-dioxotetrahydropyrimidin-1(*2H*)-yl)benzoyl)-3,9-diazaspiro[5.5]undecane-3-carboxylate.

LC-MS: (ESI, m/z): [M-Boc] ⁺= 405.1.

### Step 2: preparation of 1-(2-chloro-5-(3,9-diazaspiro[5.5]undecane-3-carbonyl)phenyl)dihydropyrimidine-2,4(1H,3H)-dione

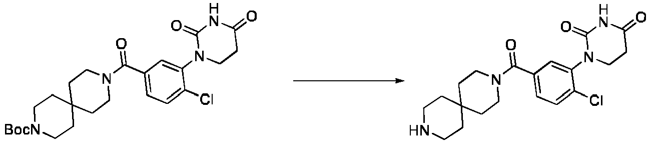

*tert-*Butyl 9-(4-chloro-3-(2,4-dioxotetrahydropyrimidin-1(2*H*)-yl)benzoyl)-3,9-diazaspiro[5.5]undecane-3-carboxylate (170 mg, 0.34 mmol) was dissolved in dichloromethane (2.5 mL). Trifluoroacetic acid (0.5 mL) was added, and the mixture was stirred at room temperature for 2 h. The reaction liquid was directly concentrated under reduced pressure to give 1-(2-chloro-5-(3,9-diazaspiro[5.5]undecane-3-carbonyl)phenyl)dihydropyrimidine-2,4(1*H*,3*H*)-dione, which was directly used in the next step without purification.

LC-MS: (ESI, m/z): [M+H] ⁺= 405.1.

### Step 3: preparation of N-(1-((1R,4R)-4-((9-(4-chloro-3-(2,4-dioxotetrahydropyrimidin-1(2H)-yl)benzoyl)-3,9-diazaspiro[5.5]undecan-3-yl)methyl)cyclohexyl)-3-(difluoromethyl)-1H-pyrazol-4-yl)-5-(2-oxa-6-azaspiro [3.3]heptan-6-yl)pyrazolo[1,5-a]pyrimidine-3-carboxamide

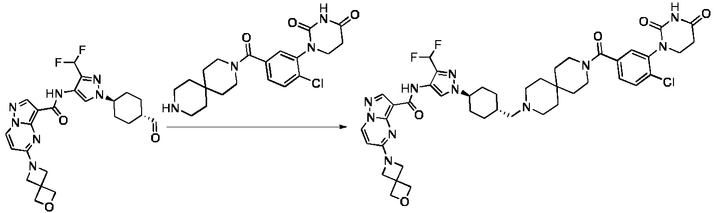

*N*-(3-(Difluoromethyl)-1-((1*R*,4*R*)-4-fortnylcyclohexyl)-1*H*-pyrazol-4-yl)-5-(2-oxa-6-azaspiro[3.3]heptan-6-yl)pyrazolo[1,5-a]pyrimidine-3-carboxamide (50 mg, 0.10 mmol) and 1-(2-chloro-5-(3,9-diazaspiro[5.5]undecane-3-carbonyl)phenyl)dihydropyrimidine-2,4(1*H*,3*H*)-dione (42 mg, 0.10 mmol) were dissolved in dichloroethane (10 mL). Sodium triacetoxyborohydride (44 mg, 0.20 mmol) was added, and the mixture was heated to 70 °C and stirred overnight. Water (50 mL) was added to the reaction liquid, and the mixture was extracted with dichloromethane (20 mL × 3), dried over anhydrous sodium sulfate, filtered, and concentrated under reduced pressure. The crude product was purified by pre-HPLC (CH₃CN/O.08% aqueous NH₄HCO₃ solution, 5%-95%) to give *N*-(1-((1*R*,4*R*)-4-((9-(4-chloro-3-(2,4-dioxotetrahydropyrimidin-1(2*H*)-yl)benzoyl)-3,9-diazaspiro[5.5]undecan-3-yl)methyl)cyclohexyl)-3-(difluoromethyl)-1*H*-pyrazol-4-yl)-5-(2-oxa-6-azaspiro[3.3]heptan-6-yl)pyrazolo[1,5-*a*]pyrimidine-3-carboxamide.

LC-MS: (ESI, m/z): [M+H] ⁺= 874.2.

¹H NMR (400 MHz, CDCl₃) δ 9.76 (s, 1H), 8.44 (s, 1H), 8.39 (d, *J* = 5.3 Hz, 2H), 8.30 (d, *J* = 7.6 Hz, 1H), 7.58-7.48 (m, 2H), 7.43 (d, *J* = 1.8 Hz, 1H), 7.38 - 7.34 (m, 1H), 6.97-6.65 (m, 1H), 6.02 (d, *J* = 7.5 Hz, 1H), 4.98-4.85 (m, 4H), 4.47-4.37 (m, 4H), 4.13-4.06 (m, 1H), 3.86-3.74 (m, 4H), 3.46-3.38 (m, 2H), 3.00-2.74 (m, 3H), 2.39-2.29 (m, 3H), 2.29-2.10 (m, 3H), 2.10-2.00 (m, 2H), 1.92-1.76 (m, 3H), 1.40-1.10 (m, 10H), 0.95-0.85 (m, 1H).

### Example 20: N-(1-(1-(2-(3-(4-chloro-3-(2,4-dioxotetrahydropyrimidin-1(2H)-yl)benzoyl)-3-azaspiro[5.5] undecan-9-yl)ethyl)piperidin-4-yl)-3-(difluoromethyl)-1H-pyrazol-4-yl)-5-morpholinopyrazolo[1,5-a]pyrimidine-3-carboxamide

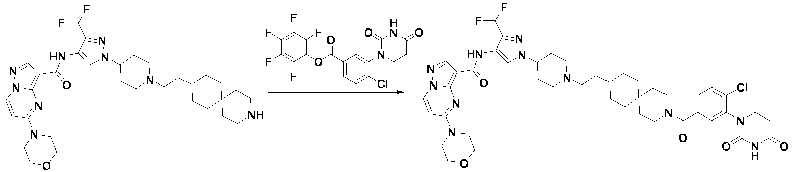

*N*-(1-(1-(2-(3-Azaspiro[5.5]undecan-9-yl)ethyl)piperidin-4-yl)-3-(difluoromethyl)-1*H*-pyrazol-4-yl)-5-morpholinopyrazolo[1,5-*a*]pyrimidine-3-carboxamide (23 mg, 0.03 mmol) was dissolved in dimethyl sulfoxide (3 mL). Pentafluorophenyl 4-chloro-3-(2,4-dioxotetrahydropyrimidin-1(2*H*)-yl)benzoate (14 mg, 0.03 mmol) and *N,N*-diisopropylethylamine (38 mg, 0.30 mmol) were added with stirring, and the mixture was stirred at room temperature for 2 h. Water (20 mL) was added to the reaction liquid. The mixture was filtered, and the filter cake was purified by p-TLC (methanol:dichloromethane = 1:9) to give *N*-(1-(1-(2-(3-(4-chloro-3-(2,4-dioxotetrahydropyrimidin-1(2*H*)-yl)benzoyl)-3-azaspiro[5.5]undecan-9-yl)ethyl)piperidin-4-yl)-3-(difluoromethyl)-1*H*-pyrazol-4-yl)-5-morpholinopyrazolo[1,5-*a*]pyrimidine-3-carboxamide.

LC-MS: (ESI, m/z): [M+H] ⁺= 876.3.

¹H NMR (400 MHz, DMSO-*d*₆) δ 10.51 (s, 1H), 9.40 (s, 1H), 8.83 (d, *J* = 7.9 Hz, 1H), 8.39 (s, 1H), 8.29 (s, 1H), 7.63 (d, *J =* 8.2 Hz, 1H), 7.55 (d, *J* = 1.9 Hz, 1H), 7.42 - 7.35 (m, 1H), 7.26-6.97 (m, 1H), 6.91 (d, *J=* 8.0 Hz, 1H), 4.31-4.11 (m, 1H), 3.86-3.66 (m, 9H), 3.61-3.50 (m, 3H), 3.29 - 3.24 (m, 1H), 2.99-2.90 (m, 2H), 2.80 - 2.71 (m, 2H), 2.43-2.30 (m, 2H), 2.15-1.84 (m, 6H), 1.73-1.60 (m, 2H), 1.55-1.23 (m, 10H), 1.16-1.01 (m, 4H).

### Example 21: 5-((1R,4R)-2-oxa-5-azabicyclo[2.2.1]heptan-5-yl)-N-(3-(difluoromethyl)-1-(1-((3-(3-(2,4-dioxotetrahydropyrimidin-1(2H)-yl)-4-methylbenzoyl)-3-azaspiro[5.5]undecan-9-yl)methyl)piperidin-4-yl)-1H-pyrazol-4-yl)pyrazolo [1,5-a]pyrimidine-3-carboxamide

### Step 1: preparation of tert-butyl 9-((4-(4-(5-((1R,4R)-2-oxa-5-azabicyclo[2.2.1]heptan-5-yl)pyrazolo[1,5-a]pyrimidine-3-carboxamido)-3-(difluoromethyl)-1H-pyrazol-1-yl)piperidin-1-yl)methyl)-3-azaspiro[5.5]undecane-3-carboxylate

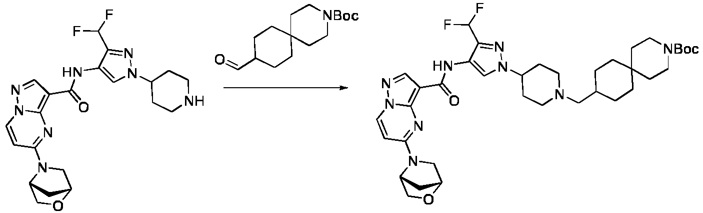

5-((1*R*,4*R*)-2-Oxa-5-azabicyclo[2.2.1]heptan-5-yl)-*N*-(3-(difluoromethyl)-1-(piperidin-4-yl)-1*H*-pyrazol-4-yl)pyrazolo[1,5-*a*]pyrimidine-3-carboxamide (100 mg, crude) was dissolved in THF (10 mL). *tert*-Butyl 9-formyl-3-azaspiro[5.5]undecane-3-carboxylate (50 mg, 0.18 mmol) and STAB (115 mg, 0.54 mmol) were added, and the mixture was reacted at room temperature for 2 h. The reaction liquid was diluted with water (50 mL), and the mixture was extracted with ethyl acetate (50 mL × 3). The organic phase was washed with saturated brine (100 mL), dried over anhydrous sodium sulfate, filtered, and concentrated under reduced pressure. The crude product was purified by column chromatography (MeOH:DCM = 0-1:9) to give *tert-*butyl 9-((4-(4-(5-((1*R*,4*R*)-2-oxa-5-azabicyclo[2.2.1]heptan-5-yl)pyrazolo[1,5-*a*]pyrimidine-3-carboxamido)-3-(difluoromethyl)-1*H*-pyrazol-1-yl)piperidin-1-yl)methyl)-3-azaspiro[5.5]undecane-3-carboxylate.

LC-MS: (ESI, m/z): [M+H] ⁺= 724.3.

### Step 2: preparation of N-(1-(1-((3-azaspiro[3.5undecan-9-yl)methyl)piperidin-4-yl)-3-(difluoromethyl)-1H-pyrazol-4-yl)-5-((1R,4R)-2-oxa-5-azabicyclo[2.2.1]heptan-5-yl)pyrazolo[1,5-a]pyrimidine-3-carboxamide

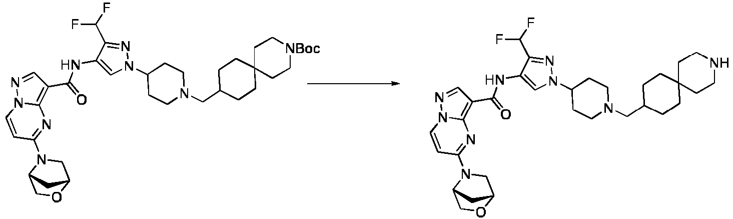

*tert-*Butyl 9-((4-(4-(5-((1*R*,4*R*)-2-oxa-5-azabicyclo[2.2.1]heptan-5-yl)pyrazolo[1,5-*a*]pyrimidine-3-carboxamido)-3-(difluoromethyl)-1*H*-pyrazol-1-yl)piperidin-1-yl)methyl)-3-azaspiro[5.5]undecane-3-carboxylate (100 mg, 0.14 mmol) was dissolved in DCM (4 mL). TFA (1 mL) was added, and the mixture was reacted at room temperature for 1 h. The reaction liquid was directly concentrated under reduced pressure to give *N*-(1-(1-((3-azaspiro[5.5]undecan-9-yl)methyl)piperidin-4-yl)-3-(difluoromethyl)-1*H*-pyrazol-4-yl)-5-((1*R*,4*R*)-2-oxa-5-azabicyclo[2.2.1]heptan-5-yl)pyrazolo[1,5-*a*]pyrimidine-3-carboxamide, which was directly used in the next step.

LC-MS: (ESI, m/z): [M+H] ⁺= 624.4.

### Step 3: preparation of 5-((1R,4R)-2-oxa-5-azabicyclo[2.2.1]heptan-5-yl)-N-(3-(difluoromethyl)-1-(1-((3-(3-(2,4-dioxotetrahydropyrimidin-1(2H)-yl)-4-methylbenzoyl)-3-azaspiro[5.5]undecan-9-yl)methyl)piperidin-4-yl)-1H-pyrazol-4-yl)pyrazolo[1,5-α]pyrimidine-3-carboxamide

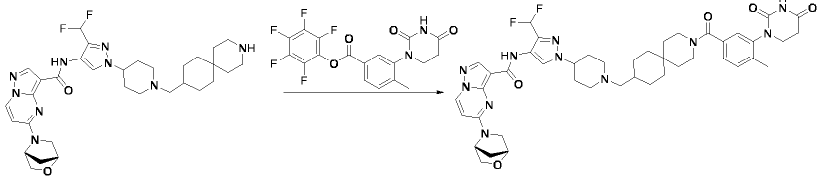

*N*-(1-(1-((3-Azaspiro[5.5]undecan-9-y)methyl)piperidin-4-yl)-3-(difluoromethyl)-1*H*-pyrazol-4-yl)-5-((1*R*,4*R*)-2-oxa-5-azabicyclo[2.2.1]heptan-5-yl)pyrazolo[1,5-*a*]pyrimidine-3-carboxamide (100 mg, 0.14 mmol) was dissolved in DMSO (10 mL). DIEA (90 mg, 0.70 mmol) and pentafluorophenyl 3-(2,4-dioxotetrahydropyrimidin-1(2*H*)-yl)-4-methylbenzoate (58 mg, 0.14 mmol) were added, and the mixture was reacted at room temperature for 2 h. Water (100 mL) was added to the reaction liquid, and the mixture was stirred for 5 min and extracted with ethyl acetate (100 mL × 3). The organic phase was washed with saturated brine (150 mL), dried over anhydrous sodium sulfate, filtered, and concentrated under reduced pressure. The crude product was purified by preparative reversed-phase chromatography to give 5-((1*R*,4*R*)-2-oxa-5-azabicyclo[2.2.1]heptan-5-yl)-*N*-(3-(diBuoromethyl)-1-(1-((3-(3-(2,4-dioxotetrahydropyrimidin-1(2*H*)-yl)-4-methylbenzoyl)-3-azaspiro[5.5]undecan-9-yl)methyl)piperidin-4-yl)-1*H*-pyrazol-4-yl)pyrazolo[1,5-*a*]pyrimidine-3-carboxamide.

LC-MS: (ESI, m/z): [M+H] ⁺= 854.4.

¹H NMR (400 MHz, DMSO-*d*₆) δ 10.37 (s, 1H), 9.50 (d, *J* = 5.7 Hz, 1H), 8.78 (d, *J* = 7.6 Hz, 1H), 8.39 (d, *J* = 3.6 Hz, 1H), 8.26 (d, *J =* 5.6 Hz, 1H), 7.36 - 7.29 (m, 2H), 7.26 - 6.96 (m, 2H), 6.88-6.44 (m, 1H), 5.28-5.07 (m, 1H), 4.77 (d, *J* = 17.6 Hz, 1H), 4.31-4.11 (m, 1H), 3.88 - 3.71 (m, 3H), 3.63-3.45 (m, 5H), 2.95 - 2.85 (m, 2H), 2.81 - 2.65 (m, 2H), 2.25 -2.10 (m, 5H), 2.07 - 1.87 (m, 8H).

### Example 22: preparation of N-(3-(difluoromethyl)-1-(1-((3-(3-(2,4-dioxotetrahydropyrimidin-1(2H)-yl)-4-methylbenzoyl)-3-azaspiro[5.5]undecan-9-yl)methyl)piperidin-4-yl)-1H-pyrazol-4-yl)-5-(1,1-dioxothiomorpholinyl)pyrazolo[1,5-a]pyrimidine-3-carboxamide

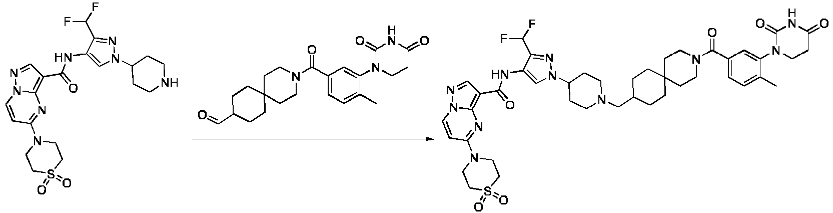

*N*-(3-(Difluoromethyl)-1-(piperidin-4-yl)-1*H*-pyrazol-4-yl)-5-(1,1-dioxothiomorpholinyl)pyrazolo[1,5-*a*]pyrimidine-3-carboxamide (60 mg, 0.1 mmol) and 3-(3-(2,4-dioxotetrahydropyrimidin-1(2*H*)-yl)-4-methylbenzoyl)-3-azaspiro[5.5]undecane-9-carbaldehyde (41 mg, 0.1 mmol) were dissolved in tetrahydrofuran (2 mL). Sodium triacetoxyborohydride (84 mg, 0.4 mmol) was added with stirring, and the mixture was stirred at room temperature for 3 h. The reaction liquid was diluted with water (50 mL), and the mixture was extracted with ethyl acetate (50 mL × 3). The organic phase was washed with saturated brine (100 mL), dried over anhydrous sodium sulfate, filtered, and concentrated under reduced pressure. The crude product was purified by pre-HPLC (CH₃CN/O.08% aqueous NH₄HCO₃ solution, 5%-95%) to give *N*-(3-(difluoromethyl)-1-(1-((3-(3-(2,4-dioxotetrahydropyrimidin-1(2*H*)-yl)-4-methylbenzoyl)-3-azaspiro[5.5]undecan-9-yl)methyl)piperidin-4-yl)-1*H*-pyrazol-4-yl)-5-(1,1-dioxothiomorpholinyl)pyrazolo[1,5-*a*]pyrimidine-3-carboxamide.
LC-MS: (ESI, m/z): [M+H] ⁺= 890.1
¹H NMR (400 MHz, CD₃OD-*d*₄) δ 8.64 (d, J = 7.9 Hz, 1H), 8.37 (d, *J =* 5.8 Hz, 2H), 7.40 (d, *J* = 7.8 Hz, 1H), 7.36-7.29 (m, 2H), 7.06-6.77 (m, 2H), 4.60-4.52 (m, 2H), 4.42-4.34 (m, 4H), 4.25-4.14 (m, 1H), 3.92-3.82 (m, 1H), 3.77-3.62 (m, 3H), 3.49-3.40 (m, 2H), 3.30-3.22 (m, 4H), 3.06-3.00 (m, 2H), 2.92-2.82 (m, 2H), 2.31 (s, 3H), 2.28-2.22 (m, 2H), 2.20-2.06 (m, 6H), 1.82-1.72 (m, 2H), 1.70-1.63 (m, 2H), 1.55-1.39 (m, 2H), 1.33-1.25 (m, 2H), 1.24-1.12 (m, 3H).

### Example 23: (S)-5-(3-aminopiperidin-1-yl)-N-(3-(difluoromethyl)-1-(1-((3-(3-(2,4-dioxotetrahydropyrimidin-1(2H)-yl)-4-methylbenzoyl)-3-azaspiro[5.5]undecan-9-yl)methyl)piperidin-4-yl)-1H-pyrazol-4-yl)pyrazolo [1,5-a]pyrimidine-3-carboxamide

### Step 1: preparation of tert-butyl (S)-(1-(3-((3-(difluoromethyl)-1-(1-((3-(3-(2,4-dioxotetrahydropyrimidin-1(2H)-yl)-4-methylbenzoyl)-3-azaspiro[5.5]undecan-9-yl)methyl)piperidin-4-yl)-1H-pyrazol-4-yl)carbamoyl) pyrazolo[1,5-a]pyrimidin-5-yl)piperidin-3-yl)carboxylate

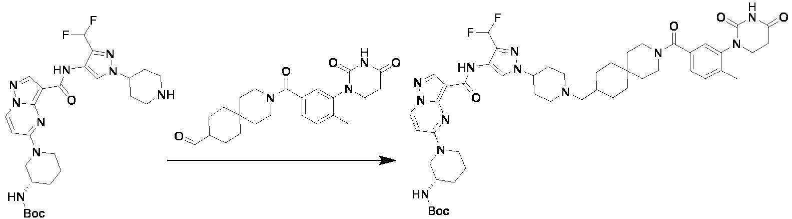

*tert*-Butyl (*S*)-(1-(3-((3-(difluoromethyl)-1-(piperidin-4-yl)-1*H*-pyrazol-4-yl)carbamoyl)pyrazolo[1,5-*a*]pyrimidin-5-yl)piperidin-3-yl)carbamate (45 mg, 0.08 mmol) and 3-(3-(2,4-dioxotetrahydropyrimidin-1(2*H*)-yl)-4-methylbenzoyl)-3-azaspiro[5.5]undecane-9-carbaldehyde (33 mg, 0.08 mmol) were dissolved in DMF (2 mL). NaBH(OAc)₃ (51 mg, 0.24 mmol) was added, and the mixture was stirred at room temperature for 2 h. Water (50 mL) was added to the reaction liquid, and the mixture was extracted with ethyl acetate (30 mL × 3). The upper organic phases were combined, washed with saturated brine (50 mL), dried over anhydrous sodium sulfate, filtered, and concentrated under reduced pressure. The crude product was purified by normal phase column chromatography to give *tert*-butyl (*S*)-(1-(3-((3-(difluoromethyl)-1-(1-((3-(3-(2,4-dioxotetrahydropyrimidin-1(2*H*)-yl)-4-methylbenzoyl)-3-azaspiro[5.5]undecan-9-yl)methyl)piperidin-4-yl)-1*H*-pyrazol-4-yl)carbatnoyl)pyrazolo[1,5-*a*]pyrimidin-5-yl)piperidin-3-yl)carboxylate.

LC-MS: (ESI, m/z): [M+H] ⁺= 955.5.

### Step 2: preparation of (S)-5-(3-aminopiperidin-1-yl)-N-(3-(difluoromethyl)-1-(1-((3-(3-(2,4-dioxotetrahydropyrimidin-1(2H)-yl)-4-methylbenzoyl)-3-azaspiro[5.5]undecan-9-yl)methyl)piperidin-4-yl)-1H-pyrazol-4-yl)pyrazolo[1,5-a]pyrimidine-3-carboxamide

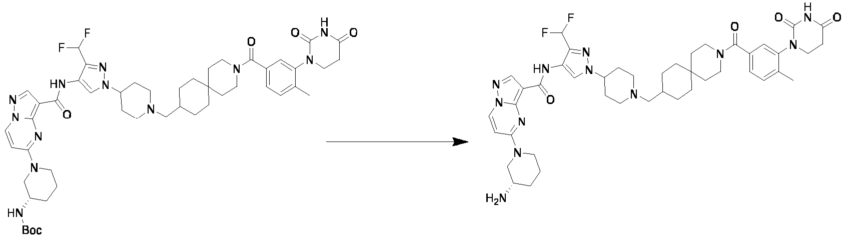

*tert*-Butyl (*S*)-(1-(3-((3-(difluoromethyl)-1-(1-((3-(3-(2,4-dioxotetrahydropyrimidin-1(2*H*)-yl)-4-methylbenzoyl)-3-azaspiro[5.5]undecan-9-yl)methyl)piperidin-4-yl)-1*H*-pyrazol-4-yl)carbamoyl)pyrazolo[1,5-*a*]pyrimidin-5-yl)piperidin-3-yl)carboxylate (30 mg, 0.03 mmol) was dissolved in DCM (0.5 mL). Then TFA (0.1 mL) was added, and the mixture was stirred at room temperature for 1 h. A saturated aqueous NaHCO₃ solution was added to the reaction liquid until the pH of the solution became slightly basic, and the mixture was extracted with a mixed organic solvent (MeOH:DCM = 1:10, 30 mL × 3), dried over anhydrous sodium sulfate, filtered, and concentrated under reduced pressure. The crude product was purified by p-HPLC to give (*S*)-5-(3-aminopiperidin-1-yl)-*N*-(3-(difluoromethyl)-1-(1-((3-(3-(2,4-dioxotetrahydropyrimidin-1(2*H*)-y)-4-methylbenzoyl)-3-azaspiro[5.5]undecan-9-yl)methyl)piperidin-4-yl)-1*H*-pyrazol-4-yl)pyrazolo[1,5-*a*]pyrimidine-3-carboxamide.

LC-MS: (ESI, m/z): [M+H] ⁺= 855.7.

¹H NMR (400 MHz, MeOD) δ 8.49 (d, *J* = 8.0 Hz, 1H), 8.35 (s, 1H), 8.29 (s, 1H), 7.40 (d, *J* = 7.9 Hz, 1H), 7.35 - 7.29 (m, 2H), 7.04 - 6.74 (m, 2H), 4.60-4.50 (m, 1H), 4.50-4.25 (m, 2H), 4.25-4.15 (m, 1H), 3.97-3.79 (m, 1H), 3.80-3.63 (m, 3H), 3.50-3.38 (m, 3H), 3.15-3.08 (m, 1H), 3.06-3.01 (m, 2H), 2.94 - 2.79 (m, 3H), 2.31 (s, 3H), 2.27-2.21 (m, 2H), 2.21-2.04 (m, 7H), 1.93-1.83 (m, 1H), 1.83-1.76 (m, 2H), 1.70-1.44 (m, 8H), 1.22-1.02 (m, 4H).

### Example 24: N-(3-(difluoromethyl)-1-(1-((3-(3-(2,4-dioxotetrahydropyrimidin-1(2H)-yl)-4-methylbenzoyl)-3-azaspiro[5.5]undecan-9-yl)methyl)piperidin-4-yl)-1H-pyrazol-4-yl)-5-(2-oxa-6-azaspiro[3.3]heptan-6-yl)pyrazolo[1,5-a]pyrimidine-3-carboxamide

### Step 1: preparation of tert-butyl 4-(4-(5-(2-oxa-6-azaspiro[3.3]heptan-6-yl)pyrazolo[1,5-a]pyrimidine-3-carboxamido)-3-(difluoromethyl)-1H-pyrazol-1-yl)piperidine-1-carboxylate

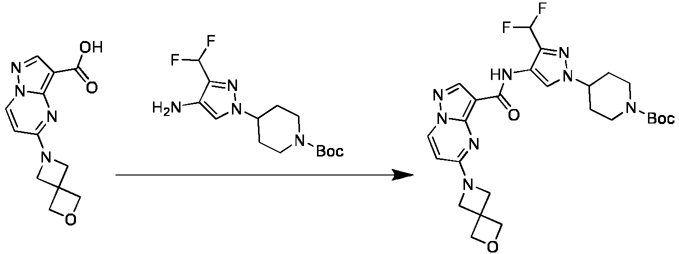

5-(2-Oxa-6-azaspiro[3.3]heptan-6-yl)pyrazolo[1,5-*a*]pyrimidine-3-carboxylic acid (130 mg, 0.5 mmol), *tert-*butyl 4-(4-amino-3-(difluoromethyl)-1*H*-pyrazol-1-yl)piperidine-1-carboxylate (158 mg, 0.5 mmol), and Py-BOP (780 mg, 1.5 mmol) were dissolved in DMF (6 mL), then DIEA (780 mg, 1.5 mmol) was added, and the mixture was stirred at room temperature for 16 h. Water (50 mL) was added to the reaction liquid, and the mixture was extracted with ethyl acetate (30 mL × 3). The upper organic phases were combined, washed with saturated brine (50 mL), dried over anhydrous sodium sulfate, filtered, and concentrated under reduced pressure. The crude product was purified by normal phase column chromatography to give *tert*-butyl 4-(4-(5-(2-oxa-6-azaspiro[3.3]heptan-6-yl)pyrazolo[1,5-*a*]pyrimidine-3-carboxamido)-3-(difluoromethyl)-1*H-*pyrazol-1-yl)piperidine-1-carboxylate.

LC-MS: (ESI, m/z): [M+H] ⁺= 559.2.

### Step 2: preparation of N-(3-(difluoromethyl)-1-(piperidin-4-yl)-1H-pyrazol-4-yl)-5-(2-oxa-6-azaspiro[3.3]heptan-6-yl)pyrazolo[1,5-a]pyrimidine-3-carboxamide

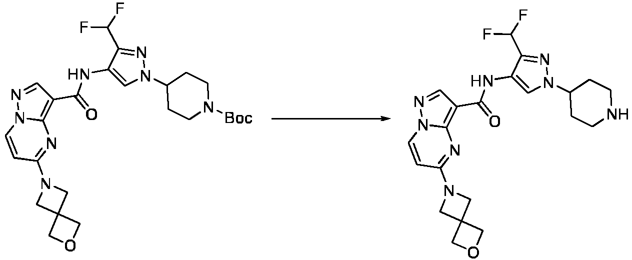

*tert-*Butyl 4-(4-(5-(2-oxa-6-azaspiro[3.3]heptan-6-yl)pyrazolo[1,5-*a*]pyrimidine-3-carboxamido)-3-(difluoromethyl)-1*H*-pyrazol-1-yl)piperidine-1-carboxylate (250 mg, 0.45 mmol) was dissolved in DCM (4 mL). Then TFA (1 mL) was added, and the mixture was stirred at room temperature for 1 h. The reaction liquid was directly concentrated under reduced pressure to give a crude product of *N*-(3-(difluoromethyl)-1-(piperidin-4-yl)-1*H*-pyrazol-4-yl)-5-(2-oxa-6-azaspiro[3.3]heptan-6-yl)pyrazolo[1,5-*a*]pyrimidine-3-carboxamide.

LC-MS: (ESI, m/z): [M+H] ⁺= 459.3.

### Step 3: preparation of N-(3-(difluoromethyl)-1-(1-((3-(3-(2,4-dioxotetrahydropyrimidin-1(2H)-yl)-4-methylbenzoyl)-3-azaspiro[5.5]undecan-9-yl)methyl)piperidin-4-yl)-1H-pyrazol-4-yl)-5-(2-oxa-6-azaspiro [3.3]heptan-6-yl)pyrazolo[1,5-a]pyrimidine-3-carboxamide

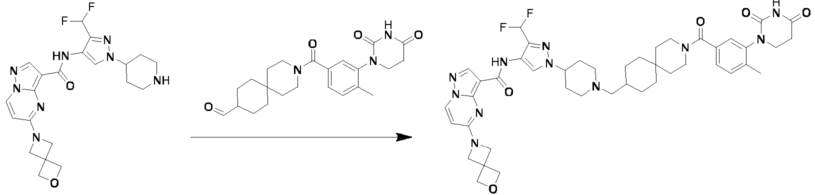

*N*-(3-(Difluoromethyl)-1-(piperidin-4-yl)-1*H*-pyrazol-4-yl)-5-(2-oxa-6-azaspiro[3.3]heptan-6-yl)pyrazolo [1,5-*a*]pyrimidine-3-carboxamide (114 mg, 0.25 mmol) and 3-(3-(2,4-dioxotetrahydropyrimidin-1(2*H*)-yl)-4-methylbenzoyl)-3-azaspiro[5.5]undecane-9-carbaldehyde (102 mg, 0.25 mmol) were dissolved in THF (5 mL). Sodium triacetoxyborohydride (160 mg, 0.75 mmol) was added, and the mixture was stirred at room temperature for 16 h. Water (50 mL) was added to the reaction liquid, and the mixture was extracted with ethyl acetate (30 mL × 3). The upper organic phases were combined, washed with saturated brine (50 mL), dried over anhydrous sodium sulfate, filtered, and concentrated under reduced pressure. The crude product was purified by prep-HPLC to give *N-*(3-(difluoromethyl)-1-(1-((3-(3-(2,4-dioxotetrahydropyrimidin-1(2*H*)-yl)-4-methylbenzoyl)-3-azaspiro[5.5]undecan-9-yl)methyl)piperidin-4-yl)-1*H*-pyrazol-4-yl)-5-(2-oxa-6-azaspiro [3.3]heptan-6-yl)pyrazolo[1,5-*a*]pyrimidine-3-carboxamide as a white solid.

LC-MS: (ESI, m/z): [M+H] ⁺= 854.6.

¹H NMR (400 MHz, MeOD) δ 8.47 (d, *J* = 7.8 Hz, 1H), 8.35 (s, 1H), 8.27 (s, 1H), 7.40 (d, *J* = 7.4 Hz, 1H), 7.36-7.27 (m, 2H), 7.05-6.95 (m, 1H), 6.31 (d, *J* = 8.0 Hz, 1H), 4.62-4.50 (m, 2H), 4.50-4.39 (m, 4H), 4.26-4.11 (m, 1H), 3.95-3.82 (m, 1H), 3.77-3.60 (m, 3H), 3.49-3.40 (m, 2H), 3.05-2.97 (m, 2H), 2.89 - 2.79 (m, 2H), 2.37-2.20 (m, 5H), 2.20-2.09 (m, 6H), 1.83-1.75 (m, 2H), 1.71-1.41 (m, 7H), 1.37-1.27 (m, 2H), 1.24-1.08 (m, 4H).

### Example 25: (S)-N-(3-(difluoromethyl)-1-(1-((3-(3-(2,4-dioxotetrahydropyrimidin-1(2H)-yl)-4-methylbenzoyl)-3-azaspiro[5.5]undecan-9-yl)methyl)piperidin-4-yl)-1H-pyrazol-4-yl)-5-(3-hydroxypiperidin-1-yl)pyrazolo [1,5-a]pyrimidine-3-carboxamide

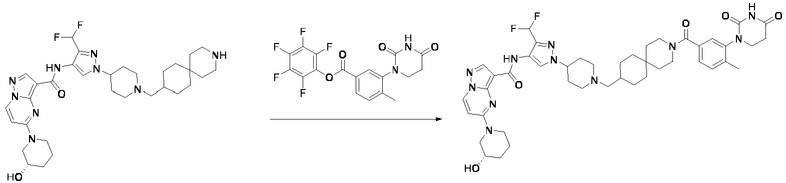

(*S*)-*N*-(1-(1-((3-Azaspiro[5.5]undecan-9-yl)methyl)piperidin-4-yl)-3-(difluoromethyl)-1*H*-pyrazol-4-yl)-5-(3-hydroxypiperidin-1-yl)pyrazolo[1,5-*a*]pyrimidine-3-carboxamide (80 mg, crude, 0.11 mmol) was dissolved in dimethyl sulfoxide (3 mL). Pentafluorophenyl 3-(2,4-dioxotetrahydropyrimidin-1(2*H*)-yl)-4-methylbenzoate (46 mg, 0.11 mmol) and *N,N*-diisopropylethylamine (142 mg, 1.1 mmol) were added with stirring, and the mixture was stirred at room temperature overnight. Water (50 mL) was added to the reaction liquid. The mixture was filtered, and the filter cake was purified by Pre-HPLC (CH₃CN/O.08% aqueous NH₄HCO₃ solution, 5%-95%) to give (*S*)-*N*-(3-(difluoromethyl)-1-(1-((3-(3-(2,4-dioxotetrahydropyrimidin-1(2*H*)-yl)-4-methylbenzoyl)-3-azaspiro[5.5]undecan-9-yl)methyl)piperidin-4-yl)-1*H*-pyrazol-4-yl)-5-(3-hydroxypiperidin-1-yl)pyrazolo [1,5-*a*]pyrimidine-3-carboxamide.

LC-MS: (ESI, m/z): [M+H] ⁺= 856.3.

¹H NMR (400 MHz, DMSO-*d*₆) δ 10.38 (s, 1H), 9.38 (s, 1H), 8.72 (d, *J* = 8.0 Hz, 1H), 8.38 (s, 1H), 8.24 (s, 1H), 7.37-7.29 (m, 2H), 7.26-6.96 (m, 2H), 6.86 (d, *J* = 8.0 Hz, 1H), 4.89 (d, *J* = 2.0 Hz, 1H), 4.26-4.14 (m, 1H), 4.00-3.75 (m, 3H), 3.70-3.39 (m, 6H), 3.30-3.24 (m, 2H), 2.93-2.64 (m, 4H), 2.21 (s, 3H), 2.18-2.10 (m, 2H), 2.06-1.65 (m, 10H), 1.61-1.22 (m, 9H), 1.16-0.95 (m, 4H).

### Example 26: (S)-N-(1-(1-((3-(4-chloro-3-(2,4-dioxotetrahydropyrimidin-1(2H)-yl)benzoyl)-3-azaspiro[5.5] undecan-9-yl)methyl)piperidin-4-yl)-3-(difluoromethyl)-1H-pyrazol-4-yl)-5-(3-hydroxypiperidin-1-yl)pyrazolo[1,5-a]pyrimidine-3-carboxamide

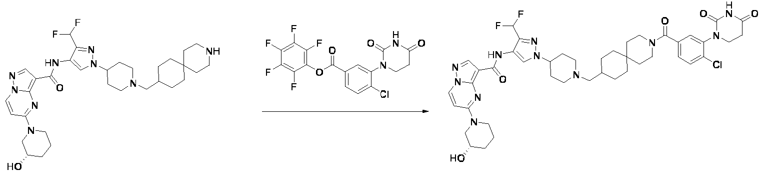

(*S*)-*N*-(1-(1-((3-Azaspiro[5.5]undecan-9-yl)methyl)piperidin-4-yl)-3-(difluoromethyl)-1*H*-pyrazol-4-yl)-5-(3-hydroxypiperidin-1-yl)pyrazolo[1,5-*a*]pyrimidine-3-carboxamide (80 mg, crude, 0.11 mmol) was dissolved in dimethyl sulfoxide (3 mL). Pentafluorophenyl 4-chloro-3-(2,4-dioxotetrahydropyrimidin-1(2*H*)-yl)benzoate (48 mg, 0.11 mmol) and *N,N*-diisopropylethylamine (142 mg, 1.1 mmol) were added with stirring, and the mixture was stirred at room temperature overnight. Water (30 mL) was added to the reaction liquid. The mixture was filtered, and the filter cake was purified by Pre-HPLC (CH₃CN/O.08% aqueous NH₄HCO₃ solution, 5%-95%) to give (*S*)-*N*-(1-(1-((3-(4-chloro-3-(2,4-dioxotetrahydropyrimidin-1(2*H*)-yl)-benzoyl)-3-azaspiro[5.5] undecan-9-yl)methyl)piperidin-4-yl)-3-(difluoromethyl)-1*H*-pyrazol-4-yl)-5-(3-hydroxypiperidin-1-yl)pyrazolo[1,5-*a*]pyrimidine-3-carboxamide.

LC-MS: (ESI, m/z): [M+H] ⁺= 876.3.

¹H NMR (400 MHz, DMSO-*d*₆) δ 10.51 (s, 1H), 9.38 (s, 1H), 8.72 (d, *J* = 7.9 Hz, 1H), 8.38 (s, 1H), 8.24 (s, 1H), 7.63 (d, *J =* 8.2 Hz, 1H), 7.55 (s, 1H), 7.39 (d, *J* = 8.5 Hz, 1H), 7.26-6.97 (m, 1H), 6.86 (d, *J* = 8.0 Hz, 1H), 4.88 (d, *J =* 2.0 Hz, 1H), 4.25-4.15 (m, 1H), 3.97-3.40 (m, 9H), 3.30-3.18 (m, 2H), 2.93-2.85 (m, 2H), 2.78-2.72 (m, 2H), 2.20-2.10 (m, 2H), 2.05-1.65 (m, 10H), 1.60-1.31 (m, 9H), 1.20-0.94 (m, 4H).

### Example 27: N-(3-(difluoromethyl)-1-((1R,4S)-4-((9-(3-(2,4-dioxotetrahydropyrimidin-1(2H)-yl)-4-methylbenzoyl)-3,9-diazaspiro[5.5]undecan-3-yl)methyl)cyclohexyl)-1H-pyrazol-4-yl)-5-((S)-3-hydroxypiperidin-1-yl)pyrazolo[1,5-a]pyrimidine-3-carboxamide

### Step 1: preparation of tert-butyl 9-(((1S,4R)-4-(3-(difluoromethyl)-4-(5-((3S)-3-((tetrahydro-2H-pyran-2-yl)oxy)piperidin-1-yl)pyrazolo[1,5-a]pyrimidine-3-carboxamido)-1H-pyrazol-1-yl)cyclohexyl)methyl)-3,9-diazaspiro[5.5]undecane-3-carboxylate

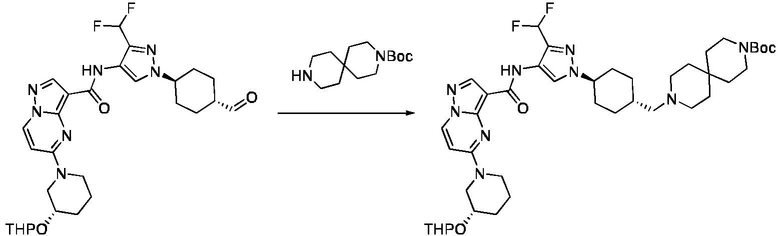

*N*-(3-(Difluoromethyl)-1-((1*R*,4*S*)-4-formylcyclohexyl)-1*H*-pyrazol-4-yl)-5-((3*S*)-3-((tetrahydro-2H-pyran-2-yl)oxy)piperidin-1-yl)pyrazolo[1,5-*a*]pyrimidine-3-carboxamide (90 mg, 0.15 mmol) and *tert-butyl* 3,9-diazaspiro[5.5]undecane-3-carboxylate (40 mg, 0.15 mmol) were dissolved in tetrahydrofuran (10 mL). Sodium triacetoxyborohydride (95 mg, 0.45 mmol) was added, and the mixture was stirred at room temperature for 2 h. Water (50 mL) was added, and the mixture was extracted with dichloromethane (20 mL × 3). The organic phase was washed with saturated brine (60 mL), dried over anhydrous sodium sulfate, filtered, and concentrated under reduced pressure. The crude product was purified by column chromatography (methanol:dichloromethane = 1:9) to give *tert-*butyl 9-(((1*S*,4*R*)-4-(3-(difluoromethyl)-4-(5-((3*S*)-3-((tetrahydro-2*H*-pyran-2-yl)oxy)piperidin-1-yl)pyrazolo[1,5-*a*]pyrimidine-3-carboxamido)-1*H*-pyrazol-1-yl)cyclohexyl)methyl)-3,9-diazaspiro[5.5]undecane-3-carboxylate.

LC-MS: (ESI, m/z): [M+H] ⁺= 810.4.

### Step 2: preparation of N-(1-((1R,4S)-4-((3,9-diazaspiro[5.5]undecan-3-yl)methyl)cyclohexyl)-3-(difluoromethyl)-1H-pyrazol-4-yl)-5-((S)-3-hydroxypiperidin-1-yl)pyrazolo[1,5-a]pyrimidine-3-carboxamide

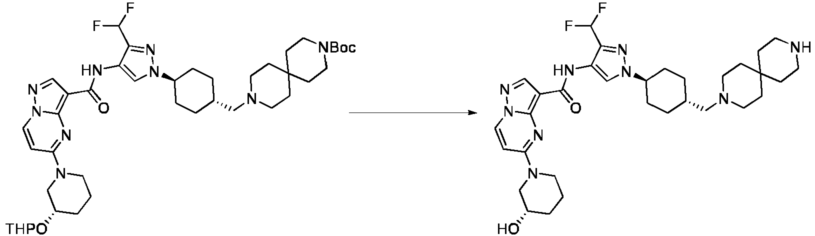

*tert-*Butyl 9-(((1*S*,4*R*)-4-(3-(difluoromethyl)-4-(5-((3*S*)-3-((tetrahydro-2H-pyran-2-yl)oxy)piperidin-1-yl)pyrazolo [1,5-*a*]pyrimidine-3-carboxamido)-1H-pyrazol-1-yl)cyclohexyl)methyl)-3,9-diazaspiro[5.5]undecane-3-carboxylate (100 mg, 0.12 mmol) was dissolved in dichloromethane (3 mL). Trifluoroacetic acid (1 mL) was added, and the mixture was stirred at room temperature for 1 h. The reaction liquid was directly concentrated under reduced pressure to give *N*-(1-((1*R*,4*S*)-4-((3,9-diazaspiro[5.5]undecan-3-yl)methyl)cyclohexyl)-3-(difluoromethyl)-1*H*-pyrazol-4-yl)-5-((*S*)-3-hydroxypiperidin-1-yl)pyrazolo[1,5-*a*]pyrimidine-3-carboxamide, which was directly used in the next step without purification.

LC-MS: (ESI, m/z): [M+H] ⁺= 626.3.

### Step 3: preparation of N-(3-(difluoromethyl)-1-((1R,4S)-4-((9-(3-(2,4-dioxotetrahydropyrimidin-1(2H)-yl)-4-methylbenzoyl)-3,9-diazaspiro[5.5]undecan-3-yl)methyl)cyclohexyl)-1H-pyrazol-4-yl)-5-((S)-3-hydroxypiperidin-1-yl)pyrazolo[1,5-a]pyrimidine-3-carboxamide

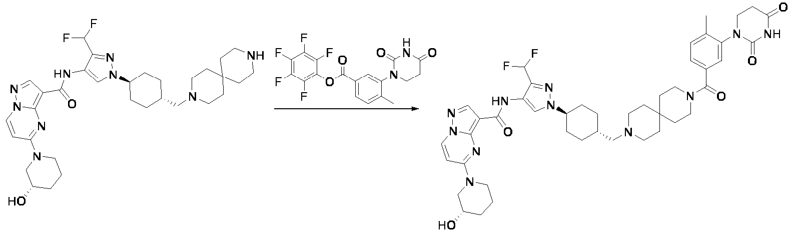

*N*-(1-((1*R*,4*S*)-4-((3,9-Diazaspiro[5.5]undecan-3-yl)methyl)cyclohexyl)-3-(difluoromethyl)-1*H*-pyrazol-4-yl)-5-((*S*)-3-hydroxypiperidin-1-yl)pyrazolo[1,5-*a*]pyrimidine-3-carboxamide (100 mg, 0.12 mmol) was dissolved in dimethyl sulfoxide (3 mL). Pentafluorophenyl 3-(2,4-dioxotetrahydropyrimidin-1(2*H*)-yl)-4-methylbenzoate (55 mg, 0.13 mmol) and *N,N*-diisopropylethylamine (155 mg, 1.2 mmol) were added, and the mixture was stirred at room temperature overnight. Water (30 mL) was added to the reaction liquid. The mixture was filtered, and the filter cake was purified by pre-HPLC (CH₃CN/O.08% aqueous NH₄HCO₃ solution, 5%-95%) to give *N*-(3-(difluoromethyl)-1-((1*R*,4*S*)-4-((9-(3-(2,4-dioxotetrahydropyrimidin-1(2*H*)-yl)-4-methylbenzoyl)-3,9-diazaspiro[5.5]undecan-3-yl)methyl)cyclohexyl)-1*H*-pyrazol-4-yl)-5-((S)-3-**hydroxypiperidin-1-yl)pyrazolo[1,5-a]pyrimidine-3-carboxamide.**

LC-MS: (ESI, m/z): [M+H] ⁺= 856.4.

¹H NMR (400 MHz, DMSO-*d*₆) δ 10.39 (s, 1H), 9.38 (s, 1H), 8.72 (d, *J* = 8.0 Hz, 1H), 8.36 (s, 1H), 8.24 (s, 1H), 7.36 - 7.29 (m, 2H), 7.27 - 6.94 (m, 2H), 6.86 (d, *J* = 8.0 Hz, 1H), 4.93-4.88 (m, 1H), 4.20-4.10 (m, 1H), 4.01 - 3.74 (m, 3H), 3.70-3.44 (m, 6H), 3.33 - 3.25 (m, 2H), 2.84-2.64 (m, 2H), 2.40-2.21 (m, 4H), 2.21 (s, 3H), 2.15-1.96 (m, 4H), 1.87-1.70 (m, 6H), 1.65-1.26 (m, 11H), 1.10-0.95 (m, 2H).

### Example 28: N-(3-(difluoromethyl)-1-(1-(3-(9-(3-(2,4-dioxotetrahydropyrimidin-1(2H)-yl)-4-methoxybenzoyl)-3,9-diazaspiro[5.5]undecan-3-yl)propyl)piperidin-4-yl)-1H-pyrazol-4-yl)-5-morpholinopyrazolo[1,5-a]pyrimidine-3-carboxamide

### Step 1: preparation of tert-butyl 9-(3-(2,4-dioxotetrahydropyrimidin-1(2H)-yl)-4-methoxybenzoyl)-3,9-diazaspiro [5.5]undecane-3-carboxylate

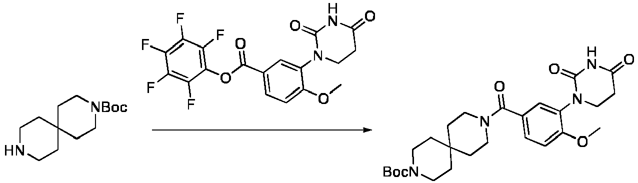

*tert-Butyl* 3,9-diazaspiro[5.5]undecane-3-carboxylate (200 mg, 0.79 mmol) was dissolved in dimethyl sulfoxide (4 mL), and pentafluorophenyl 3-(2,4-dioxotetrahydropyrimidin-1(2*H*)-yl)-4-methoxybenzoate (340 mg, 0.79 mmol) and *N,N*-diisopropylethylamine (413 mg, 3.24 mmol) were added with stirring. The reaction liquid was stirred at room temperature for 3 h. The reaction liquid was diluted with water (40 mL), and the mixture was extracted with ethyl acetate (50 mL × 3). The organic phase was washed with saturated brine (100 mL), dried over anhydrous sodium sulfate, filtered, and concentrated under reduced pressure. The crude product was purified by column chromatography (ethyl acetate:petroleum ether = 9:1) to give *tert-*butyl 9-(3-(2,4-dioxotetrahydropyrimidin-1(2*H*)-yl)-4-methoxybenzoyl)-3,9-diazaspiro[5.5]undecane-3-carboxylate.

LC-MS: (ESI, m/z): [M+H] ⁺= 501.2.

### Step 2: preparation of 1-(2-methoxy-5-(3,9-azaspiro[5.5]undecane-3-carbonyl)phenyl)dihydropyrimidine-2,4(1H,3H)-dione

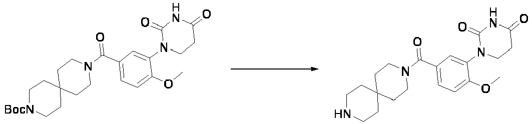

*tert-*Butyl 9-(3-(2,4-dioxotetrahydropyrimidin-1(2*H*)-yl)-4-methoxybenzoyl)-3,9-diazaspiro[5.5]undecane-3-carboxylate (230 mg, 0.46 mmol) was dissolved in dichloromethane (3 mL), and trifluoroacetic acid (0.3 mL) was added. The mixture was stirred at room temperature for 1 h. The reaction liquid was directly concentrated under reduced pressure to give 1-(2-methoxy-5-(3,9-azaspiro[5.5]undecane-3-carbonyl)phenyl) dihydropyrimidine-2,4(1H,3H)-dione, which was directly used in the next step without purification.

LC-MS: (ESI, m/z): [M+H] ⁺= 401.1.

### Step 3: preparation of 1-(5-(9-(2-(1,3-dioxolan-2-yl)ethyl)-3,9-diazaspiro[5.5]undecane-3-carbonyl)-2-methoxyphenyl)dihydropyrimidine-2,4(1H,3H)-dione

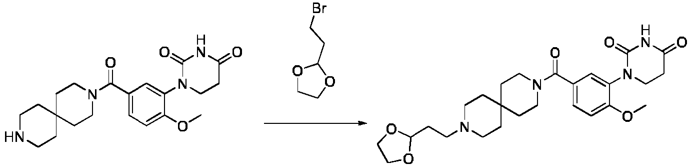

1-(2-Methoxy-5-(3,9-azaspiro[5.5]undecane-3-carbonyl)phenyl)dihydropyrimidine-2,4(1*H*,3*H*)-dione (180 mg, 0.45 mmol) and 2-(2-bromoethyl)-1,3-dioxolane (121 mg, 0.67 mmol) were dissolved in DMF (5 mL), and K₂CO₃ (186 mg,1.3 mmol) was added with stirring. The reaction liquid was heated to 80 °C and stirred for 12 h. The reaction liquid was diluted with water (80 mL), and the mixture was extracted with ethyl acetate (50 mL × 3). The organic phase was washed with saturated brine (80 mL), dried over anhydrous sodium sulfate, filtered, and concentrated under reduced pressure. The crude product was purified by column chromatography (methanol:dichloromethane = 1:7) to give 1-(5-(9-(2-(1,3-dioxolan-2-yl)ethyl)-3,9-diazaspiro[5.5]undecane-3-carbonyl)-2-methoxyphenyl)dihydropyrimidine-2,4(1*H*,3*H*)-dione.

LC-MS: (ESI, m/z): [M+H] ⁺= 501.7.

### Step 4: preparation of 3-(9-(3-(2,4-dioxotetrahydropyrimidin-1(2H)-yl)-4-methoxybenzoyl)-3,9-diazaspiro [5.5]undecan-3-yl)propanal

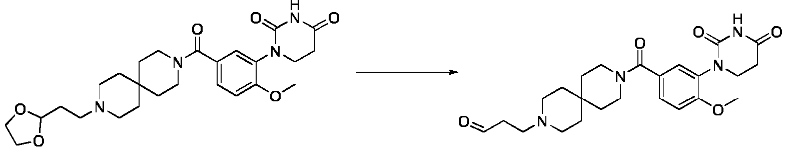

1-(5-(9-(2-(1,3-Dioxolan-2-yl)ethyl)-3,9-diazaspiro[5.5]undecane-3-carbonyl)-2-methoxyphenyl) dihydropyrimidine-2,4(1*H*,3*H*)-dione (200 mg, 0.5 mmol) was dissolved in acetone (5 mL), and hydrochloric acid (2 mol/L, 10 mL) was added. The mixture was stirred at room temperature for 6 h. The reaction liquid was adjusted to pH = 8 with sodium bicarbonate and extracted with ethyl acetate (50 mL × 3). The organic phase was dried over anhydrous sodium sulfate, filtered, and concentrated under reduced pressure to give 3-(9-(3-(2,4-dioxotetrahydropyrimidin-1(2*H*)-yl)-4-methoxybenzoyl)-3,9-diazaspiro[5.5]undecan-3-yl)propanal, which was directly used in the next step without purification.

LC-MS: (ESI, m/z): [M+H] ⁺= 457.7.

### Step 5: preparation of N-(3-(difluoromethyl)-1-(1-(3-(9-(3-(2,4-dioxotetrahydropyrimidin-1(2H)-yl)-4-methoxybenzoyl)-3,9-diazaspiro[5.5]undecan-3-yl)propyl)piperidin-4-yl)-1H-pyrazol-4-yl)-5-morpholinopyrazolo [1,5-a]pyrimidine-3-carboxamide

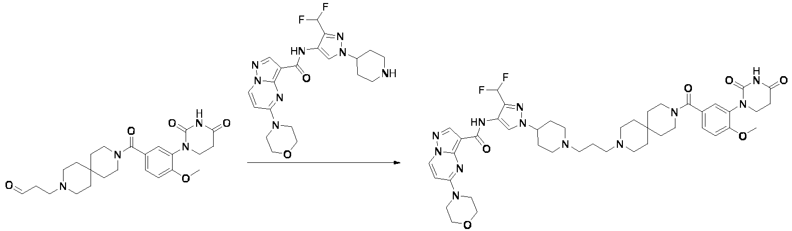

3-(9-(3-(2,4-Dioxotetrahydropyrimidin-1(2*H*)-yl)-4-methoxybenzoyl)-3,9-diazaspiro[5.5]undecan-3-yl)propanal (70 mg, 0.15 mmol) and *N*-(3-(difluoromethyl)-1-(piperidin-4-yl)-1*H-*pyrazol-4-yl)-5-morpholinopyrazolo [1,5-a]pyrimidine-3-carboxamide (67 mg, 0.15 mmol) were dissolved in dichloroethane (5 mL), and sodium triacetoxyborohydride (126 mg, 0.6 mmol) was added with stirring. The reaction liquid was stirred at room temperature for 3 h. The reaction liquid was diluted with water (50 mL), and the mixture was extracted with dichloromethane (60 mL × 3). The organic phase was dried over anhydrous sodium sulfate, filtered, and concentrated under reduced pressure. The crude product was purified by pre-HPLC (CH₃CN/O.08% aqueous NH₄HCO₃ solution, 5%-95%) to give *N*-(3-(difluoromethyl)-1-(1-(3-(9-(3-(2,4-dioxotetrahydropyrimidin-1(2*H*)-yl)-4-methoxybenzoyl)-3,9-diazaspiro[5.5]undecan-3-yl)propyl)piperidin-4-yl)-1*H*-pyrazol-4-yl)-5-morpholinopyrazolo[1,5-a]pyrimidine-3-carboxamide.

LC-MS: (ESI, m/z): [M+H] ⁺= 887.2.

¹H NMR (400 MHz, DMSO-*d*₆) δ 10.32 (s, 1H), 9.40 (s, 1H), 8.83 (d, *J* = 7.9 Hz, 1H), 8.39 (s, 1H), 8.29 (s, 1H), 7.37 (d, *J=* 8.4 Hz, 1H), 7.32 *(d, J=* 2.1 Hz, 1H), 7.26-6.97 (m, 2H), 6.91 (d, *J* = 7.9 Hz, 1H), 4.25-4.15 (m, 1H), 3.84 (s, 3H), 3.82-3.70 (m, 8H), 3.62-3.33 (m, 6H), 2.95-2.89 (m, 2H), 2.72-2.62 (m, 2H), 2.36-2.23 (m, 8H), 2.05-1.80 (m, 6H), 1.60-1.24 (m, 10H).

### Example 29: N-(1-(1-((3-(4-chloro-3-(2,4-dioxotetrahydropyrimidin-1(2H)-yl)benzoyl)-3-azaspiro[5.5] undecan-9-yl)methyl)piperidin-4-yl)-3-(difluoromethyl)-1H-pyrazol-4-yl)-5-(1,1-dioxothiomorpholinyl) pyrazolo[1,5-a]pyrimidine-3-carboxamide

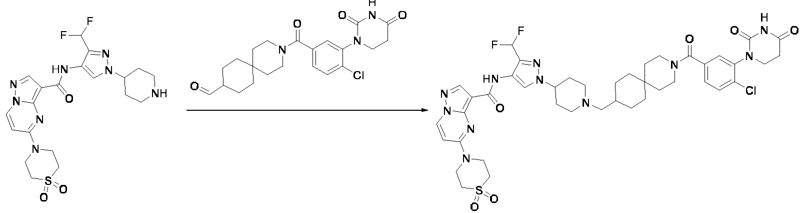

*N-(3-(Difluoromethyl)-1-(piperidin-4-yl)-1H-pyrazol-4-yl)-5-(1,1-dioxothiomorpholinyl)pyrazolo[1,5-a*]pyrimidine-3-carboxamide (80 mg, 0.16 mmol) and 3-(4-chloro-3-(2,4-dioxotetrahydropyrimidin-1(2*H*)-yl)benzoyl)-3-azaspiro[5.5]undecane-9-carbaldehyde (70 mg, 0.16 mmol) were dissolved in THF (3 mL), and sodium triacetoxyborohydride (102 mg, 0.48 mmol) was added. The mixture was stirred at room temperature for 16 h. Water (50 mL) was added to the reaction liquid, and the mixture was extracted with EA (30 mL × 3). The organic phase was washed with saturated brine (30 mL), dried over anhydrous sodium sulfate, filtered, and concentrated under reduced pressure. The crude product was separated and purified by Prep-HPLC to give *N-*(1-(1-((3-(4-chloro-3-(2,4-dioxotetrahydropyrimidin-1(2*H*)-yl)benzoyl)-3-azaspiro[5.5]undecan-9-yl)methyl) piperidin-4-yl)-3-(difluoromethyl)-1*H*-pyrazol-4-yl)-5-(1,1-dioxothiomorpholinyl)pyrazolo[1,5-*a*]pyrimidine-3-carboxamide.

LC-MS: (ESI, m/z): [M+H] ⁺= 910.4.

¹H NMR (400 MHz, DMSO-*d*₆) δ 10.50 (s, 1H), 9.25 (s, 1H), 8.92 (d, *J =* 7.9 Hz, 1H), 8.39 (s, 1H), 8.34 (s, 1H), 7.63 (d, *J =* 8.2 Hz, 1H), 7.55 (d, *J* = 1.8 Hz, 1H), 7.39 (d, *J =* 8.2 Hz, 1H), 7.25 - 6.97 (m, 2H), 4.31-4.20 (m, 5H), 3.77-3.70 (m, 1H), 3.67-3.57 (m, 3H), 3.33-3.23 (m, 5H), 2.90 (d, *J* = 9.3 Hz, 2H), 2.75-2.67 (m, 3H), 2.18-2.11 (m, 2H), 2.07-1.87 (m, 6H), 1.73-1.65 (m, 2H), 1.62-1.23 (m, 7H), 1.15-0.92 (m, 4H).

### Example 30: (S)-5-(3-aminopiperidin-1-yl)-N-(1-(1-((3-(4-chloro-3-(2,4-dioxotetrahydropyrimidin-1(2H)-yl)benzoyl)-3-azaspiro [5.5]undecan-9-yl)methyl)piperidin-4-yl)-3-(difluoromethyl)-1H-pyrazol-4-yl)pyrazolo [1,5-a]pyrimidine-3-carboxamide

### Step 1: preparation of tert-butyl (S)-(1-(3-((1-(1-((3-(4-chloro-3-(2,4-dioxotetrahydropyrimidin-1(2H)-yl)benzoyl)-3-azaspiro[5.5]undecan-9-yl)methyl)piperidin-4-yl)-3-(difluoromethyl)-1H-pyrazol-4-yl)carbamoyl) pyrazolo[1,5-a]pyrimidin-5-yl)piperidin-3-yl)carboxylate

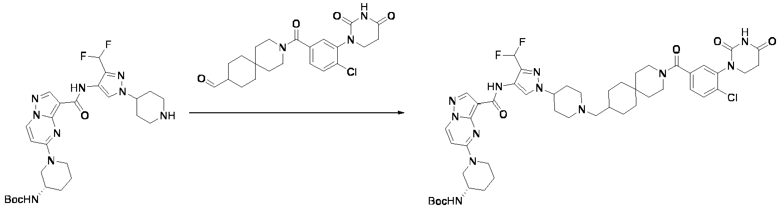

*tert-*Butyl (*S*)-(1-(3-((3-(difluoromethyl)-1-(piperidin-4-yl)-1*H*-pyrazol-4-yl)carbamoyl)pyrazolo[1,5-a]pyrimidin-5-yl)piperidin-3-yl)carbamate (70 mg, 0.12 mmol) and 3-(4-chloro-3-(2,4-dioxotetrahydropyrimidin-1(2*H*)-yl)benzoyl)-3-azaspiro[5.5]undecane-9-carbaldehyde (70 mg, 0.15 mmol) were dissolved in THF (2 mL), and NaBH(OAc)₃ (56 mg, 0.37 mmol) was added. The reaction liquid was stirred at room temperature for 2 h. The reaction liquid was diluted with water (20 mL) and extracted with ethyl acetate (20 mL × 3). The organic phases were combined, washed with saturated brine, dried over anhydrous sodium sulfate, filtered, and concentrated under reduced pressure. The crude product was purified by column chromatography (methanol:dichloromethane = 1:24) to give *tert-*butyl (*S*)-(1-(3-((1-(1-((3-(4-chloro-3-(2,4-dioxotetrahydropyrimidin-1(2*H*)-yl)benzoyl)-3-azaspiro[5.5]undecan-9-yl)methyl)piperidin-4-yl)-3-(difluoromethyl)-1*H*-pyrazol-4-yl)carbamoyl)pyrazolo[1,5-*a*]pyrimidin-5-yl)piperidin-3-yl)carboxylate. LC-MS: (ESI, m/z): [M+H] ⁺= 975.4.

### Step 2: preparation of (S)-5-(3-aminopiperidin-1-yl)-N-(1-(1-((3-(4-chloro-3-(2,4-dioxotetrahydropyrimidin-1(2H)-yl)benzoyl)-3-azaspiro[5.5]undecan-9-yl)methyl)piperidin-4-yl)-3-(difluoromethyl)-1H-pyrazol-4-yl)pyrazolo[1,5-a]pyrimidine-3-carboxamide

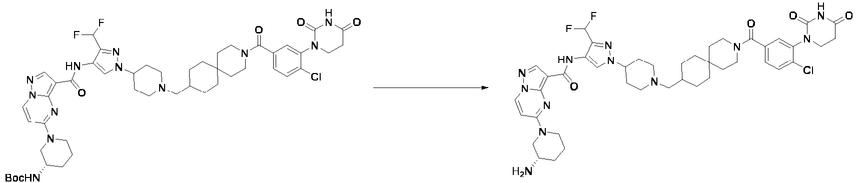

*tert-*Butyl (*S*)-(1-(3-((1-(1-((3-(4-chloro-3-(2,4-dioxotetrahydropyrimidin-1(2*H*)-yl)benzoyl)-3-azaspiro[5.5] undecan-9-yl)methyl)piperidin-4-yl)-3-(difluoromethyl)-1*H*-pyrazol-4-yl)carbamoyl)pyrazolo[1,5-a]pyrimidin-5-yl)piperidin-3-yl)carboxylate (30 mg, 0.03 mmol) was dissolved in DCM (5 mL), and TFA (1 mL) was added. The reaction liquid was stirred at room temperature for 1 h. The reaction liquid was directly concentrated under reduced pressure, and the crude product was purified by Prep-HPLC to give (*S*)-5-(3-aminopiperidin-1-yl)-*N*-(1-(1-((3-(4-chloro-3-(2,4-dioxotetrahydropyrimidin-1(2*H*)-yl)benzoyl)-3-azaspiro[5.5]undecan-9-yl)methyl)piperidin-4-yl)-3-(difluoromethyl)-1*H*-pyrazol-4-yl)pyrazolo[1,5-a]pyrimidine-3-carboxamide. LC-MS: (ESI, m/z): [M+H] ⁺= 875.3.

¹H NMR (400 MHz, DMSO-*d*₆) δ 8.77 (d, J = 7.9 Hz, 1H), 8.37 (s, 1H), 8.34-8.30 (m, 2H), 7.65 (d, *J =* 8.2 Hz, 1H), 7.53 (s, 1H), 7.40 (d, *J* = 8.4 Hz, 1H), 7.26-6.95 (m, 1H), 6.85 (d, *J* = 7.9 Hz, 1H), 4.23-4.13 (m, 2H), 3.82-3.74 (m, 1H), 3.70-3.40 (m, 7H), 3.30-3.10 (m, 3H), 2.97-2.92 (m, 2H), 2.77-2.73 (m, 2H), 2.25-1.75 (m, 11H), 1.75- 1.15 (m, 12H), 1.15-0.96 (m, 4H).

### Example 31: 5-((1R,4R)-2-oxa-5-azabicyclo[2.2.1]heptan-5-yl)-N-(1-(1-(3-((1-(4-chloro-3-(2,4-dioxotetrahydropyrimidin-1(2H)-yl)benzoyl)piperidin-4-yl)oxy)propyl)piperidin-4-yl)-3-(difluoromethyl)-1H-pyrazol-4-yl)pyrazolo [1,5-a]pyrimidine-3-carboxamide

### Step 1: preparation of benzyl 4-(3-hydroxypropoxy)piperidine-1-carboxylate

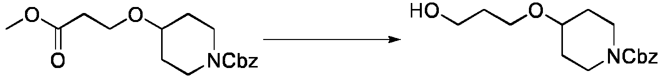

Benzyl 4-(3-methoxy-3-oxopropyl)piperidine-1-carboxylate (500 mg, 1.49 mmol) was dissolved in anhydrous THF (10 mL). A BH₃/THF solution (15 mL, 14.9 mmol) was added at 0 °C, and the mixture was stirred at room temperature for 16 h. Methanol was added to the reaction liquid to quench the reaction, and the mixture was concentrated under reduced pressure to give a crude product. The crude product was purified by column chromatography (ethyl acetate:petroleum ether = 9:1) to give benzyl 4-(3-hydroxypropoxy)piperidine-1-carboxylate.

LC-MS: (ESI, m/z): [M+H] ⁺= 294.2.

### Step 2: preparation of 3-(piperidin-4-yloxy)propan-1-ol

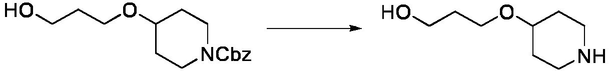

Benzyl 4-(3-hydroxypropoxy)piperidine-1-carboxylate (200 mg, 0.68 mmol) was dissolved in ethyl acetate (10 mL). Pd(OH)₂/C (80 mg) was added, and the mixture was heated to 70 °C under hydrogen atmosphere and stirred overnight. The reaction liquid was cooled to room temperature and filtered, and the filtrate was concentrated under reduced pressure to give 3-(piperidin-4-yloxy)propan-1-ol. The crude product was directly used in the next step without purification.

LC-MS: (ESI, m/z): [M+H] ⁺= 160.2.

### Step 3: preparation of 1-(2-chloro-5-(4-(3-hydroxypropoxy)piperidine-1-carbonyl)phenyl)dihydropyrimidine-2,4(1H,3H)-dione

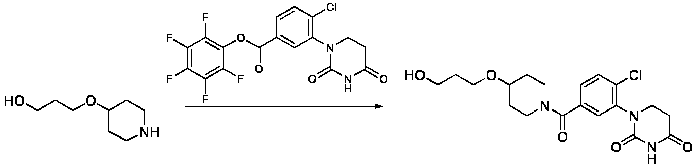

3-(Piperidin-4-yloxy)propan-1-ol (100 mg, 0.62 mmol) was dissolved in DMSO (10 mL). Pentafluorophenyl 4-chloro-3-(2,4-dioxotetrahydropyrimidin-1(2*H*)-yl)benzoate (300 mg, 0.69 mmol) and DIEA (240 mg, 1.86 mmol) were added, and the mixture was stirred at room temperature for 2 h. Water (100 mL) was added to the reaction liquid, and the mixture was extracted with dichloromethane (30 mL × 3). The organic phase was dried over anhydrous sodium sulfate, filtered, and concentrated under reduced pressure to give a crude product. The crude product was purified by column chromatography (methanol:dichloromethane = 1:9) to give 1-(2-chloro-5-(4-(3-hydroxypropoxy)piperidine-1-carbonyl)phenyl)dihydropyrimidine-2,4(1*H*,3*H*)-dione.

LC-MS: (ESI, m/z): [M+H] ⁺ = 410.1.

### Step 4: preparation of 3-((1-(4-chloro-3-(2,4-dioxotetrahydropyrimidin-1(2H)-yl)benzoyl)piperidin-4-yl)oxy)propanal

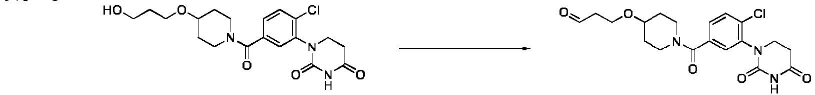

1-(2-Chloro-5-(4-(3-hydroxypropoxy)piperidine-1-carbonyl)phenyl)dihydropyrimidine-2,4(1*H*,3*H*)-dione (60 mg, 0.147 mmol) was dissolved in DCM (5 mL). PCC (63 mg, 0.294 mmol) was added, and the mixture was stirred at room temperature for 16 h. The reaction liquid was concentrated under reduced pressure, and the crude product was purified by column chromatography (methanol:dichloromethane = 1:9) to give 3-((1-(4-chloro-3-(2,4-dioxotetrahydropyrimidin-1(2*H*)-yl)benzoyl)piperidin-4-yl)oxy)propanal.

LC-MS: (ESI, m/z): [M+H] ⁺= 408.1.

### Step 5: preparation of 5-((1R,4R)-2-oxa-5-azabicyclo[2.2.1]heptan-5-yl)-N-(1-(1-(3-((1-(4-chloro-3-(2,4-dioxotetrahydropyrimidin-1(2H)-yl)benzoyl)piperidin-4-yl)oxy)propyl)piperidin-4-yl)-3-(difluoromethyl)-1H-pyrazol-4-yl)pyrazolo[1,5-a]pyrimidine-3-carboxamide

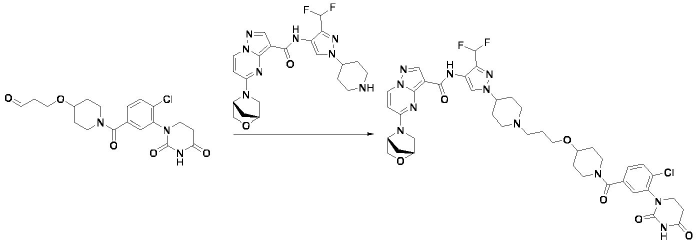

3-((1-(4-Chloro-3-(2,4-dioxotetrahydropyrimidin-1(2*H*)-yl)benzoyl)piperidin-4-yl)oxy)propanal (55 mg, 0.135 mmol) was dissolved in THF (5 mL). 5-((1*R*,4*R*)-2-Oxa-5-azabicyclo[2.2.1]heptan-5-yl)-*N-*(3-(difluoromethyl)-1-(piperidin-4-yl)-1*H*-pyrazol-4-yl)pyrazolo[1,5-*a*]pyrimidine-3-carboxamide (crude: 70 mg, 0.135 mmol) and STAB (86 mg, 0.405 mmol) were added, and the mixture was stirred at room temperature for 2 h. Water (100 mL) was added to the reaction liquid, and the mixture was extracted with ethyl acetate (30 mL × 3). The organic phase was washed with a saturated aqueous sodium chloride solution (100 mL), dried over anhydrous sodium sulfate, filtered, and concentrated under reduced pressure to give a crude product. The crude product was purified by pre-HPLC (CH₃CN/O.08% aqueous NH₄HCO₃ solution, 5%-95%) to give 5-((1*R*,4*R*)-2-oxa-5-azabicyclo[2.2.1]heptan-5-yl)-*N*-(1-(1-(3-((1-(4-chloro-3-(2,4-dioxotetrahydropyrimidin-1(2*H*)-yl)benzoyl)piperidin-4-yl)oxy)propyl)piperidin-4-yl)-3-(difluoromethyl)-1*H*-pyrazol-4-yl)pyrazolo[1,5-*a*]pyrimidine-3-carboxamide.

LC-MS: (ESI, m/z): [M+H] ⁺= 850.4.

¹H NMR (400 MHz, DMSO-*d*₆) δ 10.51 (s, 1H), 9.50 (d, *J* = 5.7 Hz, 1H), 8.79 (d, *J* = 7.8 Hz, 1H), 8.40 (d, *J* = 3.2 Hz, 1H), 8.26 (d, *J* = 5.5 Hz, 1H), 7.67 - 7.55 (m, 2H), 7.40 (d, *J* = 8.7 Hz, 1H), 7.30-6.92 (m, 1H), 6.88-6.45 (m, 1H), 5.28-5.08 (m, 1H), 4.77 (d, *J =* 17.7 Hz, 1H), 4.30-4.10 (m, 1H), 4.02 - 3.68 (m, 4H), 3.62-3.47 (m, 7H), 3.30-3.10 (m, 2H), 3.00-2.85 (m, 2H), 2.78 - 2.69 (m, 2H), 2.45-2.31 (m, 2H), 2.10-1.55 (m, 12H), 1.52-1.37 (m, 2H).

### Example 32: N-(3-(difluoromethyl)-1-((1R,4R)-4-(((2-(1-(3-(2,4-dioxotetrahydropyrimidin-1(2H)-yl)-4-methoxybenzoyl)piperidin-4-yl)ethyl)(methyl)amino)methyl)cyclohexyl)-1H-pyrazol-4-yl)-5-(2-oxa-6-azaspiro[3.3]heptan-6-yl)pyrazolo[1,5-a]pyrimidine-3-carboxamide

### Step 1: preparation of tert-butyl 4-(2-(((benzyloxy)carbonyl)(methyl)amino)ethyl)piperidine-1-carboxylate

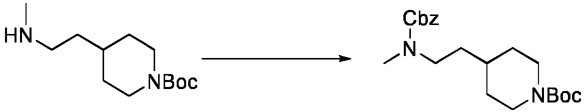

*tert-*Butyl 4-(2-(methylamino)ethyl)piperidine-1-carboxylate (200 mg, 0.82 mmol) was dissolved in dichloromethane (4 mL). Triethylamine (249, 2.46 mmol) and CbzCl (139 mg, 0.82 mmol) were added, and the reaction liquid was stirred at room temperature for 1 h. The reaction liquid was diluted with water (50 mL), and the mixture was extracted with dichloromethane (80 mL × 3), dried over anhydrous sodium sulfate, and concentrated under reduced pressure. The crude product was purified by column chromatography (PE:EA = 3:7) to give the compound *tert-*butyl 4-(2-(((benzyloxy)carbonyl)(methyl)amino)ethyl)piperidine-1-carboxylate.

LC-MS: (ESI, m/z): [M+H-100] ⁺= 277.2.

### Step 2: preparation of benzyl methyl(2-(piperidin-4-yl)ethyl)carbamate

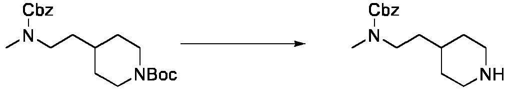

The compound *tert-*butyl 4-(2-(((benzyloxy)carbonyl)(methyl)amino)ethyl)piperidine-1-carboxylate (200 mg, 0.53 mmol) was dissolved in a hydrogen chloride/1,4-dioxane solution (4 mL), and the reaction liquid was stirred at room temperature for 1 h. The reaction liquid was directly concentrated under reduced pressure to give benzyl methyl(2-(piperidin-4-yl)ethyl)carbamate.

LC-MS: (ESI, m/z): [M+H] ⁺= 277.2.

### Step 3: preparation of benzyl (2-(1-(3-(2,4-dioxotetrahydropyrimidin-1(2H)-yl)-4-methoxybenzoyl)piperidin-4-yl)ethyl)(methyl)carbamate

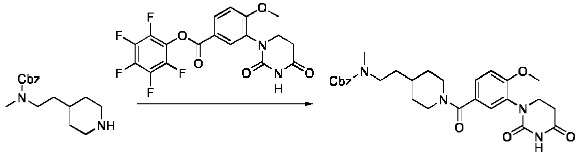

The compounds benzyl methyl(2-(piperidin-4-yl)ethyl)carbamate (200 mg, 0.72 mmol) and pentafluorophenyl 3-(2,4-dioxotetrahydropyrimidin-1(2*H*)-yl)-4-methoxybenzoate (309 mg, 0.72 mmol) were added to a DMSO solution (4 mL). Then DIEA (278 mg, 2.1 mmol) was added, and the reaction liquid was stirred at room temperature for 3 h. The reaction liquid was diluted with water (50 mL), and the mixture was extracted with ethyl acetate (70 mL × 4), washed with saturated brine, dried over anhydrous sodium sulfate, and concentrated under reduced pressure. The crude product was purified by column chromatography (PE:EA = 1:3) to give the compound benzyl (2-(1-(3-(2,4-dioxotetrahydropyrimidin-1(2*H*)-yl)-4-methoxybenzoyl)piperidin-4-yl)ethyl)(methyl)carbamate.

LC-MS: (ESI, m/z): [M+H] ⁺= 523.2.

### Step 4: preparation of 1-(2-methoxy-5-(4-(2-(methylamino)ethyl)piperidine-1-carbonyl)phenyl) dihydropyrimidine-2,4(1H,3H)-dione

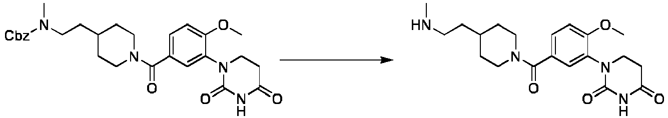

The compound benzyl (2-(1-(3-(2,4-dioxotetrahydropyrimidin-1(2*H*)-yl)-4-methoxybenzoyl)piperidin-4-yl)ethyl)(methyl)carbamate (120 mg, 0.23 mmol) was dissolved in a methanol solution (3 mL). Palladium on carbon (10%, 24 mg) was added, and the reaction liquid was stirred at room temperature for 1 h. The reaction liquid was filtered under vacuum through celite, and the filtrate was concentrated under reduced pressure to give the compound 1-(2-methoxy-5-(4-(2-(methylamino)ethyl)piperidine-1-carbonyl)phenyl)dihydropyrimidine-2,4(1*H*,3*H*)-dione.

LC-MS: (ESI, m/z): [M+H] ⁺= 389.2.

### Step 5: preparation of N-(3-(difluoromethyl)-1-((1R,4R)-4-(((2-(1-(3-(2,4-dioxotetrahydropyrimidin-1(2H)-yl)-4-methoxybenzoyl)piperidin-4-yl)ethyl)(methyl)amino)methyl)cyclohexyl)-1H-pyrazol-4-yl)-5-(2-oxa-6-azaspiro[3.3]heptan-6-yl)pyrazolo[1,5-a]pyrimidine-3-carboxamide

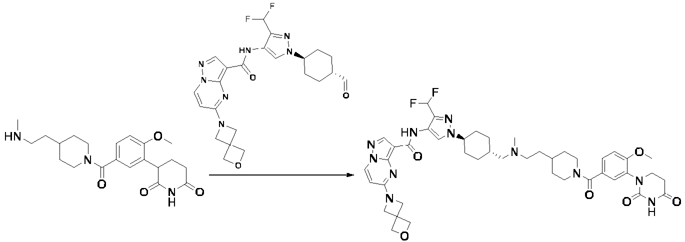

The compounds *N*-(3-(difluoromethyl)-1-((1*R*,4*R*)-4-formylcyclohexyl)-1*H*-pyrazol-4-yl)-5-(2-oxa-6-azaspiro[3.3]heptan-6-yl)pyrazolo[1,5-*a*]pyrimidine-3-carboxamide (80 mg, 0.165 mmol) and 1-(2-methoxy-5-(4-(2-(methylamino)ethyl)piperidine-1-carbonyl)phenyl)dihydropyrimidine-2,4(1*H*,3*H*)-dione (64 mg, 0.165 mmol) were dissolved in DCE (3 mL). STAB (70 mg, 0.330 mmol) was added, and the reaction liquid was stirred at room temperature for 2 h. The reaction liquid was diluted with water (20 mL), and the mixture was extracted with ethyl acetate (25 mL × 4), washed with saturated brine, dried over anhydrous sodium sulfate, and concentrated under reduced pressure. The crude product was purified by preparative reversed-phase chromatography to give the compound *N*-(3-(difluoromethyl)-1-((1*R*,4*R*)-4-(((2-(1-(3-(2,4-dioxotetrahydropyrimidin-1(2*H*)-yl)-4-methoxybenzoyl)piperidin-4-yl)ethyl)(methyl)amino)methyl)cyclohexyl)-1*H*-pyrazol-4-yl)-5-(2-oxa-6-azaspiro[3.3]heptan-6-yl)pyrazolo[1,5-*a*]pyrimidine-3-carboxatnide.

LC-MS: (ESI, m/z): [M+H] ⁺= 858.4.

¹H NMR (400 MHz, DMSO-*d*₆) δ 10.34 (s, 1H), 9.69 (s, 1H), 8.76 (d, *J* = 7.6 Hz, 1H), 8.37 (s, 1H), 8.25 (s, 1H), 7.35 (s, 1H), 7.42-7.00 (m, 3H), 6.38 (d, *J =* 7.6 Hz, 1H), 4.78-4.70 (m, 4H), 4.48-4.35 (m, 4H), 4.25-4.10 (m, 1H), 3.84 (s, 3H), 3.59 (t, *J =* 5.0 Hz, 2H), 3.30-3.10 (m, 3H), 2.75-2.63 (m, 2H), 2.36-2.25 (m, 2H), 2.20-1.99 (m, 7H), 1.93-1.83 (m, 2H), 1.77-1.50 (m, 7H), 1.44-1.30 (m, 2H), 1.20-0.96 (m, 4H).

### Example 33: N-(2-((1S,4S)-4-(((1-(4-chloro-3-(2,4-dioxotetrahydropyrimidin-1(2H)-yl)benzoyl)piperidin-4-yl)methoxy)methyl)-4-hydroxycyclohexyl)-6-methoxy-2H-indol-5-yl)-6-(trifluoromethyl)pyridinecarboxamide Step 1: preparation of tert-butyl (1-oxaspiro[2.5]octan-6-yl)carbamate

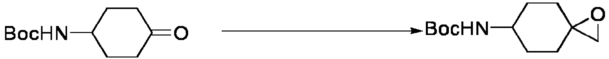

NaH (1.17 g, 29.3 mmol) was added to a solution of Me₃OS⁺I⁻ (6.2 g, 28.2 mmol) in DMSO (50 mL) at 0 °C with stirring, and the reaction mixture was stirred at room temperature for 1 h. Then a solution of *tert-*butyl (4-oxocyclohexyl)carbamate (5.0 g, 23.5 mmol) in DMSO (20 mL) was added dropwise. After the addition, the reaction liquid was stirred at room temperature for another 1 h. Water (700 mL) was added to the reaction liquid, and the mixture was extracted with EtOAc (100 mL × 3). The organic phase was washed with saturated brine (300 mL), dried over anhydrous Na₂SO₄, filtered, and concentrated under reduced pressure. The crude product was purified by column chromatography (EA:PE = 0-1:1) to give *tert-*butyl (1-oxaspiro[2.5]octan-6-yl)carbamate.

¹H NMR (400 MHz, DMSO-*d*₆) δ 6.79 (d, *J* = 7.2 Hz, 1H), 3.37 (m, 1H), 2.57 (s, 2H), 1.83 (td, *J* = 13.3, 4.1 Hz, 2H), 1.77 - 1.64 (m, 2H), 1.48 - 1.40 (m, 2H), 1.38 (s, 9H), 1.20 (d, *J* = 13.1 Hz, 2H).

### Step 2: preparation of tert-butyl (4-hydroxy-4-(hydroxymethyl)cyclohexyl)carbamate

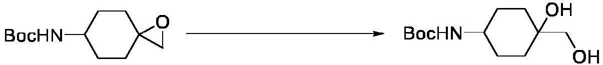

2 N NaOH (47 mL) was added to a solution of *tert-*butyl (1-oxaspiro[2.5]octan-6-yl)carbamate (4.7 g, 20.7 mmol) in NMP (50 mL) with stirring, and then the reaction liquid was stirred at 100 °C for 2 h. Water (500 mL) was added to the reaction liquid, and the mixture was extracted with EA (100 mL × 3). The organic phase was washed with saturated brine (300 mL), dried over anhydrous Na₂SO₄, filtered, and concentrated under reduced pressure. The crude product was purified by flash column chromatography (MeOH:DCM = 0-1:9) to give *tert-*butyl (4-hydroxy-4-(hydroxymethyl)cyclohexyl)carbamate.

¹H NMR (400 MHz, DMSO-*d*₆) δ 6.67 (d, *J* = 7.9 Hz, 1H), 4.46 (t, *J* = 5.8 Hz, 1H), 3.81 (s, 1H), 3.11 (t, *J* = 6.8 Hz, 3H), 1.56 - 1.45 (m, 4H), 1.44 - 1.34 (m, 13H).

### Step 3: preparation of tert-butyl (4-hydroxy-4-((pyridin-4-ylmethoxy)methyl)cyclohexyl)carbamate

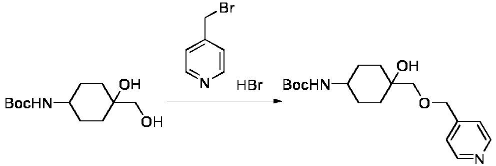

NaH (292 mg, 7.3 mmol) was added to a solution of *tert-*butyl (4-hydroxy-4-(hydroxymethyl)cyclohexyl) carbamate (600 mg, 2.4 mmol) in THF (20 mL) at 0 °C, the reaction mixture was stirred at 0 °C for 20 min and then stirred at room temperature for another 30 min, and 4-(bromomethyl)pyridine hydrobromide (617 mg, 2.4 mmol) was added. The reaction liquid was stirred at room temperature overnight. Water (30 mL) was added to the reaction liquid, and the mixture was extracted with EA (20 mL × 3). The organic phase was washed with saturated brine (60 mL), dried over anhydrous Na₂SO₄, filtered, and concentrated under reduced pressure. The resulting crude product was purified by flash column chromatography (EA:PE = 0-4:1) to give *tert*-butyl (4-hydroxy-4-((pyridin-4-ylmethoxy)methyl)cyclohexyl)carbamate.

LC-MS: (ESI, *m*/*z*): [M+H] ⁺= 337.1.

¹H NMR (400 MHz, DMSO-*d*₆) δ 8.53 (d, *J =* 5.8 Hz, 2H), 7.34 (d, *J* = 5.6 Hz, 2H), 6.69 (d, *J* = 7.6 Hz, 1H), 4.55 (s, 2H), 4.19 (s, 1H), 3.23 (s, 2H), 3.18 - 3.07 (m, 1H), 1.60-1.48 (m, 6H), 1.42-1.35 (m, *J* = 9.2 Hz, 11H).

### Step 4: preparation of tert-butyl (4-hydroxy-4-((piperidin-4-ylmethoxy)methyl)cyclohexyl)carbamate

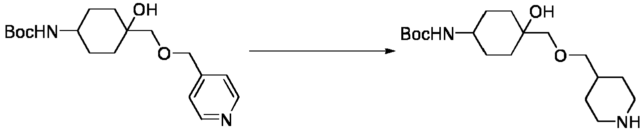

A mixed solution of *tert-*butyl (4-hydroxy-4-((pyridin-4-ylmethoxy)methyl)cyclohexyl)carbamate (700 mg, 2.07 mmol) and Pd/C (400 mg) in *i*-PrOH/H₂O (18 mL/21 mL) was stirred at 75 °C overnight under H₂ atmosphere. The reaction liquid was filtered through celite, and the filtrate was concentrated under reduced pressure to give *tert-*butyl (4-hydroxy-4-((piperidin-4-ylmethoxy)methyl)cyclohexyl)carbamate. The resulting crude product was directly used in the next step.

LC-MS: (ESI, *m*/*z*): [M+H] ⁺= 343.2.

### Step 5: preparation of benzyl 4-(((4-((tert-butoxycarbonyl)amino)-1-hydroxycyclohexyl)methoxy)methyl) piperidine-1-carboxylate

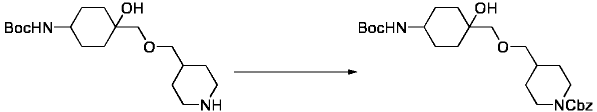

A solution of *tert-*butyl (4-hydroxy-4-((piperidin-4-ylmethoxy)methyl)cyclohexyl)carbamate (630 mg, 1.83 mmol) and CbzCl (376 mg, 2.20 mmol) in ACN (10 mL) and a saturated aqueous NaHCO₃ solution (10 mL) were stirred at room temperature overnight. The reaction liquid was concentrated under reduced pressure and then extracted with DCM (50 mL × 3). The organic phase was washed with saturated brine (150 mL), dried over anhydrous Na₂SO₄, filtered, and concentrated under reduced pressure. The crude product was purified by flash column chromatography (EA:PE = 0-7:3) to give benzyl 4-(((4-((*tert*-butoxycarbonyl)amino)-1-hydroxycyclohexyl)methoxy)methyl)piperidine-1-carboxylate.

LC-MS: (ESI, *m*/*z):* [M+Na] ⁺= 499.3.

### Step 6: preparation of benzyl 4-(((4-amino-1-hydroxycyclohexyl)methoxy)methyl)piperidine-1-carboxylate

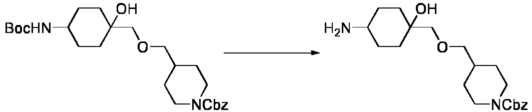

TFA (3 mL) was added to a solution of benzyl 4-(((4-((*tert*-butoxycarbonyl)amino)-1-hydroxycyclohexyl)methoxy)methyl)piperidine-1-carboxylate (300 mg, 0.63 mmol) in DCM (12 mL) at -10 to 0 °C while stirring, and then the reaction liquid was stirred at room temperature for 2 h. The pH of the reaction mixture was adjusted to 9.0 with DIEA, and then the mixture was directly concentrated under reduced pressure to give benzyl 4-(((4-amino-1-hydroxycyclohexyl)methoxy)methyl)piperidine-1-carboxylate. The resulting crude product was directly used in the next step without purification.

LC-MS: (ESI, *m*/*z):* [M+H] ⁺= 377.2.

### Step 7: preparation of benzyl 4-((((1S,4S)-1-hydroxy-4-(6-methoxy-5-nitro-2H-indol-2-yl)cyclohexyl) methoxy)methyl)piperidine-1-carboxylate

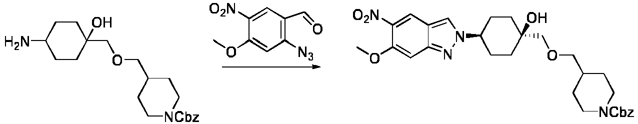

2-Azido-4-methoxy-5-nitrobenzaldehyde (170 mg, 0.76 mmol) was added to a solution of benzyl 4-(((4-amino-1-hydroxycyclohexyl)methoxy)methyl)piperidine-1-carboxylate (237 mg, 0.63 mmol) in toluene (10 mL) with stirring. The reaction liquid was stirred at 100 °C for 3 h. The reaction liquid was directly concentrated under reduced pressure. The crude product was purified by flash column chromatography (EA:PE = 0-4:1) and then purified by Prep-HPLC (CH₃CN/O.08% aqueous NH₄HCO₃ solution, 5%-95%) to give benzyl 4-((((1*S*,4*S*)-1-hydroxy-4-(6-methoxy-5-nitro-2*H*-indol-2-yl)cyclohexyl)methoxy)methyl)piperidine-1-carboxylate.

LC-MS: (ESI, *m*/*z*): [M+H] ⁺= 553.2.

¹H NMR (400 MHz, DMSO-*d*₆) δ 8.58 (s, 1H), 8.37 (s, 1H), 7.40 - 7.29 (m, 5H), 7.26 (s, 1H), 5.07 (s, 2H), 4.45 (t, *J* = 12.0 Hz, 1H), 4.36 (s, 1H), 4.06 - 3.99 (m, 2H), 3.90 (s, 3H), 3.29 (d, *J=* 6.2 Hz, 2H), 3.20 (s, 2H), 2.85-2.75 (m, 2H), 2.35 - 2.20 (m, 2H), 1.89 (d, *J* = 11.8 Hz, 2H), 1.76-1.61 (m, 7H), 1.15-1.02 (m, 2H).

### Step 8: preparation of tert-butyl 4-((((1S,4S)-4-(5-amino-6-methoxy-2H-indol-2-yl)-1-hydroxycyclohexyl) methoxy)methyl)piperidine-1-carboxylate

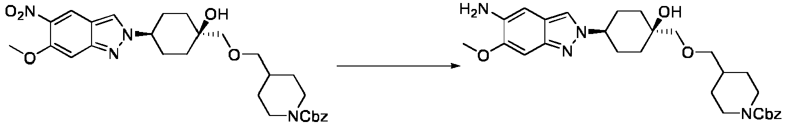

Raney-Ni (0.3 mL) and N₂H₄ H₂O (0.3 mL) were added to a solution of benzyl 4-((((1*S*,4*S*)-1-hydroxy-4-(6-methoxy-5-nitro-2*H*-indol-2-yl)cyclohexyl)methoxy)methyl)piperidine-1-carboxylate (200 mg, 0.36 mmol) in EtOH (20 mL). The reaction liquid was stirred at room temperature for 2 h. The reaction liquid was filtered through celite, and the filtrate was directly concentrated under reduced pressure to give *tert*-butyl 4-((((1*S*,4*S*)-4-(5-amino-6-methoxy-2*H*-indol-2-yl)-1-hydroxycyclohexyl)methoxy)methyl)piperidine-1-carboxylate. The resulting crude product was directly used in the next step.

LC-MS: (ESI, *m*/*z):* [M+H] ⁺= 523.2.

### Step 9: preparation of benzyl 4-((((1S,4S)-1-hydroxy-4-(6-methoxy-5-(6-(trifluoromethyl)pyridinecarboxamido)-2H-indol-2-yl)cyclohexyl)methoxy)methyl)piperidine-1-carboxylate

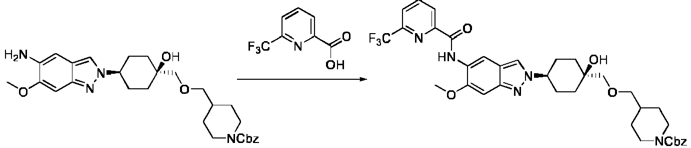

6-(Trifluoromethyl)picolinic acid (90 mg, 0.47 mmol), HATU (178 mg, 0.47 mmol), and DIEA (140 mg, 1.08 mmol) were added to a solution of *tert-*butyl 4-((((1*S*,4*S*)-4-(5-amino-6-methoxy-2*H*-indol-2-yl)-1-hydroxycyclohexyl)methoxy)methyl)piperidine-1-carboxylate (190 mg, 0.36 mmol) in DMF (5 mL) with stirring. Then the reaction liquid was stirred at room temperature for 2 h. Water (30 mL) was added to the reaction liquid, and the mixture was extracted with EA (20 mL × 3). The organic phase was washed with saturated brine (60 mL), dried over anhydrous Na₂SO₄, filtered, and concentrated under reduced pressure. The resulting crude product was purified by flash column chromatography (EA:PE = 0-4:1) to give benzyl 4-((((1*S*,4*S*)-1-hydroxy-4-(6-methoxy-5-(6-(trifluoromethyl)pyridinecarboxamido)-2*H*-indol-2-yl)cyclohexyl) methoxy)methyl)piperidine-1-carboxylate.

LC-MS: (ESI, *m*/*z*): [M+H] ⁺= 696.3.

¹H NMR (400 MHz, DMSO-*d*₆) δ 10.51 (s, 1H), 8.69 (s, 1H), 8.47 (d, *J* = 7.7 Hz, 1H), 8.41 (t, *J =* 7.8 Hz, 1H), 8.32 (s, 1H), 8.22 (d, *J* = 7.7 Hz, 1H), 7.41 - 7.29 (m, 5H), 7.16 (s, 1H), 5.08 (s, 2H), 4.40 - 4.29 (m, 2H), 4.15-3.95 (m 5H), 3.29 (d, *J* = 6.2 Hz, 2H), 3.21 (s, 2H), 2.91-2.70 (m, 2H), 2.35-2.15 (m, 2H), 1.90 (d, *J* = 9.5 Hz, 2H), 1.85-1.60 (m, 7H), 1.12-.1.02 (m, 2H).

### Step 10: preparation of N-(2-((1S,4S)-4-hydroxy-4-((piperidin-4-ylmethoxy)methyl)cyclohexyl)-6-methoxy-2H-indol-5-yl)-6-(trifluoromethyl)pyridinecarboxamide

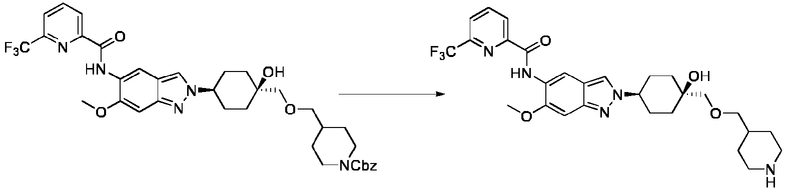

Pd(OH)₂/C (80 mg) was added to a solution of benzyl 4-((((1*S*,4*S*)-1-hydroxy-4-(6-methoxy-5-(6-(trifluoromethyl)pyridinecarboxamido)-2*H*-indol-2-yl)cyclohexyl)methoxy)methyl)piperidine-1-carboxylate (170 mg, 0.24 mmol) in EA (20 mL). Then the reaction liquid was stirred at 70 °C overnight under H₂ atmosphere. The reaction liquid was filtered through celite, and the filtrate was directly concentrated under reduced pressure to give *N*-(2-((1*S*,4*S*)-4-hydroxy-4-((piperidin-4-ylmethoxy)methyl)cyclohexyl)-6-methoxy-2*H*-indol-5-yl)-6-(trifluoromethyl)pyridinecarboxamide.

LC-MS: (ESI, *m*/*z*): [M+H] ⁺= 562.3.

### Step 11: preparation of N-(2-((1S,4S)-4-(((1-(4-chloro-3-(2,4-dioxotetrahydropyrimidin-1(2H)-yl)benzoyl) piperidin-4-yl)methoxy)methyl)-4-hydroxycyclohexyl)-6-methoxy-2H-indol-5-yl)-6-(trifluoromethyl) pyridinecarboxamide

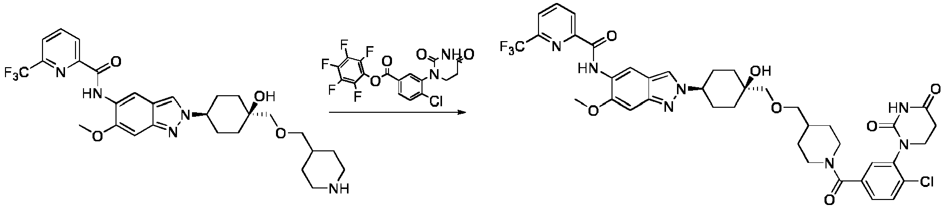

A mixed solution of *N*-(2-((1*S*,4*S*)-4-hydroxy-4-((piperidin-4-ylmethoxy)methyl)cyclohexyl)-6-methoxy-2H-indol-5-yl)-6-(trifluoromethyl)pyridinecarboxamide (130 mg, 0.23 mmol), pentafluorophenyl 4-chloro-3-(2,4-dioxotetrahydropyrimidin-1(2*H*)-yl)benzoate (121 mg, 0.28 mmol), and DIEA (89 mg, 0.69 mmol) in DMSO (6 mL) was stirred at room temperature overnight. The reaction liquid was diluted with water (50 mL), and the mixture was extracted with DCM (20 mL × 3). The organic phase was washed with saturated brine (60 mL), dried over anhydrous Na₂SO₄, filtered, and concentrated under reduced pressure. The resulting crude product was purified by Prep-HPLC (CH₃CN/O.08% aqueous NH₄HCO₃ solution, 5%-95%) to give *N*-(2-((1*S*,4*S*)-4-(((1-(4-chloro-3-(2,4-dioxotetrahydropyrimidin-1(2*H*)-yl)benzoyl)piperidin-4-yl)methoxy)methyl)-4-hydroxycyclohexyl)-6-methoxy-2*H*-indol-5-yl)-6-(trifluoromethyl)pyridinecarboxamide.

LC-MS: (ESI, *m*/*z):* [M+H] ⁺= 812.1.

¹H NMR (400 MHz, DMSO-*d*₆) δ 10.51 (d, *J =* 7.5 Hz, 2H), 8.69 (s, 1H), 8.46 *(d, J=* 7.7 Hz, 1H), 8.40 (t, *J* = 7.8 Hz, 1H), 8.32 (s, 1H), 8.21 (d, *J =* 7.6 Hz, 1H), 7.65 (d, *J* = 8.2 Hz, 1H), 7.57 (d*, J =* 1.4 Hz, 1H), 7.40 (dd, *J =* 8.2, 1.5 Hz, 1H), 7.16 (s, 1H), 4.58 - 4.29 (m, 3H), 3.99 (s, 3H), 3.84 - 3.55 (m, 3H), 3.33 - 3.28 (m, 2H), 3.22 (s, 2H), 3.13-3.03 (m, 1H), 2.88 - 2.70 (m, 3H), 2.35-2.20 (m, 2H), 1.94 - 1.59 (m, 9H), 1.21-1.15 (m, 2H).

### Example 34: N-(2-((1S,4S)-4-((2-(1-(4-chloro-3-(2,4-dioxotetrahydropyrimidin-1(2H)-yl)benzoyl)piperidin-4-yl)ethoxy)methyl)-4-hydroxycyclohexyl)-6-methoxy-2H-indol-5-yl)-6-(trifluoromethyl)pyridinecarboxamide

### Step 1: preparation of benzyl 4-(2-((4-((tert-butoxycarbonyl)amino)-1-hydroxycyclohexyl)methoxy)ethyl) piperidine-1-carboxylate

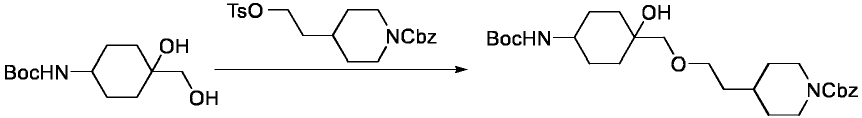

NaH (376 mg, 9.4 mmol) was added to a solution of *tert-*butyl (4-hydroxy-4-(hydroxymethyl)cyclohexyl) carbamate (770 mg, 3.13 mmol) in THF (20 mL) at 0 °C, and the mixture was stirred at 0 °C for 2 h. Then benzyl 4-(2-(*p*-toluenesulfonyl)ethyl)piperidine-1-carboxylate (2.62 g, 6.26 mmol) and KI (1.04 g, 6.26 mmol) were added. The reaction liquid was stirred at 70 °C overnight. The reaction liquid was diluted with water (50 mL), and the mixture was extracted with EA (30 mL × 3). The organic phase was washed with saturated brine (100 mL), dried over anhydrous Na₂SO₄, filtered, and concentrated under reduced pressure. The resulting crude product was purified by flash column chromatography (EA:PE = 0-3:2) to give benzyl 4-(2-((4-((*tert-*butoxycarbonyl)amino)-1-hydroxycyclohexyl)methoxy)ethyl)piperidine-1-carboxylate.

LC-MS: (ESI, *m*/*z):* [M+Na] ⁺ = 513.1.

¹H NMR (400 MHz, DMSO-*d*₆) δ 7.41 - 7.29 (m, 5H), 6.67 (d, *J* = 7.5 Hz, 1H), 5.06 (s, 2H), 4.05-3.90 (m, 3H), 3.42 (t, *J =* 6.4 Hz, 2H), 3.10 (s, 3H), 2.88-2.68 (m, 2H), 1.65 (d, *J =* 12.6 Hz, 2H), 1.55-1.32 (m, 20H), 1.13-0.92 (m, 2H).

### Step 2: preparation of benzyl 4-(2-((4-amino-1-hydroxycyclohexyl)methoxy)ethyl)piperidine-1-carboxylate

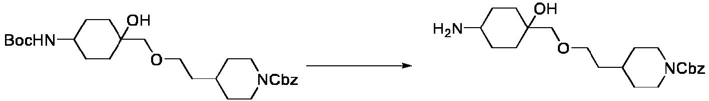

TFA (1.2 mL) was added to a solution of benzyl 4-(2-((4-((*tert*-butoxycarbonyl)amino)-1-hydroxycyclohexyl)methoxy)ethyl)piperidine-1-carboxylate (270 mg, 0.55 mmol) in DCM (3 mL) at -10 to 0 °C with stirring, and then the reaction liquid was stirred at room temperature for 2 h. DIEA was added to the reaction liquid to adjust the pH to 9.0, and the mixture was concentrated under reduced pressure to give benzyl 4-(2-((4-amino-1-hydroxycyclohexyl)methoxy)ethyl)piperidine-1-carboxylate.

LC-MS: (ESI, *m*/*z*): [M+H] ⁺= 391.2.

### Step 3: preparation of benzyl 4-(2-(((1S,4S)-1-hydroxy-4-(6-methoxy-5-nitro-2H-indol-2-yl)cyclohexyl) methoxy)ethyl)piperidine-1-carboxylate

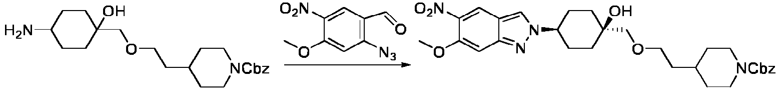

2-Azido-4-methoxy-5-nitrobenzaldehyde (150 mg, 0.66 mmol) was added to a solution of benzyl 4-(2-((4-amino-1-hydroxycyclohexyl)methoxy)ethyl)piperidine-1-carboxylate (400 mg, 0.55 mmol) in toluene (15 mL) with stirring. The reaction liquid was stirred at 100 °C for 3 h. The reaction liquid was concentrated under reduced pressure, and the resulting crude product was purified by flash column chromatography (EA:PE = 0-9:1) to give benzyl 4-(2-(((1*S*,4*S*)-1-hydroxy-4-(6-methoxy-5-nitro-2*H*-indol-2-yl)cyclohexyl)methoxy) ethyl)piperidine-1-carboxylate.

LC-MS: (ESI, *m*/*z):* [M+H] ⁺= 567.3.

¹H NMR (400 MHz, DMSO-*d*₆) δ 8.59 (s, 1H), 8.38 (s, 1H), 7.41 - 7.29 (m, 5H), 7.27 (s, 1H), 5.07 (s, 2H), 4.50-4.40 (m, 1H), 4.34 (s, 1H), 4.05-3.95 (m, 2H), 3.91 (s, 3H), 3.47 (t, *J* = 6.4 Hz, 2H), 3.21 (s, 2H), 2.90-2.70 (m, 2H), 2.35 - 2.19 (m, 2H), 1.90 (d, *J =* 9.7 Hz, 2H), 1.75-1.50 (m, 7H), 1.50-1.40 (m, 2H), 1.11 - 0.98 (m, 2H).

### Step 4: preparation of benzyl 4-(2-(((1S,4S)-4-(5-amino-6-methoxy-2H-indol-2-yl)-1-hydroxycyclohexyl) methoxy)ethyl)piperidine-1-carboxylate

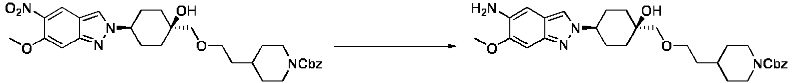

Raney-Ni (0.5 mL) and N₂H₄ H₂O (0.5 mL) were added to a solution of benzyl 4-(2-(((1*S*,4*S*)-1-hydroxy-4-(6-methoxy-5-nitro-2*H*-indol-2-yl)cyclohexyl)methoxy)ethyl)piperidine-1-carboxylate (250 mg, 0.44 mmol) in EtOH (20 mL). The reaction liquid was stirred at room temperature for 2 h. The reaction liquid was filtered through celite, and the filtrate was concentrated under reduced pressure to give benzyl 4-(2-(((1*S*,4*S*)-4-(5-amino-6-methoxy-2*H*-indol-2-yl)-1-hydroxycyclohexyl)methoxy)ethyl)piperidine-1-carboxylate. The resulting product was directly used in the next step.

LC-MS: (ESI, *m*/*z*): [M+H] ⁺= 537.3

### Step 5: preparation of benzyl 4-(2-(((1S,4S)-1-hydroxy-4-(6-methoxy-5-(6-(trifluoromethyl) pyridinecarboxamido)-2H-indol-2-yl)cyclohexyl)methoxy)ethyl)piperidine-1-carboxylate

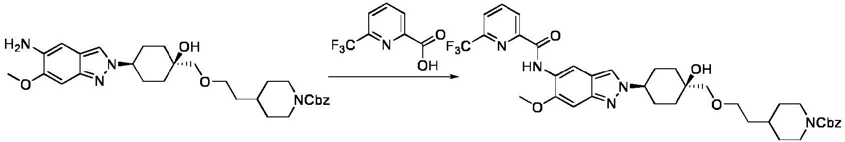

6-(Trifluoromethyl)picolinic acid (101 mg, 0.53 mmol), HATU (217 mg, 0.57 mmol), and DIEA (170 mg, 1.32 mmol) were added to a solution of benzyl 4-(2-(((1*S*,4*S*)-4-(5-amino-6-methoxy-2*H*-indol-2-yl)-1-hydroxycyclohexyl)methoxy)ethyl)piperidine-1-carboxylate (236 mg, 0.44 mmol) in DMF (5 mL) while stirring. Then the reaction liquid was stirred at room temperature for 2 h. The reaction liquid was diluted with water (50 mL), and the mixture was extracted with EA (20 mL × 3). The organic phase was washed with saturated brine (60 mL), dried over anhydrous Na₂SO₄, filtered, and concentrated under reduced pressure. The resulting crude product was purified by flash column chromatography (EA:PE = 0-4:1) to give benzyl 4-(2-(((1*S*,4*S*)-1-hydroxy-4-(6-methoxy-5-(6-(trifluoromethyl)pyridinecarboxamido)-2*H*-indol-2-yl)cyclohexyl) methoxy)ethyl)piperidine-1-carboxylate.

LC-MS: (ESI, *m*/*z*): [M+H] ⁺= 710.2.

¹H NMR (400 MHz, DMSO-*d*₆) δ 10.50 (s, 1H), 8.69 (s, 1H), 8.46 (d, *J* = 7.7 Hz, 1H), 8.40 (t, *J =* 7.8 Hz, 1H), 8.32 (s, 1H), 8.21 (d, *J =* 7.7 Hz, 1H), 7.41 - 7.28 (m, 5H), 7.16 (s, 1H), 5.06 (s, 2H), 4.40-4.20 (m, 2H), 4.10-3.90 (m, 5H), 3.47 (t, *J* = 6.4 Hz, 2H), 3.21 (s, 2H), 2.90-2.70 (m, 2H), 2.32 - 2.18 (m, 2H), 1.90 (d, *J=* 10.4 Hz, 2H), 1.72-1.53 (m, 7H), 1.52-1.40 (m, 2H), 1.15-1.00 (m, 2H).

### Step 6: preparation of N-(2-((1S,4S)-4-hydroxy-4-((2-(piperidin-4-yl)ethoxy)methyl)cyclohexyl)-6-methoxy-2H-indol-5-yl)-6-(trifluoromethyl)pyridinecarboxamide

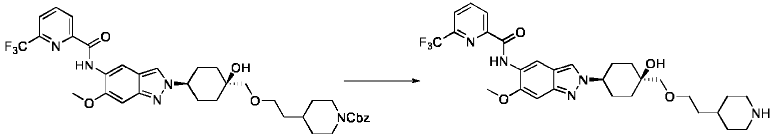

Pd(OH)₂/C (100 mg) was added to a solution of benzyl 4-(2-(((1*S*,4*S*)-1-hydroxy-4-(6-methoxy-5-(6-(trifluoromethyl)pyridinecarboxamido)-2*H*-indol-2-yl)cyclohexyl)methoxy)ethyl)piperidine-1-carboxylate (200 mg, 0.28 mmol) in EA (20 mL). Then the reaction liquid was stirred at 70 °C overnight under H₂ atmosphere. The reaction liquid was filtered through celite, and the filtrate was concentrated to give *N*-(2-((1*S*,4*S*)-4-hydroxy-4-((2-(piperidin-4-yl)ethoxy)methyl)cyclohexyl)-6-methoxy-2*H*-indol-5-yl)-6-(trifluoromethyl)pyridinecarboxamide. The resulting product was directly used in the next step.

LC-MS: (ESI, *m*/*z*): [M+H] ⁺= 576.2.

### Step 7: preparation of N-(2-((1S,4S)-4-((2-(1-(4-chloro-3-(2,4-dioxotetrahydropyrimidin-1(2H)-yl)benzoyl)piperidin-4-yl)ethoxy)methyl)-4-hydroxycyclohexyl)-6-methoxy-2H-indol-5-yl)-6-(trifluoromethyl) pyridinecarboxamide

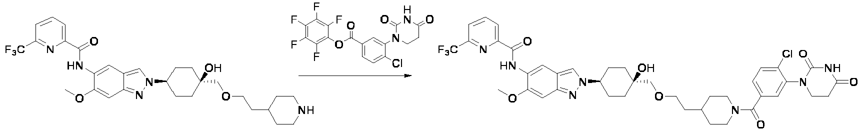

A solution of*N*-(2-((1*S*,4*S*)-4-hydroxy-4-((2-(piperidin-4-yl)ethoxy)methyl)cyclohexyl)-6-methoxy-2*H*-indol-5-yl)-6-(trifluoromethyl)pyridinecarboxamide (130 mg, 0.22 mmol), pentafluorophenyl 4-chloro-3-(2,4-dioxotetrahydropyrimidin-1(2*H*)-yl)benzoate (118 mg, 0.27 mmol), and DIEA (87 mg, 0.68 mmol) in DMSO (3 mL) was stirred at room temperature overnight. The reaction liquid was diluted with water (30 mL), and the mixture was extracted with DCM (10 mL × 3). The organic phase was washed with saturated brine (30 mL), dried over anhydrous Na₂SO₄, filtered, and concentrated under reduced pressure. The resulting crude product was purified by flash column chromatography (MeOH:DCM = 0-1:9) to give *N*-(2-((1*S*,4*S*)-4-((2-(1-(4-chloro-3-(2,4-dioxotetrahydropyrimidin-1(2*H*)-yl)benzoy])piperidin-4-yl)ethoxy)methyl)-4-hydroxycyclohexyl)-6-methoxy-2H-indol-5-yl)-6-(trifluoromethyl)pyridinecarboxamide.

LC-MS: (ESI, *m*/*z):* [M+H] ⁺= 826.1.

¹H NMR (400 MHz, DMSO-*d*₆) δ 10.51 (d, *J* = 5.7 Hz, 2H), 8.69 (s, 1H), 8.46 (d, *J =* 7.8 Hz, 1H), 8.41 (t,*J=* 7.8 Hz, 1H), 8.33 (s, 1H), 8.22 (d, *J =* 7.7 Hz, 1H), 7.64 (d, *J* = 8.2 Hz, 1H), 7.55 (d, *J =* 1.8 Hz, 1H), 7.39 (dd, *J =* 8.2, 1.8 Hz, 1H), 7.16 (s, 1H), 4.50-4.26 (m, 3H), 3.98 (s, 3H), 3.83 - 3.53 (m, 3H), 3.48 (t, *J =* 6.3 Hz, 2H), 3.21 (s, 2H), 3.13-2.97 (m, 1H), 2.86 - 2.71 (m, 3H), 2.32 - 2.18 (m, 2H), 1.90 (d, *J* = 9.8 Hz, 2H), 1.85-1.45 (m, 9H), 1.15-1.05 (m, 2H).

### Example 35: N-(2-((1S,4S)-4-((((3-(4-chloro-3-(2,4-dioxotetrahydropyrimidin-1(2H)-yl)benzoyl)-3-azaspiro[5.5] undecan-9-yl)methyl)(methyl)amino)methyl)-4-hydroxycyclohexyl)-6-methoxy-2H-indol-5-yl)-6-(trifluoromethyl)pyridinecarboxamide

### Step 1: preparation of tert-butyl (4-hydroxy-4-(methylamino)methyl)cyclohexyl)carbamate

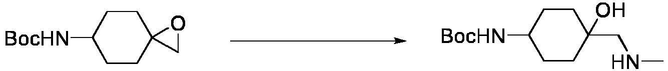

Methylamine (40 mL, 25%-30% aqueous solution) was added to a mixed solution of *tert*-butyl (1-oxaspiro[2.5]octan-6-yl)carbamate (4.2 g, 18.42 mmol) in EtOH/H₂O (90 mL/15 mL). Then the reaction liquid was stirred at 25 °C overnight. The reaction liquid was directly concentrated under reduced pressure, and the resulting crude product was purified by flash chromatography (MeOH:DCM = 0-1:1) to give *tert-*butyl (4-hydroxy-4-(methylamino)methyl)cyclohexyl)carbamate.

LC-MS: (ESI, *m*/*z):* [M+H] ⁺= 259.2.

### Step 2: preparation of benzyl ((4-((tert-butoxycarbonyl)amino)-1-hydroxycyclohexyl)methyl)(methyl)carbamate

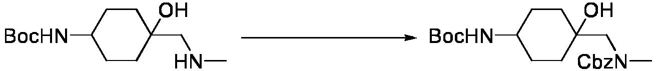

A mixed solution of *tert-*butyl (4-hydroxy-4-(methylamino)methyl)cyclohexyl)carbamate (1.2 g, 4.61 mmol) and benzyl chloroformate (941 mg, 5.53 mmol) in ACN/sat. NaHCO₃ (20 mL/20 mL) was stirred at room temperature overnight. The reaction liquid was diluted with water (50 mL), and then the mixture was extracted with EA (50 mL × 3). The organic phase was washed with saturated brine (150 mL), dried over anhydrous Na₂SO₄, filtered, and concentrated under reduced pressure. The resulting crude product was purified by flash chromatography (EA:PE = 0-7:3) to give benzyl ((4-((*tert*-butoxycarbonyl)amino)-1-hydroxycyclohexyl) methyl)(methyl)carbamate.

LC-MS: (ESI, *m*/*z*): [M+Na] ⁺ = 415.2.

### Step 3: preparation of benzyl ((4-amino-1-hydroxycyclohexyl)methyl)(methyl)carbamate

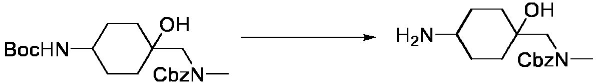

TFA (5 mL) was added to a solution of benzyl ((4-((*tert*-butoxycarbonyl)amino)-1-hydroxycyclohexyl) methyl)(methyl)carbamate (1.5 g, 3.81 mmol) in DCM (15 mL) at 0 °C while stirring, and then the reaction liquid was stirred at 0 °C for 2 h. The pH of the reaction liquid was adjusted to 9.0 with DIEA, and then the mixture was directly concentrated under reduced pressure to give benzyl ((4-amino-1-hydroxycyclohexyl) methyl)(methyl)carbamate. The resulting crude product was directly used in the next step.

LC-MS: (ESI, *m*/*z*): [M+H] ⁺= 293.1.

### Step 4: preparation of benzyl (((1S,4S)-1-hydroxy-4-(6-methoxy-5-nitro-2H-indol-2-yl)cyclohexyl)methyl) (methyl)carbamate

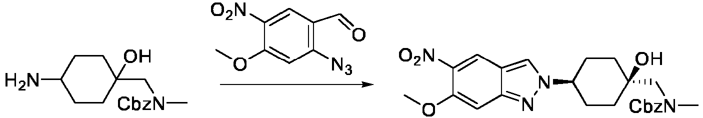

2-Azido-4-methoxy-5-nitrobenzaldehyde (1.2 g, 5.7 mmol) was added to a solution of benzyl ((4-amino-1-hydroxycyclohexyl)methyl)(methyl)carbamate (1.5 g, 3.8 mmol) in toluene (40 mL) with stirring. The reaction liquid was stirred at 100 °C for 3 h. The reaction liquid was directly concentrated under reduced pressure. The resulting crude product was purified by flash column chromatography (EA:PE = 0-1) to give benzyl (((1*S*,4*S*)-1-hydroxy-4-(6-methoxy-5-nitro-2*H*-indol-2-yl)cyclohexyl)methyl)(methyl)carbamate.

LC-MS: (ESI, *m*/*z*): [M+H] ⁺ = 469.2

¹H NMR (400 MHz, DMSO-*d*₆) δ 8.58 (s, 1H), 8.38 (s, 1H), 7.45 - 7.23 (m, 6H), 5.09 (s, 2H), 4.66-4.36 (m, 2H), 3.91 (s, 3H), 3.28 (s, 2H), 3.10-2.95 (m, 3H), 2.30-2.20 (m, 2H), 1.95-1.60 (m, 2H), 1.78-1.63 (m, 2H), 1.59 - 1.41 (m, 2H).

### Step 5: preparation of benzyl (((1S,4S)-4-(5-amino-6-methoxy-2H-indol-2-yl)-1-hydroxycyclohexylmethyl) (methyl)carbamate

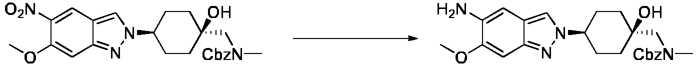

N₂H₄ H₂O (1.2 mL) and Raney-Ni (0.5 mL) were added to a solution of benzyl (((1*S*,4*S*)-1-hydroxy-4-(6-methoxy-5-nitro-2*H*-indol-2-yl)cyclohexyl)methyl)(methyl)carbamate (1.1 g, 2.34 mmol) in EtOH (20 mL). The reaction liquid was stirred at room temperature for 2 h. The reaction liquid was filtered through celite, and the filtrate was concentrated under reduced pressure to give benzyl (((1*S*,4*S*)-4-(5-amino-6-methoxy-2*H*-indol-2-yl)-1-hydroxycyclohexylmethyl)(methyl)carbamate. The resulting crude product was directly used in the next step.

LC-MS: (ESI, *m*/*z):* [M+H] ⁺= 439.2

### Step 6: preparation of benzyl (((1S,4S)-1-hydroxy-4-(6-methoxy-5-(6-(trifluoromethyl)pyridineformyl)-2H-indol-2-yl)cyclohexyl)methyl)(methyl)carbamate

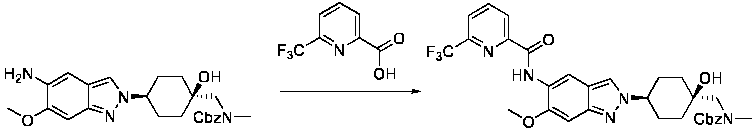

6-(Trifluoromethyl)picolinic acid (538 mg, 2.78 mmol), HATU (1.06 g, 2.78 mmol), and DIEA (828 mg, 6.42 mmol) were added to a solution of benzyl (((1*S*,4*S*)-4-(5-amino-6-methoxy-2*H*-indol-2-yl)-1-hydroxycyclohexylmethyl)(methyl)carbamate (940 mg, 2.14 mmol) in DMF (10 mL) with stirring. Then the reaction liquid was stirred at room temperature overnight. The reaction liquid was diluted with water (100 mL), and the mixture was extracted with EA (30 mL × 3). The organic phase was washed with saturated brine (100 mL), dried over anhydrous Na₂SO₄, filtered, and concentrated under reduced pressure. The resulting crude product was purified by flash column chromatography (EA:PE = 0-9:1) to give benzyl (((1*S*,4*S*)-1-hydroxy-4-(6-methoxy-5-(6-(trifluoromethyl)pyridineformyl)-2*H*-indol-2-yl)cyclohexyl)methyl)(methyl)carbamate.

LC-MS: (ESI, *m*/*z):* [M+H] ⁺= 612.2.

¹H NMR (400 MHz, DMSO-*d*₆) δ 10.50 (s, 1H), 8.69 (s, 1H), 8.46 (d, *J=* 7.5 Hz, 1H), 8.40 (t, *J =* 7.8 Hz, 1H), 8.31 (s, 1H), 8.21 (dd, *J =* 7.7, 1.0 Hz, 1H), 7.42 - 7.28 (m, 5H), 7.16 (s, 1H), 5.09 (s, 2H), 4.60-4.31 (m, 2H), 3.98 (s, 3H), 3.28 (s, 2H), 3.10-2.95 (m, 3H), 2.30-2.15 (m, 2H), 1.95-1.60 (m, 2H), 1.67 (t, *J* = 14.9 Hz, 2H), 1.57 - 1.40 (m, 2H).

### Step 7: preparation of N-(2-((1S,4S)-4-hydroxy-4-((methylamino)methyl)cyclohexyl)-6-methoxy-2H-indol-5-yl)-6-(trifluoromethyl)pyridinecarboxamide

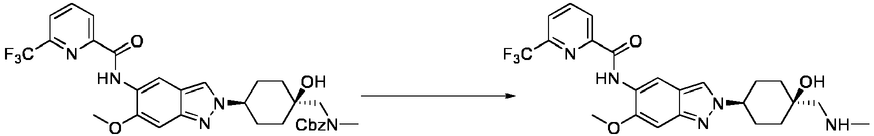

Pd(OH)₂/C (200 mg) was added to a solution of benzyl (((1*S*,4*S*)-1-hydroxy-4-(6-methoxy-5-(6-(trifluoromethyl)pyridineformyl)-2*H*-indol-2-yl)cyclohexyl)methyl)(methyl)carbamate (500 mg, 0.81 mmol) in EA (20 mL). Then the reaction liquid was stirred at 70 °C overnight under H₂ atmosphere. The reaction liquid was filtered through celite, and the filtrate was concentrated under reduced pressure to give *N*-(2-((1*S*,4*S*)-4-hydroxy-4-((methylamino)methyl)cyclohexyl)-6-methoxy-2*H*-indol-5-yl)-6-(trifluoromethyl)pyridinecarboxamide. The resulting crude product was directly used in the next step. LC-MS: (ESI, *m*/*z*): [M+H] ⁺= 478.1.

### Step 8: preparation of tert-butyl 9-(((((1S,4S)-1-hydroxy-4-(6-methoxy-5-(6-(trifluoromethyl) pyridinecarboxamido)-2H-indol-2-yl)cyclohexyl)methyl)(methyl)amino)methyl)-3-azaspiro[5.5]undecane-3-carboxylate

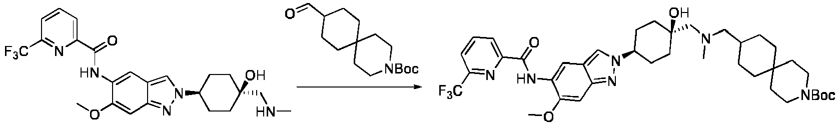

A mixed solution of *N*-(2-((1*S*,4*S*)-4-hydroxy-4-((methylamino)methyl)cyclohexyl)-6-methoxy-2*H*-indol-5-yl)-6-(trifluoromethyl)pyridinecarboxamide (200 mg, 0.41 mmol), *tert*-butyl 9-formyl-3-azaspiro[5.5]undecane-3-carboxylate (141 mg, 0.50 mmol), and STAB (434 mg, 2.05 mmol) in THF (10 mL) was stirred at room temperature overnight. The reaction liquid was diluted with water (30 mL), and the mixture was extracted with DCM (10 mL × 3). The organic phase was washed with saturated brine (30 mL), dried over anhydrous Na₂SO₄, filtered, and concentrated under reduced pressure. The resulting crude product was purified by flash column chromatography (MeOH:DCM = 0-1:9) to give *tert-*butyl 9-(((((1*S*,4*S*)-1-hydroxy-4-(6-methoxy-5-(6-(trifluoromethyl)pyridinecarboxamido)-2*H*-indol-2-yl)cyclohexyl)methyl)(methyl)amino)methyl)-3-azaspiro [5.5]undecane-3-carboxylate.

LC-MS: (ESI, *m*/*z):* [M+H] ⁺ = 743.3.

### Step 9: preparation of N-(2-((1S,4S)-4-((((3-azaspiro[5.5]undecan-9-yl)methyl)(methyl)amino)methyl)-4-hydroxycyclohexyl)-6-methoxy-2H-indol-5-yl)-6-(trifluoromethyl)pyridinecarboxamide

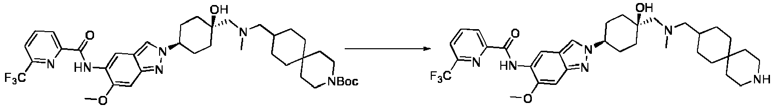

TFA (2 mL) was added to a solution of *tert-*butyl 9-(((((1*S*,4*S*)-1-hydroxy-4-(6-methoxy-5-(6-(trifluoromethyl)pyridinecarboxamido)-2*H*-indol-2-yl)cyclohexyl)methyl)(methyl)amino)methyl)-3-*a*zaspiro[5.5]undecane-3-carboxylate (200 mg, 0.27 mmol) in DCM (5 mL) at 0 °C. Then the reaction liquid was stirred at room temperature for 2 h. The pH of the reaction liquid was adjusted to 9.0 with DIE. The organic phase was washed with water (100 mL), dried over anhydrous Na₂SO₄, filtered, and concentrated under reduced pressure to give *N*-(2-((1*S,*4*S*)-4-((((3-azaspiro[5.5]undecan-9-yl)methyl)(methyl)amino)methyl)-4-hydroxycyclohexyl)-6-methoxy-2*H*-indol-5-yl)-6-(trifluoromethyl)pyridinecarboxamide. The resulting crude product was directly used in the next step.

LC-MS: (ESI, *m*/*z*): [M+H] ⁺ = 643.3.

### Step 10: preparation of (2-((1S,4S)-4-((((3-(4-chloro-3-(2,4-dioxotetrahydropyrimidin-1(2H)-yl)benzoyl)-3-azaspiro[5.5]undecan-9-yl)methyl)(methyl)amino)methyl)-4-hydroxycyclohexyl)-6-methoxy-2H-indol-5-yl)-6-(trifluoromethyl)pyridinecarboxamide

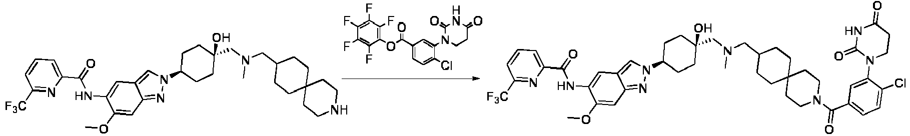

A mixed solution of *N*-(2-((1*S*,4*S*)-4-((((3-azaspiro[5.5]undecan-9-yl)methyl)(methyl)amino)methyl)-4-hydroxycyclohexyl)-6-methoxy-2*H*-indol-5-yl)-6-(trifluoromethyl)pyridinecarboxamide (130 mg, 0.20 mmol), pentafluorophenyl 4-chloro-3-(2,4-dioxotetrahydropyrimidin-1(2*H*)-yl)benzoate (105 mg, 0.24 mmol), and DIEA (77 mg, 0.60 mmol) in DMSO (5 mL) was stirred at room temperature overnight. The reaction liquid was diluted with water (50 mL), and then the mixture was extracted with DCM (20 mL × 3). The organic phase was washed with saturated brine (60 mL), dried over anhydrous Na₂SO₄, filtered, and concentrated under reduced pressure. The resulting crude product was purified by Prep-HPLC (CH₃CN/O.08% aqueous NH₄HCO₃ solution, 5%-95%) to give *N*-(2-((1*S*,4*S*)-4-((((3-(4-chloro-3-(2,4-dioxotetrahydropyrimidin-1(2*H*)-yl)benzoyl)-3-azaspiro[5.5]undecan-9-yl)methyl)(methyl)amino)methyl)-4-hydroxycyclohexyl)-6-methoxy-2*H*-indol-5-yl)-6-(trifluoromethyl)pyridinecarboxamide.

LC-MS: (ESI, *m*/*z):* [M+H] ⁺= 893.1.

¹H NMR (400 MHz, DMSO-*d*₆) δ 10.51 (s, 2H), 8.69 (s, 1H), 8.47 (d, *J =* 7.8 Hz, 1H), 8.41 (t, *J =* 7.8 Hz, 1H), 8.32 (s, 1H), 8.22 (d, *J =* 7.8 Hz, 1H), 7.63 (d, *J =* 8.2 Hz, 1H), 7.55 (d, *J =* 1.7 Hz, 1H), 7.39 (d, *J* = 8.3 Hz, 1H), 7.15 (s, 1H), 4.37-4.27 (m, 1H), 4.10-3.90 (m, 4H), 3.82 - 3.49 (m, 4H), 3.35-3.25 (m, 1H), 2.80-2.70 (m, 2H), 2.36-2.15 (m, 9H), 1.95-1.85 (m, 2H), 1.78 - 1.22 (m, 14H), 1.18-0.98 (m, 4H).

### Example 36: N-(2-((1S,4S)-4-((2-(1-(4-chloro-3-(2,4-dioxotetrahydropyrimidin-1(2H)-yl)benzoyl)piperidin-4-yl)ethoxy)methyl)-4-methoxycyclohexyl)-6-methoxy-2H-indol-5-yl)-6-(trifluoromethyl)picolinamide Step 1: preparation of benzyl 4-(2-(((1S,4S)-4-(6-methoxy-5-nitro-2H-indazol-2-yl)-1-((methylthio)methoxy) cyclohexyl)methoxy)ethyl)piperidine-1-carboxylate

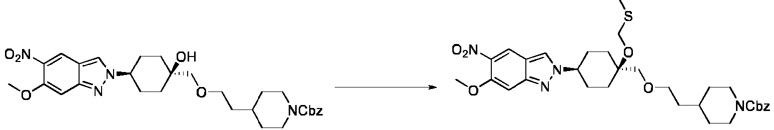

The compound benzyl 4-(2-(((1*S*,4*S*)-1-hydroxy-4-(6-methoxy-5-nitro-2*H*-indazol-2-yl)cyclohexyl)methoxy) ethyl)piperidine-1-carboxylate (700 mg, 1.24 mmol) and Ac₂O (151 mg, 1.48 mmol) were dissolved in anhydrous DMSO (20 mL), and the mixture was stirred at room temperature for 16 h under nitrogen atmosphere. Water (50 mL) was added to the reaction liquid, and the mixture was extracted with ethyl acetate (50 mL × 3). The organic phase was washed with saturated brine (150 mL), dried over anhydrous sodium sulfate, filtered, and concentrated under reduced pressure. The crude product was purified by column chromatography (ethyl acetate: petroleum ether = 1:9) to give benzyl 4-(2-(((1*S*,4*S*)-4-(6-methoxy-5-nitro-2H-indazol-2-yl)-1-((methylthio)methoxy)cyclohexyl)methoxy)ethyl)piperidine-1-carboxylate.

LC-MS: (ESI, m/z): [M+H] ⁺= 627.2.

### Step 2: preparation of benzyl 4-(2-(((1S,4S)-4-(5-amino-6-methoxy-2H-indazol-2-yl)-1-methoxycyclohexyl) methoxy)ethyl)piperidine-1-carboxylate

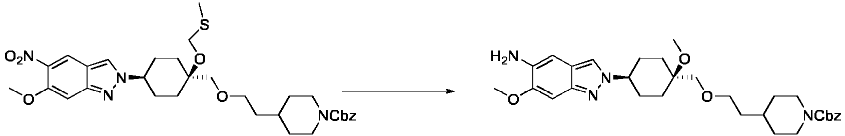

The compound benzyl 4-(2-(((1*S*,4*S*)-4-(6-methoxy-5-nitro-2*H*-indazol-2-yl)-1-((methylthio)methoxy) cyclohexyl)methoxy)ethyl)piperidine-1-carboxylate (600 mg, 0.96 mmol) and pretreated Raney-Ni (200 mg) (pretreatment method: washing with water 3 times, then washing with acetone 3 times, and finally washing with ethanol 3 times) were dissolved in anhydrous ethanol (50 mL), and the mixture was stirred at room temperature for 3 h under hydrogen atmosphere and filtered. The filtrate was directly concentrated under reduced pressure, and the crude product was purified by reversed-phase silica gel column chromatography (ACN:H₂O = 1:1, flow rate 40 mL/min) to give benzyl 4-(2-(((1*S*,4*S*)-4-(5-amino-6-methoxy-2H-indazol-2-yl)-1-methoxycyclohexyl)methoxy)ethyl)piperidine-1-carboxylate.

LC-MS: (ESI, m/z): [M+H] ⁺= 551.3.

### Step 3: preparation of benzyl 4-(2-(((1S,4S)-1-methoxy-4-(6-methoxy-5-(6-(trifluoromethyl) pyridinecarboxamido)-2H-indazol-2-yl)cyclohexyl)methoxy)ethyl)piperidine-1-carboxylate

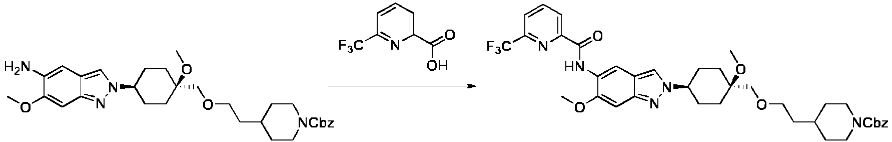

The compound 6-(trifluoromethyl)picolinic acid (60 mg, 0.3 mmol), HATU (114 mg, 0.3 mmol), and DIEA (100 mg, 0.78 mmol) were dissolved in DMF (10 mL), and the mixture was stirred at room temperature for 20 min. Benzyl 4-(2-(((1*S*,4*S*)-4-(5-amino-6-methoxy-2H-indazol-2-yl)-1-methoxycyclohexyl)methoxy)ethyl) piperidine-1-carboxylate (150 mg, 0.26 mmol) was added, and the mixture was reacted at room temperature for 1 h. Water (100 mL) was added to the reaction liquid, and the mixture was extracted with ethyl acetate (30 mL × 3). The organic phase was washed with saturated brine (100 mL), dried over anhydrous sodium sulfate, filtered, and concentrated under reduced pressure. The crude product was purified by column chromatography (ethyl acetate:petroleum ether = 1:1) to give benzyl 4-(2-(((1*S*,4*S*)-1-methoxy-4-(6-methoxy-5-(6-(trifluoromethyl)pyridinecarboxamido)-2*H*-indazol-2-yl)cyclohexyl)methoxy)ethyl)piperidine-1-carboxylate. LC-MS: (ESI, m/z): [M+H] ⁺= 724.4.

### Step 4: preparation of N-(6-methoxy-2-((1S,4S)-4-methoxy-4-((2-(piperidin-4-yl)ethoxy)methyl)cyclohexyl)-2H-indazol-5-yl)-6-(trifluoromethyl)pyridinecarboxamide

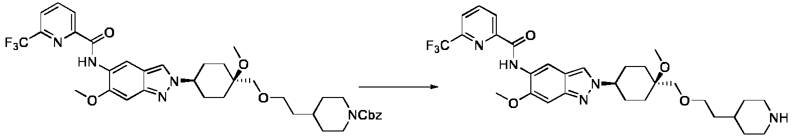

Benzyl 4-(2-(((1*S*,4*S*)-1-methoxy-4-(6-methoxy-5-(6-(trifluoromethyl)pyridinecarboxamido)-2*H*-indazol-2-yl)cyclohexyl)methoxy)ethyl)piperidine-1-carboxylate (70 mg, 0.097 mmol) was dissolved in trifluoroacetic acid (3 mL), and the mixture was heated to 90 °C and stirred for 1 h. The reaction liquid was directly concentrated under reduced pressure to give *N*-(6-methoxy-2-((1*S*,4*S*)-4-methoxy-4-((2-(piperidin-4-yl)ethoxy)methyl)cyclohexyl)-2*H*-indazol-5-yl)-6-(trifluoromethyl)pyridinecarboxamide, which was directly used in the next step without purification.

LC-MS: (ESI, m/z): [M+H] ⁺ = 590.4.

### Step 5: preparation of N-(2-((1S,4S)-4-((2-(1-(4-chloro-3-(2,4-dioxotetrahydropyrimidin-1(2H)-yl)benzoyl) piperidin-4-yl)ethoxy)methyl)-4-methoxycyclohexyl)-6-methoxy-2H-indazol-5-yl)-6-(trifluoromethyl) pyridinecarboxamide

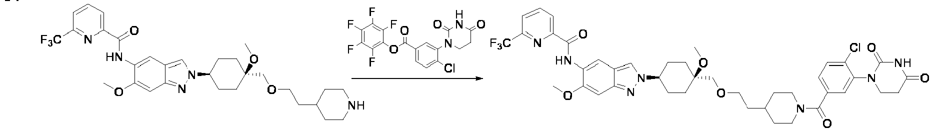

*N*-(6-Methoxy-2-((1*S*,4*S*)-4-methoxy-4-((2-(piperidin-4-yl)ethoxy)methyl)cyclohexyl)-2*H*-indazol-5-yl)-6-(trifluoromethyl)pyridinecarboxamide (70 mg, 0.097 mmol) was dissolved in dimethyl sulfoxide (3 mL). Pentafluorophenyl 4-chloro-3-(2,4-dioxotetrahydropyrimidin-1(2*H*)-yl)benzoate (48 mg, 0.11 mmol) and*N,N-*diisopropylethylamine (37 mg, 0.29 mmol) were added with stirring. After the mixture was stirred at room temperature for 2 h, water (50 mL) was added, and the mixture was extracted with dichloromethane (20 mL × 3). The organic phase was washed with saturated brine (3 × 60 mL), dried over anhydrous sodium sulfate, filtered, and concentrated under reduced pressure to give a crude product. The crude product was purified by pre-HPLC (CH₃CN/0.08% aqueous NH₄HCO₃ solution, 5%-95%) to give *N*-(2-((1*S*,4*S*)-4-((2-(1-(4-chloro-3-(2,4-dioxotetrahydropyrimidin-1(2*H*)-yl)benzoyl)piperidin-4-yl)ethoxy)methyl)-4-methoxycyclohexyl)-6-methoxy-2*H*-indazol-5-yl)-6-(trifluoromethyl)pyridinecarboxamide.

LC-MS: (ESI, m/z): [M+H] ⁺= 840.3.

¹H NMR (400 MHz, DMSO-*d*₆) δ 10.51 (d, *J* = 4.5 Hz, 2H), 8.68 (s, 1H), 8.49 - 8.37 (m, 2H), 8.32 (s, 1H), 8.22 (d, *J* = 7.6 Hz, 1H), 7.64 (d, *J =* 8.2 Hz, 1H), 7.55 (d, *J* = 1.9 Hz, 1H), 7.39 (dd, *J =* 8.2, 2.0 Hz, 1H), 7.18 (s, 1H), 4.52-4.30 (m, 2H), 3.98 (s, 3H), 3.80 - 3.52 (m, 3H), 3.47 (t, *J* = 6.2 Hz, 2H), 3.34 (s, 2H), 3.19 (s, 3H), 3.15-2.95 (m, 1H), 2.85 - 2.70 (m, 3H), 2.19-1.97 (m, 2H), 1.96-1.85 (m, 4H), 1.85-1.55 (m, 3H), 1.55-1.45 (m, 4H), 1.25-1.05 (m, 2H).

### II. Biological Activity Test Examples

### Test Example 1: IRAK4 Kinase Activity Test

The KinEASE-STK S1 serine/threonine kinase kit (Cisbio) was used to test the inhibitory effects of the compounds on the IRAK4 kinase activity, and the specific procedures were as follows: A compound was dissolved in dimethyl sulfoxide, and the solution was diluted in the buffer of the kit with an equal gradient, such that the final concentration range of the test compound in the reaction systems was 10000-0.038 nM. Then 2.5 nM IRAK4 kinase (Carna), 1 µM biotinylated polypeptide substrate (Cisbio), and 7 µM adenosine triphosphate (Sigma-Aldrich) were sequentially added, and the mixtures were incubated at 37 °C for 120 min. Subsequently, an anti-phosphoserine/threonine antibody (Cisbio) conjugated with a europium element compound and streptavidin conjugated with a modified XL665 (Cisbio) were added to the reaction system to stop the reaction. After 1 h of incubation at room temperature, the fluorescence intensity of each well was measured at the excitation wavelength of 337 nm and the emission wavelengths of 620 nm and 665 nm with a microplate reader EnVision (PerkinElmer) in the HTRF mode, and Ratio values were calculated using the formula: Ratio = (665 nm/620 nm) × 10⁴. The inhibition rates of the compounds at each concentration were calculated by comparison with the fluorescence intensity ratio of the control group. Then non-linear curve fittings for logarithmic concentration-inhibition rate were performed using GraphPad Prism 7, and the IC₅₀ values of the compounds were obtained.

**Table 1:**

| Example | IC₅₀ (nM) |
|---|---|
| Example 1 | 30.3 |
| Example 2 | 15.5 |
| Example 3 | 19.3 |
| Example 4 | 14.2 |
| Example 5 | 3.8 |
| Example 6 | 8.8 |
| Example 7 | 9.9 |
| Example 8 | 17.04 |
| Example 9 | 4.64 |
| Example 10 | 8.05 |
| Example 11 | 7.74 |
| Example 12 | 6.08 |
| Example 13 | 11.03 |
| Example 14 | 3.36 |
| Example 15 | 3.86 |
| Example 16 | 12.87 |
| Example 17 | 9.82 |
| Example 18 | 7.86 |
| Example 19 | 36.07 |
| Example 20 | 18.64 |
| Example 21 | 13.45 |
| Example 22 | 21.31 |
| Example 23 | 7.05 |
| Example 24 | 36.74 |
| Example 25 | 3.87 |
| Example 26 | 4.57 |
| Example 27 | 2.98 |
| Example 28 | 21.94 |
| Example 29 | 36.24 |
| Example 30 | 5.93 |
| Example 31 | 22.96 |
| Example 32 | 17.04 |
| Example 33 | 10.6 |
| Example 34 | 12.92 |
| Example 35 | 15.1 |
| Example 36 | 9.29 |

Experimental results: The compounds of the present disclosure are capable of effectively binding to the target protein and inhibiting IRAK4 kinase activity.

### Test Example 2: Degradation of IRAK4 in THP-1 Cells by Compounds

THP-1 cells (Stem Cell Bank, Chinese Academy of Sciences) were inoculated onto a 24-well cell culture plate at 0.95 mL/well, with a cell density of 5 × 10⁵ cells/well. The cell plate was incubated in an incubator at 37 °C with 5% carbon dioxide overnight, and 50 µL of a solution of a compound in dimethyl sulfoxide was added, such that the final concentration of the compound fell within the range of 0.03-3000 nM. After another 24 h of incubation, cells were collected into 1.5 mL centrifuge tubes and centrifuged at 1000 rpm at 4 °C for 5 min. The cell pellets were washed twice with 1× DPBS. The resuspended cells were lysed with 200 µL of lysis buffer (Western and IP cell lysis buffer (Beyotime), supplemented with 1 mM phenylmethylsulfonyl fluoride and a protease inhibitor cocktail (Beyotime)), left to stand on ice for 30 min, and then centrifuged at 14000 g at 4 °C for 10 min. The supernatant samples were assayed by Western Blot for IRAK4 protein levels.

### Human IRAK4 AlphaLISA Assay

The above cell lysates were also tested for IRAK4 degradation using a human IRAK4 AlphaLISA assay kit (PerkinElmer, AL3117C). The method is specifically as follows: 2-µL test samples of cell lysate supernatant were added to a white 384-well plate (PerkinElmer, 6007299), and 4 µL of 5× anti-IRAK4 receptor beads (PerkinElmer, final concentration of 10 µg/mL) was then added. The plate was incubated at 23 °C for 30 min. Then 4 µL of 5× biotin-labeled anti-IRAK4 antibody (PerkinElmer, final concentration of 1 nM) was added, and the plate was incubated at 23 °C for 60 min. Finally, 10 µL of 2× streptavidin-labeled donor beads (final concentration of 40 µg/mL) was added to each well, and the plate was incubated in the dark at 23 °C for 30 min. After the incubation was completed, AlphaLISA signal values were measured on a microplate reader EnVision (PerkinElmer, 2105) in the AlphaScreen standard mode, and IRAK4 concentrations were calculated from these signal values. Then non-linear curve fittings for logarithmic concentration-inhibition rate were performed using GraphPad Prism 7, and the DC₅₀ and Dₘₐₓ values of the compound were obtained.

**Table 2:**

| Example | DC₅₀ (nM) | Dₘₐₓ (%) |
|---|---|---|
| Example 1 | 3.7 | 90.0 |
| Example 2 | 1.8 | 95.8 |
| Example 3 | 5.5 | 84.4 |
| Example 4 | 2.5 | 92.1 |
| Example 5 | 5.1 | 97.7 |
| Example 6 | 6.4 | 87.9 |
| Example 7 | 9.6 | 95.5 |
| Example 8 | 2.75 | 98.48 |
| Example 9 | 5.23 | 90.09 |
| Example 10 | 6.10 | 91.10 |
| Example 11 | 3.46 | 90.53 |
| Example 12 | 6.16 | 96.32 |
| Example 13 | 3.55 | 87.11 |
| Example 14 | 0.78 | 100 |
| Example 15 | 3.45 | 96.02 |
| Example 16 | 3.18 | 89.42 |
| Example 17 | 8.13 | 88.18 |
| Example 18 | 4.84 | 99.01 |
| Example 19 | 5.55 | 94.41 |
| Example 20 | 3.66 | 91.70 |
| Example 21 | 6.30 | 80.45 |
| Example 22 | 7.52 | 79.18 |
| Example 23 | 3.22 | 97.07 |
| Example 24 | 5.94 | 82.09 |
| Example 25 | 0.41 | 96.44 |
| Example 26 | 0.58 | 96.57 |
| Example 27 | 0.82 | 96.16 |
| Example 28 | 9.36 | 96.59 |
| Example 29 | 9.18 | 79.3 |
| Example 30 | 7.47 | 96.06 |
| Example 31 | 10.10 | 93.65 |
| Example 32 | 10.86 | 93.89 |
| Example 33 | 8.8 | 86.8 |
| Example 34 | 2.8 | 99.3 |
| Example 35 | 10.37 | 99.4 |
| Example 36 | 3.42 | 96.53 |

Experimental results: The compounds of the present disclosure can effectively degrade the IRAK4 kinase protein in cells, and have excellent degradation activity.

### Test Example 3: Determination of Concentration of Cytokines Secreted by LPS-Induced Normal Human Whole Blood and PBMCs

### (1) Determination of concentration of cytokines secreted by LPS-induced normal human whole blood

Fresh normal human whole blood (healthy people aged 22-50, freshly collected blood) anticoagulated with heparin sodium was plated onto a 96-well clear cell plate (Labserv, 310109008) at 190 µL/well, and 10 µL of diluted compound solution was then added to corresponding wells, such that the final concentration of the compound fell within the range of 10-20000 nM. The final concentration of dimethyl sulfoxide was 0.1%. The compound-treated cell plate was sealed with a plate-sealing membrane. After the plate was incubated in an incubator at 37 °C with 5% carbon dioxide for 20 h, 10 µL of LPS (0111:B4) (Sigma, L4391) was added, making a final concentration of 100 ng/mL. The cell plate was sealed with a plate-sealing membrane. After the plate was incubated in an incubator at 37 °C with 5% carbon dioxide for another 5 h, the plate was centrifuged at 2000 rpm for 10 min, and 30 µL of supernatant plasma was transferred from each well to a new 96-well clear cell plate for cytokine concentration testing and cryopreserved at -80 °C before testing.

Interleukin-6 (IL-6) AlphaLISA assay.

Supernatant plasma samples of LPS-induced human whole blood (healthy people aged 22-50, freshly collected blood) were assayed for IL-6 concentrations using an interleukin-6 (IL-6) AlphaLISA assay kit (PerkinElmer, AL223 C).

IL-6 standard solutions with different concentrations, ranging from 0 to 100000 pg/mL, were prepared by following the instructions for use of the product. 2 µL of each of the IL-6 standard solutions and test samples of cell supernatant were added to a white 384-well plate (PerkinElmer, 6007299), and 8 µL of a mixed solution of 5× anti-IL-6 receptor beads (PerminElmer, final concentration of 10 µg/mL) and biotin-labeled anti-IL-6 antibody (PerminElmer, final concentration of 1 nM) was then added to each well. The plate was incubated at 23 °C for 60 min. Finally, 10 µL of 2× streptavidin-labeled donor beads (final concentration of 40 µg/mL) was added to each well, and the plate was incubated in the dark at 23 °C for another 30 min. After the incubation was completed, AlphaLISA signal values were measured on a microplate reader EnVision (PerkinElmer, 2105) in the AlphaScreen standard mode. A standard curve was plotted from the AlphaLISA signal values of the IL-6 standard solutions with different concentrations, and the IL-6 concentration of each cell lysate supernatant was determined according to the corresponding concentrations on the standard curve for the AlphaLISA signal values of the test samples. The inhibition rates of the compounds at each concentration were calculated by comparing the IL-6 concentration with that of the control group. Then non-linear curve fittings for logarithmic concentration-inhibition rate were performed using GraphPad Prism 7, and the IC₅₀ values of the compounds were calculated.

### (2) Determination of concentration of cytokines secreted by LPS-induced human PBMCs

The cryopreserved human PBMCs (Milestone, PB010C) were thawed and resuspended in RPMI 1640 medium (Gibco, A1049101) supplemented with 10% fetal bovine serum (Gibco, 10099141), 100 U/mL penicillin, and 100 µg/mL streptomycin (Gibco, 15140122). On the same day, PBMCs were plated onto a 96-well clear cell plate (Labserv, 310109008) at 150 µL/well, with a cell density of 2 × 10⁵ cells/well. Then 50 µL of diluted compound solution was added to corresponding wells with cells, such that the final concentration of the compound fell within the range of 0.026-10000 nM. The final concentration of DMSO was 0.25%. After the compound-treated cell plate was incubated in an incubator at 37 °C with 5% CO₂ for 1 h or 20 h, 10 µL of LPS (0111:B4) (Sigma, L4391) was added, making a final concentration of 100 ng/mL. After the cell plate was incubated in an incubator at 37 °C with 5% CO₂ for another 5 h, the plate was centrifuged at 2000 rpm for 10 min, and 100 µL of cell supernatant was transferred from each well to a new 96-well clear cell plate for cytokine concentration testing and cryopreserved at -80 °C before testing.

Interleukin-6 (IL-6) AlphaLISA assay

The LPS-induced human PBMC cell supernatants were assayed for IL-6 concentrations using an interleukin-6 (IL-6) AlphaLISA assay kit (PerkinElmer, AL223 C).

IL-6 standard solutions with different concentrations, ranging from 0 to 100000 pg/mL, were prepared by following the instructions for use of the product. 2 µL of each of the IL-6 standard solutions and test samples of cell supernatant were added to a white 384-well plate (PerkinElmer, 6007299), and 8 µL of a mixed solution of 5× anti-IL-6 receptor beads (final concentration of 10 µg/mL) and biotin-labeled anti-IL-6 antibody (final concentration of 1 nM) was then added to each well. The plate was incubated at 23 °C for 60 min. Finally, 10 µL of 2× streptavidin-labeled donor beads (final concentration of 40 µg/mL) was added to each well, and the plate was incubated in the dark at 23 °C for another 30 min. After the incubation was completed, AlphaLISA signal values were measured on a microplate reader EnVision (PerkinElmer, 2105) in the AlphaScreen standard mode. A standard curve was plotted from the AlphaLISA signal values of the IL-6 standard solutions with different concentrations, and the IL-6 concentration of each cell lysate supernatant was determined according to the corresponding concentrations on the standard curve for the AlphaLISA signal values of the test samples. The inhibition rates of the compounds at each concentration were calculated by comparing the IL-6 concentration with that of the control group. Then non-linear curve fittings for logarithmic concentration-inhibition rate were performed using GraphPad Prism 7, and the IC₅₀ values of the compounds were calculated.

### Test Example 4: IRAK4 Degradation by Compounds

### (1) Degradation of IRAK4 protein in normal human PBMCs by compounds

The cryopreserved normal human PBMCs (Milestone, PB010C) were thawed and resuspended in RPMI 1640 medium (Gibco, A1049101) supplemented with 10% fetal bovine serum (Gibco, 10099141), 100 U/mL penicillin, and 100 µg/mL streptomycin (Gibco, 15140122). After 0.95 mL of cells were plated in each well of a 24-well cell culture plate, 50 µL of diluted compound solution was added to corresponding wells with cells, such that the final concentration of the compound fell within the range of 0.01-3000 nM. The final concentration of DMSO was 0.25%. After the compound-treated cell plate was incubated in an incubator at 37 °C with 5% CO₂ for 24 h, the cells were collected into 1.5 mL centrifuge tubes and centrifuged at 1000 rpm at 4 °C for 5 min. The cell pellets were washed twice with 1× DPBS. The resuspended cells were lysed with 100 µL of lysis buffer, left to stand on ice for 30 min, and then centrifuged at 14000 g at 4 °C for 10 min. The samples of the cell lysate supernatant after centrifugation were cryopreserved at -80 °C before testing. The cell lysis buffer was a Western and IP cell lysis buffer (Beyotime, P0013) supplemented with 1 mM phenylmethylsulfonyl fluoride and a protease inhibitor cocktail (Beyotime, P1008).

The total protein concentrations of the cell lysate samples were determined using a BCA protein quantification kit (Tiangen, PA115-02).

The prepared PBMC lysate samples were assayed for IRAK4 concentrations using a human IRAK4 AlphaLISA assay kit (PerkinElmer, AL3117C) to determine the degradation effects of the compounds on IRAK4. The method is as follows:
5 µL of each of IRAK4 standard solutions with different concentrations and test samples of cell lysate supernatant were added to a white 384-well plate (PerkinElmer, 6007299), and then 10 µL of 5× anti-IRAK4 receptor beads was added to each well (final concentration of 10 µg/mL). The plate was incubated at room temperature for 30 min. Then 10 µL of 5× biotin-labeled anti-IRAK4 antibody was added to each well (final concentration of 1 nM), and the plate was incubated at room temperature for another 60 min. Finally, 25 µL of 2× streptavidin-labeled donor beads was added to each well (final concentration of 40 µg/mL), and the plate was incubated at room temperature for another 30 min. After the incubation was completed, AlphaLISA signal values were measured on a microplate reader EnVision (PerkinElmer, 2105) in the AlphaScreen standard mode. A standard curve was plotted from the AlphaLISA signal values of the IRAK4 standard solutions with different concentrations, and the IRAK4 concentration of each cell lysate supernatant was determined according to the corresponding concentrations on the standard curve for the AlphaLISA signal values of the test samples and by performing corresponding correction on the total protein concentration of each sample obtained by BCA quantification. The degradation rates for the compounds at each concentration were calculated by comparing the IRAK4 concentration with that of the control group. Then non-linear curve fittings for logarithmic concentration-inhibition rate were performed using GraphPad Prism 8, and the DC₅₀ and Dₘₐₓ values of the compound were obtained.

**Table 4:**

| Example | DC₅₀ (nM) | Dₘₐₓ (%) |
|---|---|---|
| Example 1 | 1.28 | 98.99 |
| Example 2 | 1.56 | 99.11 |
| Example 3 | 0.28 | 97.68 |
| Example 4 | 0.39 | 97.15 |
| Example 5 | 0.60 | 98.76 |
| Example 6 | 0.56 | 97.86 |
| Example 7 | 0.39 | 98.08 |
| Example 8 | 0.80 | 99.09 |
| Example 9 | 0.39 | 98.04 |
| Example 10 | 0.67 | 97.59 |
| Example 11 | 0.88 | 97.37 |
| Example 12 | 0.37 | 98.00 |
| Example 13 | 0.21 | 98.21 |
| Example 14 | 0.27 | 98.19 |
| Example 15 | 1.49 | 98.85 |
| Example 16 | 0.70 | 98.36 |
| Example 17 | 0.92 | 98.88 |
| Example 18 | 0.77 | 99.46 |
| Example 19 | 0.47 | 98.04 |
| Example 20 | 0.72 | 95.73 |
| Example 21 | 0.45 | 96.16 |
| Example 22 | 0.53 | 98.07 |
| Example 23 | 2.17 | 99.00 |
| Example 24 | 0.48 | 96.20 |
| Example 25 | 0.32 | 97.50 |
| Example 26 | 0.35 | 97.77 |
| Example 27 | 0.28 | 97.85 |
| Example 28 | 4.73 | 107.10 |
| Example 29 | 0.53 | 98.22 |
| Example 30 | 1.00 | 100.20 |
| Example 31 | 0.84 | 99.25 |
| Example 32 | 1.11 | 98.12 |
| Example 33 | 1.58 | 98.58 |
| Example 34 | 1.32 | 99.10 |
| Example 35 | 0.65 | 99.71 |
| Example 36 | 2.03 | 98.61 |

### (2) Degradation of IRAK4 protein in patient whole blood by compounds

Fresh whole blood from normal or patient (rheumatoid arthritis and dermatomyositis) volunteers was collected and anticoagulated with heparin sodium. After 0.95 mL of whole blood was plated in each well of a 24-well cell culture plate, 50 µL of diluted compound solution was added to corresponding wells with whole blood, such that the final concentration of the compound fell within the range of 10-10000 nM. The final concentration of DMSO was 0.1%. After the compound-treated cell plate was incubated in an incubator at 37 °C with 5% CO₂ for 24 h, the whole blood sample was collected from each well, and PBMCs were isolated from the whole blood using FICOLL isolation solution (GE, 17-1440-02) following the procedures in the instructions for use of the product. The isolated cells were washed once with 1× DPBS. Then the red blood cells were lysed using red blood cell lysis buffer (Nanjing SenBeiJia, BL-051-100ml) for 5 min, and the cells were washed once with 1× DPBS. Finally, the white precipitated cells collected after centrifugation at 3000 rpm for 5 min were PBMCs.

The isolated PBMCs were lysed with a cell lysis buffer after the supernatant was removed, left to stand on ice for 30 min, and then centrifuged at 14000 g at 4 °C for 10 min. The cell lysis buffer was a Western and IP cell lysis buffer (Beyotime, P0013) supplemented with 1 mM phenylmethylsulfonyl fluoride and a protease inhibitor cocktail (Beyotime, P1008). The protein samples of the cell lysate supernatant after centrifugation were cryopreserved at -80 °C before testing.

The total protein concentrations in the prepared whole blood cell lysate samples from normal humans or patients (rheumatoid arthritis and dermatomyositis) were determined using a BCA protein quantification kit (Tiangen, PA115-02).

The prepared whole blood cell lysate samples from normal humans or patients (rheumatoid arthritis and dermatomyositis) were assayed for IRAK4 concentrations using a human IRAK4 AlphaLISA assay kit (PerkinElmer, AL3117C) to determine the degradation effects of the compounds on IRAK4. The method is as follows:
5 µL of each of IRAK4 standard solutions with different concentrations and test samples of cell lysate supernatant were added to a white 384-well plate (PerkinElmer, 6007299), and then 10 µL of 5× anti-IRAK4 receptor beads was added to each well (final concentration of 10 µg/mL). The plate was incubated at room temperature for 30 min. Then 10 µL of 5× biotin-labeled anti-IRAK4 antibody was added to each well (final concentration of 1 nM), and the plate was incubated at room temperature for another 60 min. Finally, 25 µL of 2× streptavidin-labeled donor beads was added to each well (final concentration of 40 µg/mL), and the plate was incubated at room temperature for another 30 min. After the incubation was completed, AlphaLISA signal values were measured on a microplate reader EnVision (PerkinElmer, 2105) in the AlphaScreen standard mode. A standard curve was plotted from the AlphaLISA signal values of the IRAK4 standard solutions with different concentrations, and the IRAK4 concentration of each cell lysate supernatant was determined according to the corresponding concentrations on the standard curve for the AlphaLISA signal values of the test samples and by performing corresponding correction on the total protein concentration of each sample obtained by BCA quantification. The degradation rates for the compounds at each concentration were calculated by comparing the IRAK4 concentration with that of the control group. Then non-linear curve fittings for logarithmic concentration-inhibition rate were performed using GraphPad Prism 8, and the DC₅₀ and Dₘₐₓ values of the compound were obtained.

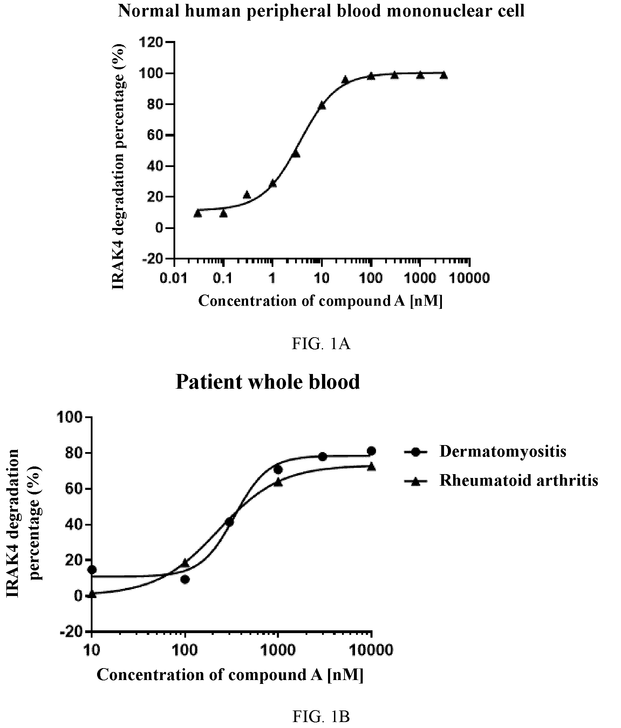

FIGs. 1A and 1B show that compound A of the present disclosure has a good degradation effect on IRAK4 of normal human peripheral blood mononuclear cells and patients with dermatomyositis and rheumatoid arthritis. Test Example 5: Determination of Intracellular IRAK4 Protein Expression Level in PBMCs of Various Patients Fresh whole blood of volunteers was collected and anticoagulated with heparin sodium, and then PBMCs were isolated from the whole blood using FICOLL isolation solution (GE, 17-1440-02) following the procedures in the instructions for use of the product. The isolated cells were washed once with 1× DPBS. Then the red blood cells were lysed using red blood cell lysis buffer (Nanjing SenBeiJia, BL-051-100ml), and the cells were washed once with 1× DPBS. Finally, the white precipitated cells collected after centrifugation at 3000 rpm for 5 min were PBMCs. The method is suitable for isolating PBMCs from the whole blood of patients with osteoarthritis, rheumatoid arthritis, systemic lupus erythematosus, dermatomyositis, systemic sclerosis, etc., and the whole blood of normal humans.

The isolated PBMCs were lysed with a cell lysis buffer after the supernatant was removed, left to stand on ice for 30 min, and then centrifuged at 14000 g at 4 °C for 10 min. The cell lysis buffer was a Western and IP cell lysis buffer (Beyotime, P0013) supplemented with 1 mM phenylmethylsulfonyl fluoride and a protease inhibitor cocktail (Beyotime, P1008). The protein samples of the cell lysate supernatant after centrifugation were cryopreserved at -80 °C before testing.

The total protein concentrations of the cell protein samples were determined using a BCA protein quantification kit (Tiangen, PA115-02).

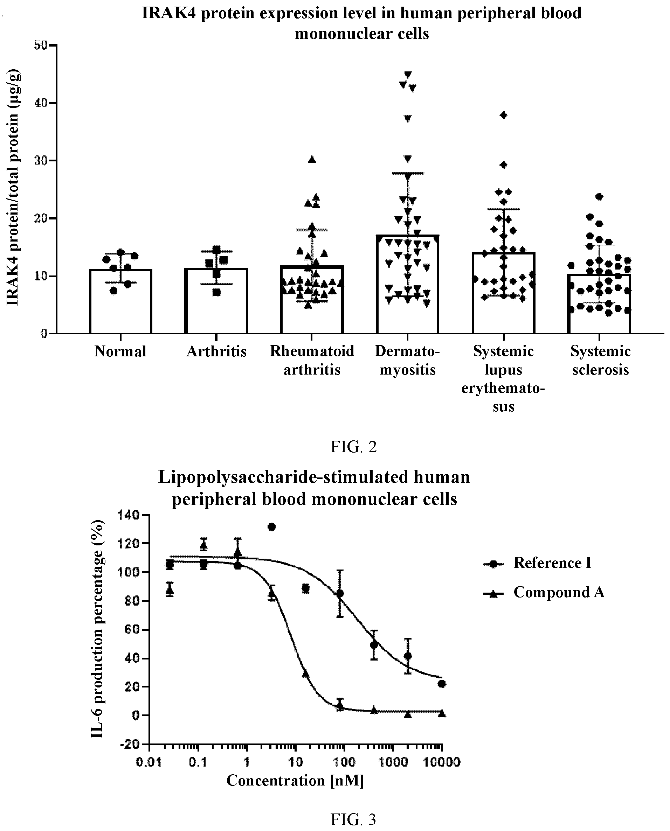

The prepared cell protein samples were assayed for the expression levels of IRAK4 proteins in PBMCs of various patients or normal humans using a human IRAK4 AlphaLISA assay kit (PerkinElmer, AL3117C). The method is as follows:
5 µL of each of IRAK4 standard solutions with different concentrations and test samples of cell lysate supernatant were added to a white 384-well plate (PerkinElmer, 6007299), and then 10 µL of 5× anti-IRAK4 receptor beads was added to each well (final concentration of 10 µg/mL). The plate was incubated at room temperature for 30 min. Then 10 µL of 5× biotin-labeled anti-IRAK4 antibody was added to each well (final concentration of 1 nM), and the plate was incubated at room temperature for another 60 min. Finally, 25 µL of 2× streptavidin-labeled donor beads was added to each well (final concentration of 40 µg/mL), and the plate was incubated at room temperature for another 30 min. After the incubation was completed, AlphaLISA signal values were measured on a microplate reader EnVision (PerkinElmer, 2105) in the AlphaScreen standard mode. A standard curve was plotted from the AlphaLISA signal values of the IRAK4 standard solutions with different concentrations, and the IRAK4 concentration of each cell lysate sample was determined according to the corresponding concentrations on the standard curve for the AlphaLISA signal values of the test samples and by performing corresponding correction on the total protein concentration of each sample obtained by BCA quantification. The histogram was plotted by GraphPad Prism 8 software, and the results are shown in FIG. 2. FIG. 2 shows that the intracellular IRAK4 proteins in PBMCs of patients with rheumatoid arthritis, dermatomyositis, systemic lupus erythematosus, and systemic sclerosis have high-level expression.

### Test Example 6: IL-6 Inhibition in LPS-Induced Normal Human PMBCs by Compounds

The cryopreserved normal human PBMCs (Milestone, PB010C) were thawed and resuspended in RPMI 1640 medium (Gibco, A1049101) supplemented with 10% fetal bovine serum (Gibco, 10099141), 100 U/mL penicillin, and 100 µg/mL streptomycin (Gibco, 15140122). PBMCs were plated onto a 96-well clear cell plate (Labserv, 310109008) at 150 µL/well, with a cell density of 2 × 105 cells/well. Then 50 µL of diluted compound solution was added to corresponding wells with cells, such that the final concentration of the compound fell within the range of 0.026-10000 nM. The final concentration of DMSO was 0.25%. After the compound-treated cell plate was incubated in an incubator at 37 °C with 5% CO₂ for 20 h, 10 µL of LPS (0111:B4) (Sigma, L4391) was added. After the cell plate was incubated in an incubator at 37 °C with 5% CO₂ for another 5 h, the plate was centrifuged at 2000 rpm for 10 min, and 100 µL of cell supernatant was transferred from each well to a new 96-well clear cell plate for cytokine concentration testing and cryopreserved at -80 °C before testing.

The IL-6 concentrations in normal human PBMC cell supernatants were determined using an IL-6 AlphaLISA assay kit (PerkinElmer, AL223 C). The method is as follows:
IL-6 standard solutions with different concentrations, ranging from 0 to 100000 pg/mL, were prepared by following the instructions for use of the product. 2 µL of each of the IL-6 standard solutions and test samples of cell supernatant were added to a white 384-well plate (PerkinElmer, 6007299), and 8 µL of a mixed solution of 5× anti-IL-6 receptor beads (final concentration of 10 µg/mL) and biotin-labeled anti-IL-6 antibody (final concentration of 1 nM) was then added to each well. The plate was incubated at 23 °C for 60 min. Finally, 10 µL of 2× streptavidin-labeled donor beads (final concentration of 40 µg/mL) was added to each well, and the plate was incubated in the dark at 23 °C for another 30 min. After the incubation was completed, AlphaLISA signal values were measured on a microplate reader EnVision (PerkinElmer, 2105) in the AlphaScreen standard mode. A standard curve was plotted from the AlphaLISA signal values of the IL-6 standard solutions with different concentrations, and the IL-6 concentration of each cell lysate supernatant was determined according to the corresponding concentrations on the standard curve for the AlphaLISA signal values of the test samples. The inhibition rates of the compounds at each concentration were calculated by comparing the IL-6 concentration with that of the control group. Then non-linear curve fittings for logarithmic concentration-inhibition rate were performed using GraphPad Prism 8, and the IC₅₀ values of the compounds were calculated. FIG. 3 shows that compound A of the present disclosure has a very good inhibitory effect on IL-6 in human peripheral blood mononuclear cells stimulated by lipopolysaccharide.

### Test Example 7: Human PBMC Cytokine Assay

Testing apparatus and reagents:
Normal human peripheral blood mononuclear cells (PBMCs) (TPCS, PB010C)
RPMI 1640 medium (Gibco, A1049101)
Fetal bovine serum (Gibco, 10099141)
Bispecific antibody (Gibco, 15140122)
Lipopolysaccharide (LPS) (0111:B4) (Sigma-Aldrich, L4391)
Resiquimod (R848) (MCE, HY-13740)
DMSO (Sigma-Aldrich, D8418-500ML)
Ficoll isolation solution (Cytiva, 17544602)
ACK red blood cell lysis buffer (sterile) (Sbjbio, BL-O51)
96-well cell culture plate (Corning, 3596)
Vacuum blood collection tube (heparin lithium anticoagulation) (BD, 367880)
Centrifuge (Thermo Scientific, ST16R)
Pipettor (Eppendorf)

### (1) Lipopolysaccharide- or resiquimod-induced normal human PMBC cytokine assay

The cryopreserved normal human PBMCs were thawed and resuspended in RPMI 1640 medium (supplemented with 10% fetal bovine serum, 100 U/mL penicillin, and 100 µg/mL streptomycin). PBMCs were plated onto a 96-well clear cell plate, with a cell density of 2 × 10⁵ cells/well. The test compounds diluted with a medium were then added to the compound wells to a final concentration of 500 nM (containing 0.25% DMSO) while the medium containing 0.25% DMSO was added to each of the positive and negative control wells. After sample loading, the cell plate was incubated in an incubator at 37 °C with 5% CO₂ for 20 h, and LPS or R848 was added to the compound wells and the positive control well. After sample loading, the cell plate was incubated in an incubator at 37 °C with 5% CO₂ for another 5 h, and then centrifuged at 2000 rpm for 10 min. Finally, the cell supernatant was taken from each well and cryopreserved at -80 °C for later use.

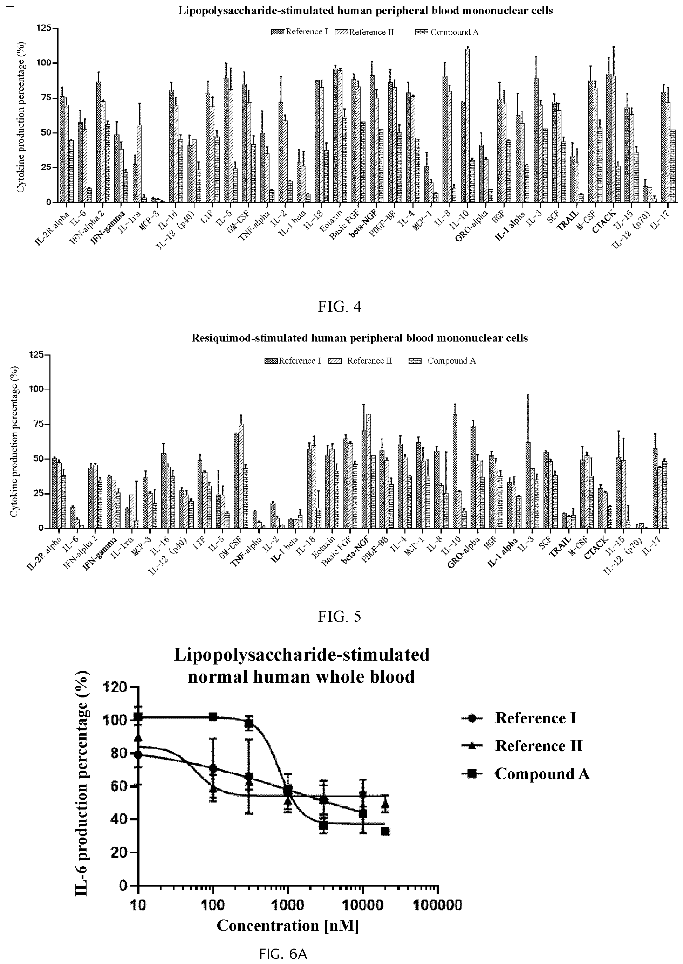

The detection of 48 human cytokines (Shanghai Universal Biotech Co., Ltd.) was conducted according to the instructions of the Bio-Plex Pro Human Cytokine Screening Panel kit (Bio-Rad, 12007283), and the samples were analyzed using the Luminex (X-200) instrument. The detection results for the expression of each cytokine in the samples are shown in FIGs. 4 and 5.

FIGs. 4 and 5 show that at 500 nM, the degrader compound A of the present disclosure has a general inhibitory effect on lipopolysaccharide- or resiquimod-stimulated cytokine secretion from human peripheral blood mononuclear cells, and is superior to inhibitor compounds (reference I and reference II) at the same concentration.

### Test Example 8: IL-6 Inhibition in Normal Human or Patient Whole Blood by Compounds

Fresh whole blood from normal or patient (rheumatoid arthritis, systemic lupus erythematosus, and dermatomyositis) volunteers was collected and anticoagulated with heparin sodium. After 190 µL of whole blood was plated onto a 96-well clear cell plate (Labserv, 310109008), 10 µL of diluted compound solution was added to corresponding wells with whole blood, such that the final concentration of the compound fell within the range of 10-10000 nM. The final concentration of DMSO was 0.25%. After the compound-treated cell plate was incubated in an incubator at 37 °C with 5% CO₂ for 20 h, 10 µL of LPS was added. After the cell plate was incubated in an incubator at 37 °C with 5% CO₂ for another 5 h, the plate was centrifuged at 2000 rpm for 10 min, and 50 µL of supernatant was transferred from each well to a new 96-well clear cell plate for cytokine concentration testing and cryopreserved at -80 °C before testing.

The IL-6 concentrations in the whole blood supernatants of normal humans or patients (dermatomyositis, rheumatoid arthritis, and systemic lupus erythematosus) were determined using a human IL-6 AlphaLISA assay kit (PerkinElmer, AL223 C). The method is as follows:
IL-6 standard solutions with different concentrations, ranging from 0 to 100000 pg/mL, were prepared by following the instructions for use of the product. 2 µL of each of the IL-6 standard solutions and test samples of cell supernatant were added to a white 384-well plate (PerkinElmer, 6007299), and 8 µL of a mixed solution of 5× anti-IL-6 receptor beads (final concentration of 10 µg/mL) and biotin-labeled anti-IL-6 antibody (final concentration of 1 nM) was then added to each well. The plate was incubated at 23 °C for 60 min. Finally, 10 µL of 2× streptavidin-labeled donor beads (final concentration of 40 µg/mL) was added to each well, and the plate was incubated in the dark at 23 °C for another 30 min. After the incubation was completed, AlphaLISA signal values were measured on a microplate reader EnVision (PerkinElmer, 2105) in the AlphaScreen standard mode. A standard curve was plotted from the AlphaLISA signal values of the IL-6 standard solutions with different concentrations, and the IL-6 concentration of each cell lysate supernatant was determined according to the corresponding concentrations on the standard curve for the lphaLISA signal values of the test samples. The inhibition rates of the compounds at each concentration were calculated by comparing the IL-6 concentration with that of the control group. Then non-linear curve fittings for logarithmic concentration-inhibition rate were performed using GraphPad Prism 7, and the IC₅₀ values of the compounds were calculated.

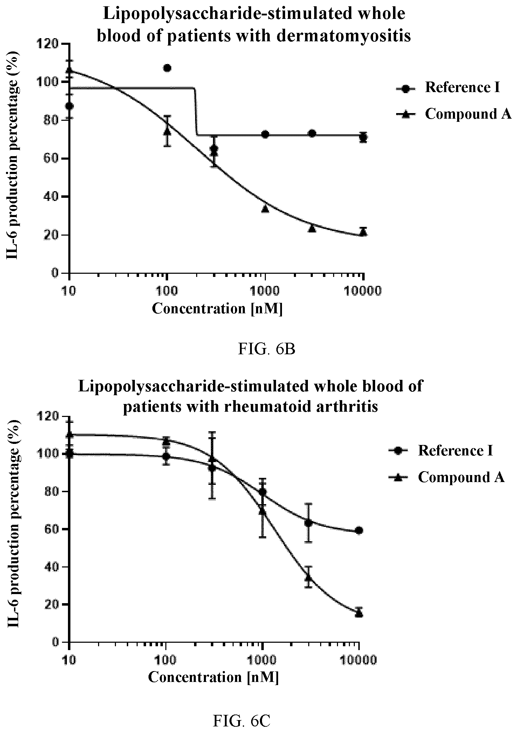

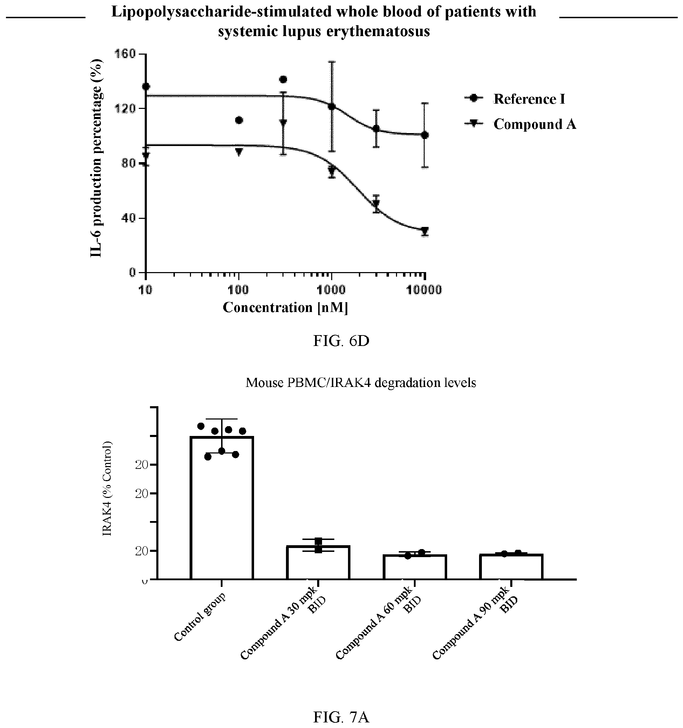

FIGs. 6A, 6B, 6C, and 6D show that compound A of the present disclosure has a very good inhibitory effect on lipopolysaccharide-induced IL-6 in normal human whole blood, IL-6 in whole blood of patients with dermatomyositis, IL-6 in whole blood of patients with rheumatoid arthritis, and IL-6 in whole blood of patients with systemic lupus erythematosus.

Test Example 9: Test for Effect of Compound A on Tissue IRAK4 Protein Expression Following Multiple Oral Administrations in Male BALB/C Mice
Subject information
Male BALB/C mice, 6-8 weeks old.
Test reagents
DMSO, Solutol, and Glucose (batch No. 20181102, AR, Sinopharm Chemical Reagent Co., Ltd.).
Preparation of formulation for administration

A certain amount of compound A was weighed and then dissolved in a 5% DMSO solution to form a clear solution. Then a 15% Solutol solution was added, and the mixture was shaken to ensure homogeneity. Finally, the mixture was added with 80% normal saline, and the resulting mixture was mixed well.

### Dose setting and grouping

The dose of compound A was set at 30 mpk, 60 mpk, and 90 mpk; oral administration, twice daily; Vehicle was 5% DMSO + 15% Solutol + 80% normal saline; grouping information is shown in Table 5:

**Table 5. Compound dose setting and grouping information**

| **Group** | **Test compounds** | **Number/gender of animals** | **Administration dose** | **Administration volume** | **Administration route** |
|---|---|---|---|---|---|
| G1 (Normal) | Vehicle | 4/Male | -- | 10 mL/kg | PO |
| G2 | Compound A | 4/Male | 30 mg/kg | 10 mL/kg | PO |
| G3 | | 4/Male | 60 mg/kg | 10 mL/kg | PO |
| G4 | | 4/Male | 90 mg/kg | 10 mL/kg | PO |

### Experimental procedures

Animals were grouped by weight, the grouping information is shown in Table 5, and then administration was performed. Multiple gavage administrations were performed, and the animals were euthanized 3 days later with CO₂. The blood from the heart was collected after CO₂ euthanasia and anticoagulated with heparin, PBMCs were prepared by isolation, and the IRAK4 degradation was detected; spleen and skin tissues were also taken and used to detect IRAK4 protein levels.
Detection indices
IRAK4 protein levels in PBMCs, spleen, and skin.

### Test results

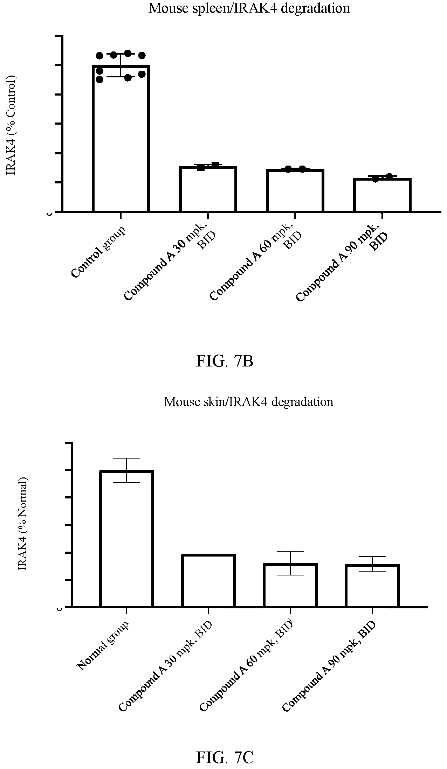

FIGs. 7A, 7B, and 7C show that after the oral administration of compound A, IARK4 was degraded well in animal PBMCs, spleen, and skin. With the prolonging of the administration time, the IRAK4 degradation rates in PBMCs and spleen tissues were increased, reaching 80% and more.

Test Example 10: Efficacy of Compound A on Imiquimod-Induced C57 Mouse Psoriasis Model
Reagents
5% imiquimod cream, Aldara, 3M Pharmaceuticals, LOT: GVJ005C
VecticalTM (calcitriol) Ointment, Galderma, LOT: 321449
Dexamethasone acetate tablets, Cisen Pharmaceutical, Chinese medicine approval No. H37021898
Subject information
Female C57BL/6 mice (weight 19-21 g), 9 weeks, 10 mice in each group.

### Experimental method

A. Preparation of reagents
   i. Imiquimod cream: 62.5 mg of imiquimod cream was weighed correspondingly for direct application on the back of each mouse, and 20 mg of imiquimod cream was weighed for direct application on the right ear;
   ii. calcitriol ointment: 75 mg of calcitriol ointment was weighed correspondingly for direct application on the back of each mouse, and 24 mg of calcitriol ointment was weighed for direct application on the right ear;
   iii. test compound Dex was formulated into the corresponding concentration for oral administration;
      5 mg/kg: 5 Dex tablets were taken and added with 7.5 mL of normal saline. The mixture was subjected to vortex ultrasonic treatment until uniformly suspended, and then stored at 4 °C for later use; the drug was subjected to vortex ultrasonic treatment to uniformity in advance before administration, and formulated every three days;
   iv. test compound A was formulated into the corresponding concentration for oral administration.
   Vehicle: 1% DMSO + 10% Solutol + 89% (5% Dextrose), formulated fresh for each administration.
   10 mg/kg and 100 mg/kg groups of test compound A were formulated in EP tubes. Compound A was weighed and added with 1% of the total volume of DMSO, and the mixture was subjected to vortex ultrasonic treatment until completely dissolved. Then 10% of the total volume of Solutol was added, and the mixture was subjected to vortex ultrasonic treatment until clear. 89% of the total volume of 5% glucose solution was added in steps while performing vortex, and the mixture was under magnetic stirring continuously before administration. The compound was formulated fresh for each use.
B. Imiquimod sensitization and drug intervention on C57BL/6J mice were conducted, with specific administration times shown in Table 6.
   i. On Day 1, the backs of the mice were shaved, ensuring consistent area using a mold, approximately 2 cm × 3 cm, and 60 mice were selected;
   ii. the mice were randomly divided into 6 groups based on body weight and right ear thickness, with 10 mice per group;
   iii. to induce psoriasis symptoms in the model mice, imiquimod was applied on the back skin and right ear of the mice in groups G2-G6 for sensitization at noon on Day 0, and this continued for 7 days.
   iv. Starting on Day 0, clinical scoring of the back skin of the mice was conducted before administration every morning, and ear thickness was measured every other day; compound A was administered at 9:00 and 17:00; imiquimod was applied at 13:30 for sensitization, with the detailed group administration regimen shown in Table 6.
   v. Endpoint sampling of the mice was performed on Day 5 or Day 7.

**Table 6. Grouping information**

| Group | Treatment method | Dose | Model | Therapeutic dose | | |
|---|---|---|---|---|---|---|
| | | | IMQ(topical) | Administration mode | Administration time | Administration frequency |
| 1 | Vehicle | - | - | p.o. | 9:00 | BID x 7 |
| 2 | Vehicle | - | 62.5 mg back + 20 mg right ear/mouse | p.o. | 9:05 | BID x 7 |
| 3 | Calcitriol | 75 mg back + 24 mg right ear/mouse | 62.5 mg back + 20 mg right ear/mouse | topical | 9:20 | QD x 7 |
| 4 | Dexamethasone | 5 mpk | 62.5 mg back + 20 mg right ear/mouse | p.o. | 9:10 | QD x 7 |
| 5 | Compound A | 10 mpk | 62.5 mg back + 20 mg right ear/mouse | p.o. | 9:15 | BID x 7 |
| 6 | Compound A | 100 mpk | 62.5 mg back + 20 mg right ear/mouse | p.o. | 9:30 | BID x 7 |

### Detection indices

Clinical score and right ear thickness.

### Test results

### Clinical score

This experiment evaluated the effect of the compounds on improving clinical scores in an imiquimod-induced mouse psoriasis model. The mean clinical score for the vehicle control group was gradually increased to 8.00 points by Day 5, suggesting the successful establishment of an imiquimod-induced mouse psoriasis model. Compound A has an inhibitory effect on the clinical score of psoriasis mice at both doses of 10 mg/kg and 100 mg/kg, which is dose-dependent. Significant differences were observed for compound A at both doses of 10 mg/kg and 100 mg/kg compared to the vehicle control group starting from Day 3 and continued until the end of the experiment (Day 5).

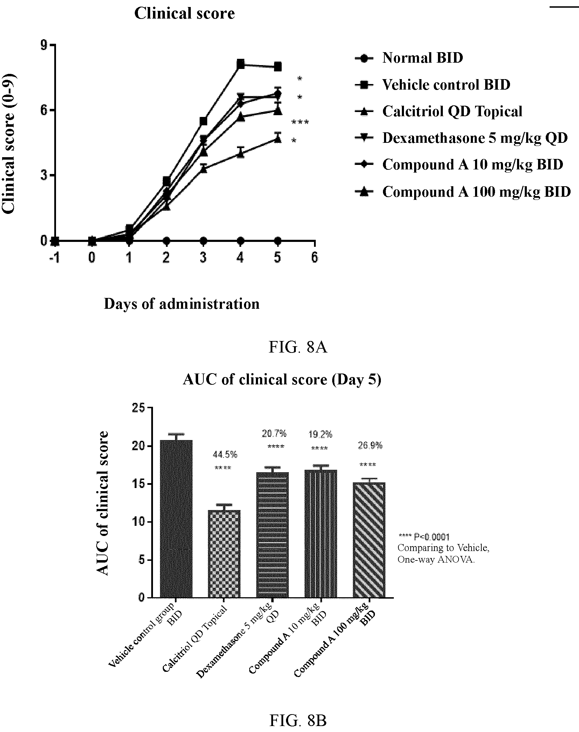

The area under the curve (AUC) was calculated by analyzing the clinical score curve for each animal in each group, and the inhibition rate of each treatment group relative to the vehicle control group was calculated based on the average AUC between groups, as shown in FIGs. 8A and 8B. The inhibition rates of compound A at doses of 10 mg/kg and 100 mg/kg were 19.2% and 26.9%, respectively (p < 0.0001). The inhibition rates for the positive control drugs calcitriol and dexamethasone were 44.5% and 20.7%, respectively (p < 0.0001).

### Thickness of right ear

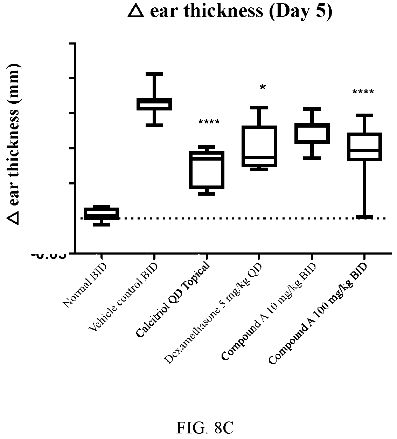

FIG. 8C indicates that compound A at doses of 10 mg/kg and 100 mg/kg had an inhibitory effect on the ear thickness difference (Δ ear thickness = the ear thickness value on the day of administration - the ear thickness value before group administration) on the Day 5, and at 100 mg/kg, compound A showed a significant statistical difference in the inhibition of the ear thickness difference (Δ ear thickness) (p < 0.0001).

### Experimental conclusion

Based on the above biological activity test experimental data, it can be concluded that the compound of the present disclosure, as an IRAK4 degrader, can be used to treat rheumatoid arthritis, dermatomyositis, systemic lupus erythematosus, systemic sclerosis, and/or psoriasis.

## Claims

1. An IRAK4 degrader, and/or a stereoisomer, an enantiomer, a diastereomer, a deuteride, a hydrate, a solvate, or a prodrug thereof and/or a pharmaceutically acceptable salt thereof, and use thereof for the treatment and/or prevention of an immunological and/or inflammatory disease.

2. An IRAK4 degrader, and/or a stereoisomer, an enantiomer, a diastereomer, a deuteride, a hydrate, a solvate, or a prodrug thereof and/or a pharmaceutically acceptable salt thereof, and use thereof for the treatment and/or prevention of a disease caused by abnormal expression of IRAK4 and IRAK4-associated proteins in an IL-1R/TLR pathway and/or abnormal secretion or proliferation of chemical factors, cytokines, or immune cells mediated by IRAK4.

3. The use according to claim 1 or 2, wherein the IRAK4 degrader is a compound of formula I or formula II or a pharmaceutically acceptable salt thereof: 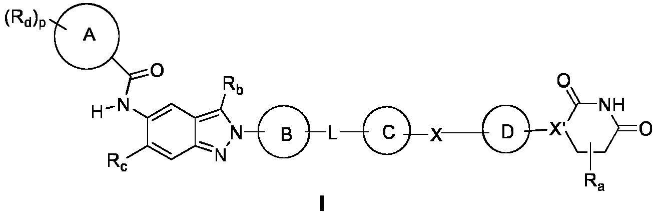 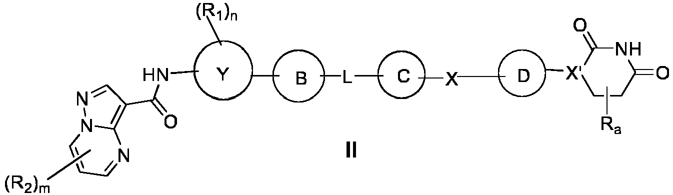 wherein:
ring A is phenyl or pyridyl;
ring B is C₆-C₁₀ cycloalkyl or 6- to 10-membered heterocycloalkyl containing 1-2 heteroatoms selected from N, O, and S, wherein the C₆-C₁₀ cycloalkyl and 6- to 10-membered heterocycloalkyl are optionally substituted with substituents selected from halogen, oxo, cyano, amino, hydroxy, C₁-C₆ alkyl, C₁-C₆ haloalkyl, C₁-C₆ hydroxyalkyl, and -O-(C₁-C₆ alkyl);
ring C is C₆-C₁₂ cycloalkyl or 6- to 12-membered heterocycloalkyl containing 1-2 heteroatoms selected from N, O, and S, wherein the C₆-C₁₂ cycloalkyl and 6- to 12-membered heterocycloalkyl are optionally substituted with substituents selected from halogen, cyano, amino, hydroxy, C₁-C₆ alkyl, C₁-C₆ haloalkyl, C₁-C₆ hydroxyalkyl, and -O-(C₁-C₆ alkyl);
ring D is C₆-C₁₀ aryl, wherein the C₆-C₁₀ aryl is optionally substituted with substituents of halogen, cyano, amino, hydroxy, C₁-C₆ alkyl, C₁-C₆ haloalkyl, C₁-C₆ hydroxyalkyl, or -O-(C₁-C₆ alkyl);
ring Y is 5- to 6-membered heteroaryl;
X is a bond, -O-, -NH-, -C(O)-, -OC(O)-, -C(O)O-, -NHC(O)-, or -C(O)NH-;
L is -(CH₂)ⱼ-, wherein 1 or more methylene groups in the -(CH₂)ⱼ- are optionally replaced with a group selected from -NR^{3'}-, -O-, -S-, -S(O)-, -S(O)₂-, -S(O)₂NR^{3'}-, -CR^{1'}R^{2'}-, -C(O)-, -C(O)O-, -OC(O)-, -NR^{3'}C(O)O-, - OC(O)NR^{3'}-, -C(O)NR^{3'}-, -NR^{3'}C(O)-, -NR^{4'}C(O)NR^{3'}-, ethenylene, and ethynylene;
R^{1'} and R^{2'} are each independently halogen, -OH, -NH₂, C₁-C₄ alkyl, C₁-C₄ haloalkyl, C₁-C₄ hydroxyalkyl, - O(C₁-C₄ alkyl), or -NH(C₁-C₄ alkyl);
R^{3'} and R^{4'} are each independently hydrogen or C₁-C₆ alkyl;
each R_{d} is independently hydrogen, deuterium, halogen, cyano, or C₁-C₆ alkyl, wherein the alkyl is optionally substituted with 1 or more groups selected from halogen, hydroxy, and amino;
R_{c} is -O(C₁-C₃ alkyl), -N(C₁-C₃ alkyl)₁₋₂, or C₁-C₆ alkyl, wherein the alkyl is optionally substituted with 1 or more groups independently selected from hydroxy, amino, halogen, cyano, and -O-(C₁-C₃ alkyl);
R_{b} is hydrogen or C₁-C₆ alkyl, wherein the alkyl is optionally substituted with 1 or more groups independently selected from hydroxy, amino, halogen, and cyano;
Rₐ is hydrogen, halogen, C₁-C₆ alkyl, or -O-(C₁-C₆ alkyl), wherein the alkyl is optionally substituted with halogen or hydroxy;
each R₁ is independently selected from C₁-C₄ alkyl, -O(C₁-C₄ alkyl), -N(C₁-C₄ alkyl)₁₋₂, CN, halogen, -OH, and -NH₂, wherein the C₁-C₄ alkyl is optionally substituted with a group selected from halogen, cyano, -OH, C₁-C₄ alkyl, and -O(C₁-C₄ alkyl);
each R₂ is independently hydrogen, C₁-C₄ alkyl, -O(C₁-C₄ alkyl), C₃-C₈ cycloalkyl, 3- to 8-membered heterocycloalkyl, C₆-C₁₀ aryl, 5- to 6-membered heteroaryl, CN, halogen, or -OH, wherein the C₃-C₈ cycloalkyl, 3- to 8-membered heterocycloalkyl, C₆-C₁₀ aryl, and 5- to 6-membered heteroaryl are optionally selected from halogen, cyano, -OH, -NH₂, C₁-C₄ alkyl, and -O(C₁-C₄ alkyl);
X' is CH or N;
m is 0, 1, 2, 3, or 4;
n is 0, 1, 2, 3, or 4;
p is 1 or 2;
j is 0, 1, 2, 3, 4, or 5.

4. The use according to claim 3, wherein the IRAK4 degrader is a compound of formula I-1, formula I-2, or formula II-1 or a pharmaceutically acceptable salt thereof: 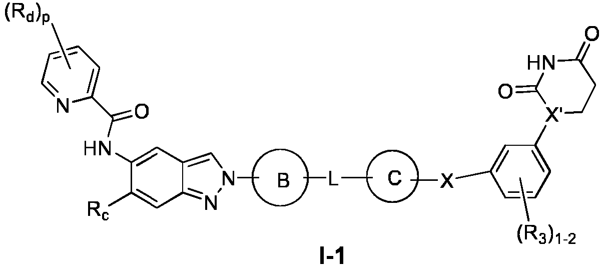 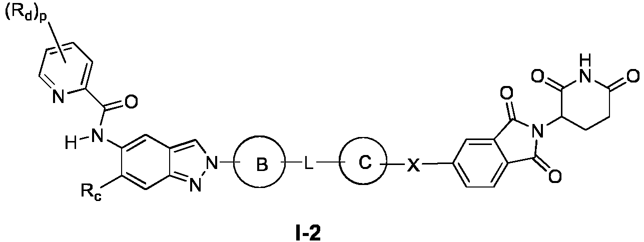 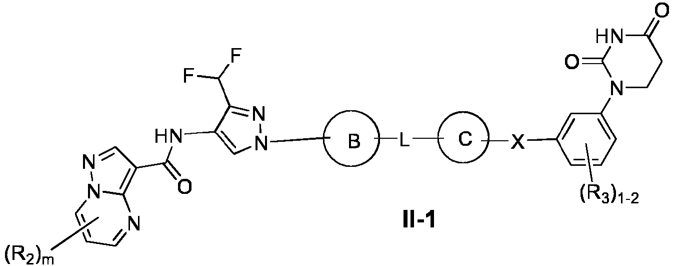 wherein:
R₃ is H, halogen, C₁-C₆ alkyl, or -O-(C₁-C₆ alkyl);
R_{c}, R_{d}, ring B, L, ring C, X, X', p, R₂, and m are as defined and described in claim 3.

5. The use according to claim 3 or 4, wherein the IRAK4 degrader is a compound selected from A1-A52, B1-B5, and C1-C36, 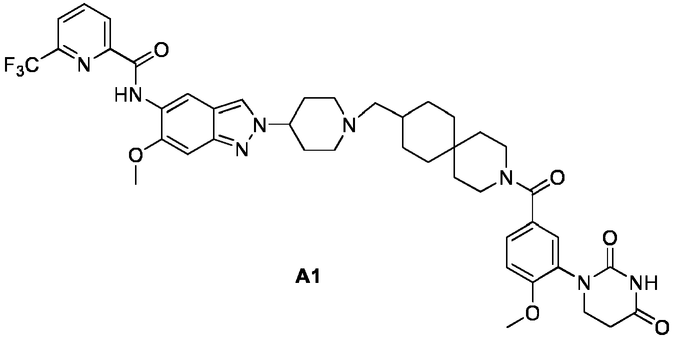 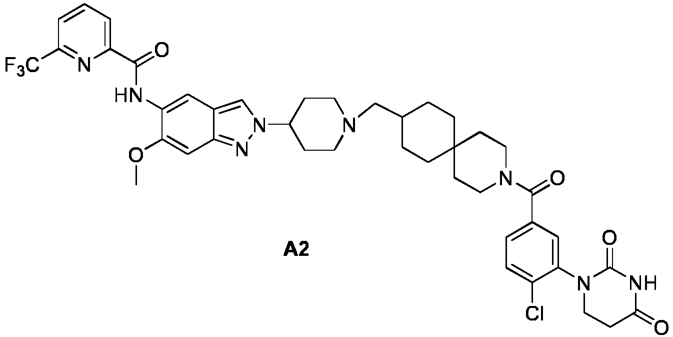 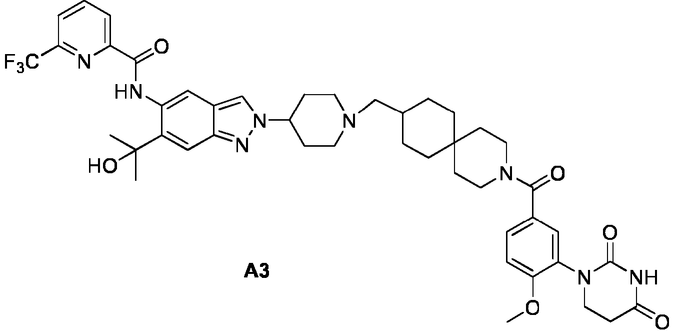 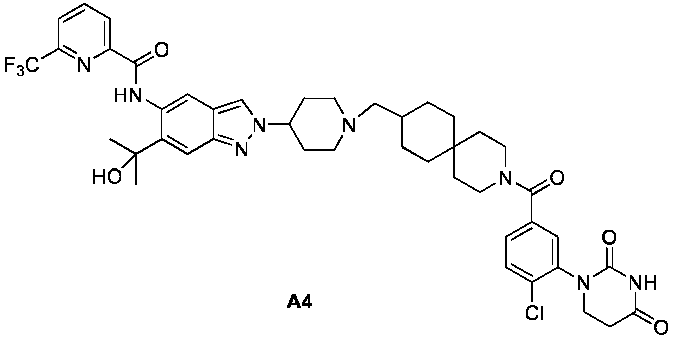 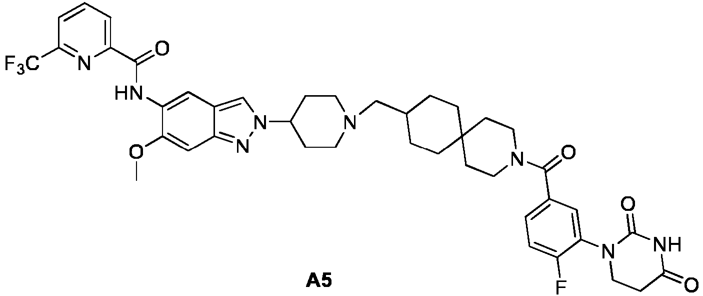 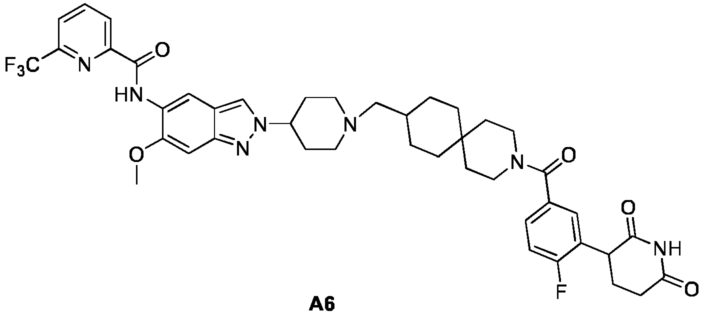 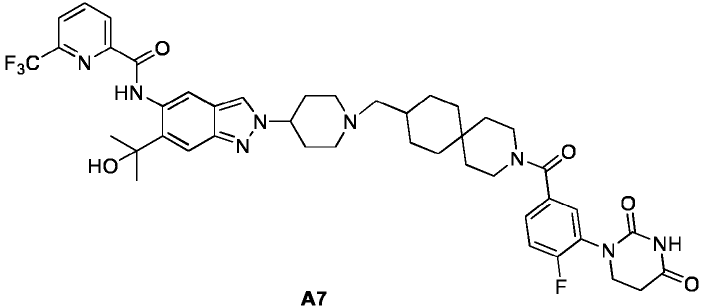 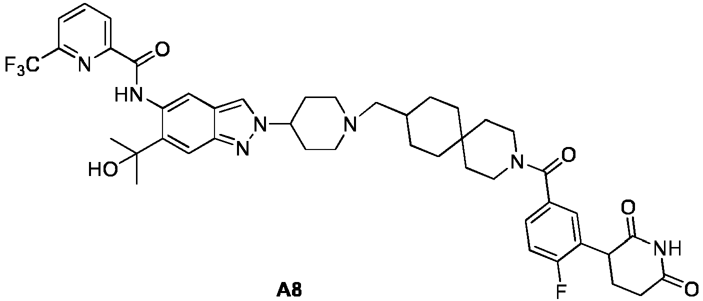 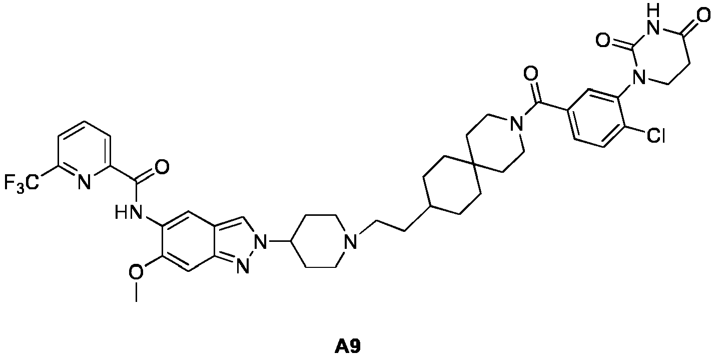 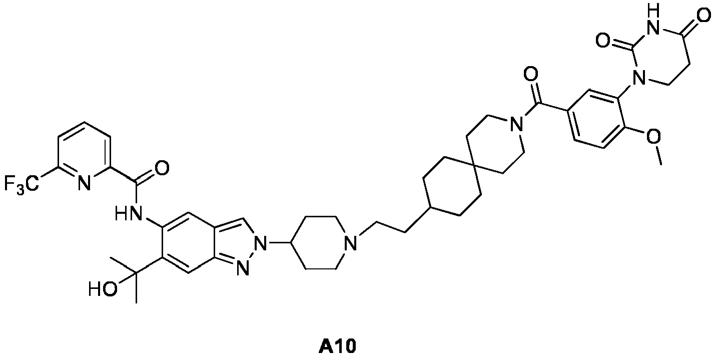 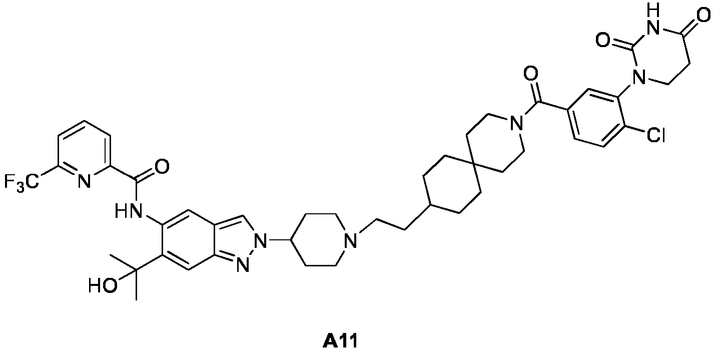 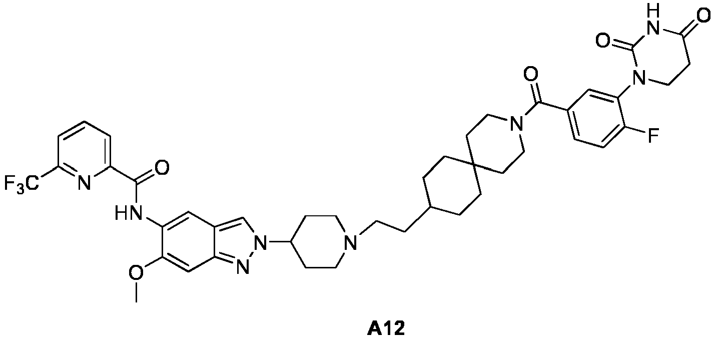 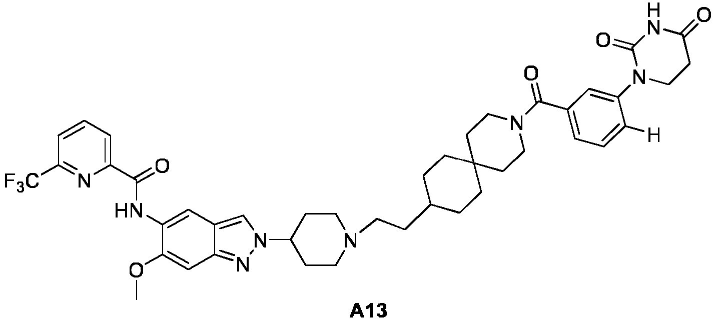 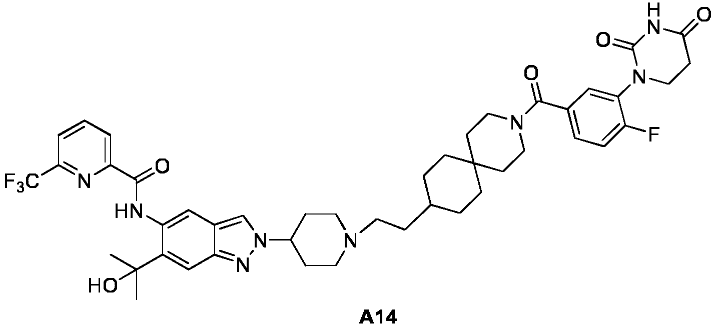 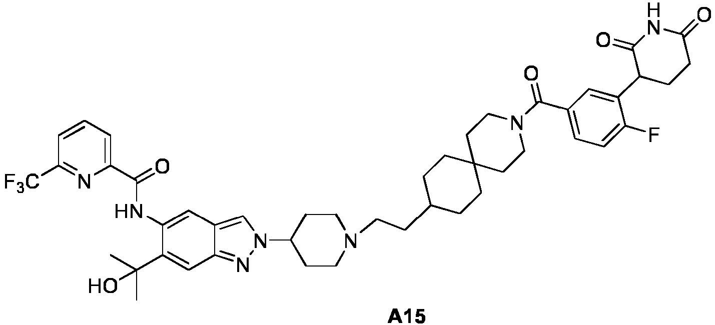 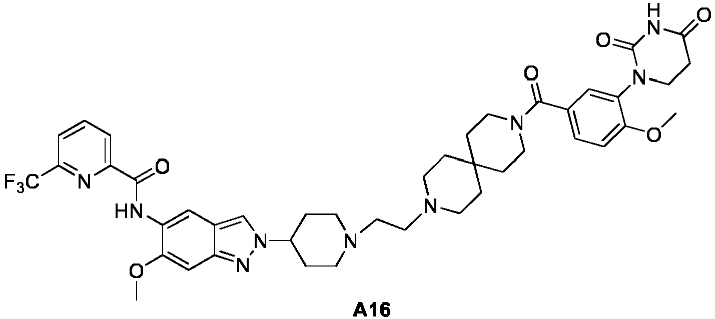 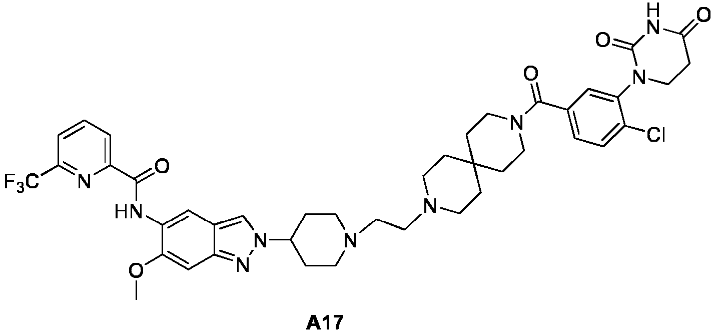 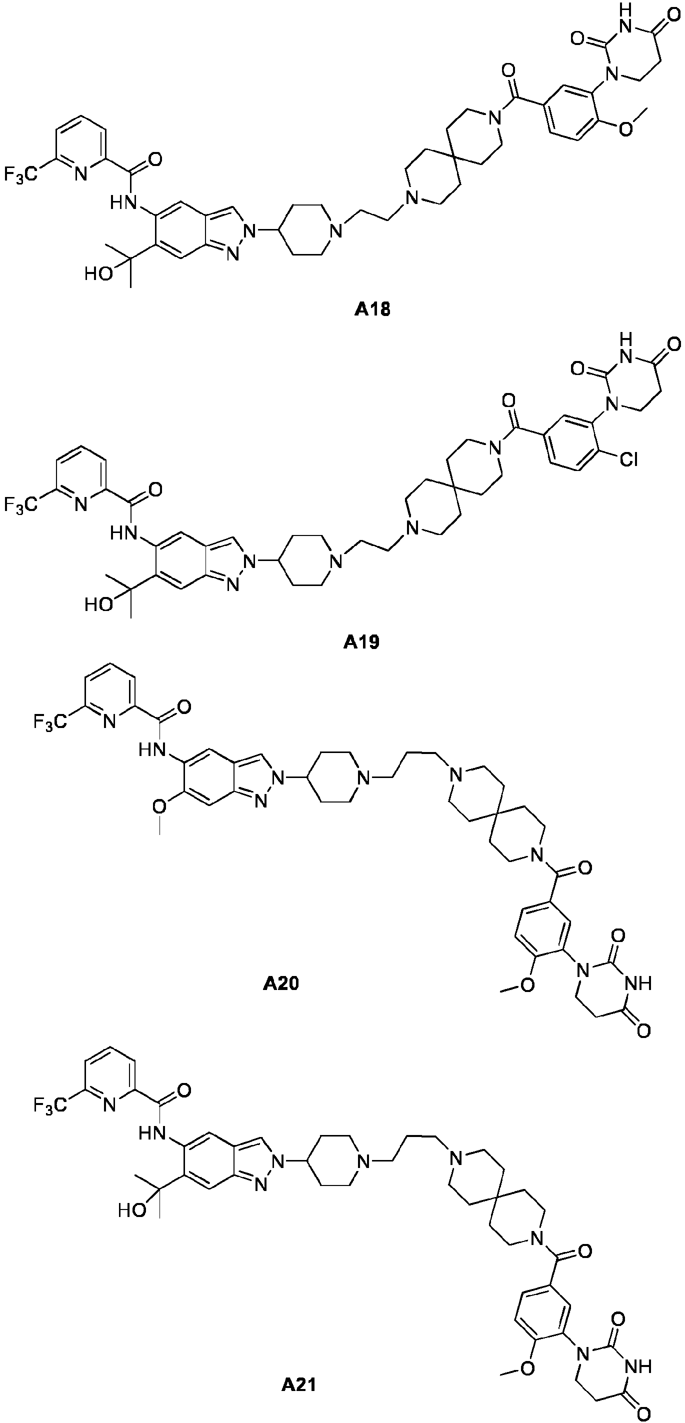 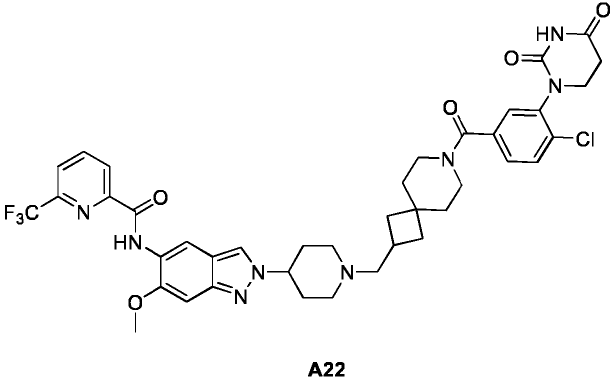 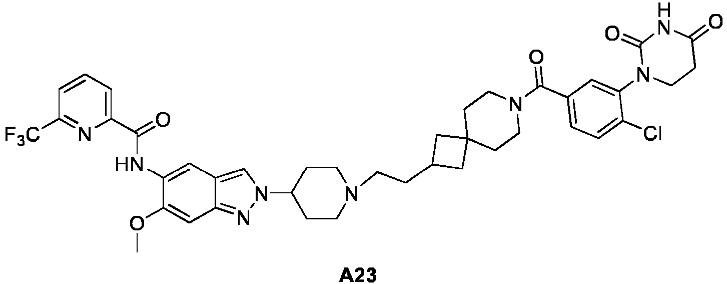 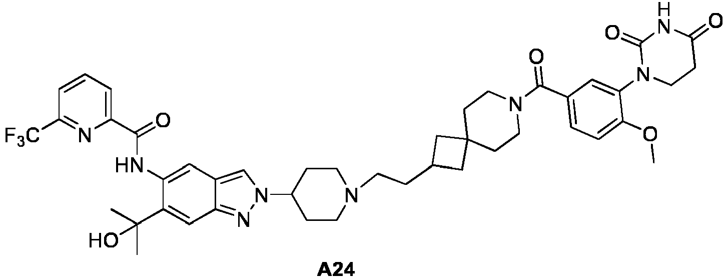 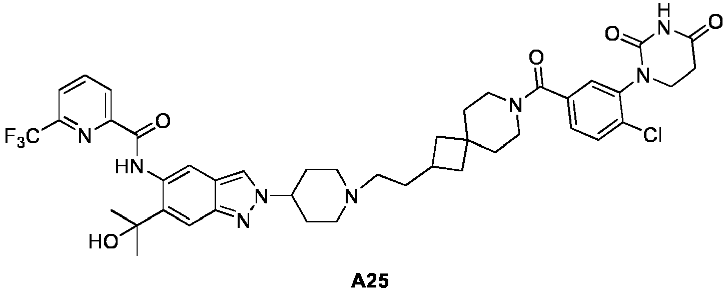 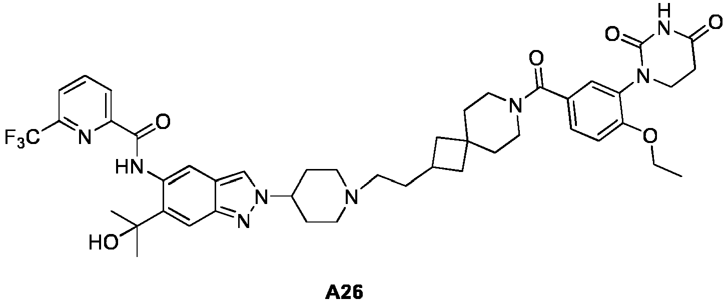 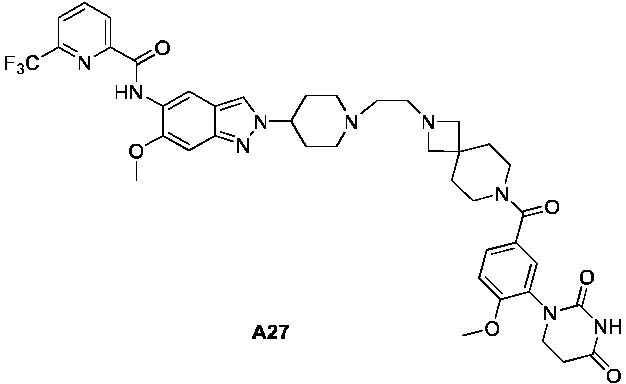 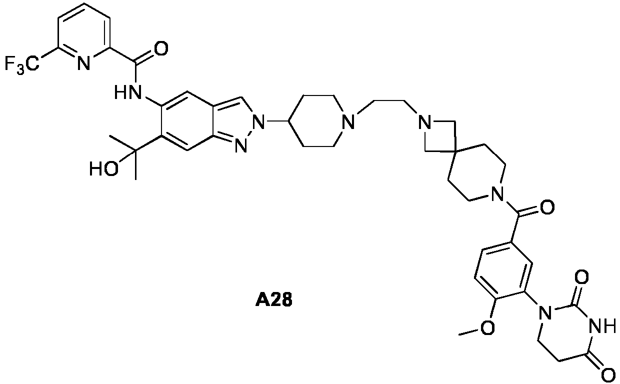 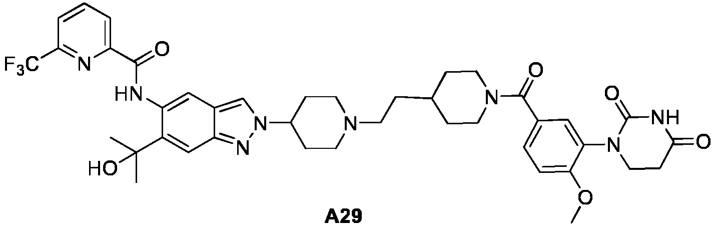 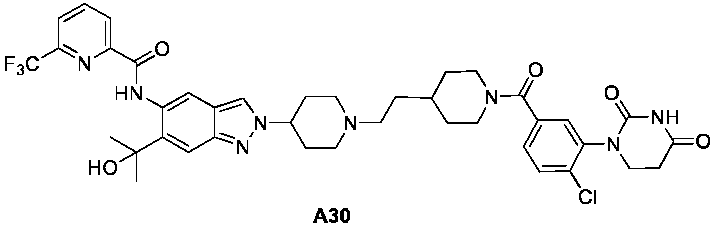 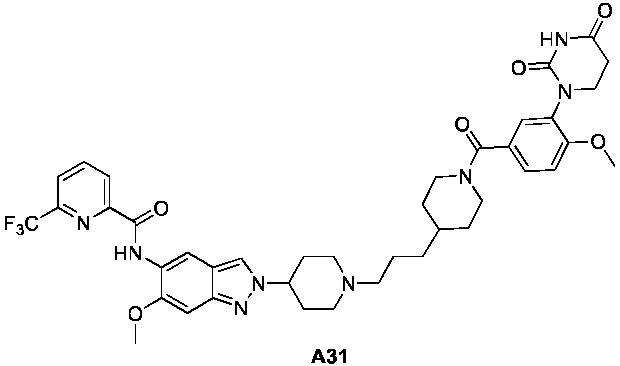 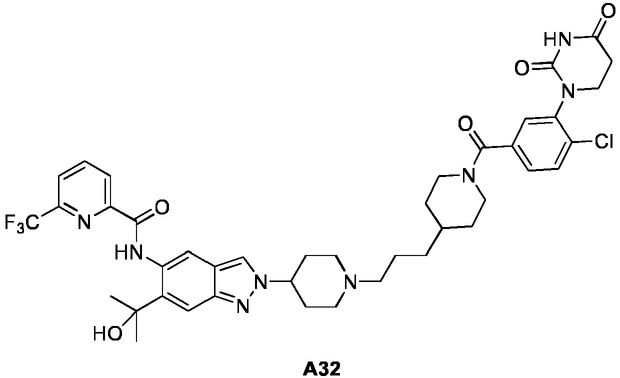 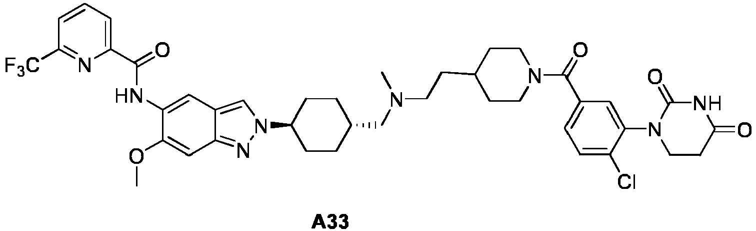 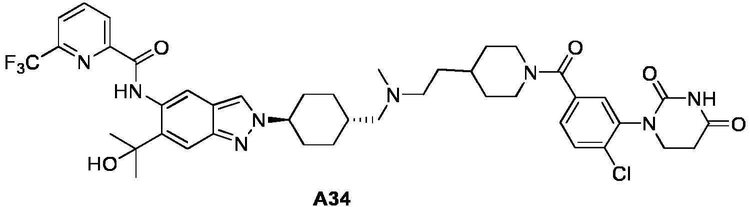 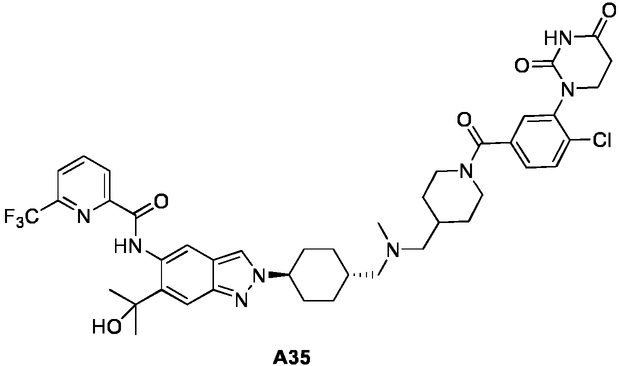 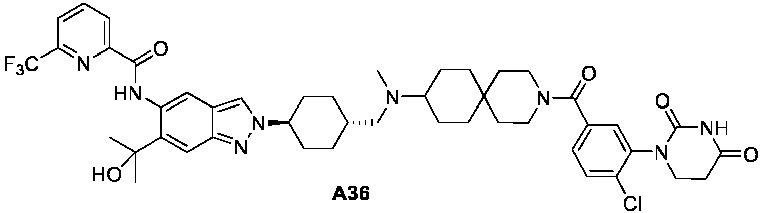 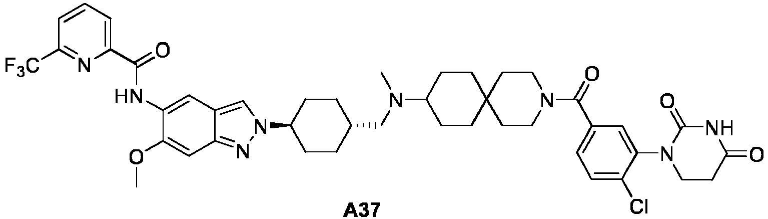 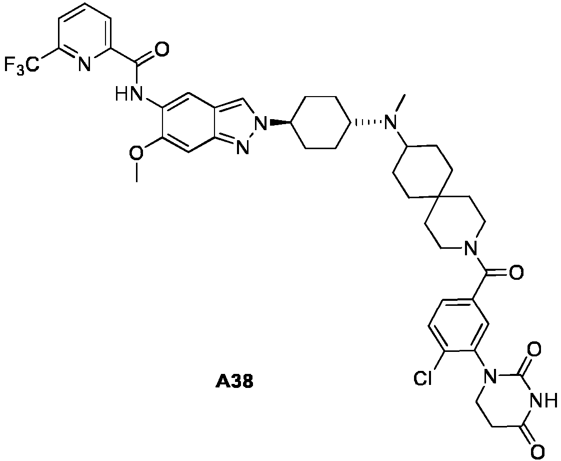 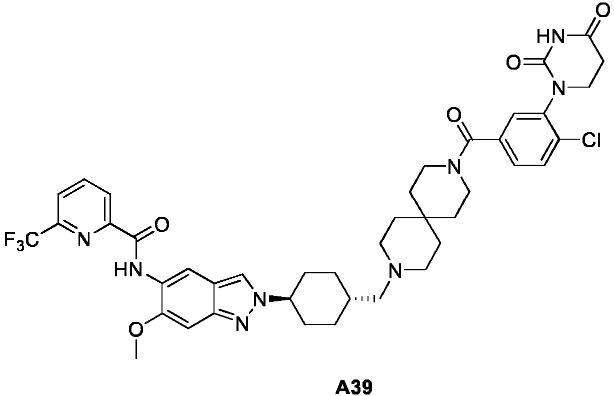 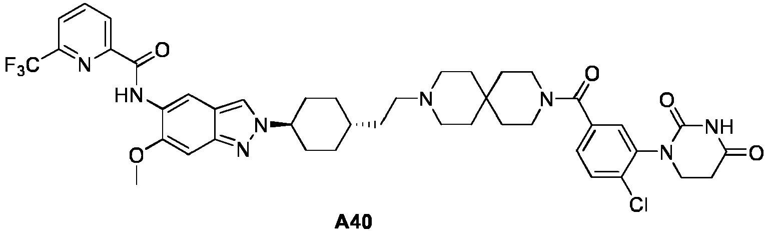 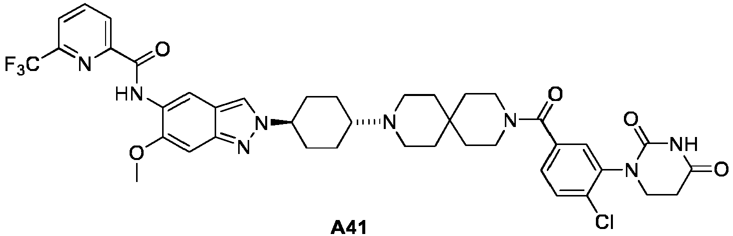 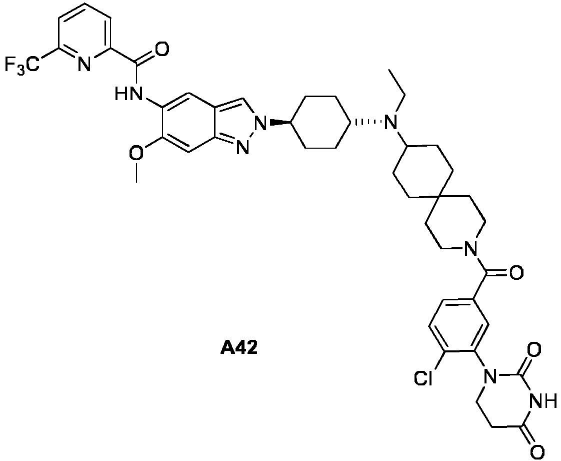 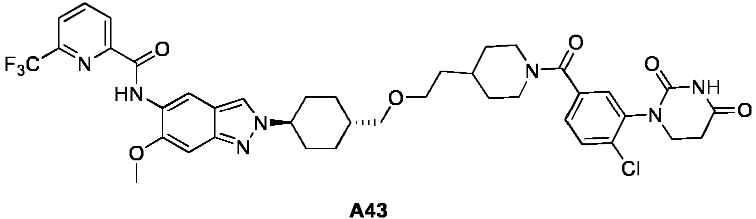 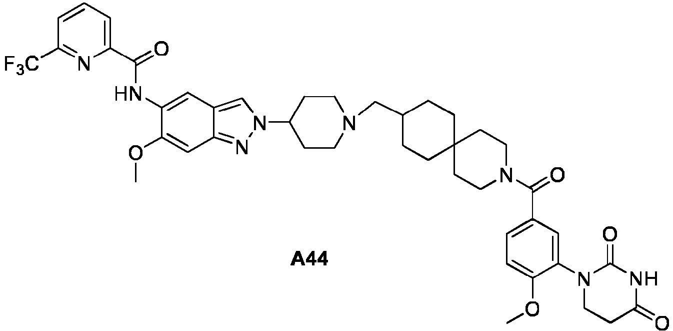 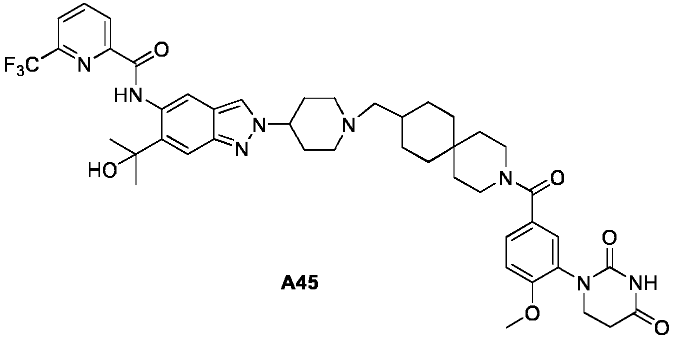 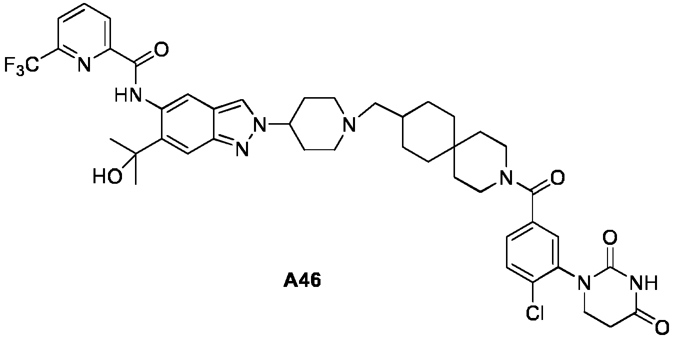 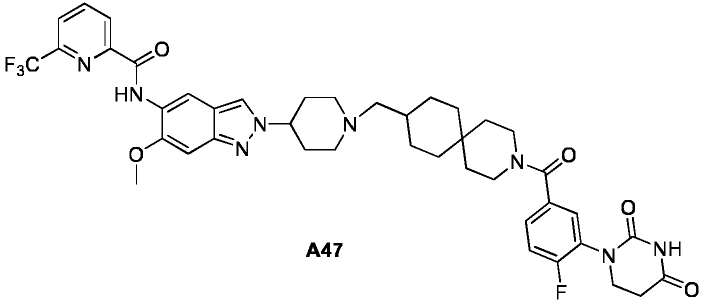 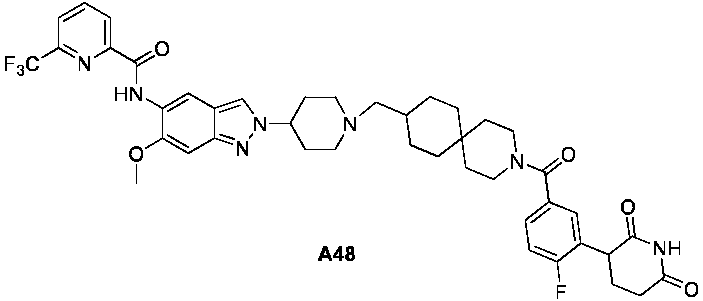 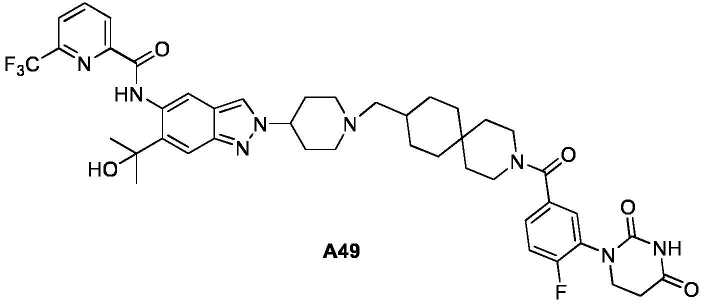 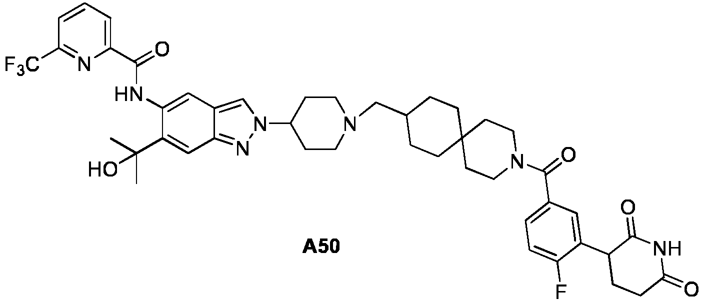 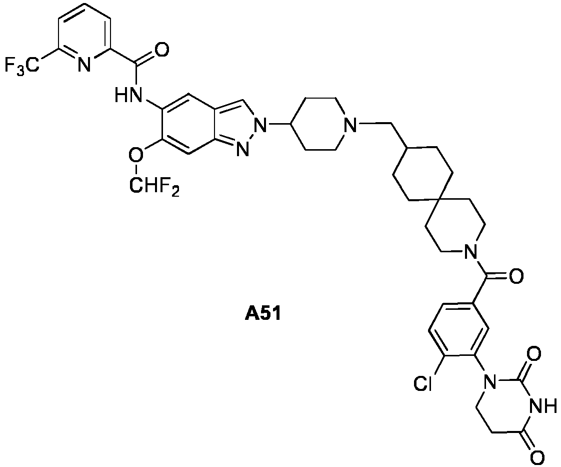 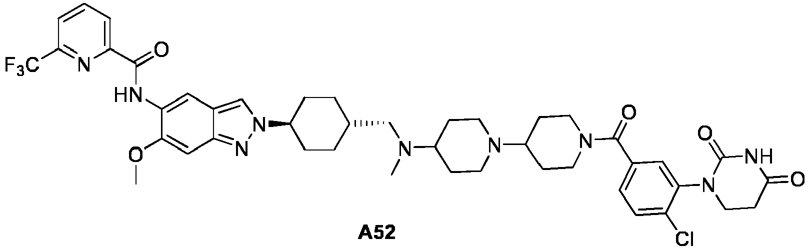 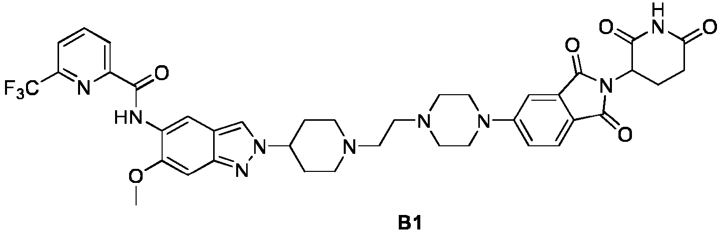 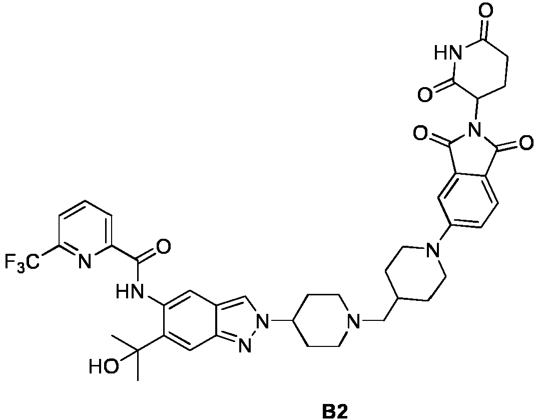 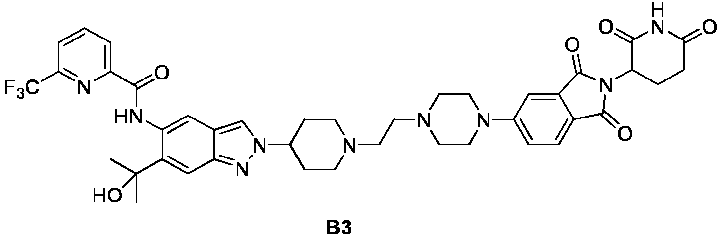 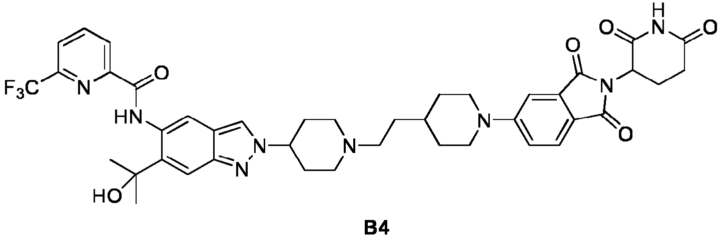 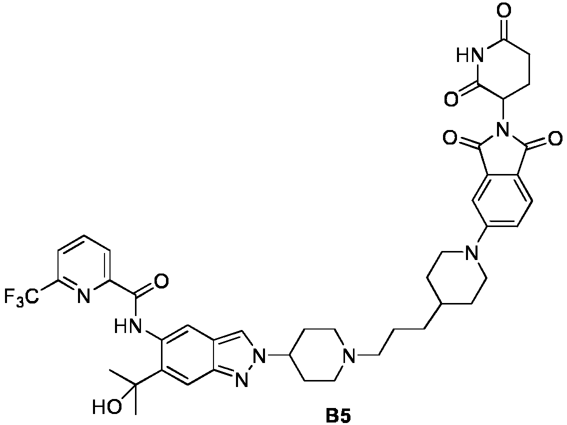 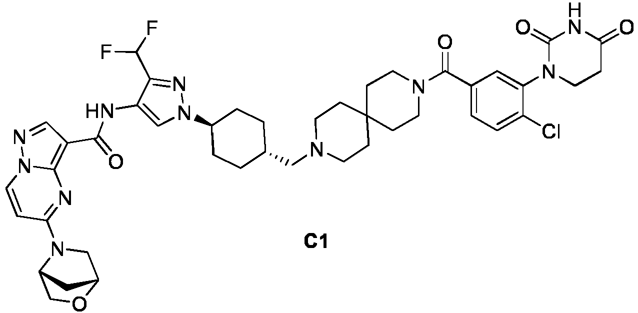 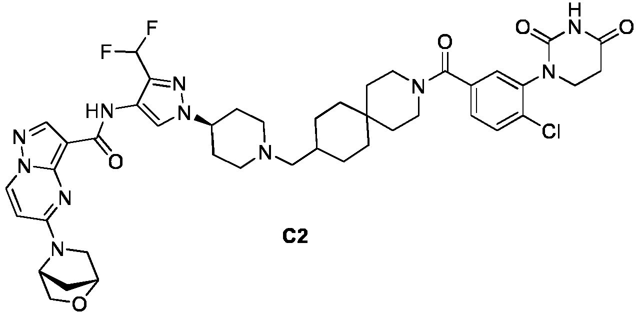 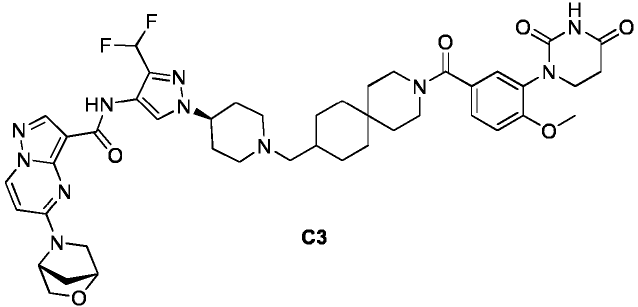 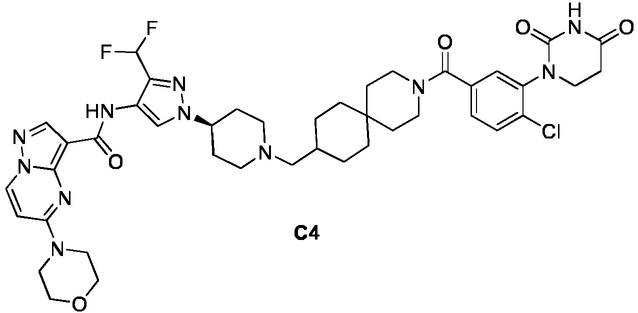 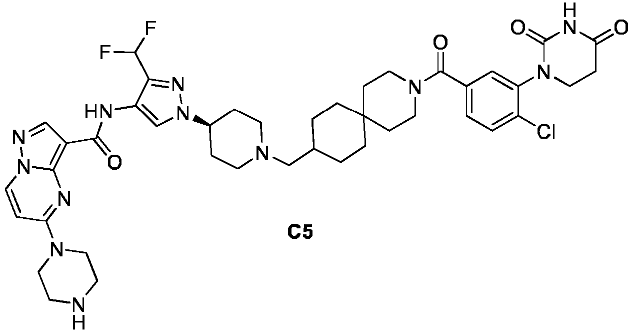 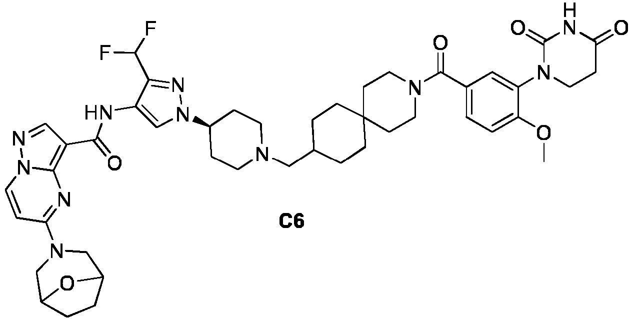 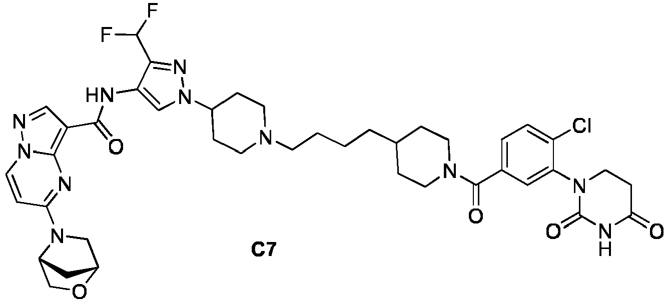 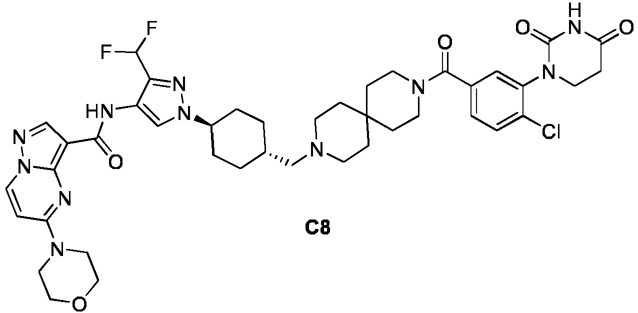 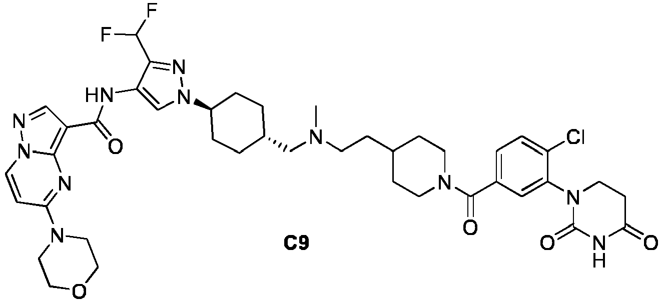 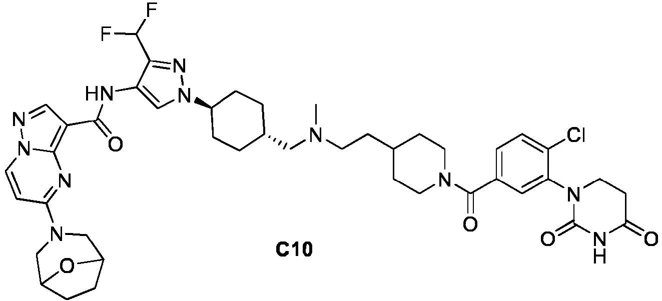 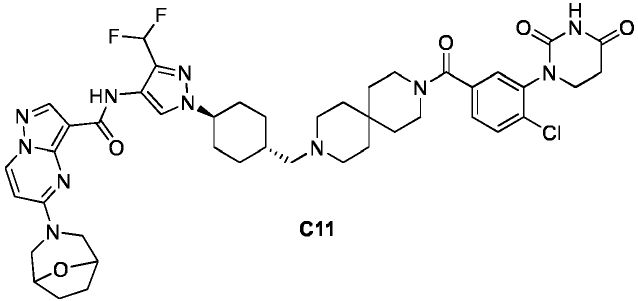 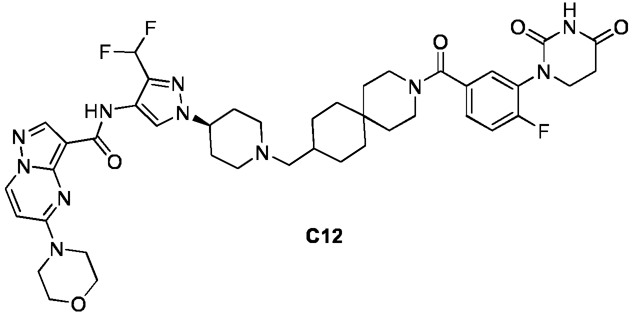 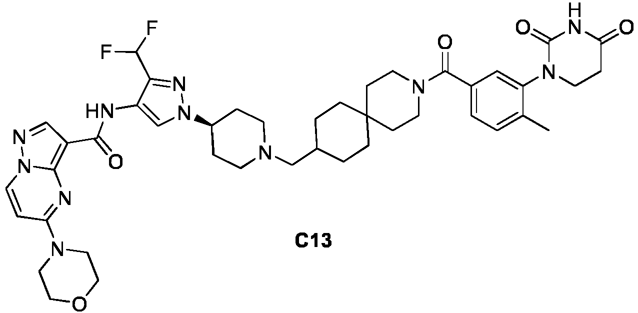 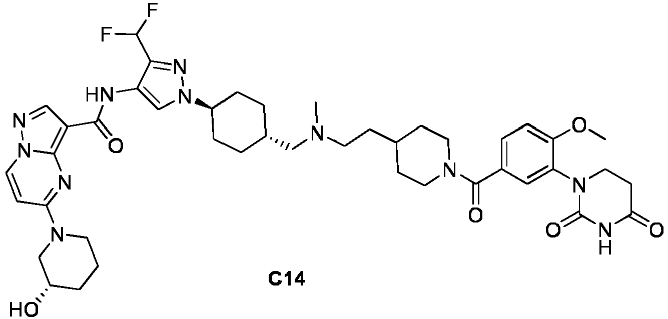 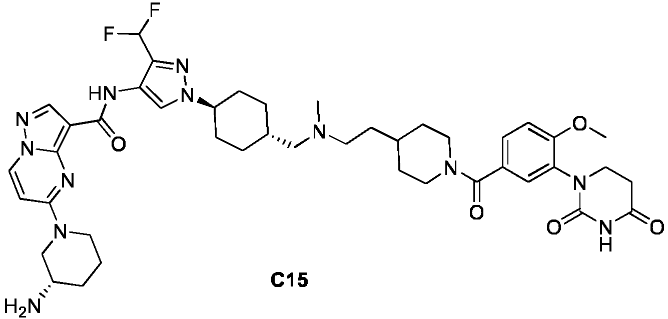 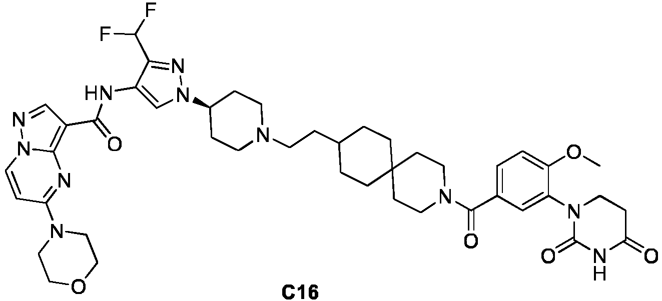 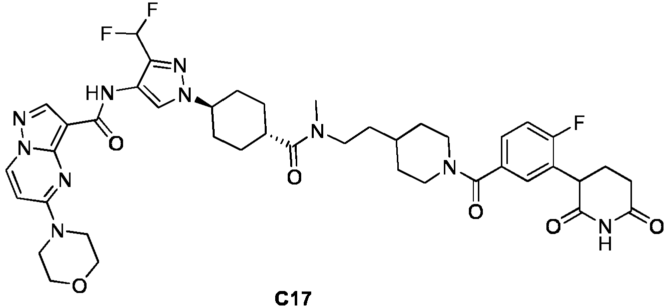 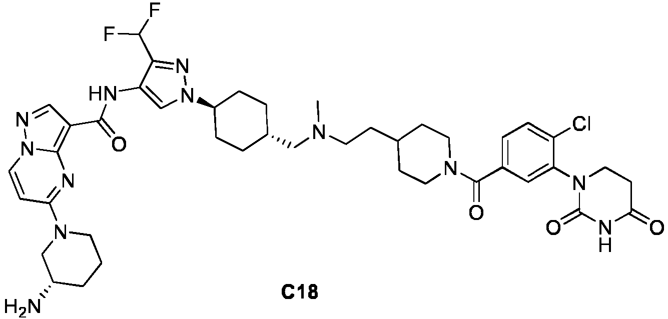 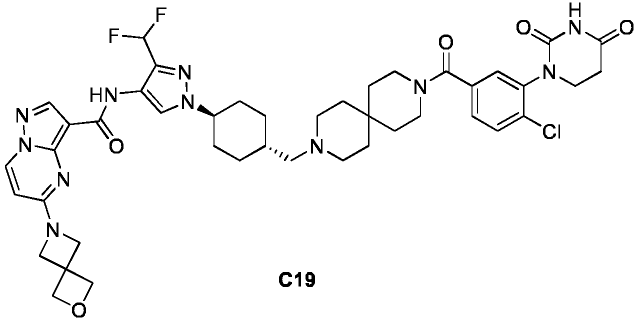 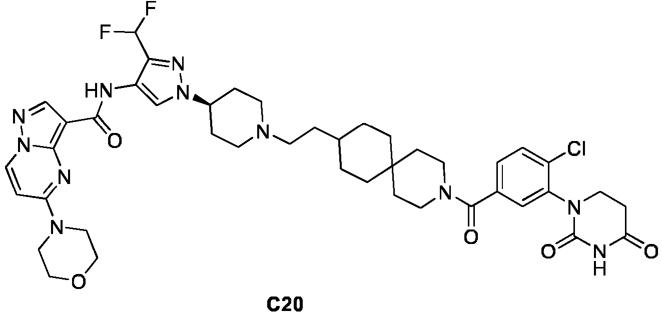 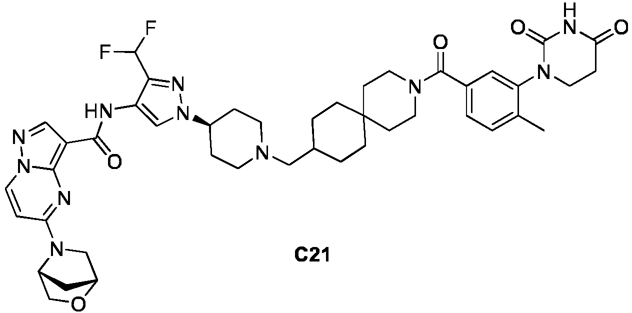 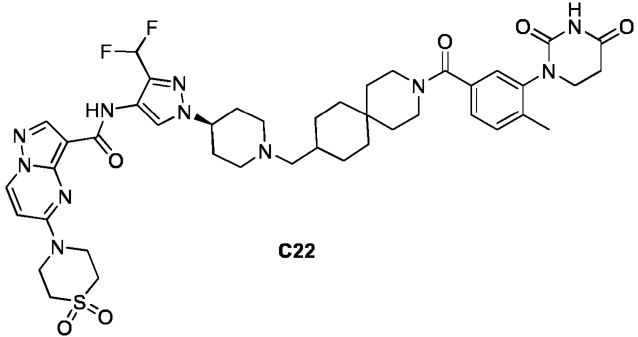 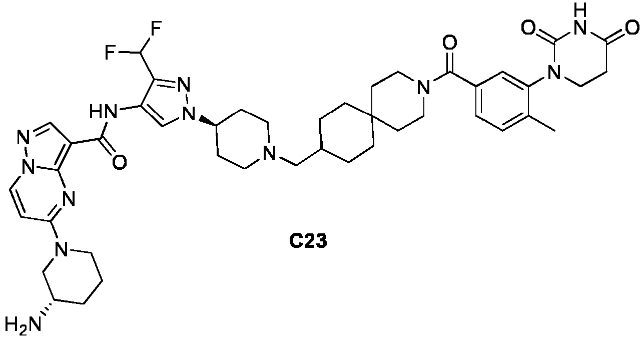 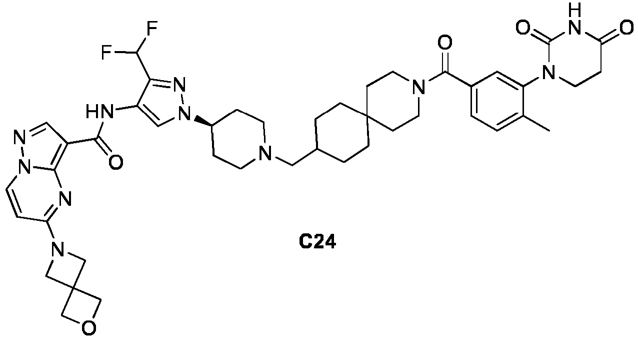 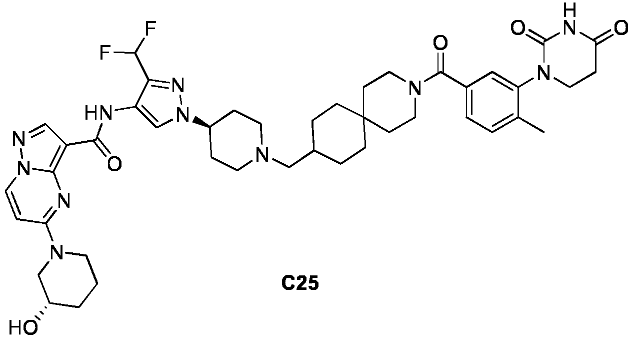 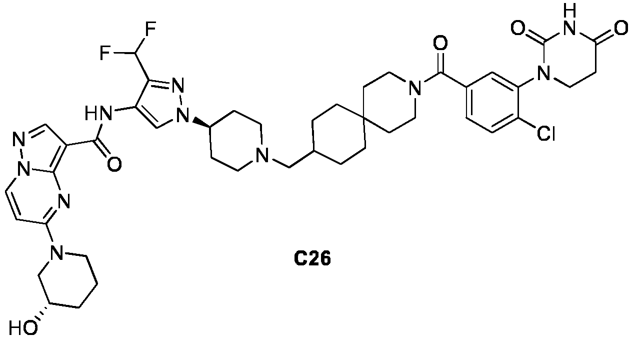 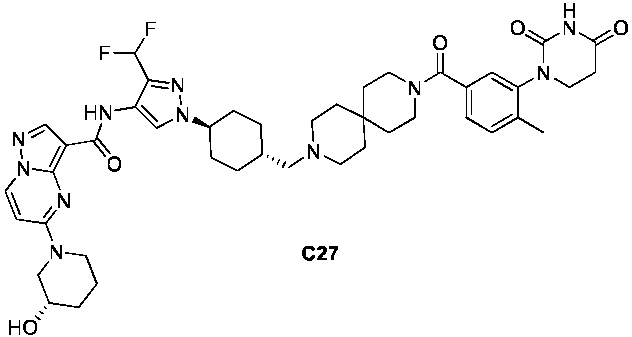 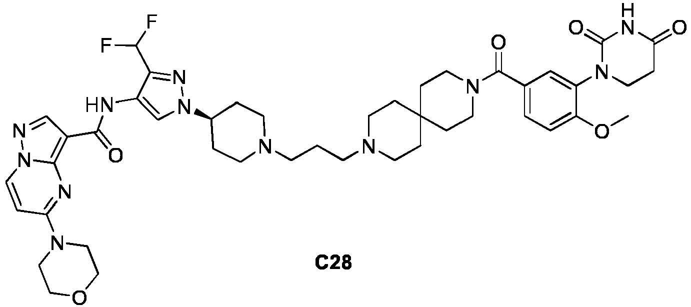 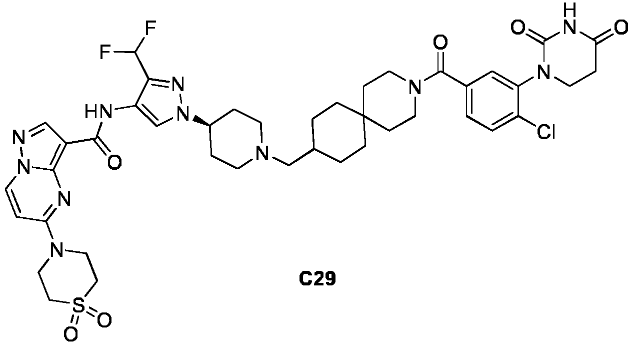 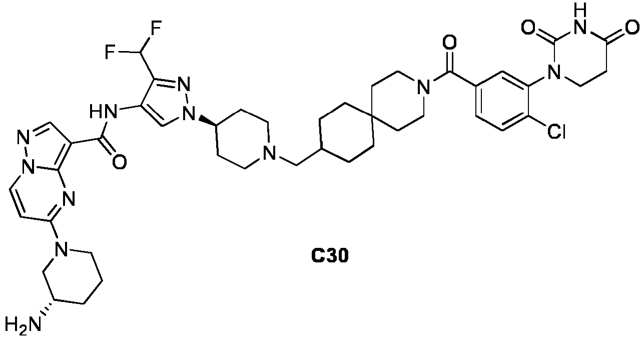 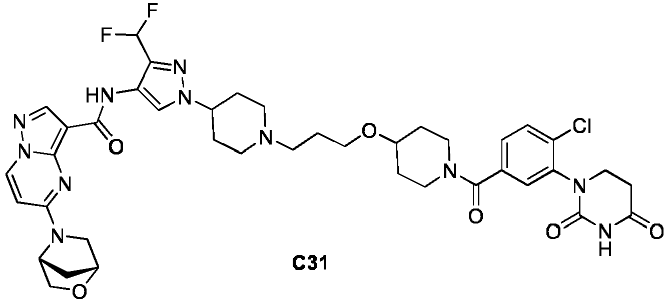 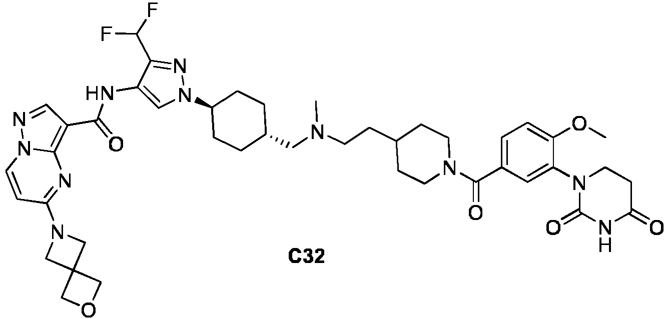 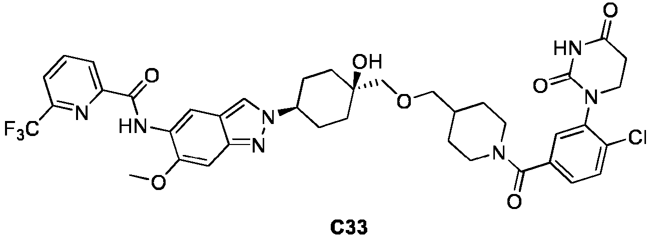 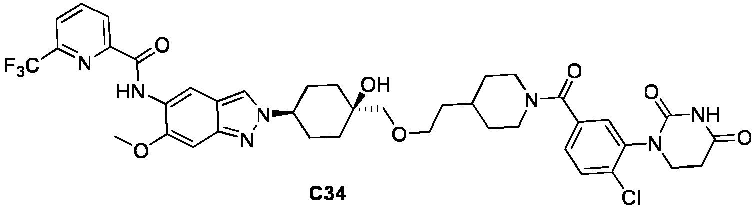 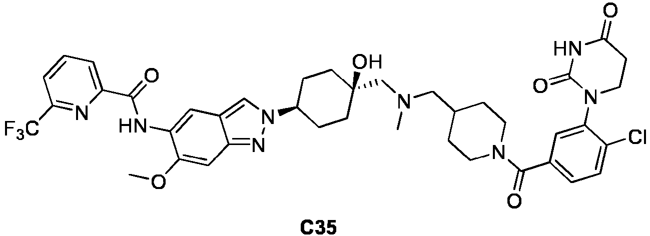 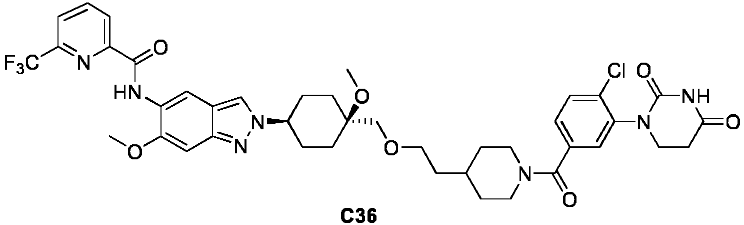 or a pharmaceutically acceptable salt, a deuteride, a solvate, or a prodrug thereof.

6. The use according to claim 1, wherein the IRAK4 degrader is for use for the treatment and/or prevention of an immunological disease.

7. The use according to claim 6, wherein the immunological disease is selected from: adult-onset Still's disease, alopecia areata, ankylosing spondylitis, autoimmune hepatitis, autoimmune myocarditis, autoimmune pancreatitis, autoimmune retinopathy, autoimmune urticaria, behcet's disease, benign mucosal pemphigoid (mucosal pemphigoid), bullous pemphigoid, Castleman's disease (CD), celiac disease, coxsackie myocarditis, Crohn's disease, dermatitis herpetiformis, atopic dermatitis, dermatomyositis, discoid lupus, adenomyosis, eosinophilic fasciitis, erythema nodosum, fibrosing alveolitis, primary glomerulonephritis, Goodpasture's syndrome, autoimmune hemolytic anemia, Henoch-Schonlein purpura (HSP), hidradenitis suppurativa (HS), IgG4-associated sclerosing disease, inclusion body myositis (IBM), interstitial cystitis (IC), Lambert-Eaton syndrome, linear IgA disease (LAD), systemic lupus erythematosus, chronic lyme disease, multiple sclerosis, systemic sclerosis, idiopathic inflammatory myopathy (IIM), ocular cicatricial pemphigoid optic neuritis, palindromic rheumatism (PR), pemphigus, autoimmune encephalomyelitis, POEMS syndrome, polyarteritis nodosa, primary biliary cholangitis, primary sclerosing cholangitis, psoriasis, psoriatic arthritis, reactive arthritis, relapsing polychondritis, retroperitoneal fibrosis, rheumatic fever, rheumatoid arthritis, sarcoidosis, autoimmune scleritis, morphea, Sjogren's syndrome, Takayasu arteritis, temporal arteritis, idiopathic thrombocytopenic purpura (ITP), thrombotic thrombocytopenic purpura (TTP), thyroid eye disease (Ted), Tolosa-Hunt syndrome (THS), transverse myelitis, ulcerative colitis (UC), undifferentiated connective tissue disease (UCTD), uveitis, systemic vasculitis, vitiligo, Vogt-Koyanagi-Harada disease, myasthenia gravis, gout, spondyloarthropathy, crystal arthropathy, osteoarthritis, rheumatoid arthritis, multiple myositis, interstitial lung disease, giant cell arteritis, polymyalgia rheumatica, granulomatous vasculitis, eosinophilic granulomatous vasculitis, eosinophilic vasculitis, microscopic polyangiitis, cryoglobulinemia, cutaneous leukocytoclastic vasculitis, mixed connective tissue disease, Steven-Johnson syndrome, pulmonary hypertension, endocarditis, atherosclerosis, erythema multiforme, acute coronary syndrome, idiopathic pulmonary fibrosis, nonalcoholic fatty liver, renal fibrosis, type 1 diabetes, primary dryness, Kawasaki disease, pustular psoriasis, chronic granulomatous disease, neuromyelitis optica urticaria, palmoplantar pustulosis, septicemia, bullous skin disease, Alzheimer's disease, chronic spontaneous urticaria, chronic psoriasis, juvenile idiopathic arthritis, axial spondyloarthritis, and Graves' disease.

8. The use according to claim 7, wherein the immunological disease is selected from: rheumatoid arthritis, atopic dermatitis, hidradenitis suppurativa (HS), vitiligo, dermatomyositis, alopecia areata, urticaria, multiple myositis, interstitial lung disease, systemic lupus erythematosus, systemic sclerosis, and/or psoriasis.

9. The use according to claim 1, wherein the IRAK4 degrader is for use for the treatment and/or prevention of an inflammatory disease.

10. The use according to claim 9, wherein the inflammatory disease is selected from: familial Mediterranean fever, tumor necrosis factor-associated periodic syndrome, mevalonate kinase deficiency, pyrin-associated autoinflammation with neutrophilic dermatosis (PAAND), pyogenic aseptic arthritis, pyoderma gangrenosum, and acne (PAPA), familial cold autoinflammatory syndrome (FCAS), familial keratosis fichenoides chronica (FKLC), NLRP1-associated autoinflammation with arthritis and dyskeratosis (NAIAD), deficiency of the IL-1 receptor antagonist (DIRA), deficiency of the IL-36 receptor antagonist (DITRA), allergic contact dermatitis, CAR-T cell-induced cytokine release syndrome, Crohn's disease, chronic bronchitis, COPD, active ankylosing spondylitis, axial spondyloarthritis, pityriasis rubra pilaris, inflammatory bowel disease, spinal arthritis, acute lung injury, generalized pustular psoriasis, acne vulgaris, and colitis.

11. The use according to claim 2, wherein the IRAK4 degrader is for use for the treatment and/or prevention of a disease mediated by IL-2R alpha, IL-6, IFA-alpha2, IFN-gamma, IL-1ra, MCP-3, IL-16, IL-12 (p40), LIF, IL-5, GM-CSF, TNF-alpha, IL-2, IL-1alpha, IL-1beta, IL-18, Eotaxin, Basic FGF, beta-NGF, PDGF-BB, IL-4, MCP-1, IL-8, IL-10, GRO-alpha, HGF, IL-1alpha, IL-1beta, IL-3, SCF, TRAIL, M-CSF, CTACK, IL-15, IL-12 (P70), IL-17, IL-23, IL-33, and/or IL-36 cytokines.

12. The use according to claim 11, wherein the IRAK4 degrader is for use for the treatment and/or prevention of a disease mediated by IL-4, IL-6, IL-12(p40), GM-CSF, TNF-alpha, IL-2, IL-1alpha, IL-1beta, IL-18, IL-8, IL-10, IL-17, IL-23, IL-33, and/or IL-36 cytokines.

13. The use according to any one of claims 1-12, wherein a sample of the disease is a spleen, skin, and/or blood sample.

14. The use according to claim 13, wherein the blood sample is normal human whole blood and/or patient whole blood.

15. A method for the treatment of the disease according to any one of claims 1-12, comprising administering to a subject an effective amount of an IRAK4 degrader.

16. The treatment method according to claim 15, wherein the IRAK4 degrader is the single compound according to claim 5 or in combination with an additional drug.
